# EUROPEAN PATENT APPLICATION

(11) **EP 4 273 125 A1**
(43) Date of publication of application: **08.11.2023**
(21) Application number: 23171358.7
(22) Date of filing: 03.05.2023
(51) Int. Cl.: C07D 209/86, H10K 85/60

(54) **HETEROCYCLIC COMPOUND, ORGANIC LIGHT-EMITTING DEVICE INCLUDING THE SAME, AND ELECTRONIC APPARATUS INCLUDING THE ORGANIC LIGHT-EMITTING DEVICE**

(30) Priority: 04.05.2022 KR 20220055745; 02.05.2023 KR 20230057363
(71) Applicant: Samsung Electronics Co., Ltd., Suwon-si, Gyeonggi-do 16677 (KR)
(72) Inventor: CHUNG, Yeon Sook, 16678 Suwon-si (KR); CHOI, Eunjeong, 17084 Yongin-si (KR); AN, Eunhye, 17084 Yongin-si (KR); AHN, Heechoon, 17113 Yongin-si (KR); KANG, Giwook, 17084 Yongin-si (KR); KWON, Eunsuk, 16678 Suwon-si (KR); LEE, Yeseul, 17113 Yongin-si (KR); JANG, Dongjin, 17084 Yongin-si (KR); CHO, Seowon, 17113 Yongin-si (KR); JUNG, Yongsik, 16678 Suwon-si (KR); KIM, Hwang Suk, 16678 Suwon-si (KR); CHOI, Hyeonho, 16678 Suwon-si (KR); CHOI, Byoungki, 16678 Suwon-si (KR)
(74) Representative: Elkington and Fife LLP

(57) **Abstract**

Provided are a heterocyclic compound represented by Formula 1, an organic light-emitting device including the same, and an electronic apparatus including the organic light-emitting device: 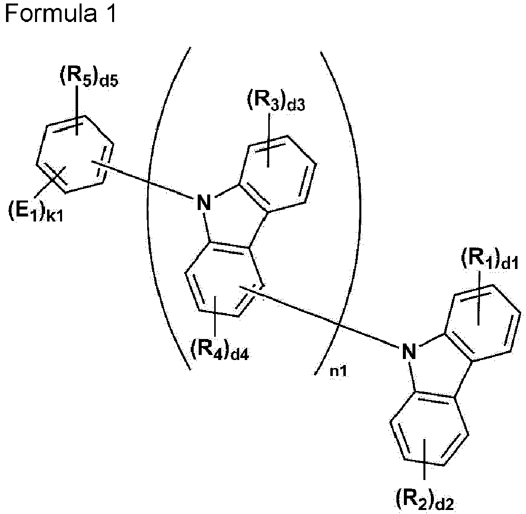 wherein a detailed description of Formula 1 is provided in the specification.

## Description

### FIELD OF THE INVENTION

The present disclosure relates to a heterocyclic compound, an organic light-emitting device including the same, and an electronic apparatus including the organic light-emitting device.

### BACKGROUND OF THE INVENTION

Organic light-emitting devices are self-emissive devices, which have improved characteristics in terms of viewing angles, response time, brightness, driving voltage, and response speed, and produce full-color images.

In an example, an organic light-emitting device includes an anode, a cathode, and an organic layer that is arranged between the anode and the cathode and includes an emission layer. A hole transport region may be provided between the anode and the emission layer, and an electron transport region may be provided between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. The excitons may transition from an excited state to a ground state, thereby generating light.

### SUMMARY OF THE INVENTION

Provided are a novel heterocyclic compound, an organic light-emitting device including the same, and an electronic apparatus including the organic light-emitting device.

Additional aspects will be set forth in part in the description which follows and, in part, will be apparent from the description, or may be learned by practice of the presented embodiments of the disclosure.

According to an aspect of the disclosure, there is provided a heterocyclic compound represented by Formula 1:

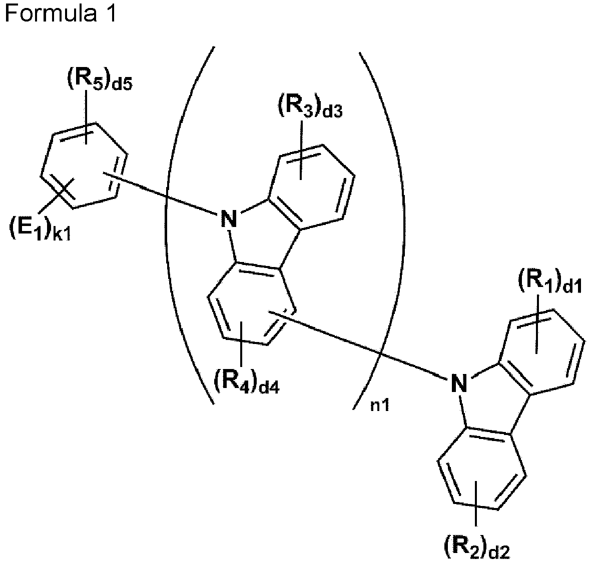

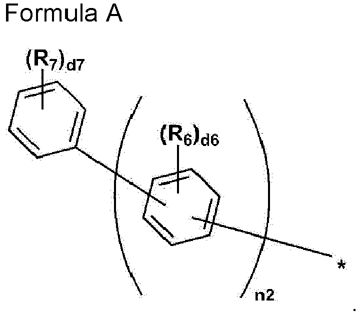

In Formula 1,
E₁ may be a group represented by Formula A, and
k1 may be an integer from 1 to 5, and
in Formulae 1 and A,
n1 may be 1,
n2 may be 0,
R₁ to R₄ may each independently be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or - P(Q₈)(Q₉),
R₅ to R₇ may each independently be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryloxy group, a substituted or unsubstituted C₆-C₆₀ arylthio group, -N(Q₁)(Q₂), -Si(Q₃)(Q₄)(Q₅), - Ge(Q₃)(Q₄)(Q₅), -B(Q₆)(Q₇), -P(=O)(Q₈)(Q₉), or -P(Q₈)(Q₉),
d1, d2, d3, d5, and d6 may each independently be an integer from 0 to 4,
d4 may be an integer from 0 to 3,
d7 may be an integer from 0 to 5,
the heterocyclic compound represented by Formula 1 may include at least one deuterium, and
a moiety represented by 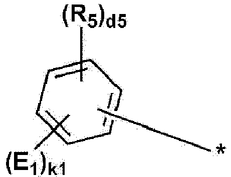 in Formula 1 may be a group represented by Formula 4-2 or 4-3: 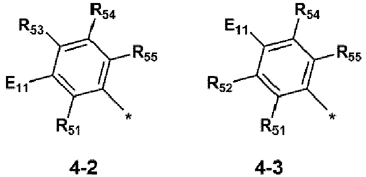
wherein, in Formulae 4-2 and 4-3,
E₁₁ is the same as described in connection with E₁,
R₅₁ to R₅₅ are each independently the same as described in connection with R₅,
* indicates a binding site to a neighboring atom,
substituents of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may each be:
   deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group,
   a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -Ge(Q₁₃)(Q₁₄)(Q₁₅), - B(Q₁₆)(Q₁₇), -P(=O)(Q₁₈)(Q₁₉), -P(Q₁₈)(Q₁₉), or any combination thereof,
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with at least one of deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), - Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), -P(Q₂₈)(Q₂₉) or any combination thereof,
   -N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉), or
   any combination thereof, and
   Q₁ to Q₉, Q₁₁ to Q₁₉, Q₂₁ to Q₂₉, and Q₃₁ to Q₃₉ may each independently be hydrogen, deuterium, -F, -Cl, -Br, -I, a hydroxyl group, a cyano group, a nitro group, an amidino group, a hydrazine group, a hydrazone group, a carboxylic acid or a salt thereof, a sulfonic acid or a salt thereof, a phosphoric acid or a salt thereof, a C₁-C₆₀ alkyl group which is unsubstituted or substituted with deuterium, a C₁-C₆₀ alkyl group, a C₆-C₆₀ aryl group, or any combination thereof, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group which is unsubstituted or substituted with deuterium, a C₁-C₆₀ alkyl group, a C₆-C₆₀ aryl group, or any combination thereof, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group.

According to another aspect of the disclosure, an organic light-emitting device includes a first electrode, a second electrode, and an organic layer arranged between the first electrode and the second electrode and including an emission layer, wherein the organic layer includes at least one heterocyclic compound represented by Formula 1.

According to another aspect of the present disclosure, an electronic apparatus includes the organic light-emitting device.

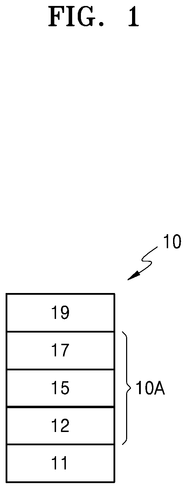

### BRIEF DESCRIPTION OF THE DRAWING

The above and other aspects, features, and advantages of certain embodiments of the disclosure will be more apparent from the following description taken in conjunction with FIGURE which is a schematic cross-sectional view of an organic light-emitting device 10 according to an embodiment.

### DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

According to an aspect, a heterocyclic compound may be represented by Formula 1: 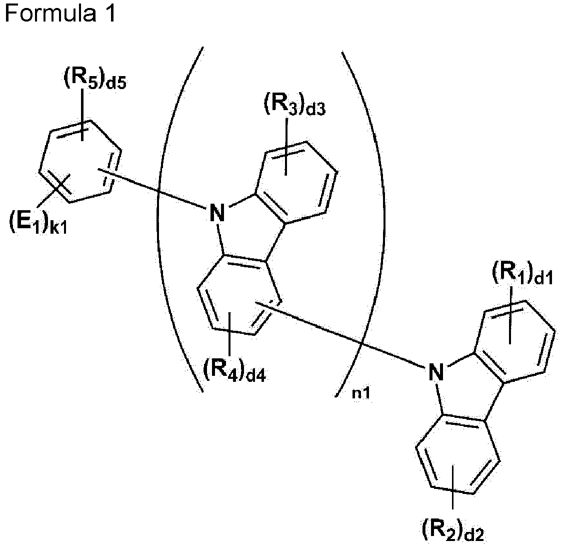 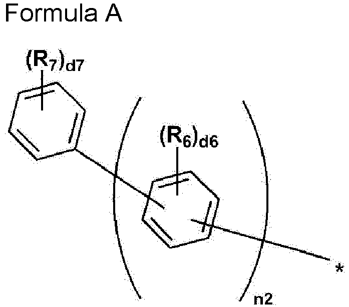
wherein, in Formula 1,
E₁ may be a group represented by Formula A, and
k1 may be an integer from 1 to 5.
In an embodiment, k1 may be an integer from 1 to 3.
In Formulae 1 and A,
n1 may be 1.
n2 may be 0.
R₁ to R₄ may each independently be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or - P(Q₈)(Q₉).
R₅ to R₇ may each independently be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryloxy group, a substituted or unsubstituted C₆-C₆₀ arylthio group, -N(Q₁)(Q₂), -Si(Q₃)(Q₄)(Q₅), - Ge(Q₃)(Q₄)(Q₅), -B(Q₆)(Q₇), -P(=O)(Q₈)(Q₉), or -P(Q₈)(Q₉).

Substituents of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may each be:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -Ge(Q₁₃)(Q₁₄)(Q₁₅), - B(Q₁₆)(Q₁₇), -P(=O)(Q₁₈)(Q₁₉), -P(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with at least one of deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), - Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), -P(Q₂₈)(Q₂₉) or any combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉); or
any combination thereof.

Q₁ to Q₉, Q₁₁ to Q₁₉, Q₂₁ to Q₂₉, and Q₃₁ to Q₃₉ may each independently be: hydrogen; deuterium; -F; -Cl; -Br; -I; a hydroxyl group; a cyano group; a nitro group; an amidino group; a hydrazine group; a hydrazone group; a carboxylic acid group or a salt thereof; a sulfonic acid group or a salt thereof; a phosphoric acid group or a salt thereof; a C₁-C₆₀ alkyl group unsubstituted or substituted with deuterium, a C₁-C₆₀ alkyl group, a C₆-C₆₀ aryl group, or any combination thereof; a C₂-C₆₀ alkenyl group; a C₂-C₆₀ alkynyl group; a C₁-C₆₀ alkoxy group; a C₃-C₁₀ cycloalkyl group; a C₁-C₁₀ heterocycloalkyl group; a C₃-C₁₀ cycloalkenyl group; a C₁-C₁₀ heterocycloalkenyl group; a C₆-C₆₀ aryl group unsubstituted or substituted with deuterium, a C₁-C₆₀ alkyl group, a C₆-C₆₀ aryl group, or any combination thereof; a C₆-C₆₀ aryloxy group; a C₆-C₆₀ arylthio group; a C₁-C₆₀ heteroaryl group; a monovalent non-aromatic condensed polycyclic group; or a monovalent non-aromatic condensed heteropolycyclic group.

In Formula 1, d1, d2, d3, d5, and d6 indicate the number of R₁(s), R₂(s), R₃(s), R₅(s), and R₆(s), respectively, and may each independently be an indicates the number of 0 to 4 (e.g., 0, 1, 2, 3, or 4). When d1 is 2 or more, two or more of R₁ may be identical to or different from each other, when d2 is 2 or more, two or more of R₂ may be identical to or different from each other, when d3 is 2 or more, two or more of R₃ may be identical to or different from each other, when d5 is 2 or more, two or more of R₅ may be identical to or different from each other, and when d6 is 2 or more, two or more of R₆ may be identical to or different from each other.

In Formula 1, d4 indicates the number of R₄(s), and may be an integer from 0 to 3 (e.g., 0, 1, 2, or 3). When d4 is 2 or more, two or more of R₄ may be identical to or different from each other.

In Formula 1, d7 indicates the number of R₇(s), and may be an integer from 0 to 5 (e.g., 0, 1, 2, 3, 4, or 5). When d7 is 2 or more, two or more of R₇ may be identical to or different from each other.

In an embodiment, at least one of d2 and d1 may be an integer of 1 or more In one or more embodiments, at least one of d2 and d1 may be 4.

In an embodiment, R₁ to R₄ may each independently be:
hydrogen, deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, or a C₁-C₂₀ alkoxy group;
a C₁-C₂₀ alkyl group or a C₁-C₂₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, or a combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purynyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purynyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, -Si(Q₃₃)(Q₃₄)(Q₃₅), or any combination thereof; or
-N(Q₁)(Q₂), -Si(Q₃)(Q₄)(Q₅), -Ge(Q₃)(Q₄)(Q₅), -B(Q₆)(Q₇), or -P(=O)(Q₈)(Q₉), and
Q₁ to Q₉ and Q₃₁ to Q₃₃ may each independently be:
   -CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group; or
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof.

In an embodiment, R₅ to R₇ may each independently be:
hydrogen, deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, or a C₁-C₂₀ alkoxy group;
a C₁-C₂₀ alkyl group or a C₁-C₂₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, or any combination thereof; or
-N(Q₁)(Q₂), -Si(Q₃)(Q₄)(Q₅), -Ge(Q₃)(Q₄)(Q₅), -B(Q₆)(Q₇), or -P(=O)(Q₈)(Q₉), and
Q₁ to Q₉ and Q₃₁ to Q₃₃ may each independently be:
   -CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group; or
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof.

In one or more embodiments, R₁ to R₄ may each independently be:
hydrogen, deuterium, -F, -Cl, -Br, -I, -CD₃, -CD₂H, -CDH₂, -CF₃, -CF₂H, -CFH₂, a C₁-C₆₀ alkyl group, a C₂-C₆₀ alkenyl group, a C₂-C₆₀ alkynyl group, or a C₁-C₆₀ alkoxy group; or
a group represented by one of Formulae 9-1 to 9-61, 9-201 to 9-240, 10-1 to 10-129, and 10-201 to 10-355; or
-N(Q₁)(Q₂), -Si(Q₃)(Q₄)(Q₅), or -Ge(Q₃)(Q₄)(Q₅), and
Q₁ to Q₅ are each the same as described herein:

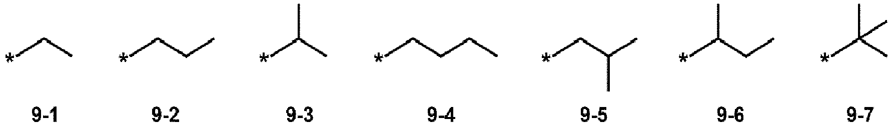

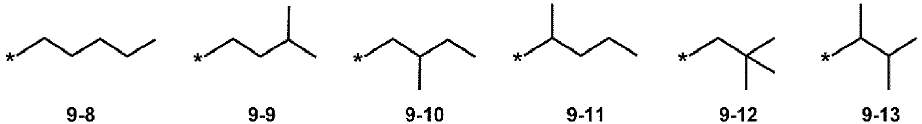

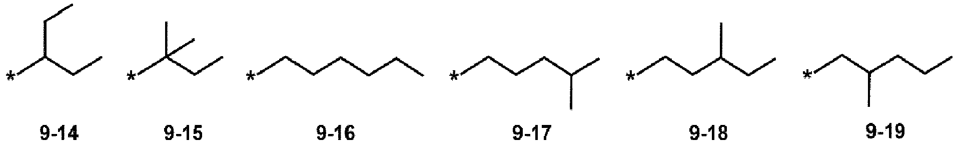

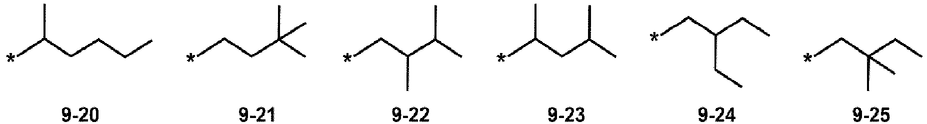

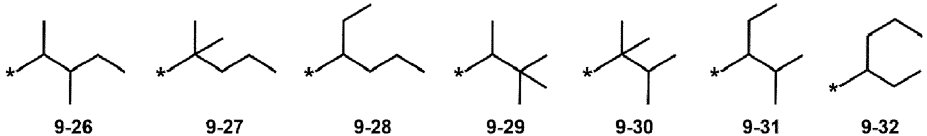

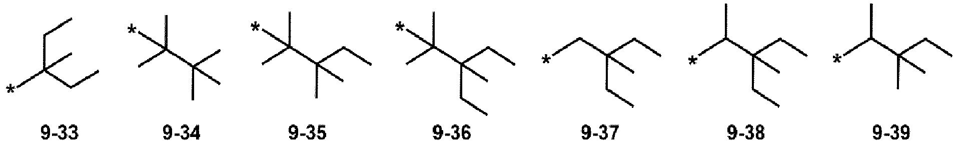

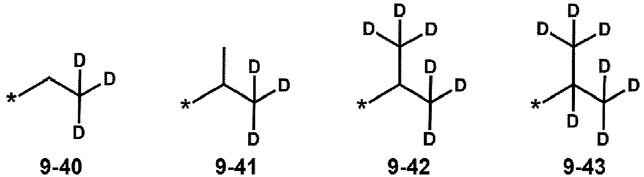

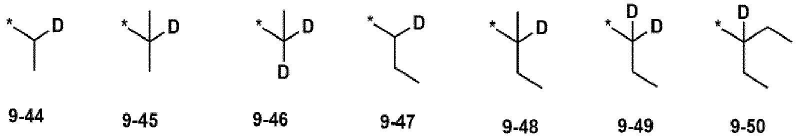

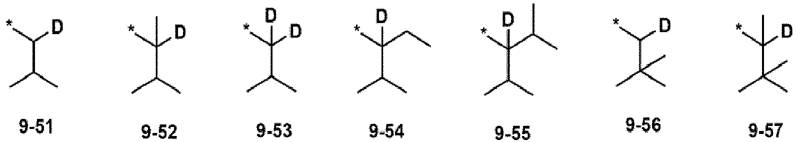

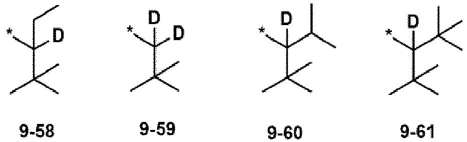

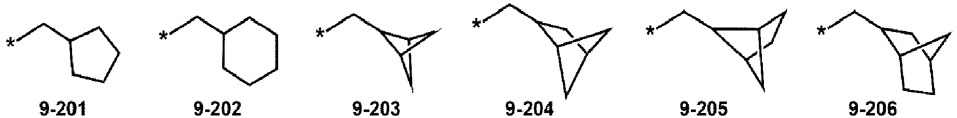

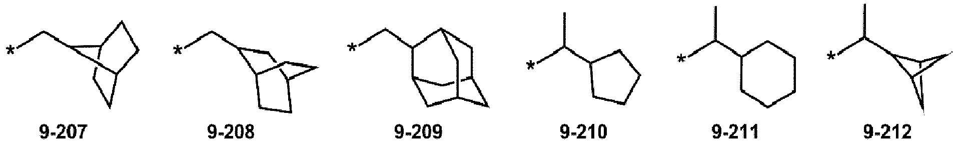

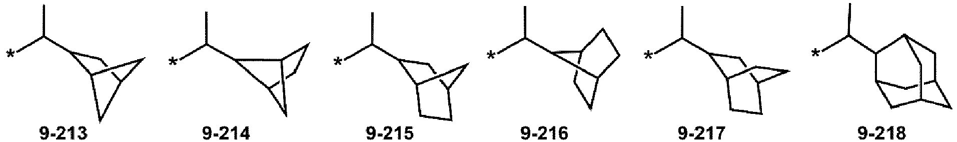

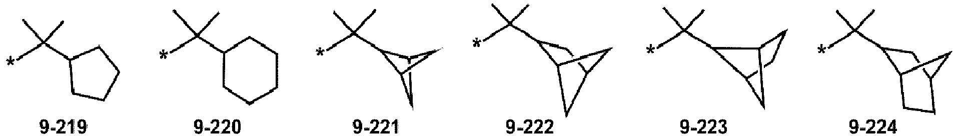

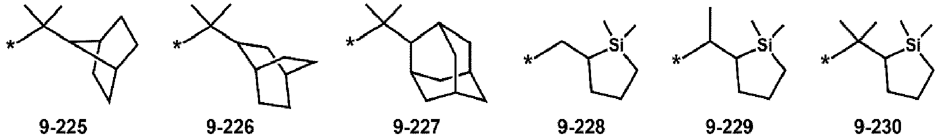

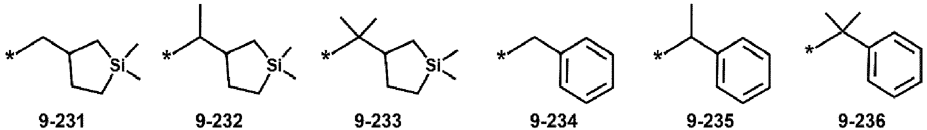

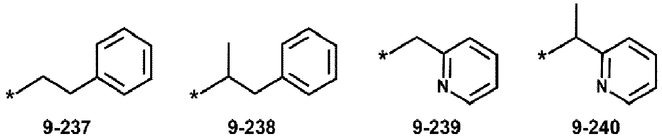

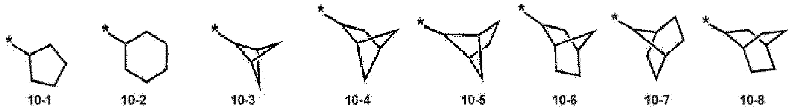

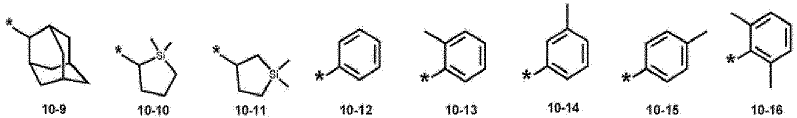

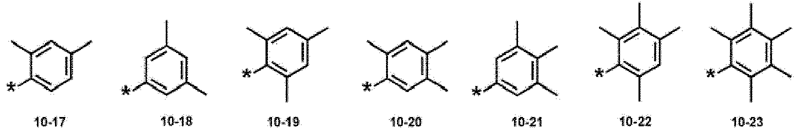

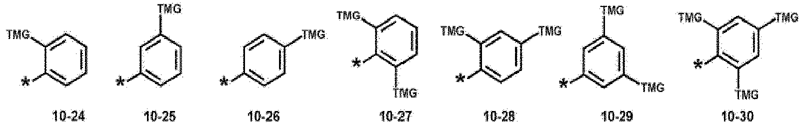

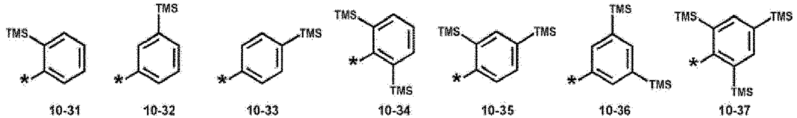

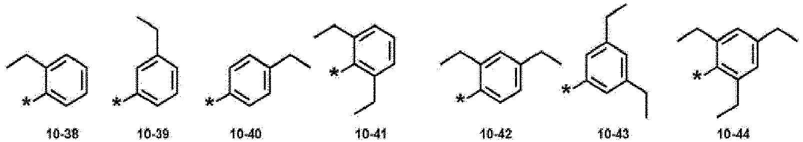

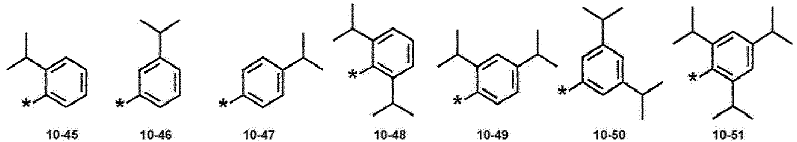

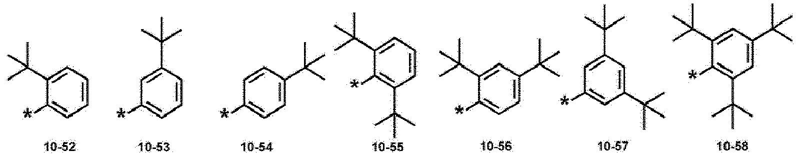

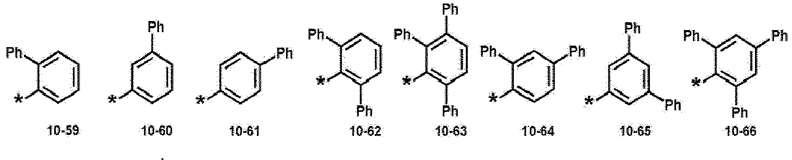

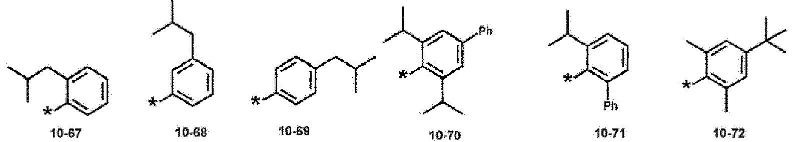

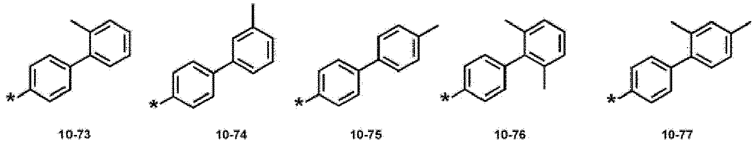

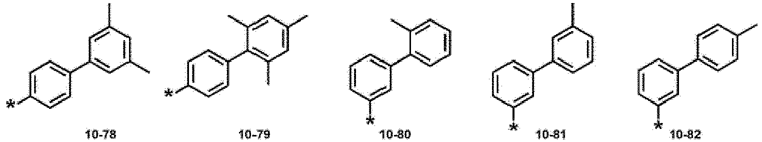

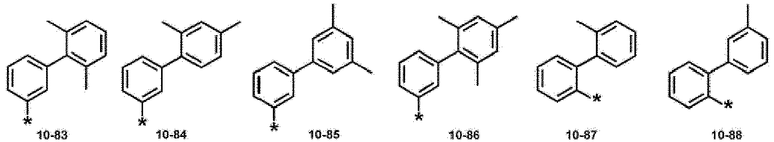

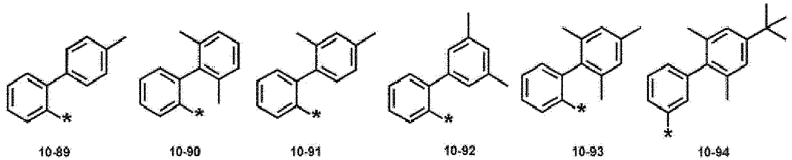

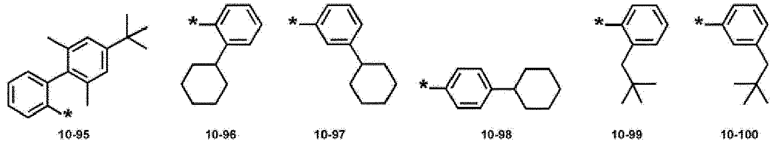

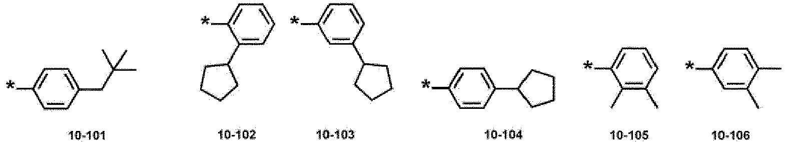

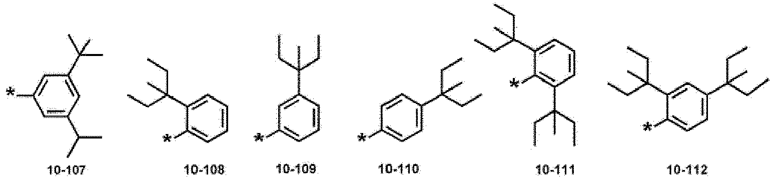

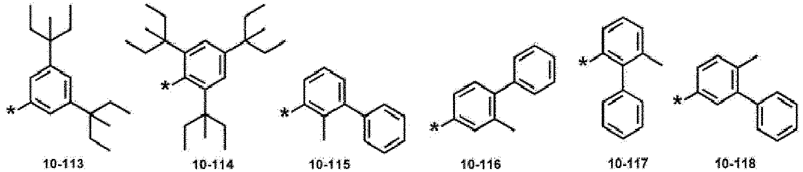

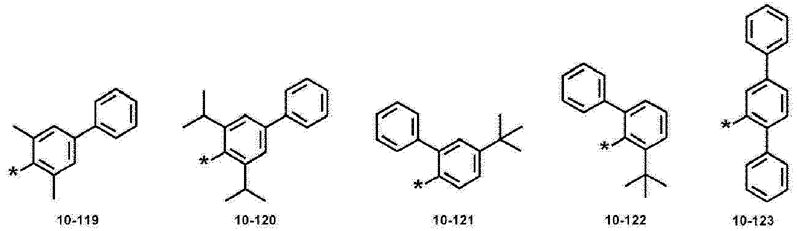

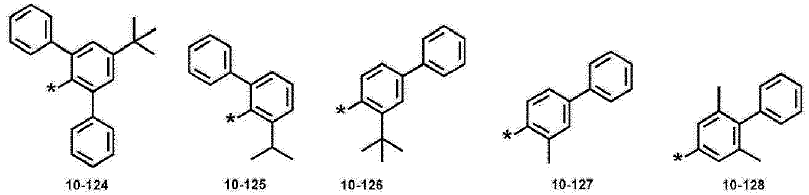

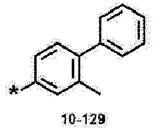

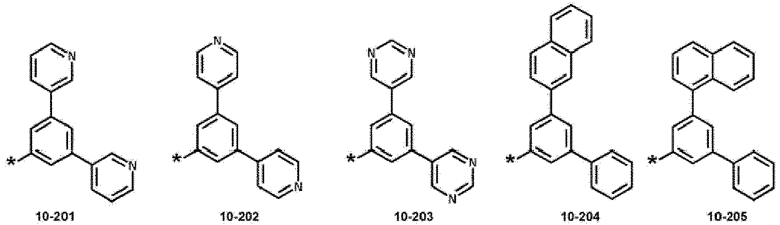

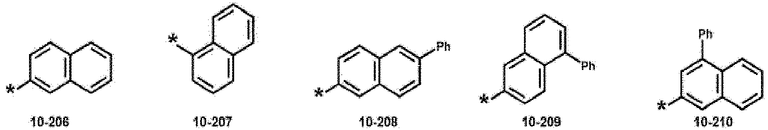

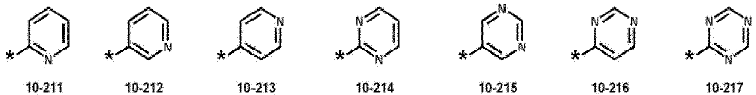

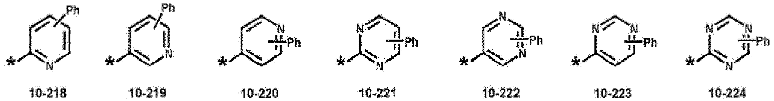

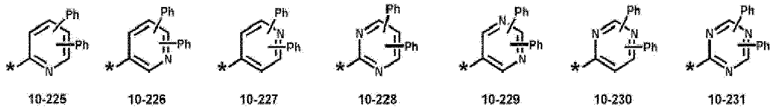

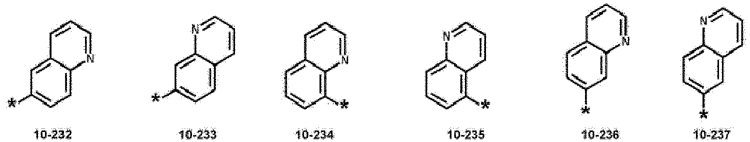

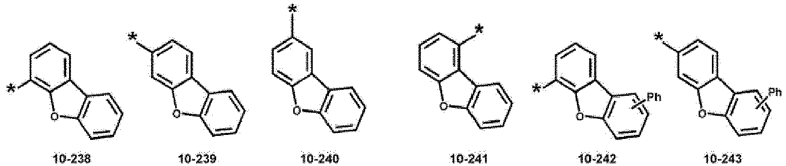

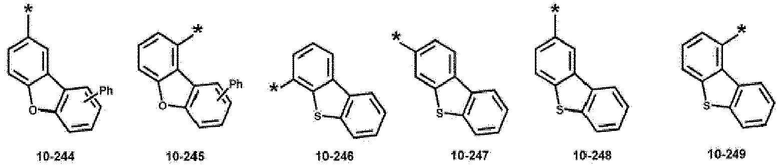

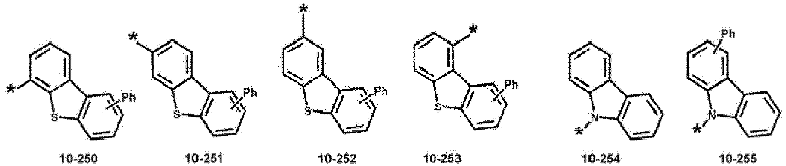

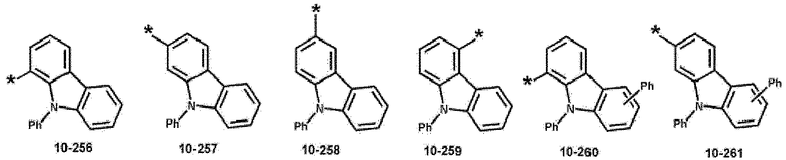

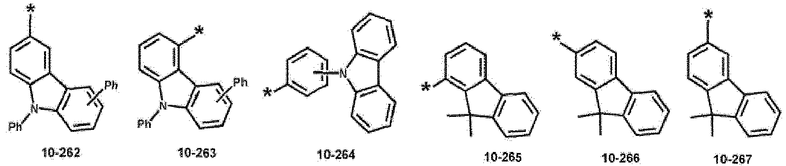

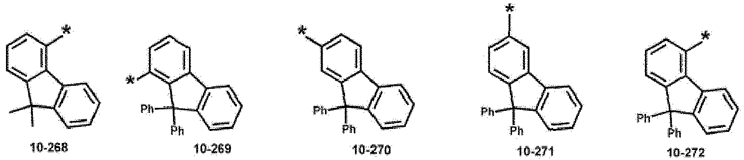

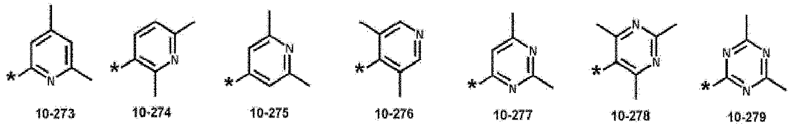

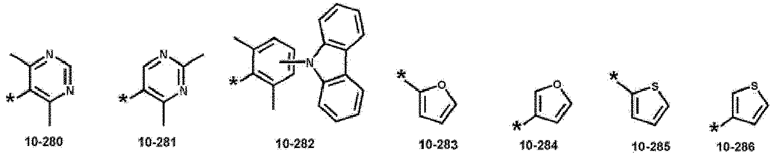

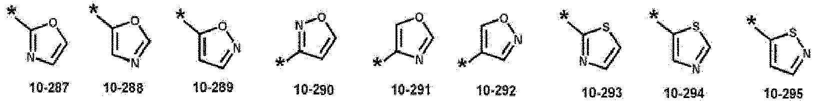

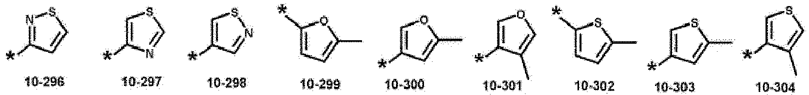

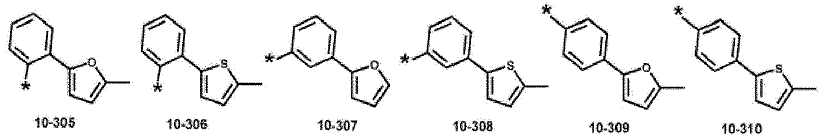

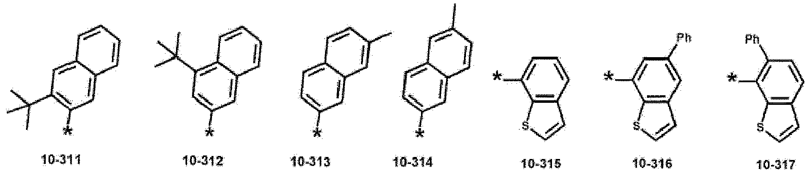

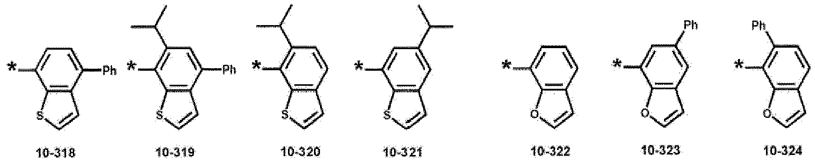

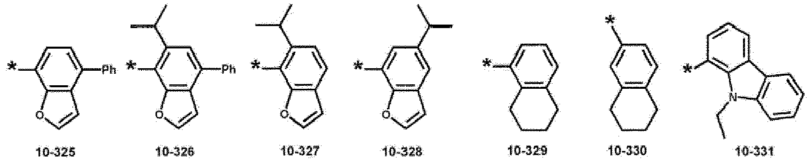

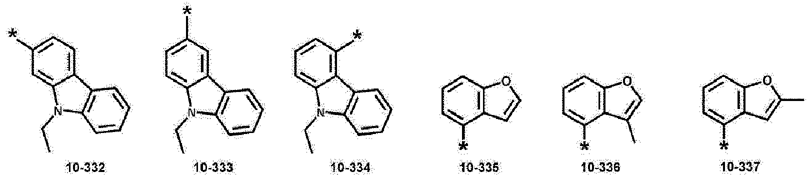

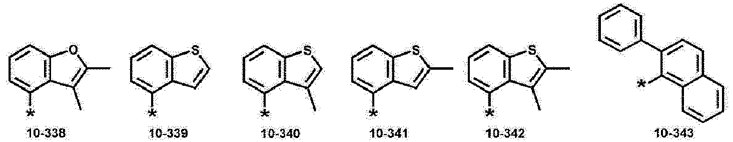

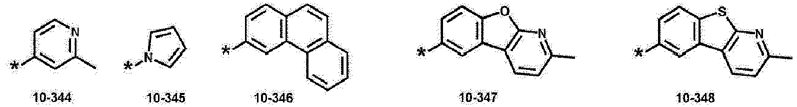

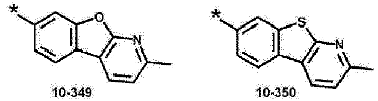

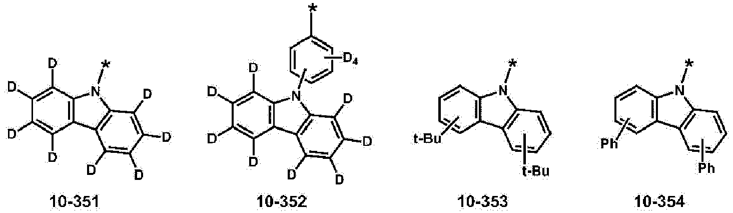

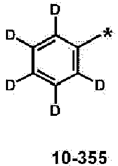

In Formulae 9-1 to 9-61, 9-201 to 9-240, 10-1 to 10-129, and 10-201 to 10-355, * indicates a binding site to a neighboring atom, Ph represents a phenyl group, TMS represents a trimethylsilyl group, TMG represents a trimethylgermyl group, and t-Bu represents a t-butyl group.

In one or more embodiments, R₅ to R₇ may each independently be:
hydrogen, deuterium, -F, -Cl, -Br, -I, -CD₃, -CD₂H, -CDH₂, -CF₃, -CF₂H, -CFH₂, a C₁-C₆₀ alkyl group, a C₂-C₆₀ alkenyl group, a C₂-C₆₀ alkynyl group, or a C₁-C₆₀ alkoxy group;
a group represented by one of Formulae 9-1 to 9-61 and 9-201 to 9-240; or
-N(Q₁)(Q₂), -Si(Q₃)(Q₄)(Q₅) or -Ge(Q₃)(Q₄)(Q₅), and
Q₁ to Q₅ are each the same as described herein:

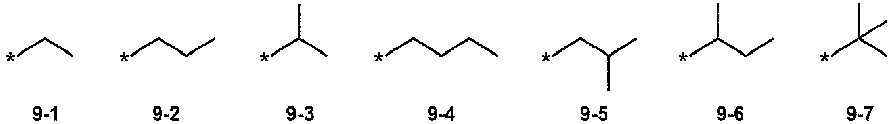

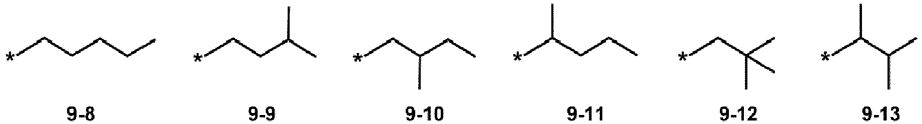

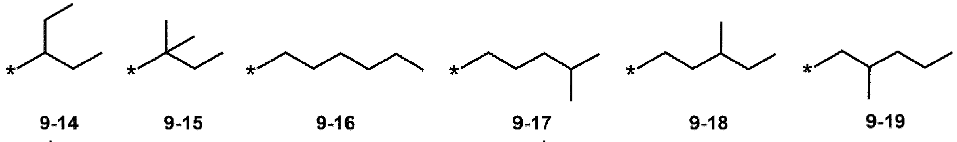

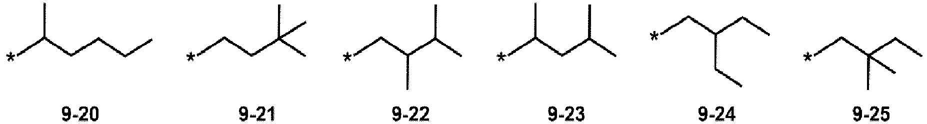

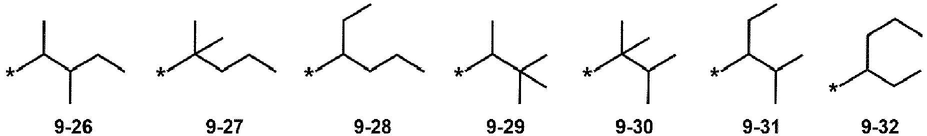

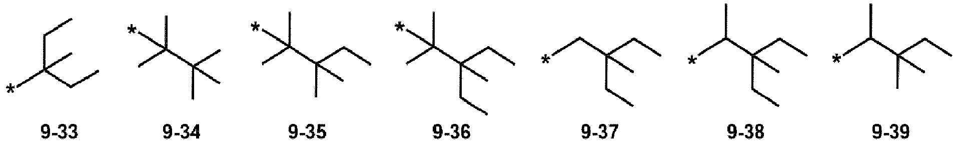

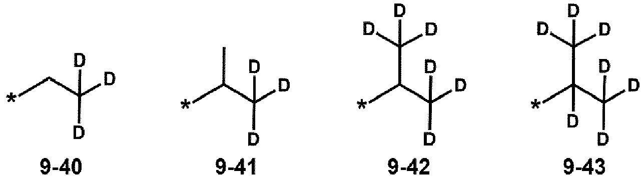

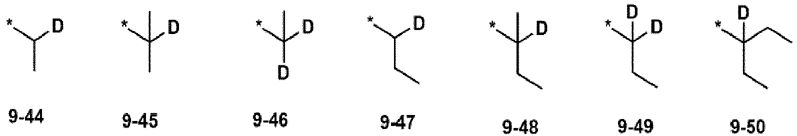

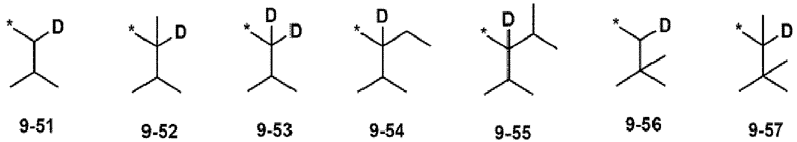

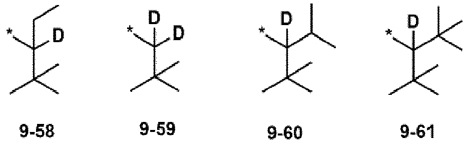

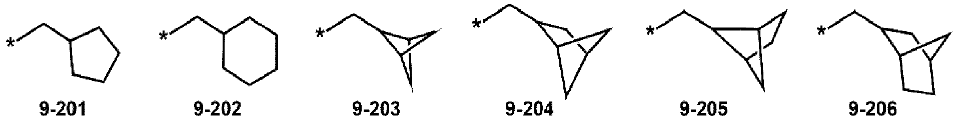

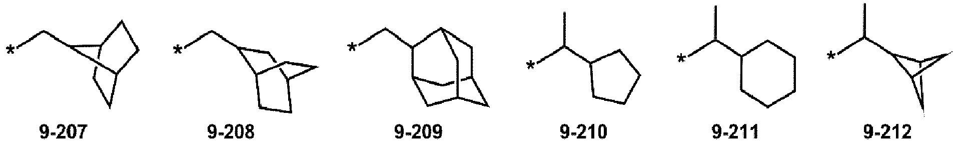

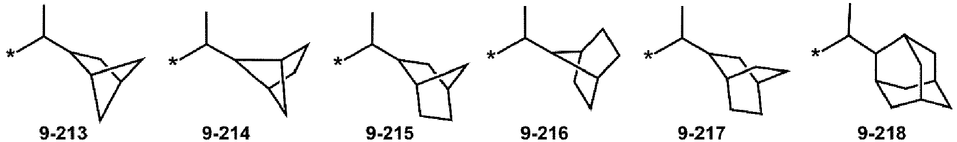

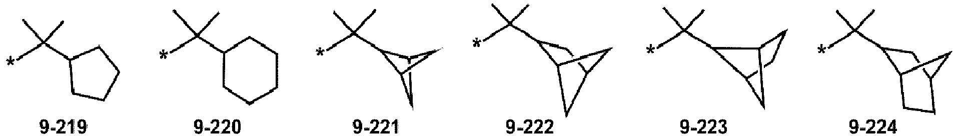

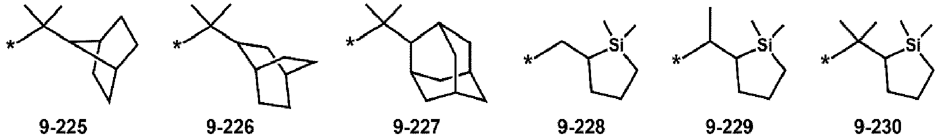

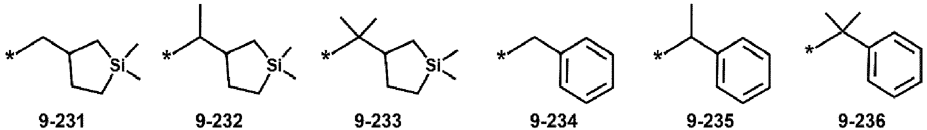

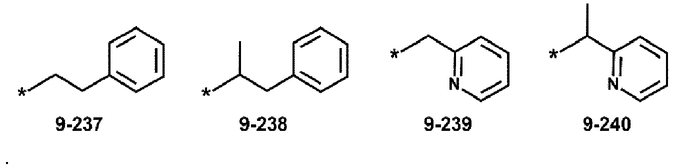

In Formulae 9-1 to 9-61 and 9-201 to 9-240, * indicates a binding site to a neighboring atom.

The heterocyclic compound represented by Formula 1 may include at least one deuterium.

In an embodiment, the heterocyclic compound may include deuterium as a substituent or a part of the substituent.

In an embodiment, the heterocyclic compound represented by Formula 1 may satisfy at least one of Conditions 1 to 7:
Condition 1
   At least one of R₁(s) in the number of d1 is deuterium;
Condition 2
   At least one of R₂(s) in the number of d2 is deuterium;
Condition 3
   At least one of R₃(s) in the number of n1*d3 is deuterium;
Condition 4
   At least one of R₄(s) in the number of n1*d4 is deuterium;
Condition 5
   At least one of R₅(s) in the number of d5 is deuterium;
Condition 6
   At least one of R₆(s) in the number of k1*n2*d6 is deuterium; and
Condition 7
   A least one of R₇(s) in the number of k1*d7 is deuterium.

For example, the heterocyclic compound may satisfy at least one of Conditions 1 and 2.

In an embodiment, the heterocyclic compound may satisfy at least two of Conditions 1 to 7.

In an embodiment, the heterocyclic compound may satisfy at least three of Conditions 1 to 7.

In an embodiment, the heterocyclic compound may satisfy at least four of Conditions 1 to 7.

In an embodiment, the heterocyclic compound may satisfy at least five of Conditions 1 to 7.

In an embodiment, the heterocyclic compound may satisfy at least six of Conditions 1 to 7.

In an embodiment, the heterocyclic compound may satisfy all of Conditions 1 to 7.

In an embodiment, the heterocyclic compound represented by Formula 1 may satisfy at least one of Conditions 1' to 7':
Condition 1'
   R₁(s) in the number of d1 are all deuterium, and d1 is 4;
Condition 2'
   R₂(s) in the number of d2 are all deuterium, and d2 is 4;
Condition 3'
   R₃(s) in the number of n1 *d3 are all deuterium, and d3 is 4;
Condition 4'
   R₄(s) in the number of n1 *d4 are all deuterium, and d4 is 3;
Condition 5'
   R₅(s) in the number of d5 are all deuterium,
   k1 is 1 and d5 is 4; or k1 is 2 and d5 is 3;
Condition 6'
   R₆(s) in the number of k1*n2*d6 are all deuterium, and d6 is 4; and
Condition 7'
   R₇(s) in the number of k1*d7 are all deuterium, and d7 is 4.

In an embodiment, the heterocyclic compound may satisfy at least two of Conditions 1' to 7'.

In an embodiment, the heterocyclic compound may satisfy at least three of Conditions 1' to 7'.

In an embodiment, the heterocyclic compound may satisfy at least four of Conditions 1' to 7'.

In an embodiment, the heterocyclic compound may satisfy at least five of Conditions 1' to 7'.

In an embodiment, the heterocyclic compound may satisfy at least six of Conditions 1' to 7'.

In an embodiment, the heterocyclic compound may satisfy all of Conditions 1' to 7'.

In an embodiment, the substitution ratio of deuterium of the heterocyclic compound represented by Formula 1 may be greater than about 0 % and less than or equal to about 100 %.

In an embodiment, the substitution ratio of deuterium of the heterocyclic compound represented by Formula 1 may be greater than or equal to about 10 % and less than or equal to about 100 %. For example, the substitution ratio of deuterium may be greater than or equal to about 10 % and less than or equal to about 100 %, greater than or equal to about 10 % and less than or equal to about 90 %, greater than or equal to about 15 % and less than or equal to about 100 %, greater than or equal to about 15 % and less than or equal to about 90 %, greater than or equal to about 20 % and less than or equal to about 100 %, greater than or equal to about 20 % and less than or equal to about 90 %, greater than or equal to about 40 % and less than or equal to about 100 %, or greater than or equal to about 40 % and less than or equal to about 90 %.

In an embodiment, the substitution ratio of deuterium of heterocyclic compound may be greater than or equal to about 40 % and less than or equal to about 90 %.

The term "substitution ratio of deuterium" as used herein refers to the ratio of the number of substituents substituted with deuterium to the number of substituents that can be substituted in the core of the heterocyclic compound represented by Formula 1. That is, the substitution ratio of deuterium refers to, in Formulae 1 and A, the ratio of the number of deuterium in R₁ to R₇ to the total number of R₁ to R₇.

In an embodiment, among R₁(s) in the number of d1, each of R₂(s) in the number of d2, R₃(s) in the number of n1*d3, R₄(s) in the number of n1*d4, R₅(s) in the number of d5, Re(s) in the number of k1*n2*d6, and R₇(s) in the number of k1*d7, at least 10 % of the substituents may be deuterium.

In an embodiment, among R₁(s) in the number of d1, R₂(s) in the number of d2, R₃(s) in the number of n1*d3, R₄(s) in the number of n1*d4, R₅(s) in the number of d5, Re(s) in the number of k1*n2*d6, and R₇(s) in the number of k1*d7, at least 20 % of the substituents may be deuterium.

In an embodiment, among R₁(s) in the number of d1, R₂(s) in the number of d2, R₃(s) in the number of n1*d3, R₄(s) in the number of n1*d4, R₅(s) in the number of d5, Re(s) in the number of k1*n2*d6, and R₇(s) in the number of k1*d7, at least 30 % of the substituents may be deuterium.

In an embodiment, among R₁(s) in the number of d1, R₂(s) in the number of d2, R₃(s) in the number of n1*d3, R₄(s) in the number of n1*d4, R₅(s) in the number of d5, Re(s) in the number of k1*n2*d6, and R₇(s) in the number of k1*d7, at least 40 % of the substituents may be deuterium.

In an embodiment, among R₁(s) in the number of d1, R₂(s) in the number of d2, R₃(s) in the number of n1*d3, R₄(s) in the number of n1*d4, R₅(s) in the number of d5, Re(s) in the number of k1*n2*d6, and R₇(s) in the number of k1*d7, at least 80 % of the substituents may be deuterium.

In an embodiment, among R₁(s) in the number of d1, R₂(s) in the number of d2, R₃(s) in the number of n1*d3, R₄(s) in the number of n1*d4, R₅(s) in the number of d5, Re(s) in the number of k1*n2*d6, and R₇(s) in the number of k1*d7, at least 90 % of the substituents may be deuterium.

In an embodiment, the heterocyclic compound represented by Formula 1 may be represented by Formula 1A: 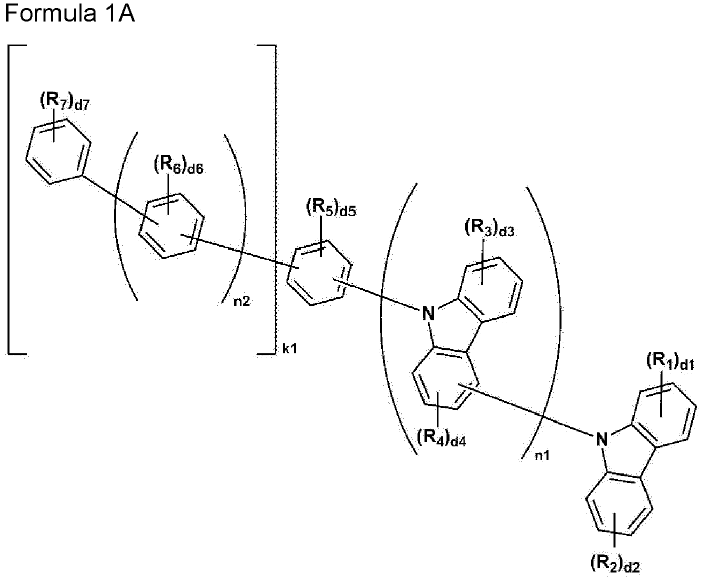
wherein, in Formula 1A,
k1, n1, n2, R₁ to R₇, and d1 to d7 are each the same as described herein.

In an embodiment, a moiety represented by 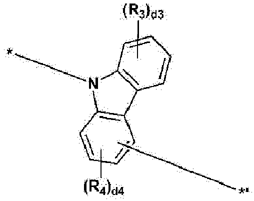 in Formula 1 may be a group represented by one of Formulae 2-1 to 2-4: 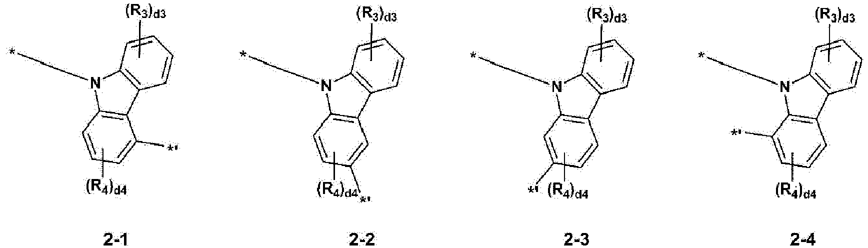
wherein, in Formulae 2-1 to 2-4,
R₃, R₄, d3, and d4 are each the same as described herein, and
* and *' each indicate a binding site to a neighboring atom.

In an embodiment, a moiety represented by 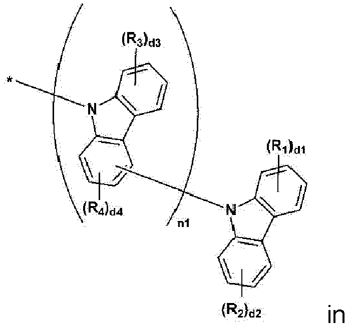 Formula 1 may be a group represented by one of Formulae 3-1 to 3-4: 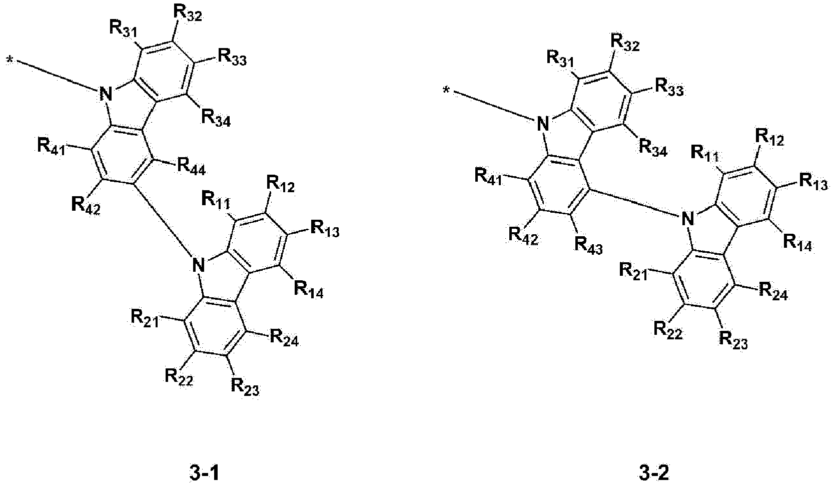 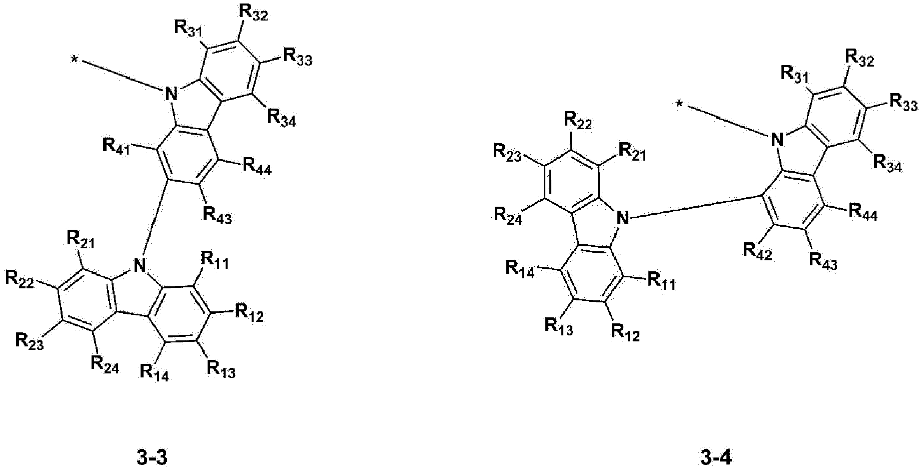
wherein, in Formulae 3-1 to 3-4,
R₁₁ to R₁₄ are each the same as described in connection with R₁,
R₂₁ to R₂₄ are each the same as described in connection with R₂,
R₃₁ to R₃₄ are each the same as described in connection with R₃,
R₄₁ to R₄₄ are each the same as described in connection with R₄, and
* indicates a binding site to a neighboring atom.

In an embodiment, in Formulae 3-1 to 3-4, at least one of R₁₁ to R₁₄, R₂₁ to R₂₄, R₃₁ to R₃₄, and R₄₁ to R₄₄ may be deuterium.

In an embodiment, in Formulae 3-1 to 3-4, some or all of R₁₁ to R₁₄, R₂₁ to R₂₄, R₃₁ to R₃₄, and R₄₁ to R₄₄ may be deuterium.

In an embodiment, a group represented by one of Formulae 3-1 to 3-4 may satisfy one of Conditions 8-1 to 8-5:
Condition 8-1
   At least one of R₁₁ to R₁₄ is deuterium;
Condition 8-2
   At least one of R₂₁ to R₂₄ is deuterium;
Condition 8-3
   At least one of R₃₁ to R₃₄ is deuterium; and
Condition 8-4
   At least one of R₄₁ to R₄₄ is deuterium.

In an embodiment, a moiety represented by 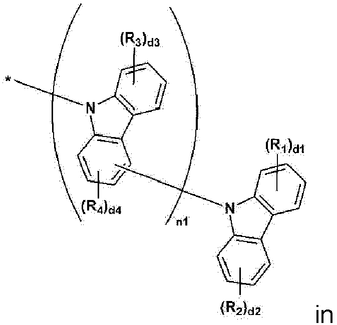 Formula 1 may be a group represented by one of Formulae 3-101 to 3-112, 3-201 to 3-212, 3-301 to 3-312, and 3-401 to 3-412: 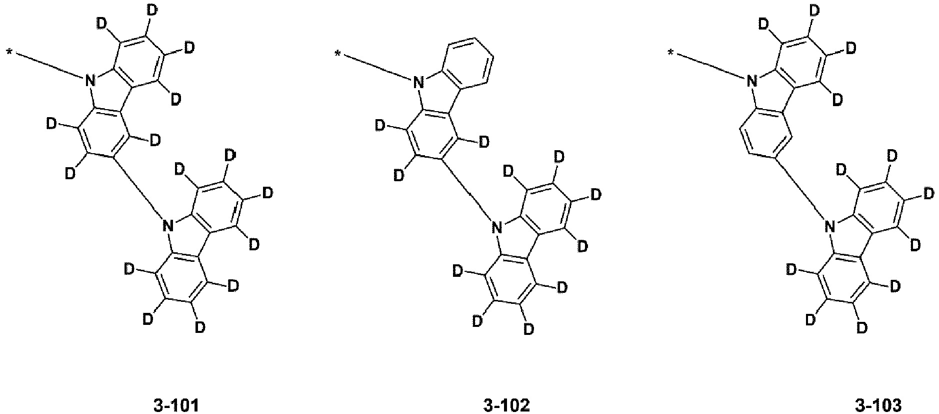 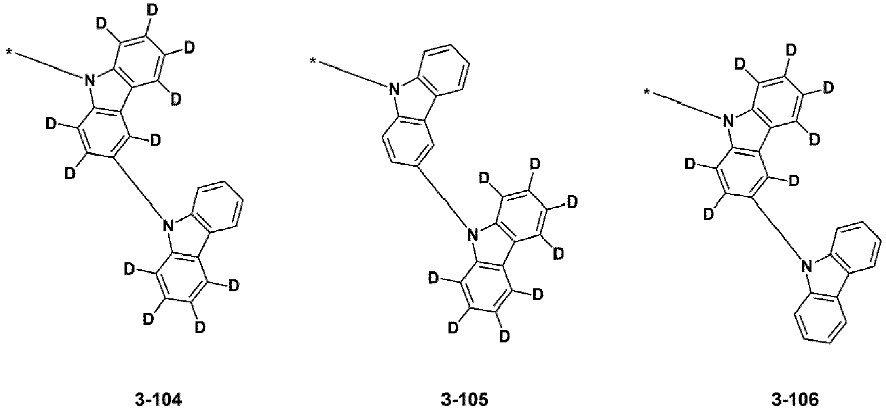 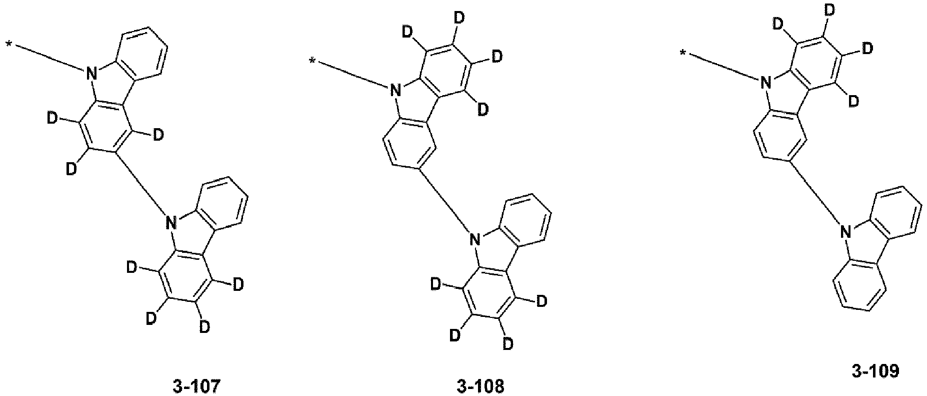 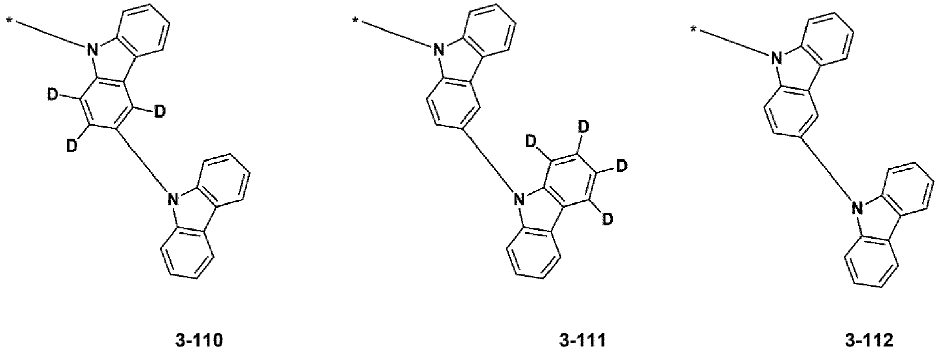 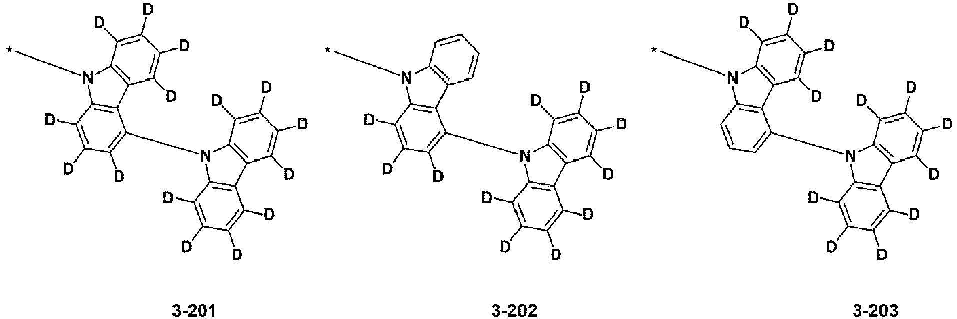 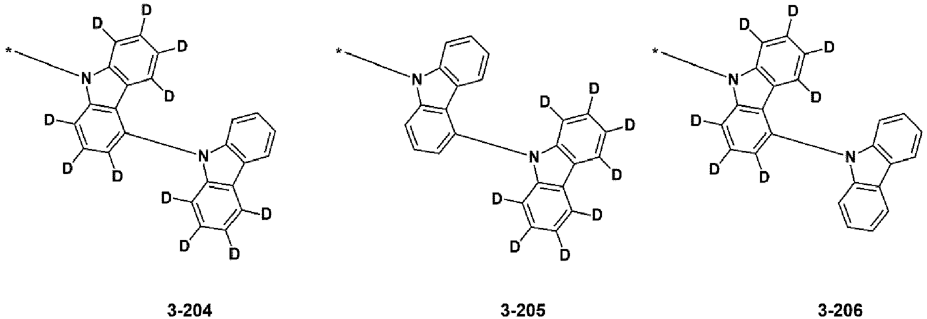 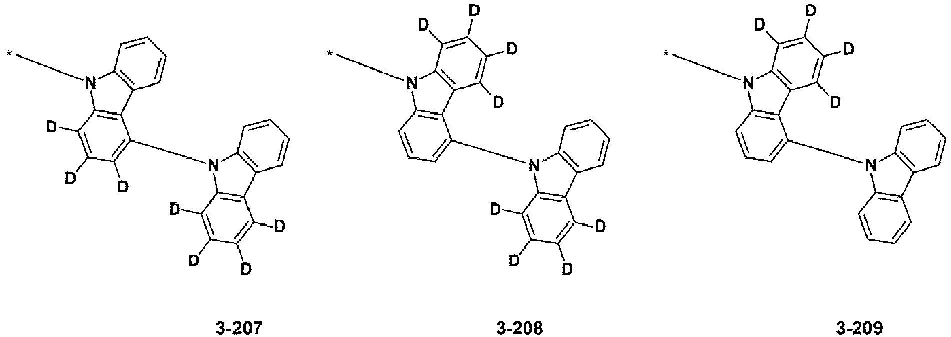 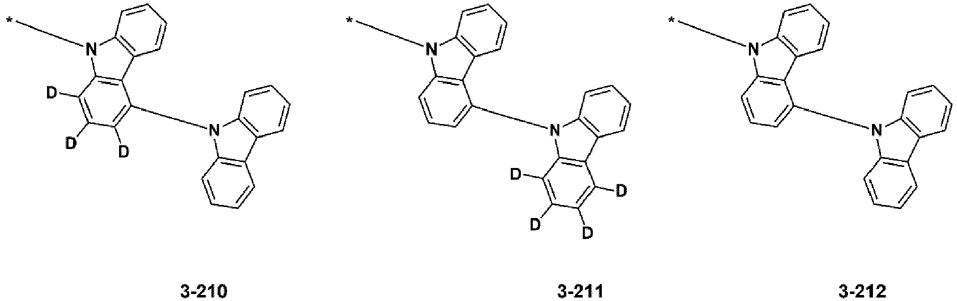 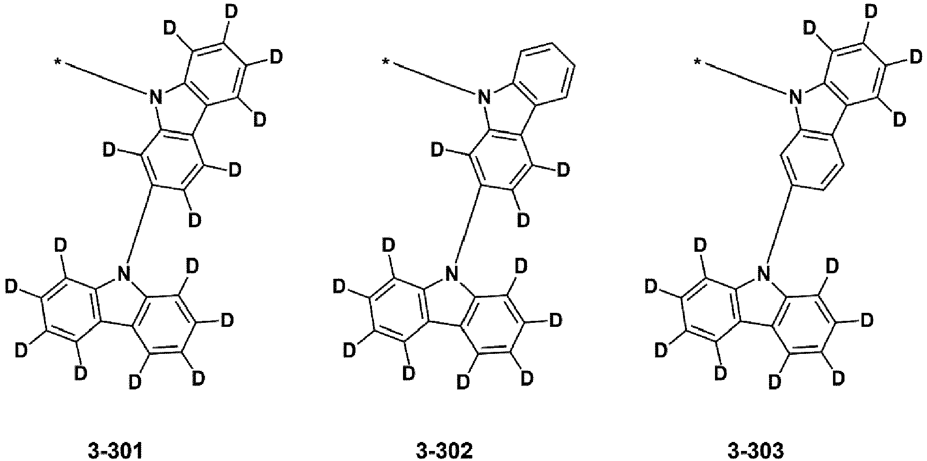 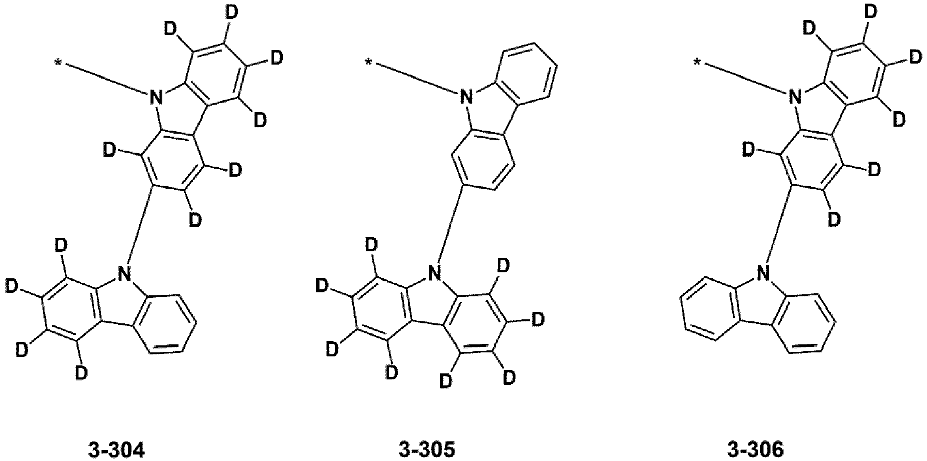 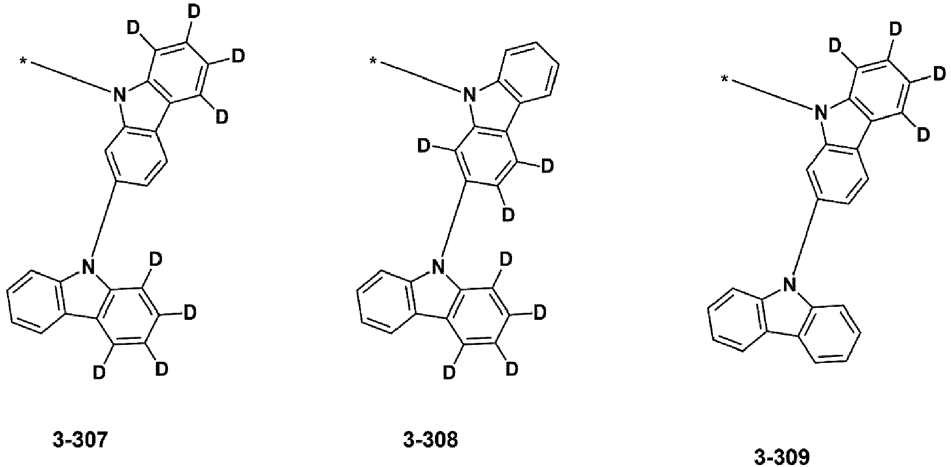 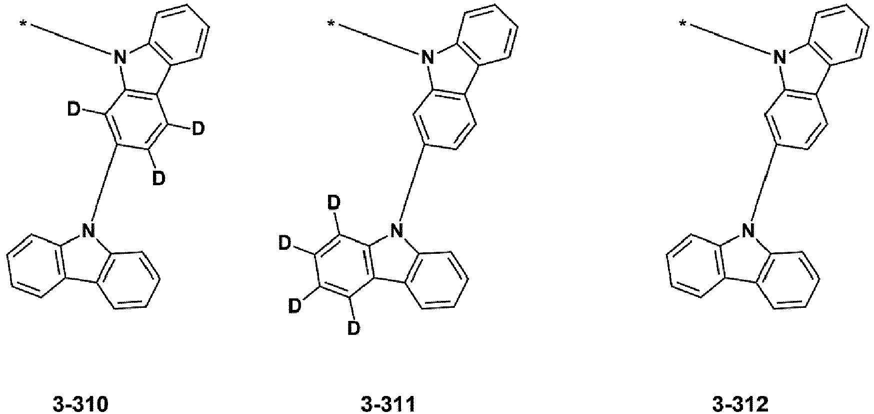 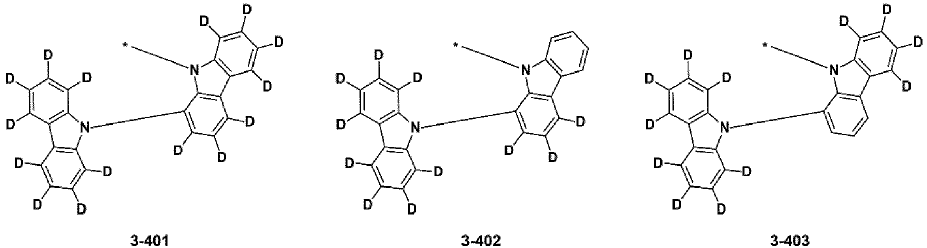 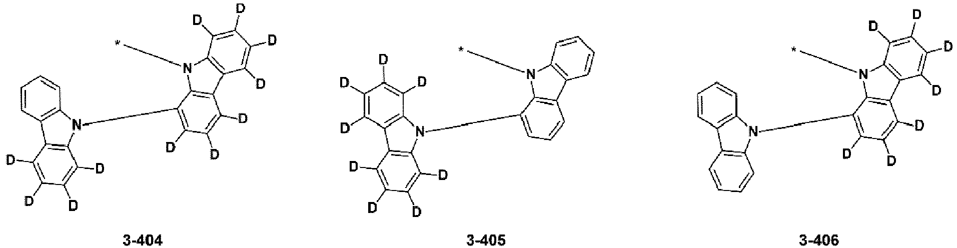 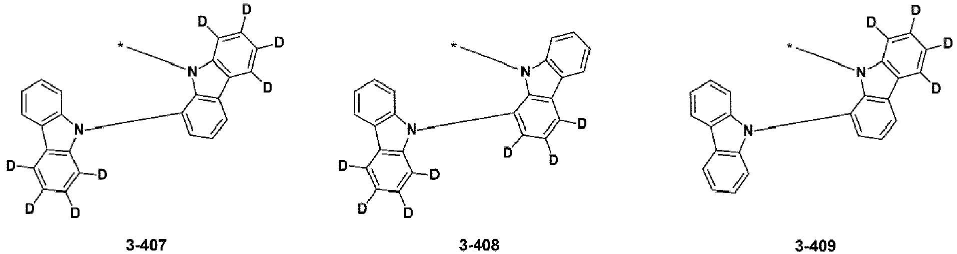 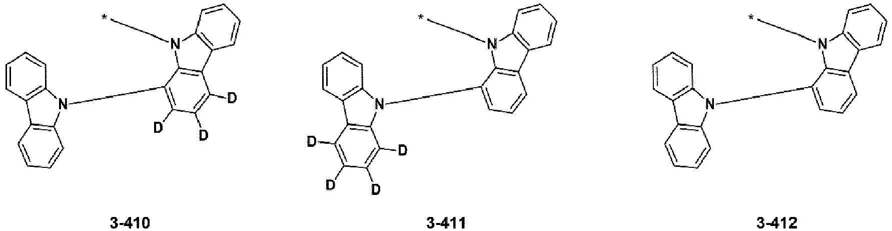
wherein, in Formulae 3-101 to 3-112, 3-201 to 3-212, 3-301 to 3-312, and 3-401 to 3-412,
D is a deuterium atom, and
* indicates a binding site to a neighboring atom.

In an embodiment, the group represented by Formula A may be a group represented by Formula A-1: 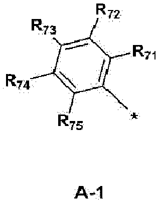
wherein, in Formula A-1,
R₇₁ to R₇₅ are each the same as described in connection with R₇, and
* indicates a binding site to a neighboring atom.

In an embodiment, in Formula A-1, at least one of R₇₁ to R₇₅ may be deuterium.

In an embodiment, some or all of R₇₁ to R₇₅ in Formula A-1 may be deuterium.

In an embodiment, a moiety represented by 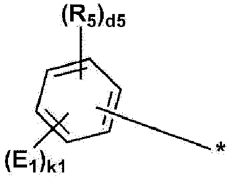 in Formula 1 may be a group represented by one of Formula 4-2 or 4-3: 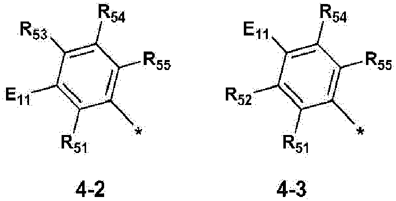
wherein, in Formulae 4-2 and 4-3,
E₁₁ is the same as described in connection with E₁,
R₅₁ to R₅₅ are each the same as described in connection with R₅, and
* indicates a binding site to a neighboring atom.

In an embodiment, at least one of R₅₁ to R₅₅ in Formulae 4-2 and 4-3 may be deuterium.

In an embodiment, some or all of R₅₁ to R₅₅ in Formula 4-2 and 4-3 may be deuterium.

In an embodiment, a moiety represented by 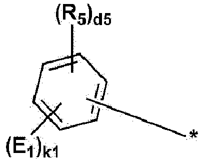 in Formula 1 may be a group represented by one of Formulae 4-201 to 4-279 and 4-301 to 4-356: 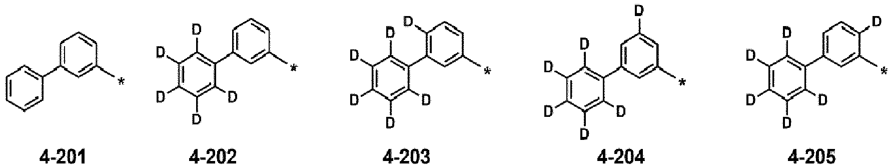 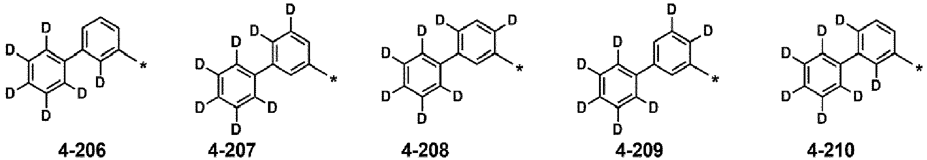 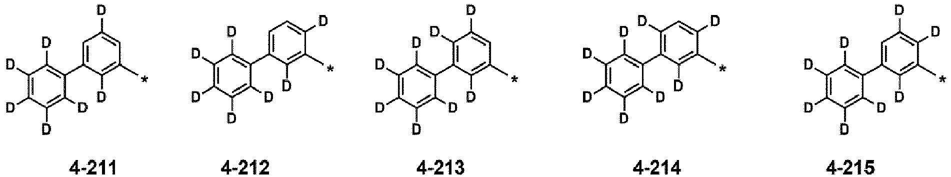 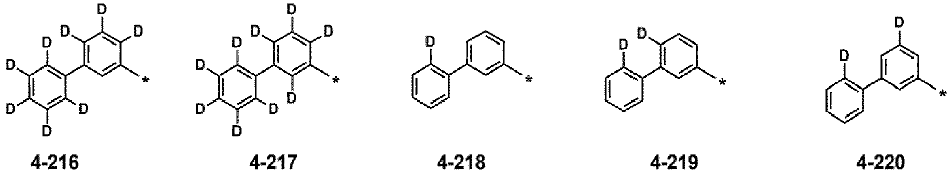 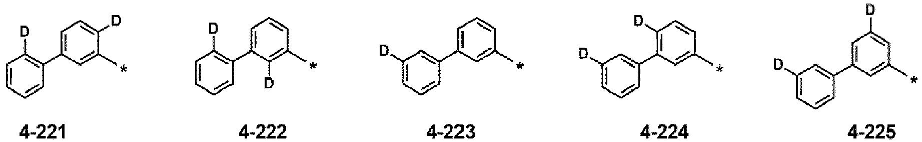 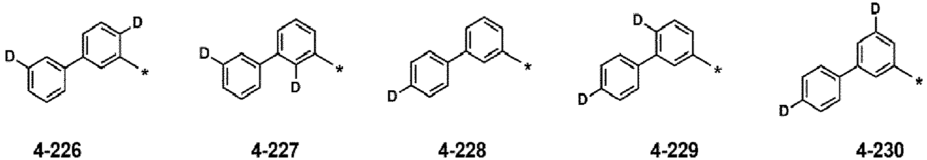 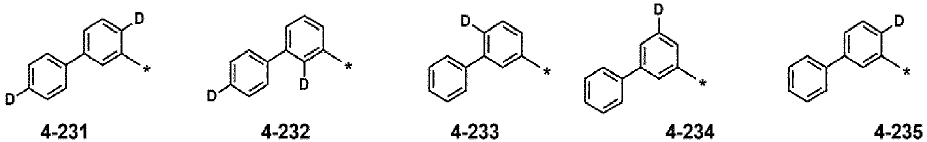 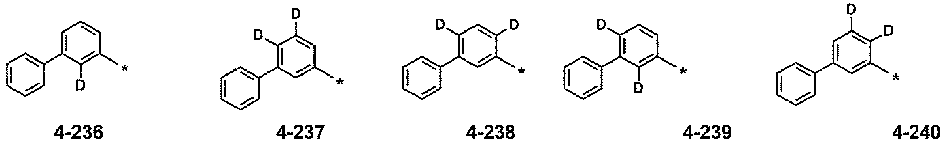 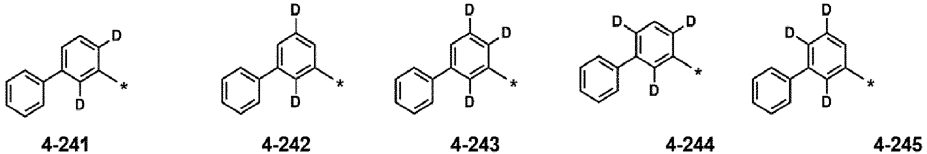 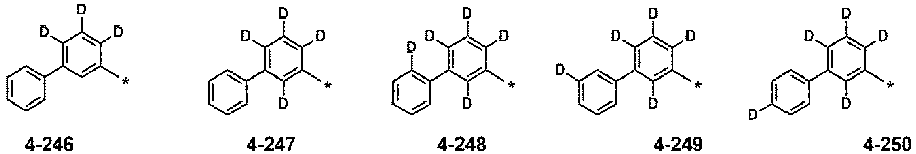 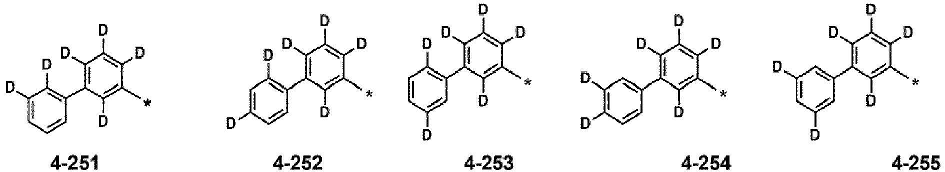 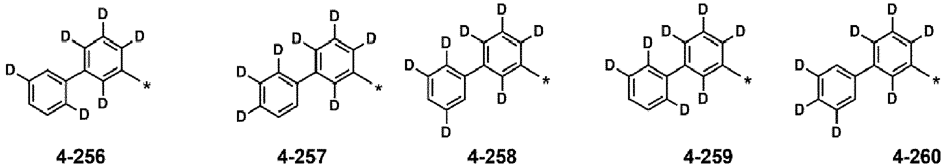 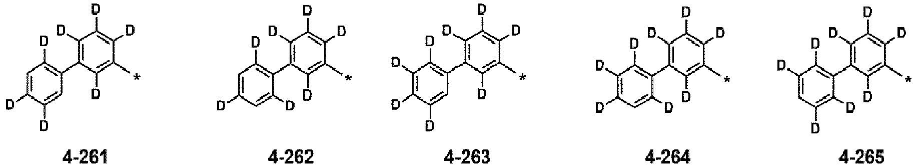 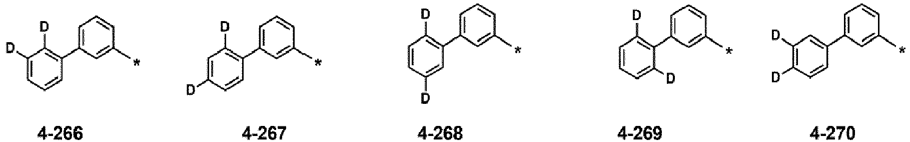 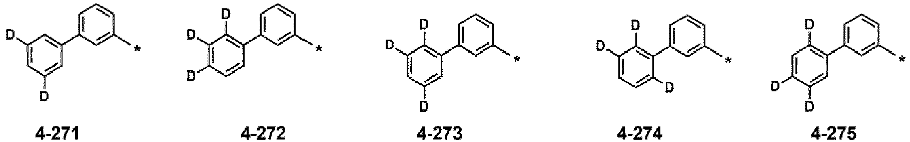 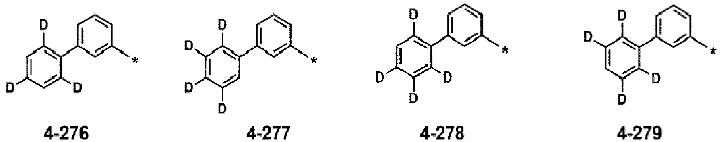 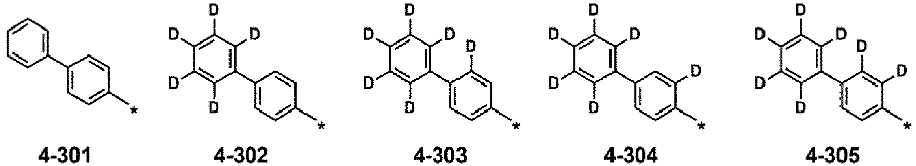 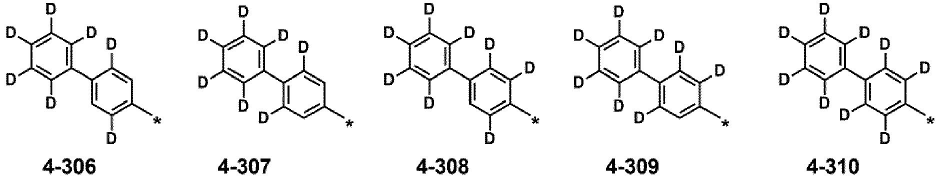 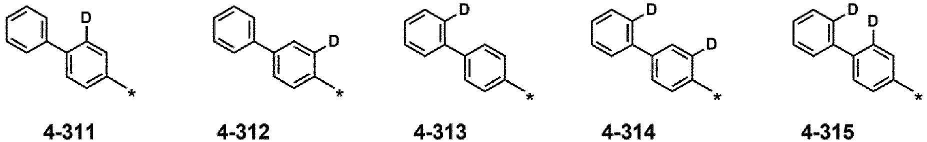 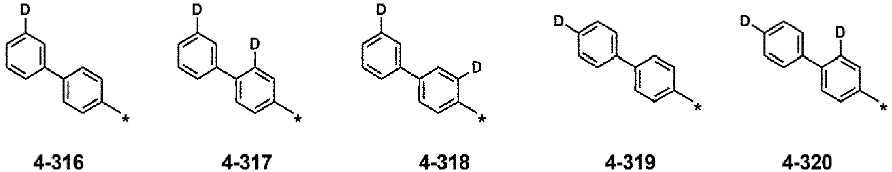 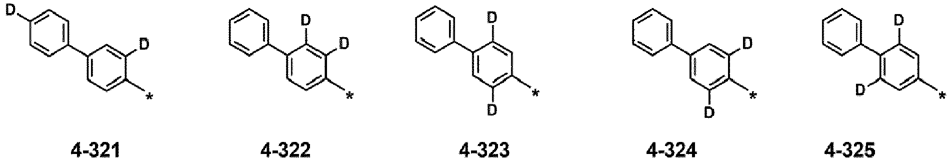 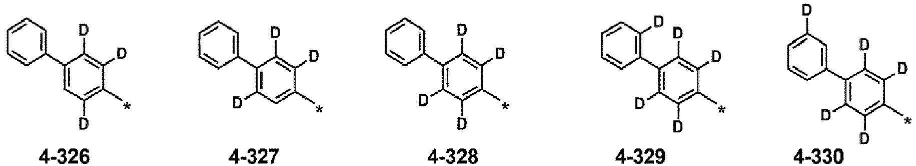 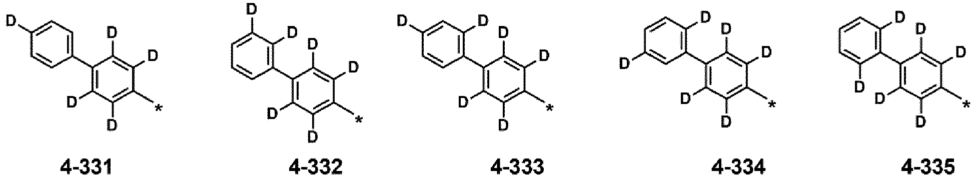 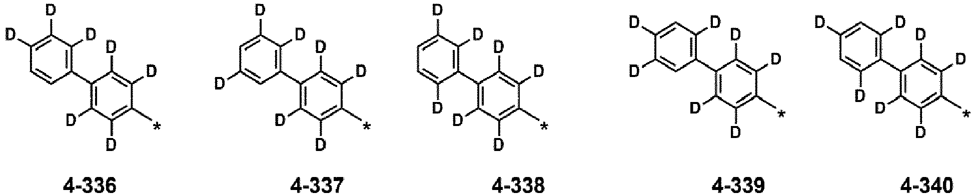 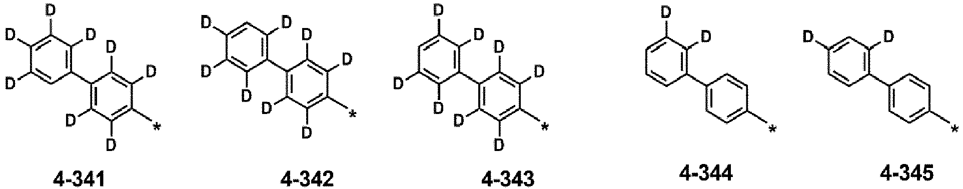 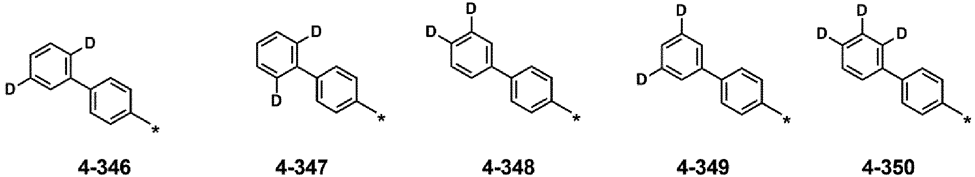 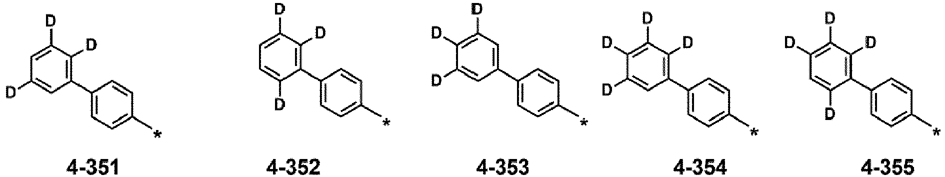 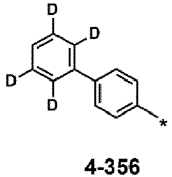
wherein, in Formulae 4-201 to 4-279 and 4-301 to 4-356,
D is a deuterium atom, and
* indicates a binding site to a neighboring atom.

The heterocyclic compound represented by Formula 1 may be represented by Formula 1-1 or 1-2: 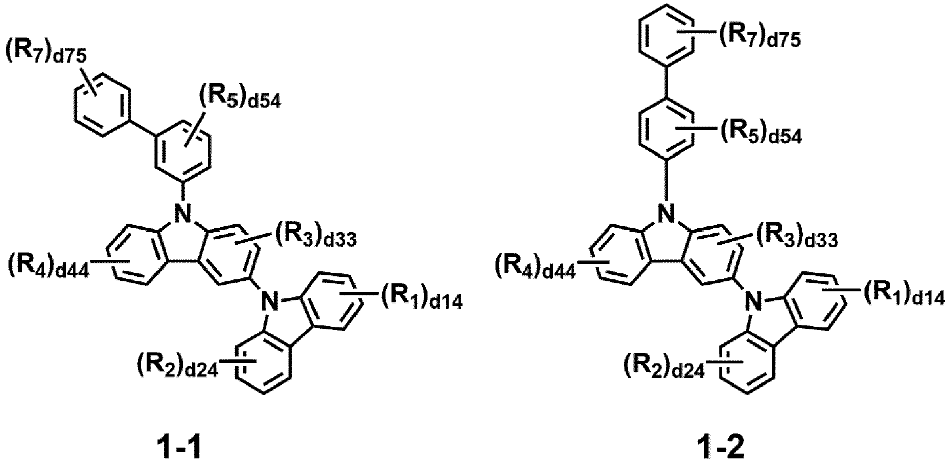
wherein, in Formulae 1-1 and 1-2,
R₁ to R₅ are each the same as described herein,
d75 may be an integer from 0 to 5,
d14, d24, d34, and d54 may each independently be an integer from 0 to 4,
d43 may be an integer from 0 to 3, and
the heterocyclic compounds represented by Formulae 1-1 and 1-2 may each include at least one deuterium.

In one or more embodiments, the heterocyclic compound represented by Formula 1 may be one of Compounds 1 to 160:

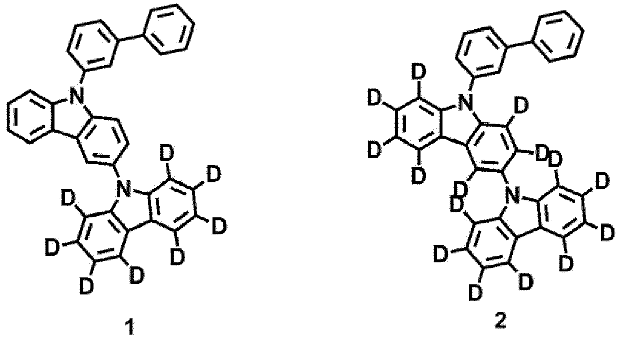

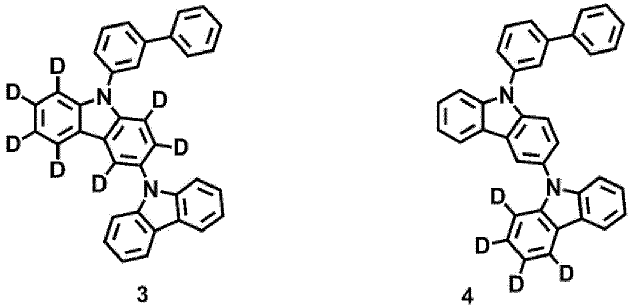

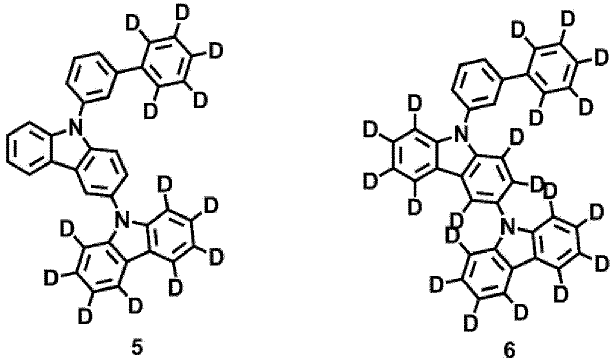

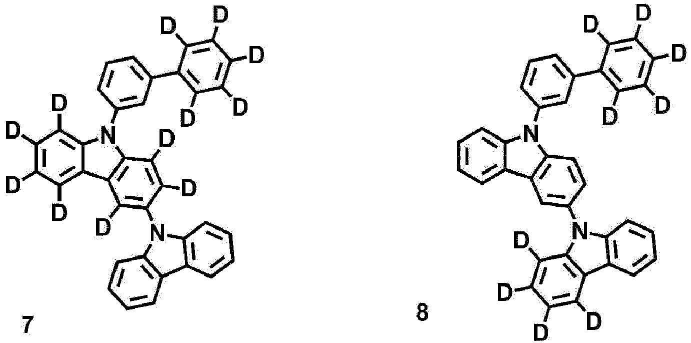

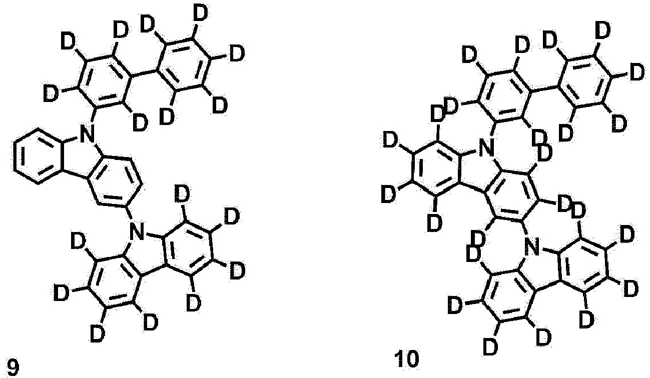

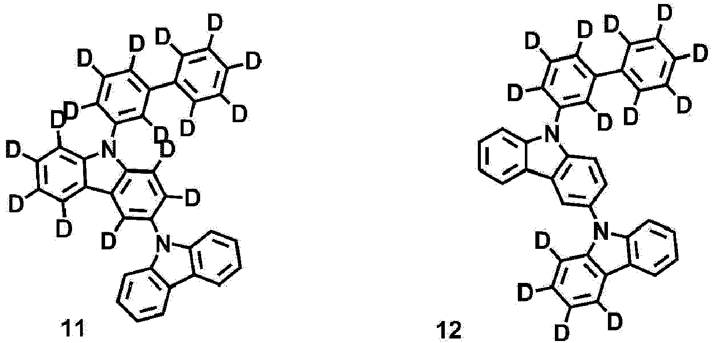

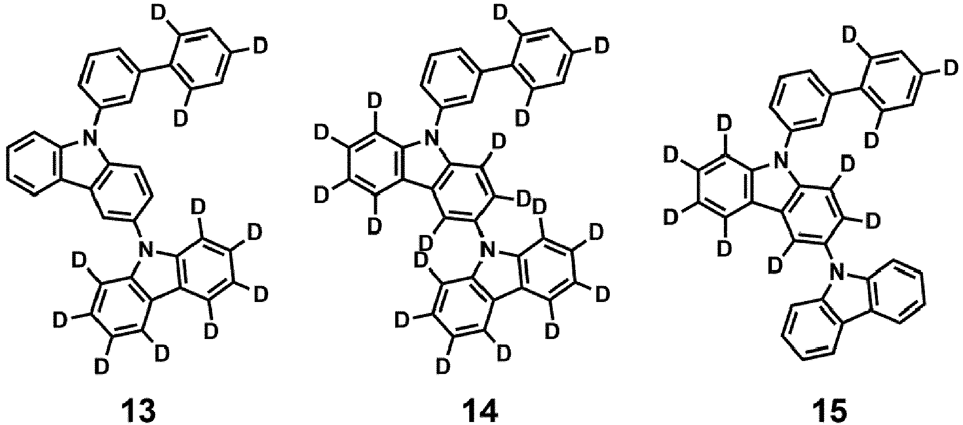

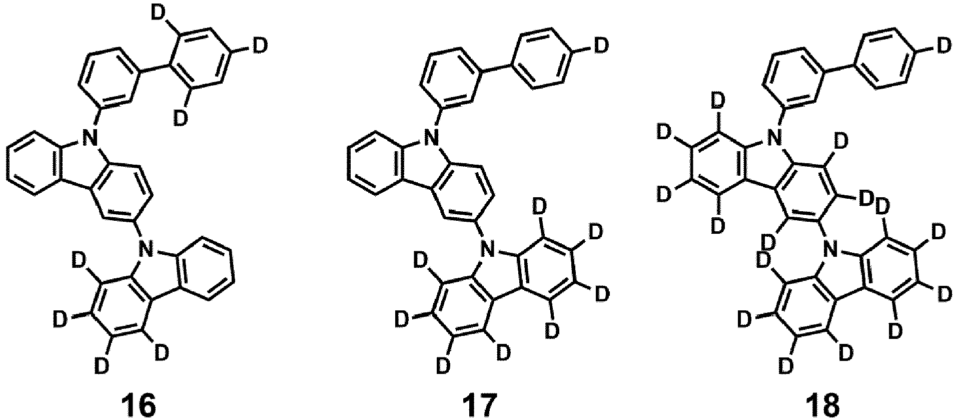

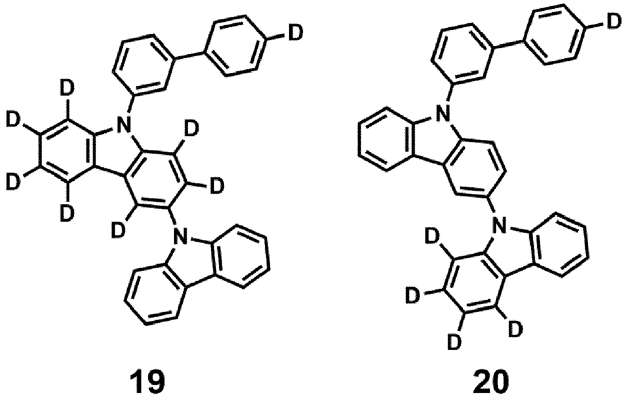

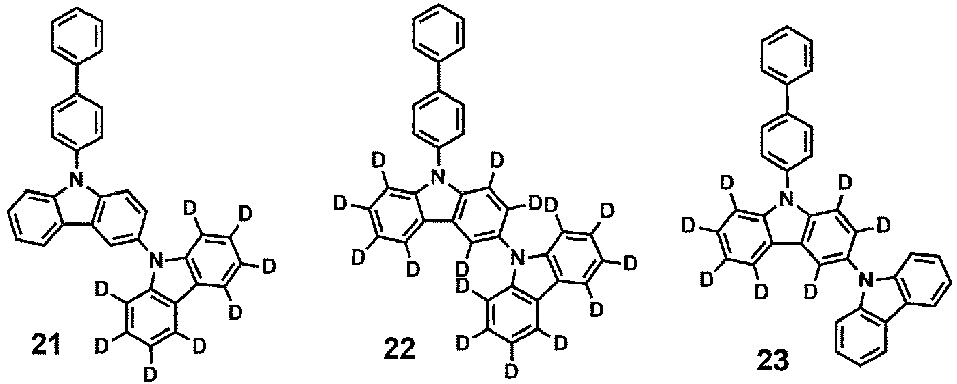

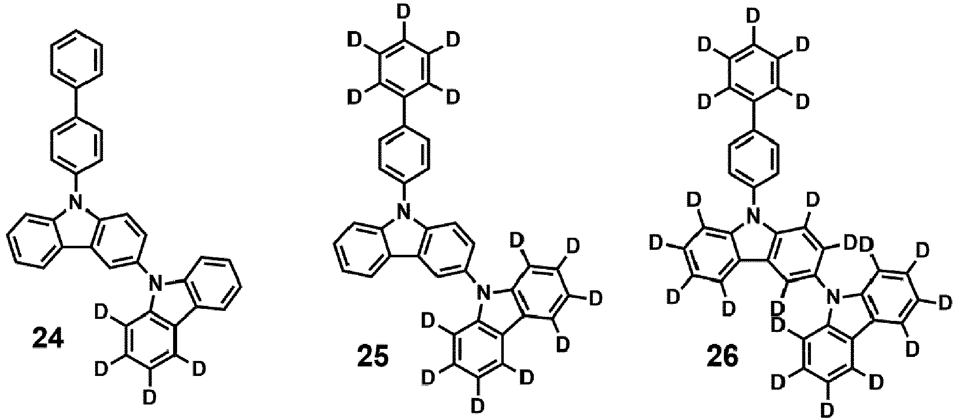

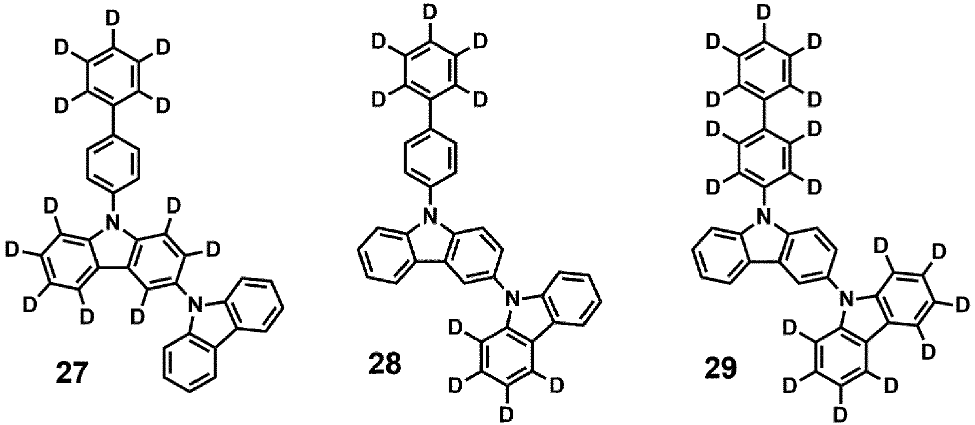

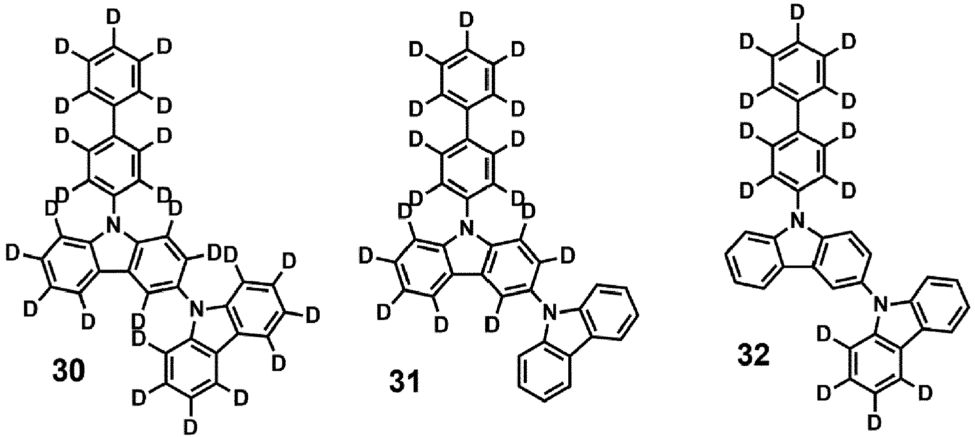

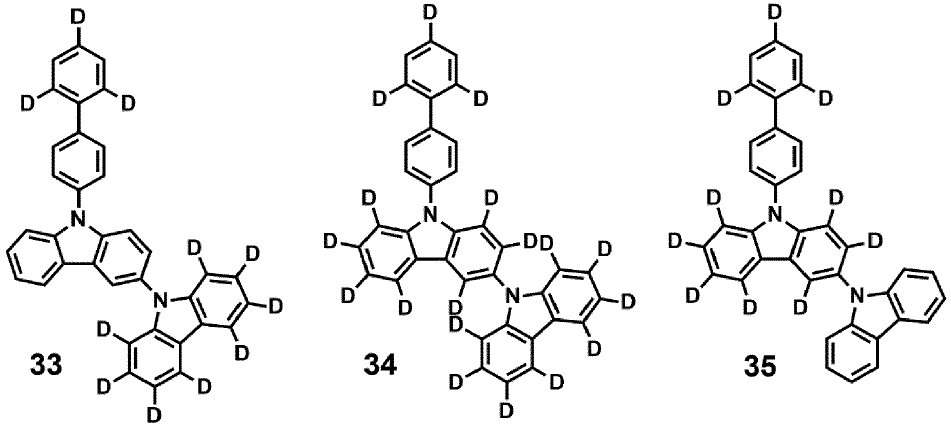

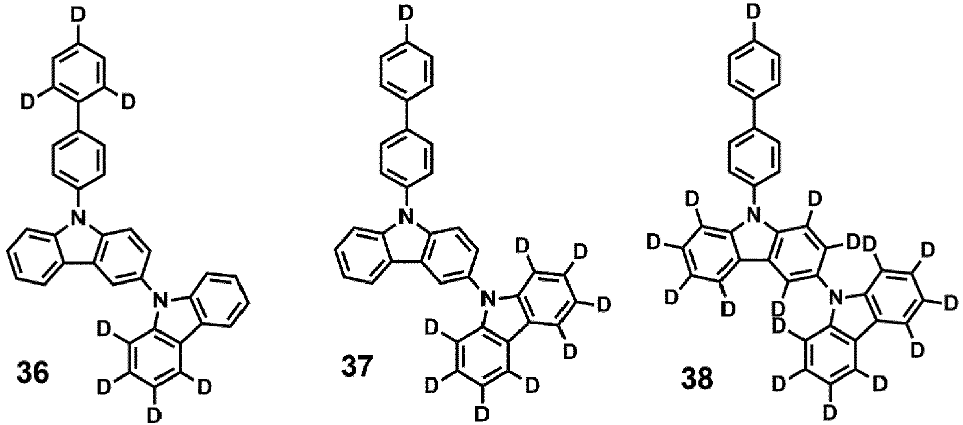

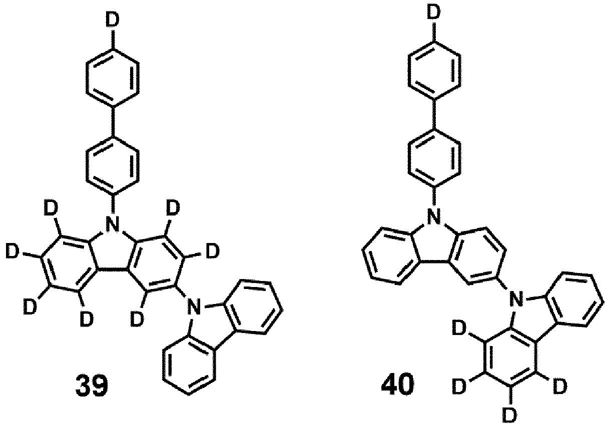

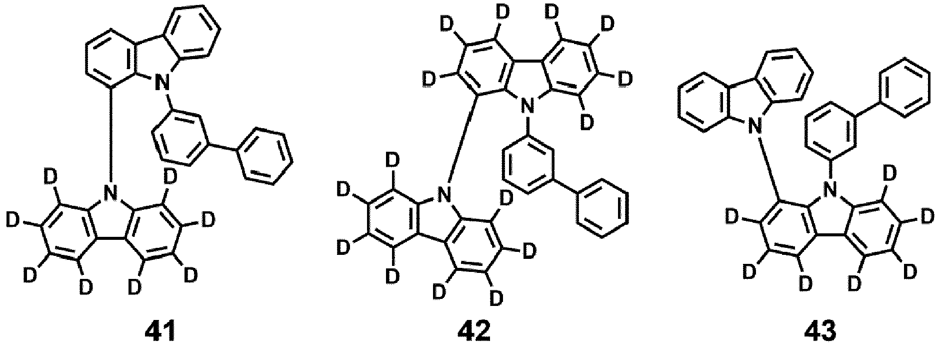

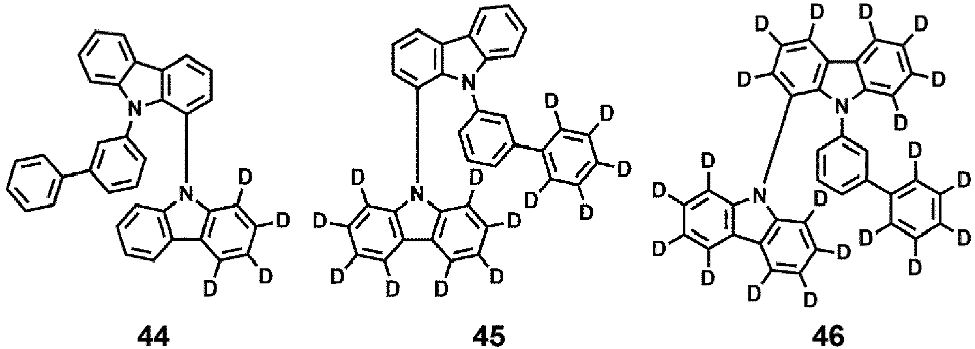

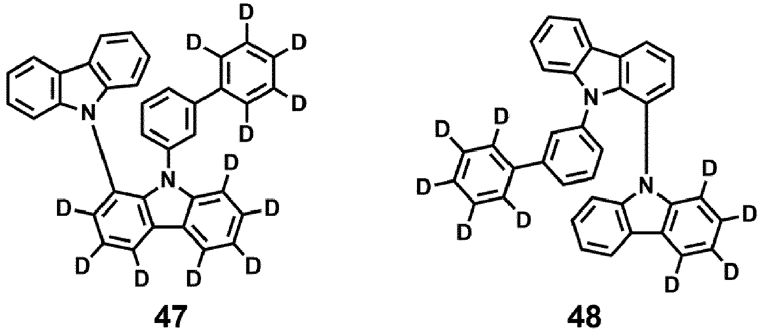

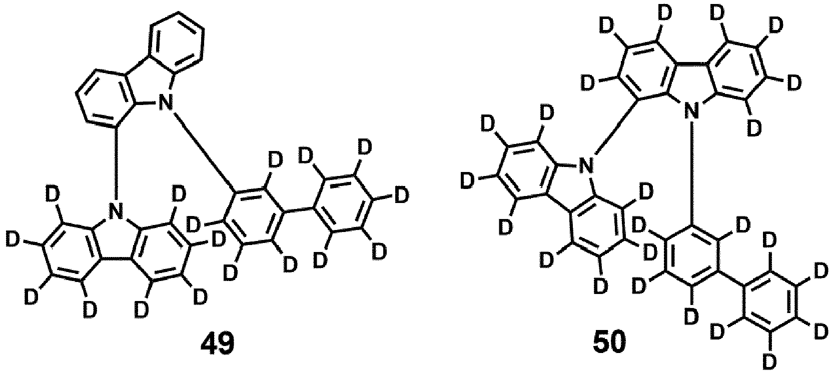

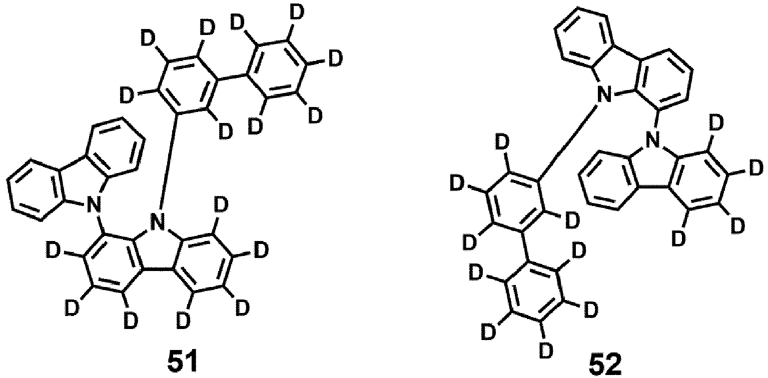

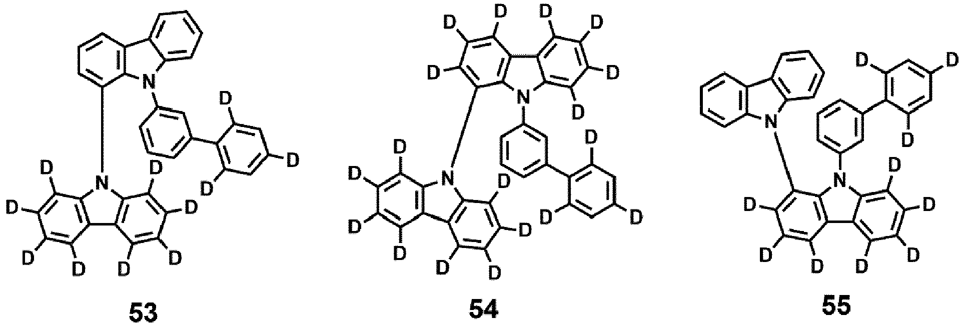

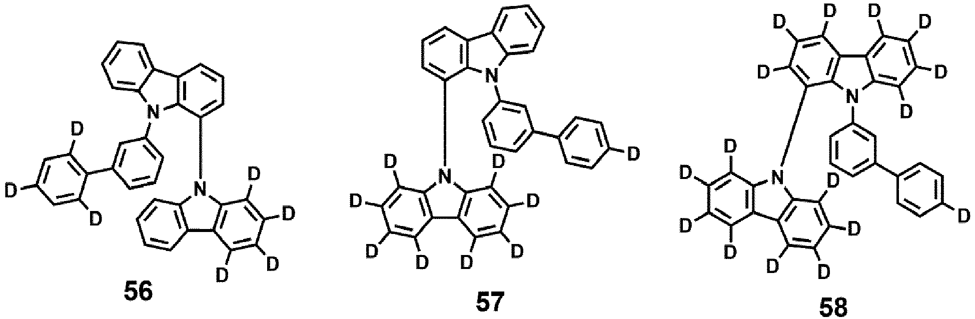

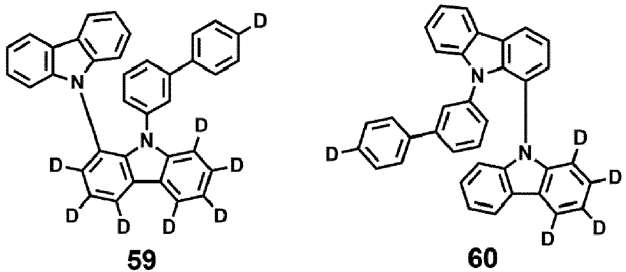

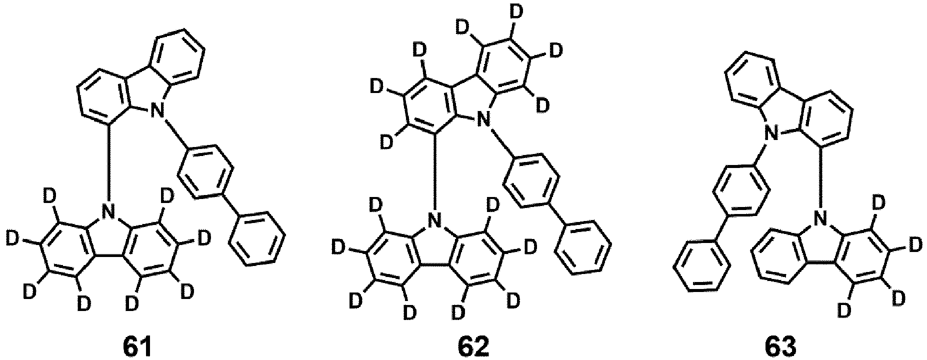

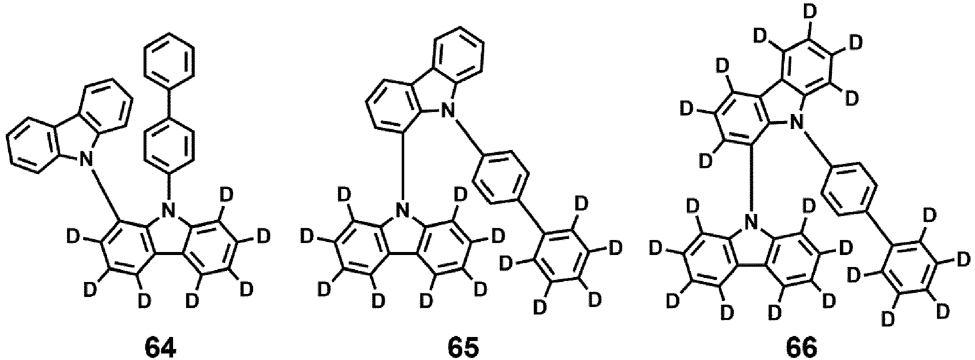

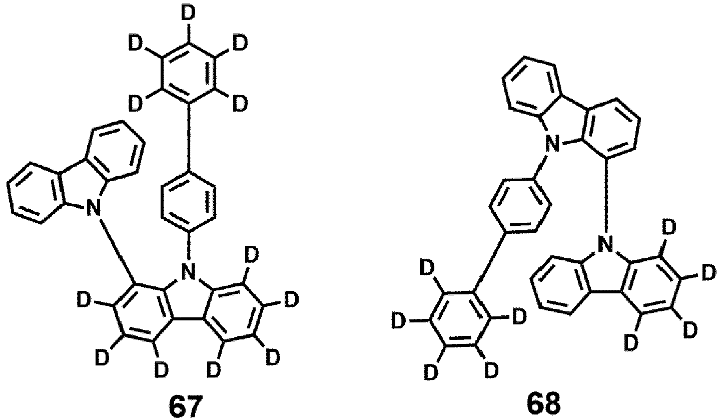

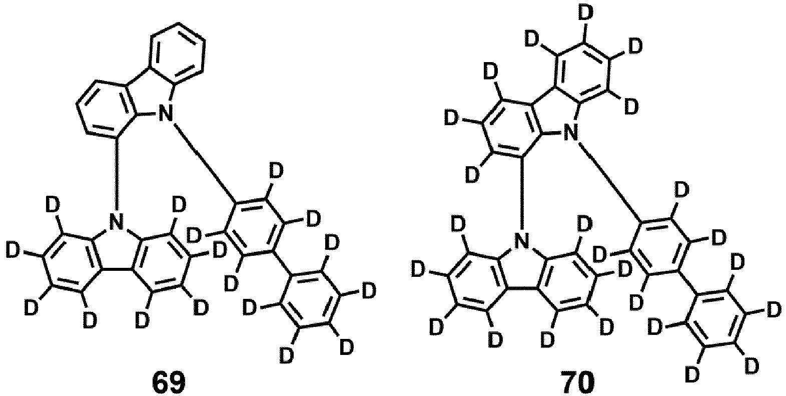

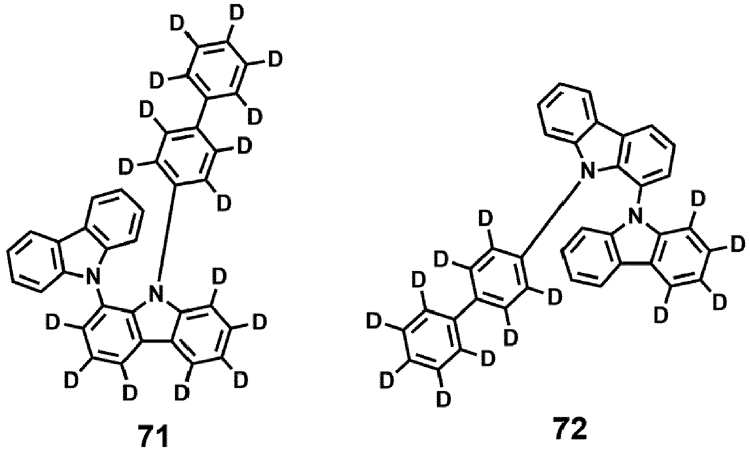

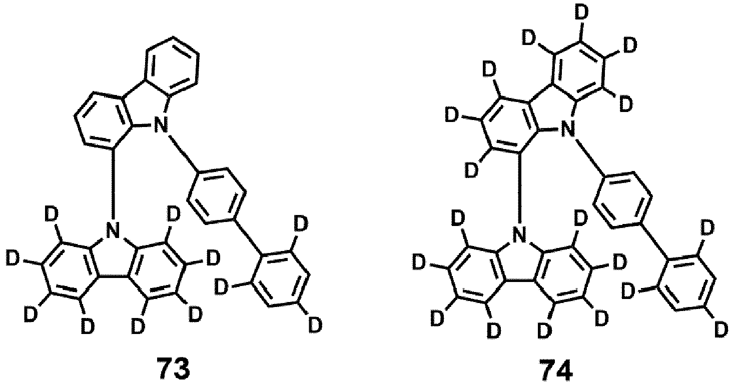

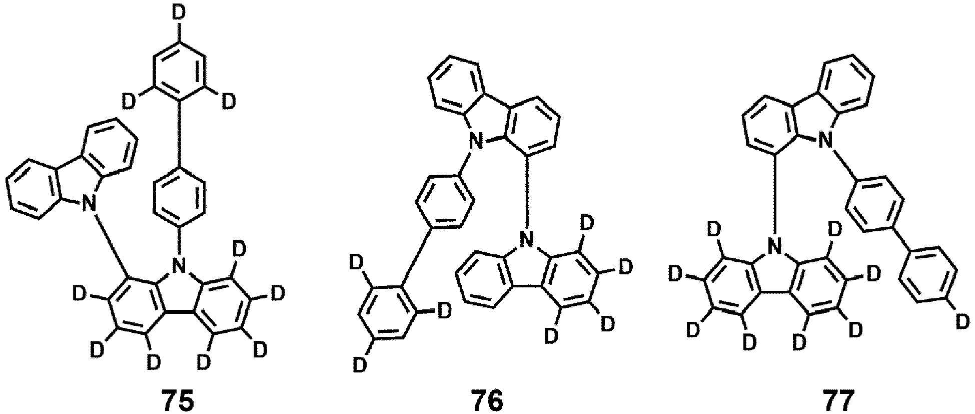

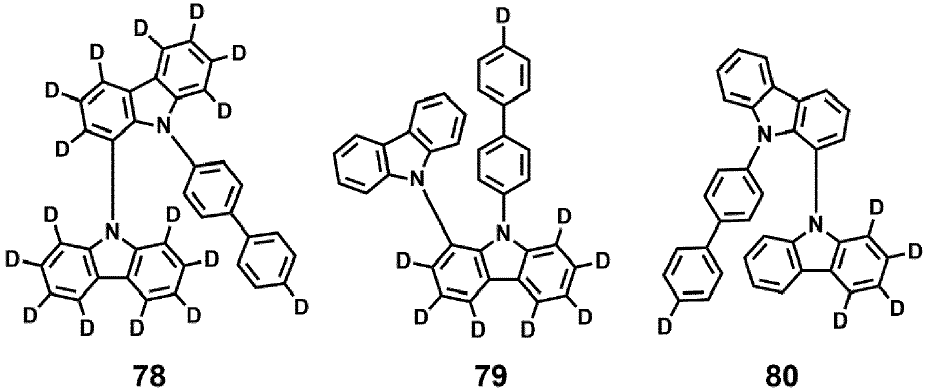

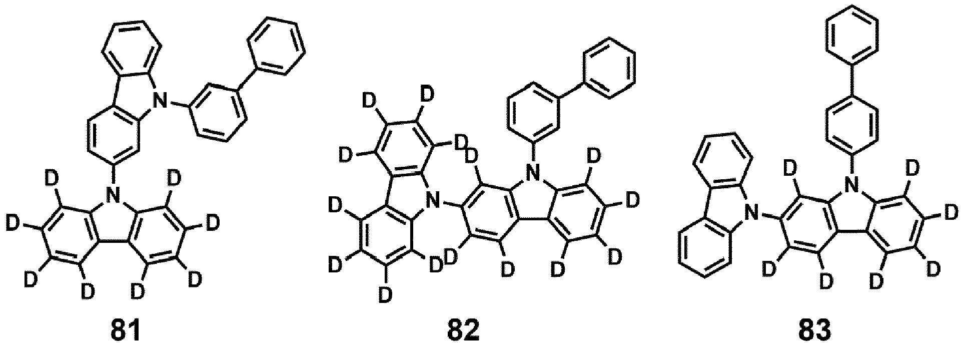

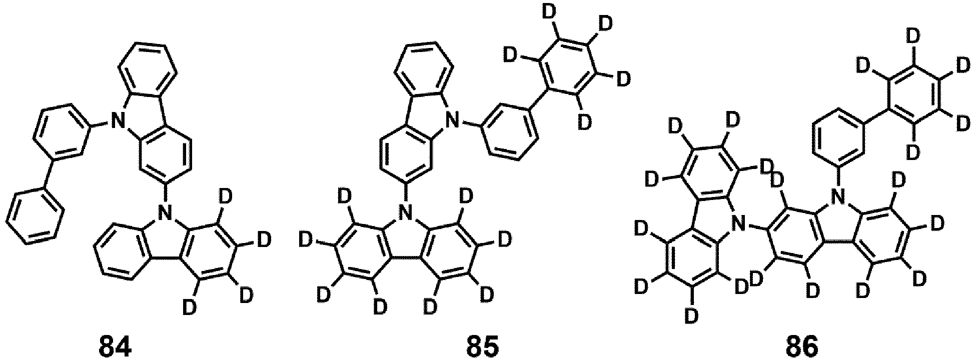

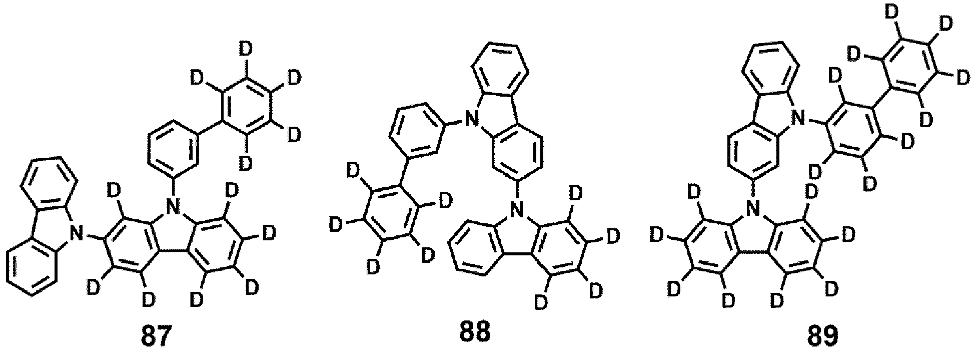

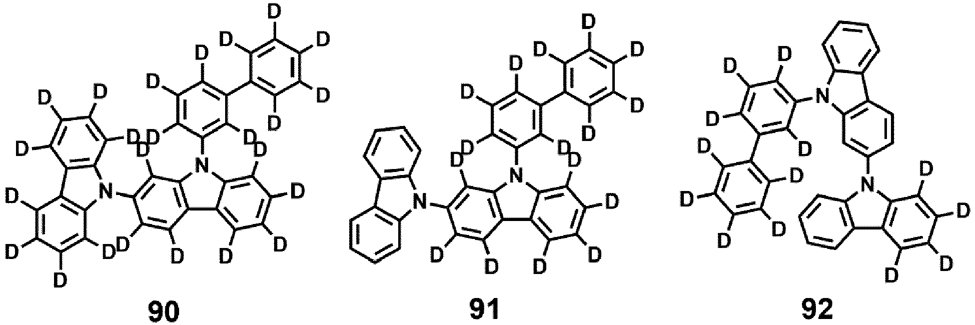

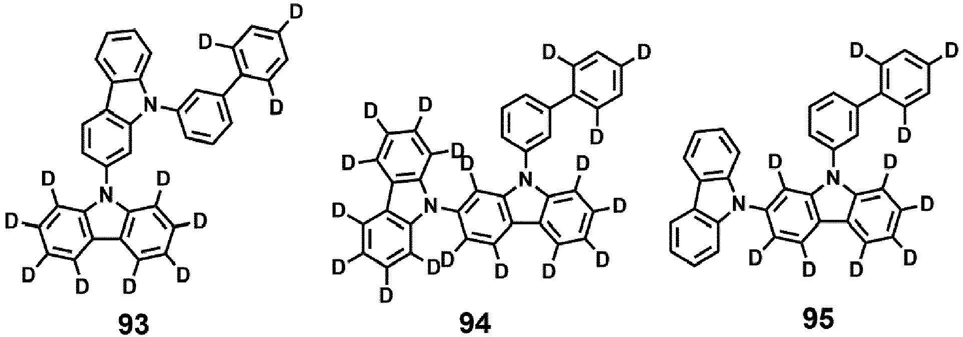

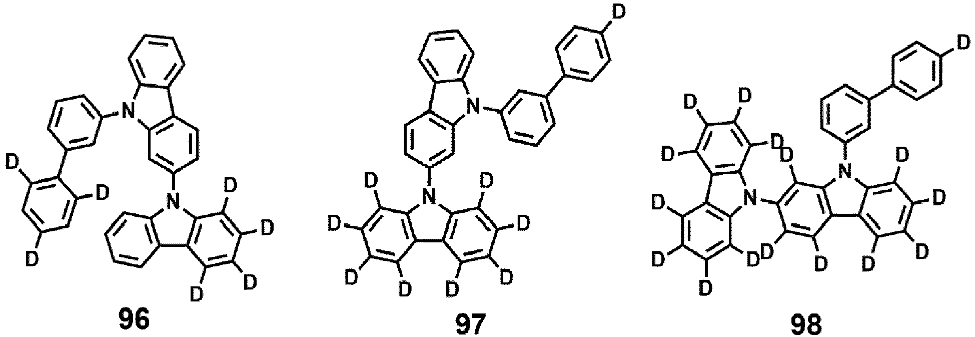

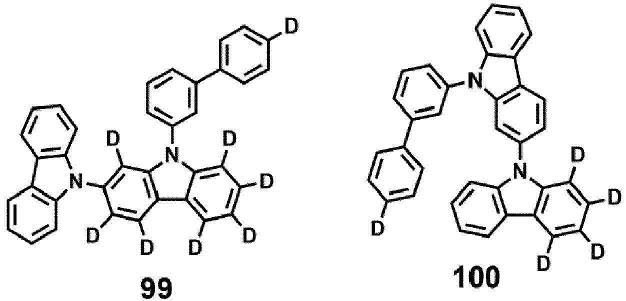

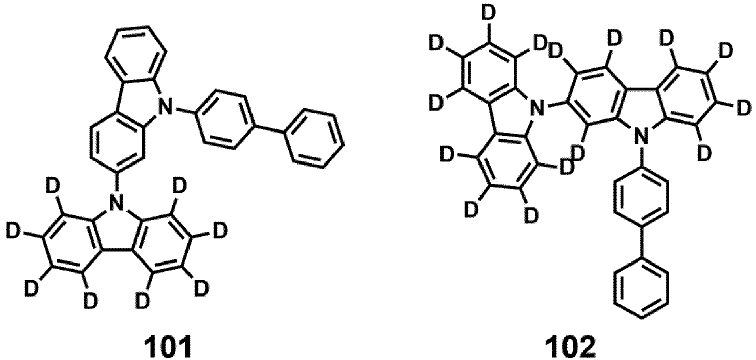

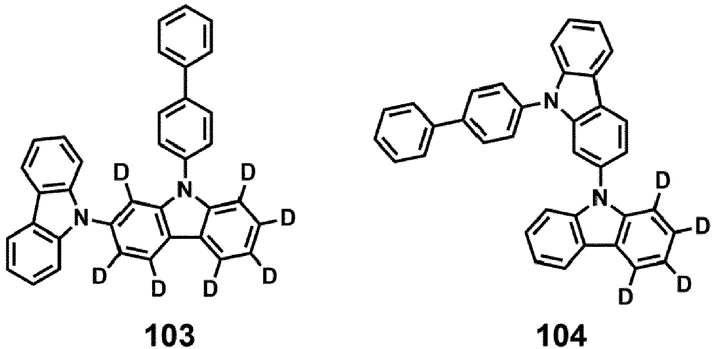

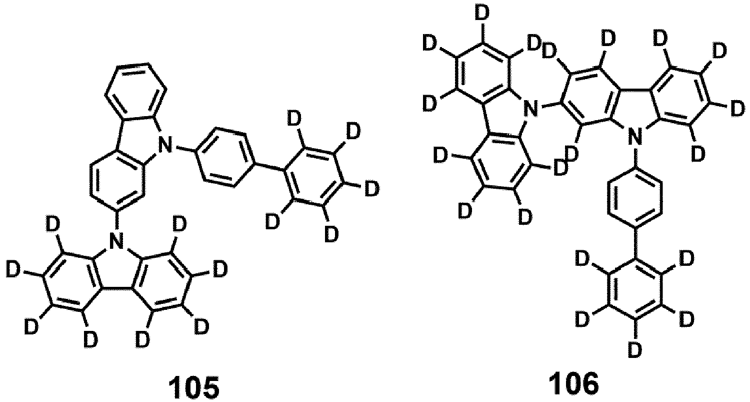

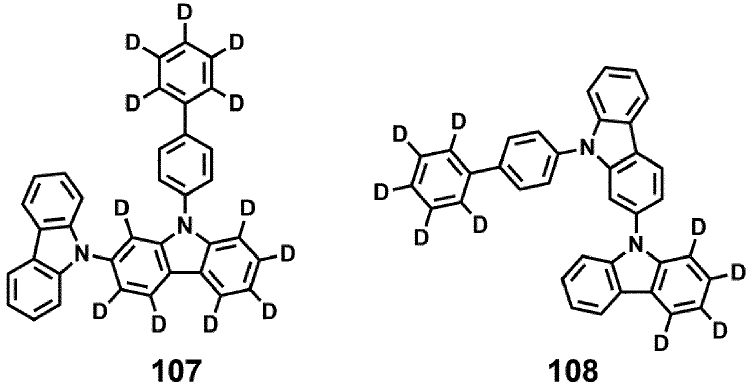

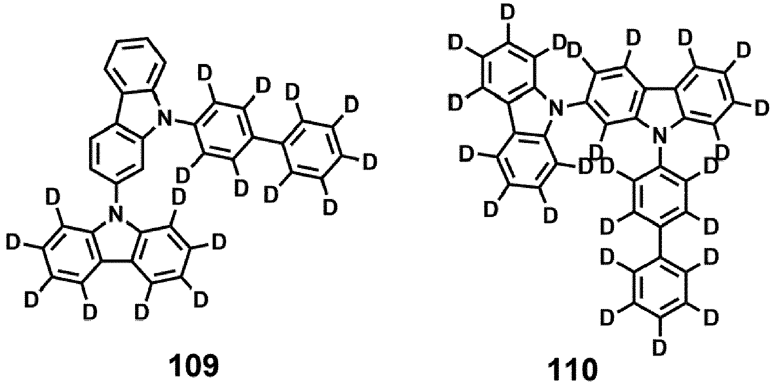

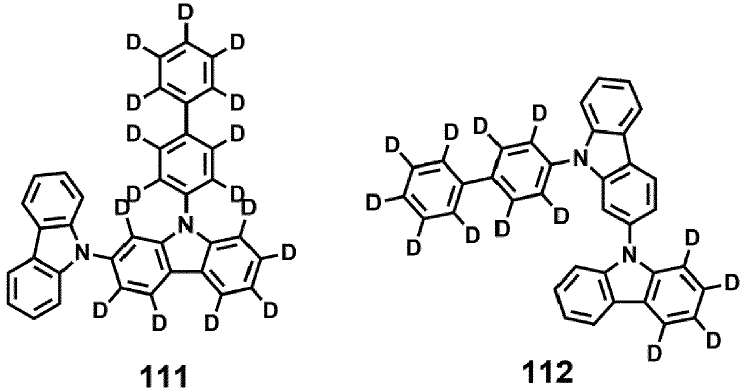

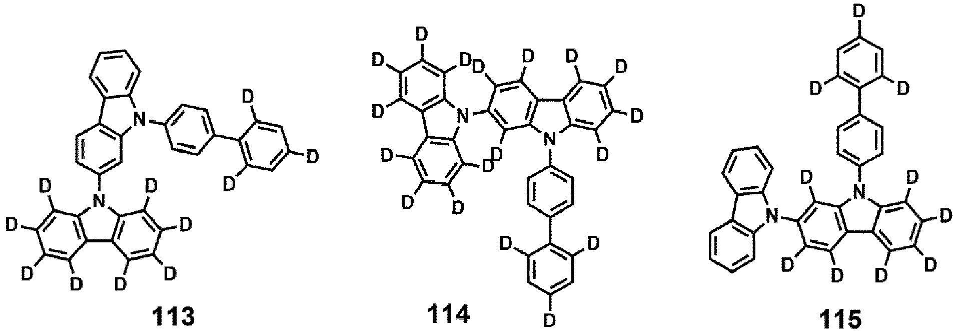

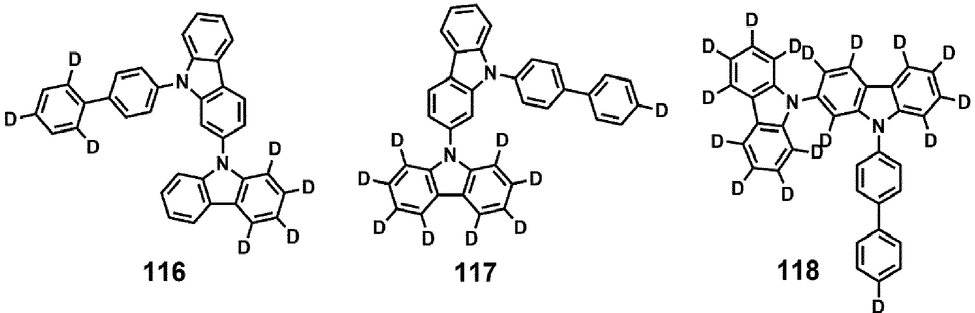

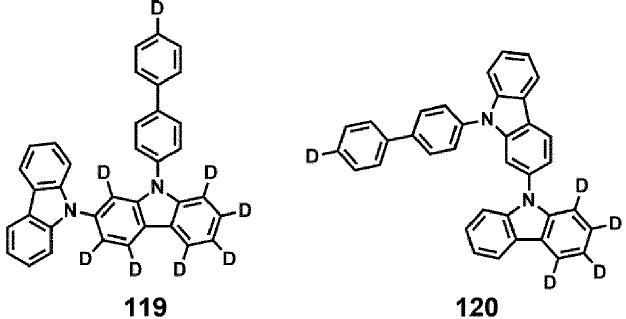

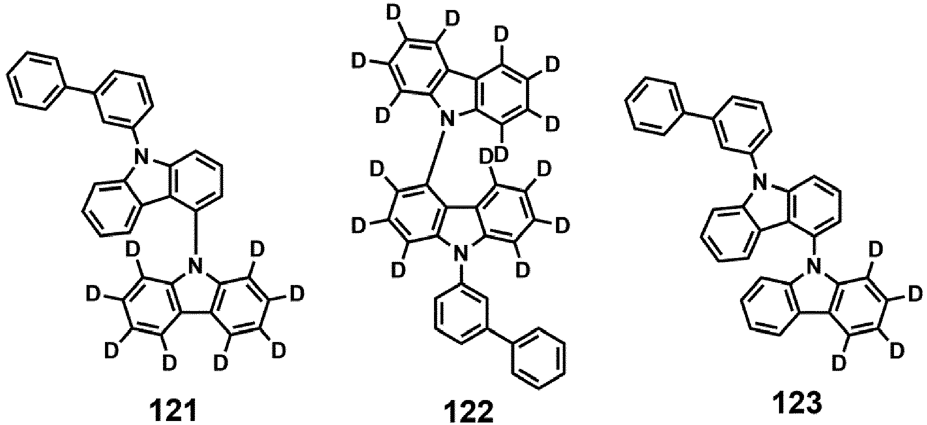

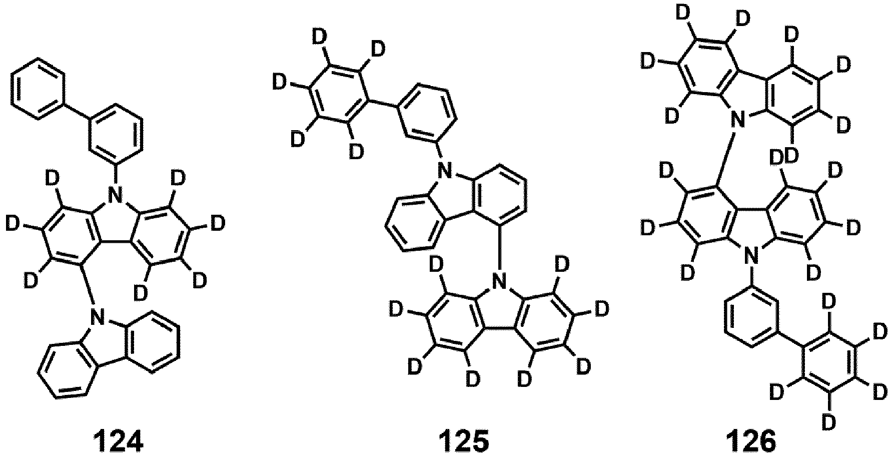

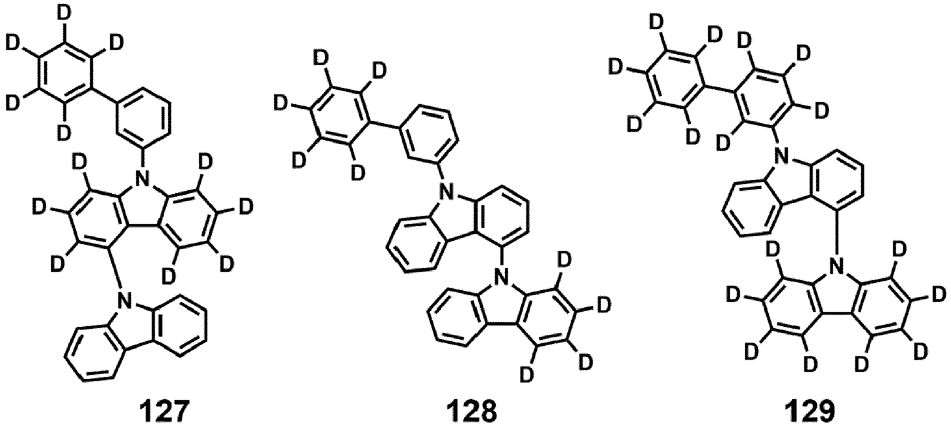

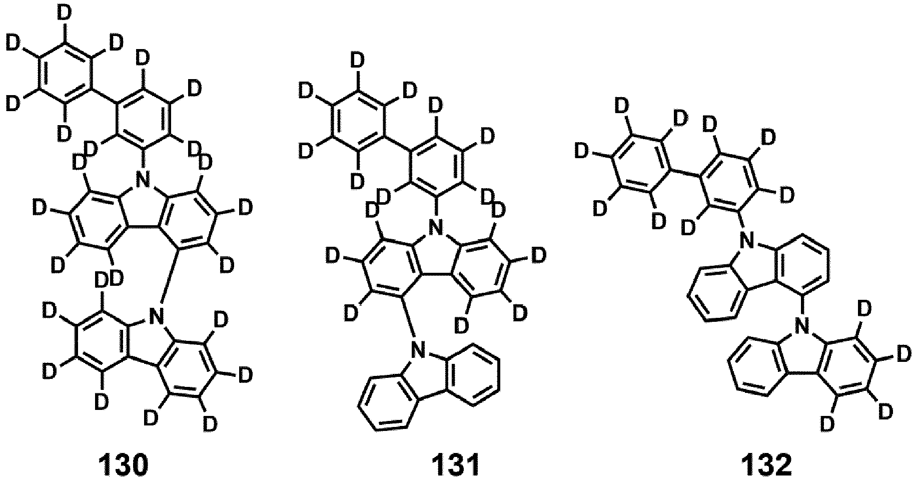

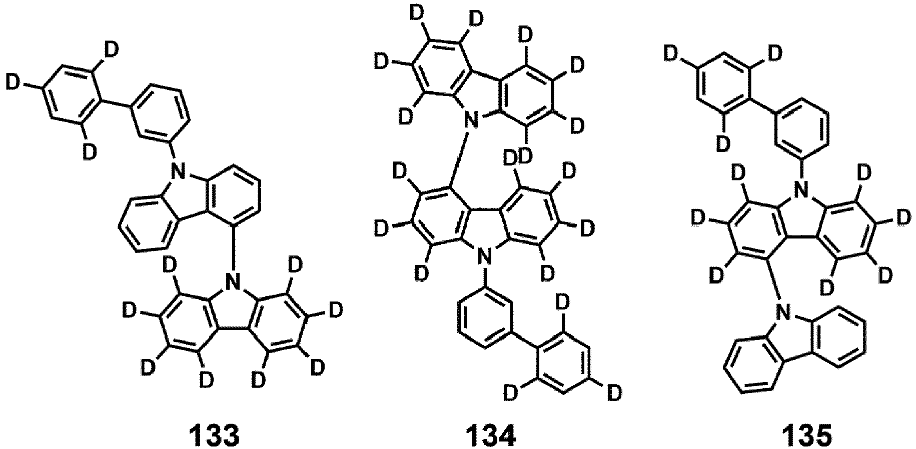

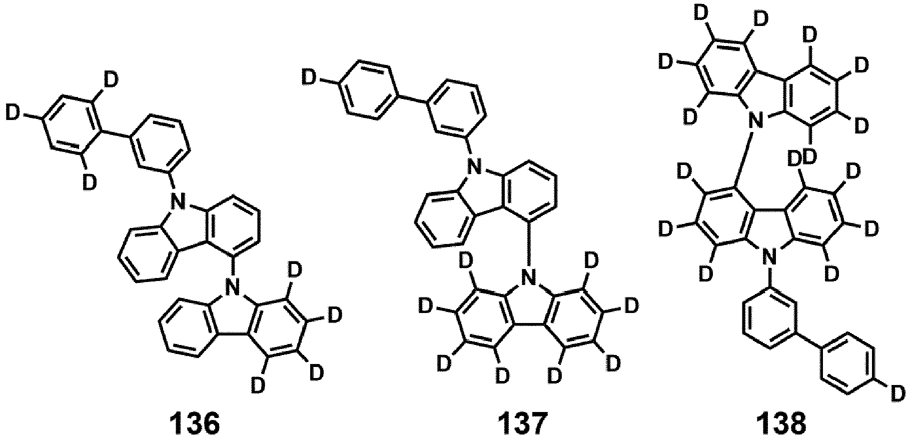

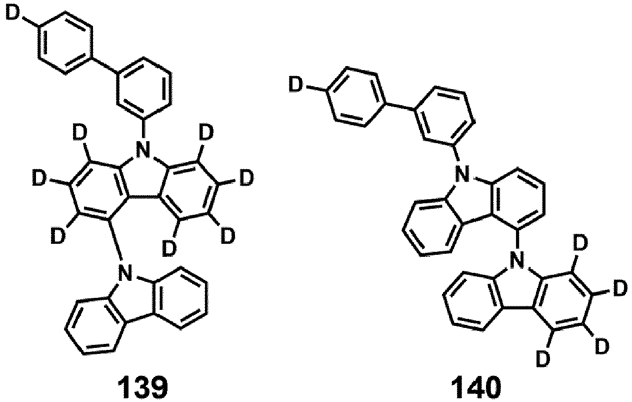

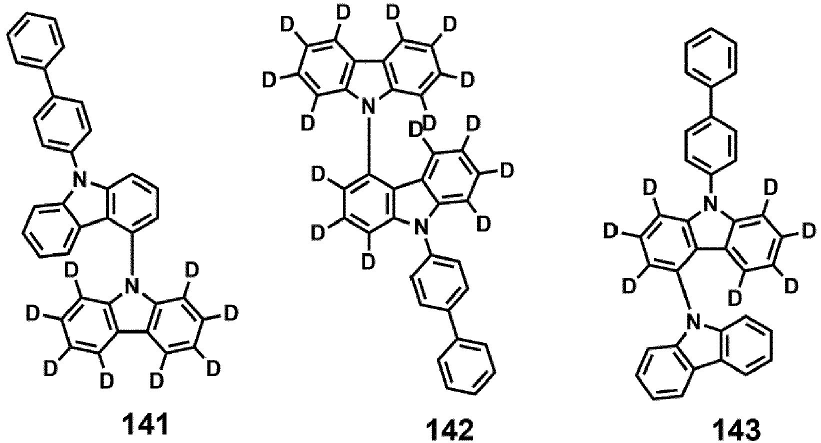

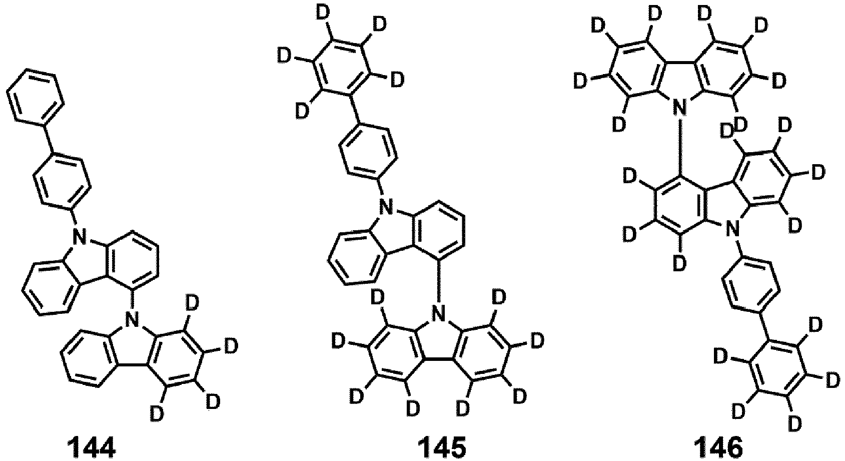

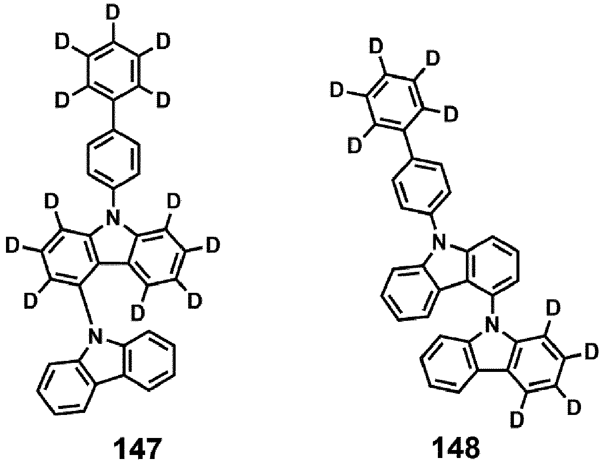

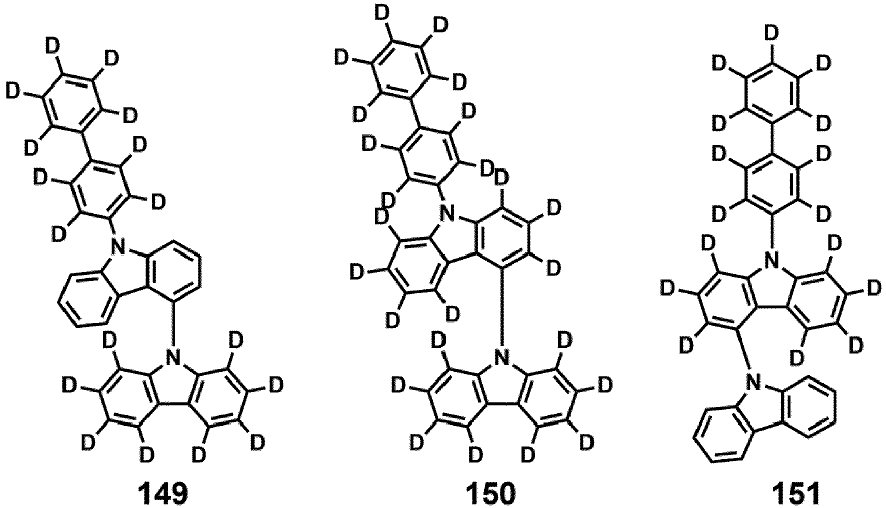

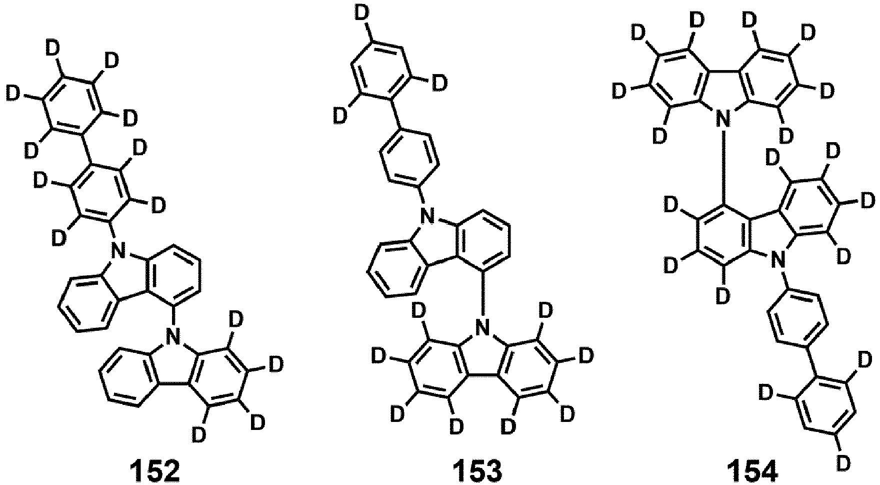

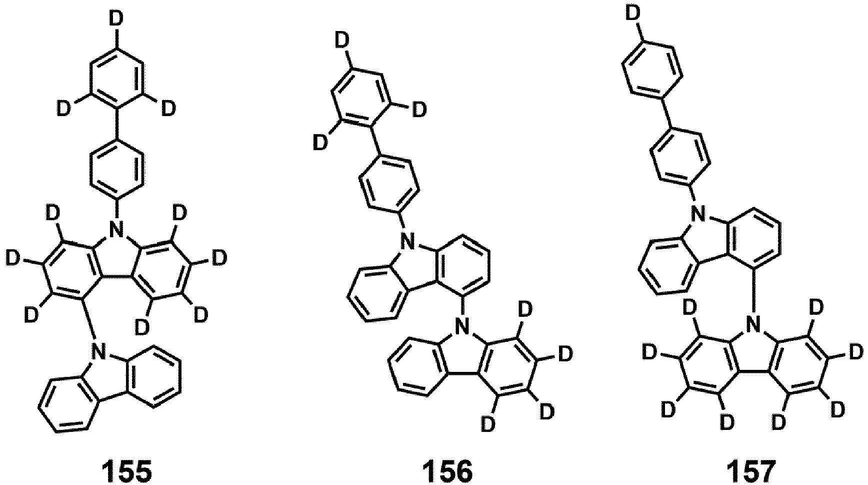

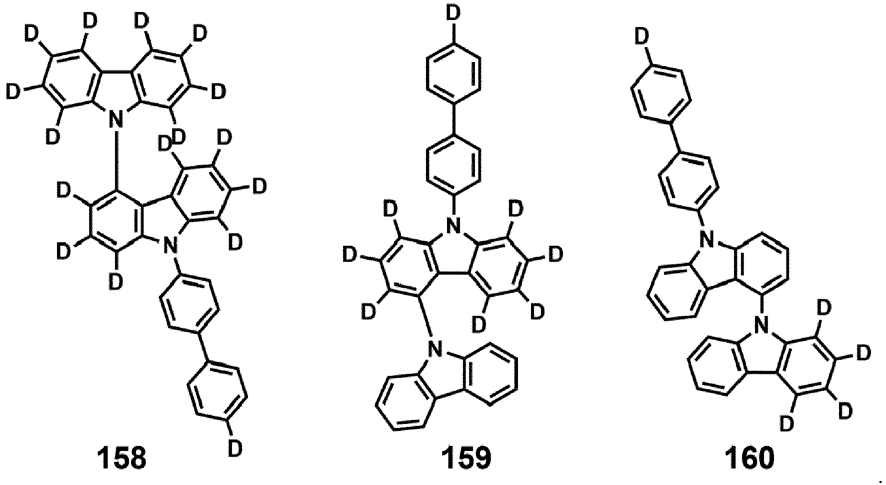

In the heterocyclic compound represented by Formula 1, a first benzene group, a second benzene group, a first carbazole group, and a second carbazole group are sequentially bonded through a single bond, and moreover, a benzene group and a carbazole group may be further included, wherein "N" in the first carbazole group is bonded to the benzene group, and "N" in the second carbazole group is bonded to the first carbazole group (see Formula 1'). Accordingly, the heterocyclic compound represented by Formula 1 may have a relatively high T₁ energy level.

In addition, the heterocyclic compound represented by Formula 1 may include at least one deuterium. A distance of the C-D bond is shorter than a distance of the C-H bond, the bond energy is high, and the frequency is large so that the range of structural fluctuation is small, and thus the heterocyclic compound may have structural stability. In addition, due to the kinetic isotope effect, the heterocyclic compound may have hole stability in structures related to hole movement.

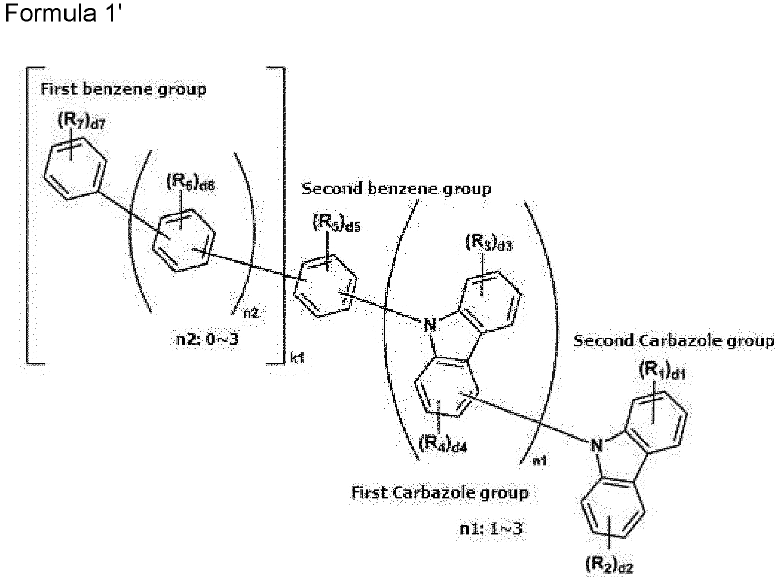

In an embodiment, the heterocyclic compound represented by Formula 1 may have a triplet energy level of greater than or equal to 2.8 eV or in a range of about 2.8 eV to about 3.5 eV (e.g., see Table 1).

In one or more embodiments, the heterocyclic compound represented by Formula 1 may have an absolute value of a highest occupied molecular orbital (HOMO) energy level of less than or equal to 5.2 eV or in a range of about 4.8 eV to about 5.2 eV or about 5.0 eV to about 5.2 eV (e.g., see Table 1).

The triplet energy level and the HOMO energy level may be evaluated based on the density functional theory (DFT).

For example, regarding Compound 6 belonging to the heterocyclic compound represented by Formula 1, the HOMO energy level, lowest unoccupied molecular orbital (LUMO) energy level, singlet (S₁) energy level, triplet (T₁) energy level were evaluated by using the DFT that is structurally optimized at the level of B3LYP/6-31G(d,p) (e.g., a DFT method of the Gaussian program), and results are shown in Table 1:

**Table 1**

| Compound No. | HOMO (eV) | LUMO (eV) | S₁ (eV) | T₁ (eV) |
|---|---|---|---|---|
| 6 | -5.125 | -1.081 | 3.794 | 3.292 |
| 26 | -5.131 | -1.130 | 3.603 | 3.124 |

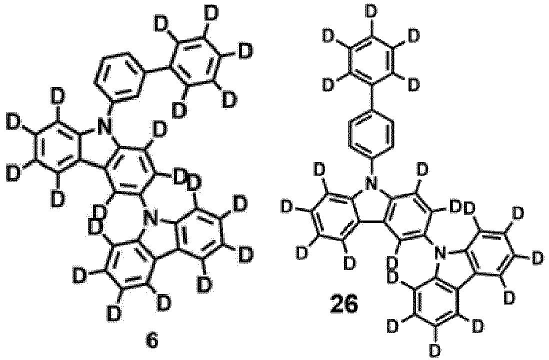

The method of synthesizing the heterocyclic compound represented by Formula 1 may be recognized by those skilled in the art with reference to Synthesis Example to be described later.

According to another aspect, an organic light-emitting device includes: a first electrode; a second electrode; and an organic layer arranged between the first electrode and the second electrode and including an emission layer, wherein the organic layer includes at least one heterocyclic compound represented by Formula 1.

When the organic light-emitting device includes such an organic layer including the aforementioned heterocyclic compound represented by Formula 1, long lifespan characteristics or the like may be resulted.

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

For example, in the organic light-emitting device, the first electrode may be an anode, the second electrode may be a cathode, and the organic layer may further include a hole transport region between the first electrode and the emission layer and an electron transport region between the emission layer and the second electrode, wherein the hole transport region may include a hole injection layer, a hole transport layer, an electron blocking layer, an auxiliary layer, or a combination thereof, and the electron transport region may include a buffer layer, a hole blocking layer, an electron transport layer, an electron injection layer, or a combination thereof.

The heterocyclic compound represented by Formula 1 may be used between a pair of electrodes of an organic light-emitting device. In an embodiment, the heterocyclic compound represented by Formula 1 may be included in the emission layer. In this regard, the heterocyclic compound may act as a host, and the emission layer may further include a dopant (that is, the amount of the heterocyclic compound represented by Formula 1 is greater than the amount of the dopant). The emission layer may emit, for example, blue light.

In an embodiment, the heterocyclic compound may be included in the hole transport region. For example, the heterocyclic compound may be included in the auxiliary layer of the hole transport region.

In an embodiment, the heterocyclic compound may be included in the electron transport region. For example, the heterocyclic compound may be included in the buffer layer of the electron transport region.

In an embodiment, the emission layer may include a host and a dopant, and the heterocyclic compound may be included in the host. In an embodiment, the amount (weight) of the host may be greater than the amount (weight) of the dopant.

In one or more embodiments, the emission layer may emit blue light, but embodiments are not limited thereto.

The dopant may be a fluorescent dopant, a phosphorescent dopant, or any combination thereof. In an embodiment, the dopant may be a phosphorescent dopant.

In an embodiment, the emission layer may include a host, a fluorescent dopant, and a phosphorescent dopant, and the host may include the heterocyclic compound. In this regard, the phosphorescent dopant may be a sensitizer compound that is used together with a fluorescent dopant to transfer excitons to the fluorescent dopant.

The expression that an "(organic layer) includes at least one heterocyclic compound" as used herein may be construed as meaning that the "(organic layer) may include one heterocyclic compound of Formula 1 or two or more different heterocyclic compounds of Formula 1".

In an embodiment, the organic layer may include only Compound 1 as the heterocyclic compound. In this embodiment, Compound 1 may be included in the emission layer of the organic light-emitting device. In one or more embodiments, the organic layer may include Compounds 1 and 2 as the heterocyclic compounds. In this embodiment, Compound 1 and Compound 2 may exist in an identical layer (for example, Compound 1 and Compound 2 all may exist in an emission layer).

The term "organic layer" as used herein refers to a single layer and/or a plurality of layers arranged between the first electrode and the second electrode of the organic light-emitting device. The "organic layer" may include, in addition to an organic compound, an organometallic complex including metal.

### Description of FIGURE

FIGURE is a schematic cross-sectional view of an organic light-emitting device 10 according to an embodiment. Hereinafter, the structure and manufacturing method of the organic light-emitting device 10 according to an embodiment of the present disclosure will be described in connection with the FIGURE.

In the FIGURE, an organic light-emitting device 10 includes a first electrode 11, a second electrode 19 facing the first electrode 11, and an organic layer 10A between the first electrode 11 and the second electrode 19.

In the FIGURE, the organic layer 10A includes an emission layer 15, a hole transport region 12 is between the first electrode 11 and an emission layer 15, and an electron transport region 17 is between the emission layer 15 and the second electrode 19.

A substrate may be additionally disposed under the first electrode 11 or on the second electrode 19. The substrate may be a conventional substrate used in organic light-emitting devices, e.g., a glass substrate or a transparent plastic substrate, each having excellent mechanical strength, thermal stability, transparency, surface smoothness, ease of handling, and water repellency.

### [First electrode 11]

The first electrode 11 may be formed by, for example, depositing or sputtering, onto the substrate, a material for forming the first electrode 11. The first electrode 11 may be an anode. The material for forming the first electrode 11 may include materials with a high work function to facilitate hole injection.

The first electrode 11 may be a reflective electrode, a semi-transmissive electrode, or a transmissive electrode. In an embodiment, when the first electrode 11 is a transmissive electrode, the material for forming the first electrode 11 may include indium tin oxide (ITO), indium zinc oxide (IZO), tin oxide (SnO₂), zinc oxide (ZnO), or any combination thereof. In one or more embodiments, when the first electrode 11 is a semi-transmissive electrode or a reflective electrode, the material for forming the first electrode 11 may include magnesium (Mg), silver (Ag), aluminum (Al), aluminum-lithium (Al-Li), calcium (Ca), magnesium-indium (Mg-In), magnesium-silver (Mg-Ag), or any combination thereof.

The first electrode 11 may have a single-layer structure or a multi-layer structure including a plurality of layers.

### [Emission layer 15]

A thickness of the emission layer 15 may be in a range of about 100 Å to about 1,000 Å, for example, about 200 Å to about 600 Å. When the thickness of the emission layer 15 is within these ranges, excellent luminescence characteristics may be obtained without a substantial increase in driving voltage.

### [Host in emission layer 15]

A host in the emission layer 15 may include the heterocyclic compound represented by Formula 1.

In addition to the heterocyclic compound represented by Formula 1 (for example, refer to the first host in Examples below), an arbitrary host (for example, refer to the second host in the Examples) may be further included. Hereinafter, a host that may be included in the emission layer 15 in addition to the heterocyclic compound represented by Formula 1 will be described below.

The host may not include a transition metal.

The host may be one kind of compound, or a mixture of two or more different types of compounds.

In an embodiment, the host may include at least one of a bipolar host, an electron-transporting host, and a hole-transporting host. The bipolar host, the electron-transporting host, and the hole-transporting host may be different from each other.

The electron-transporting host may include at least one electron-transporting group.

The hole-transporting host may not include an electron-transporting group.

The term "electron-transporting group" as used herein may include a cyano group, a π electron-deficient nitrogen-containing C₁-C₆₀ cyclic group, a group represented by one of the following formulae, or any combination thereof: 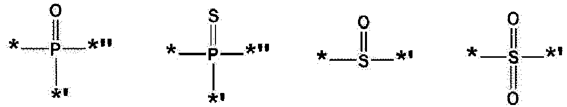 wherein, in the formulae above, *, *', and *" each indicate a binding site to a neighboring atom.

In an embodiment, the electron-transporting host may include at least one of a cyano group and a π-electron deficient nitrogen-containing C₁-C₆₀ cyclic group.

In one or more embodiments, the electron-transporting host may include at least one cyano group.

In one or more embodiments, the electron-transporting host may include at least one cyano group and a π electron deficient nitrogen-containing C₁-C₆₀ cyclic group.

In one or more embodiments, the host may include a bipolar host.

In one or more embodiments, the host may include an electron-transporting host.

In one or more embodiments, the host may include a hole-transporting host.

In one or more embodiments, the hole-transporting host may not be 1,3-bis(9-carbazolyl)benzene (mCP), tris(4-carbazoyl-9-ylphenyl)amine (TCTA), 4,4'-bis(N-carbazolyl)-1,1'-biphenyl (CBP), 3,3-bis(carbazol-9-yl)biphenyl (mCBP), N,N'-di(1-naphthyl)-N,N'-diphenyl-(1,1'-biphenyl)-4,4'-diamine (NPB), 4,4',4"-tris[phenyl(m-tolyl)amino]triphenylamine (m-MTDATA), and N,N'-bis(3-methylphenyl)-N,N'-diphenylbenzidine (TPD).

In one or more embodiments,
the host may include an electron-transporting host and a hole-transporting host,
the electron-transporting host may include at least one π electron-rich C₃-C₆₀ cyclic group and at least one electron-transporting group,
the hole-transporting host may include at least one π electron-rich C₃-C₆₀ cyclic group, and may not include an electron-transporting group, and
the electron-transporting group may include a cyano group, a π electron deficient nitrogen-containing C₁-C₆₀ cyclic group, or any combination thereof.

In one or more embodiments, the electron-transporting host may include i) at least one of a cyano group, a pyrimidine group, a pyrazine group, and a triazine group, and ii) at least one of a triphenylene group and a carbazole group.

In one or more embodiments, the hole-transporting host may include at least one carbazole group.

In one or more embodiments, the electron-transporting host may include a compound represented by Formula E-1, and
the hole-transporting host may include a compound represented by Formula H-1:

   Formula E-1 [Ar₃₀₁]_{xb11}-[(L₃₀₁)_{xb1}-R₃₀₁]_{xb21}
wherein, in Formula E-1,
Ar₃₀₁ may be a C₆-C₆₀ carbocyclic group unsubstituted or substituted with at least one R₃₀₁ₐ or a C₁-C₆₀ heterocyclic group unsubstituted or substituted with at least one R₃₀₁ₐ,
xb11 may be 1, 2, or 3,
L₃₀₁ may each independently be a single bond, a group represented by one of the following formulae, a C₆-C₆₀ carbocyclic group unsubstituted or substituted with at least one R₃₀₁ₐ, or a C₁-C₆₀ heterocyclic group unsubstituted or substituted with at least one R₃₀₁ₐ, and *, *', and *" in the following formulae each indicate a binding site to a neighboring atom, 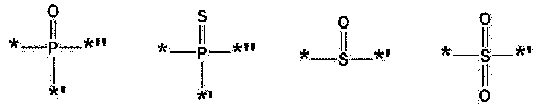
xb1 may be an integer from 1 to 5,
R₃₀₁ₐ and R₃₀₁ may each independently be hydrogen, deuterium, -F, -Cl, -Br, - I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), - S(=O)₂(Q₃₀₁), -S(=O)(Q₃₀₁), -P(=O)(Q₃₀₁)(Q₃₀₂), or -P(=S)(Q₃₀₁)(Q₃₀₂),
xb21 may be an integer from 1 to 5,
Q₃₀₁ to Q₃₀₃ may each independently be a C₁-C₁₀ alkyl group, a C₁-C₁₀ alkoxy group, a phenyl group, a biphenyl group, a terphenyl group, or a naphthyl group, and
at least one of Conditions 1" to 3" may be satisfied:
   Condition 1"
      At least one of Ar₃₀₁, L₃₀₁, and R₃₀₁ in Formula E-1 each independently includes a π electron-deficient nitrogen-containing C₁-C₆₀ cyclic group;
   Condition 2"
      L₃₀₁ in Formula E-1 is a group represented by one of the following Formulae; and 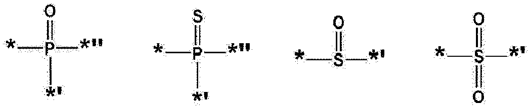
   Condition 3"
      R₃₀₁ in Formula E-1 is a cyano group, -S(=O)₂(Q₃₀₁), -S(=O)(Q₃₀₁), - P(=O)(Q₃₀₁)(Q₃₀₂), or -P(=S)(Q₃₀₁)(Q₃₀₂).

      Formula H-1 Ar₄₀₁-(L₄₀₁)_{xc1}-(Ar₄₀₂)_{xc11}

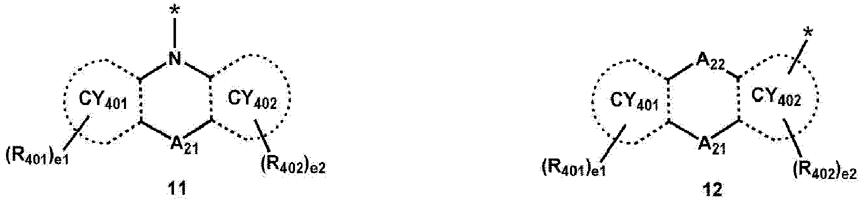

In Formulae H-1, 11, and 12,
L₄₀₁ may be:
a single bond; or
a π electron-rich C₃-C₆₀ cyclic group, unsubstituted or substituted with deuterium, a C₁-C₁₀ alkyl group, a C₁-C₁₀ alkoxy group, a phenyl group, a naphthyl group, a fluorenyl group, a carbazolyl group, a dibenzofuranyl group, a dibenzothiophenyl group, a triphenylenyl group, a biphenyl group, a terphenyl group, a tetraphenyl group, -Si(Q₄₀₁)(Q₄₀₂)(Q₄₀₃), or any combination thereof,
xc1 may be an integer from 1 to 10, wherein, when xc1 is 2 or more, two or more of L₄₀₁ may be identical to or different from each other,
Ar₄₀₁ may be a group represented by Formula 11 or 12,
Ar₄₀₂ may be:
   a group represented by Formula 11 or 12; or
   a π electron-rich C₃-C₆₀ cyclic group (for example, a phenyl group, a naphthyl group, a fluorenyl group, a carbazolyl group, a dibenzofuranyl group, a dibenzothiophenyl group, a biphenyl group, a terphenyl group, or a triphenylenyl group), each unsubstituted or substituted with deuterium, a C₁-C₁₀ alkyl group, a C₁-C₁₀ alkoxy group, a phenyl group, a naphthyl group, a fluorenyl group, a carbazolyl group, a dibenzofuranyl group, a dibenzothiophenyl group, a triphenylenyl group, a biphenyl group, a terphenyl group, a tetraphenyl group, or any combination thereof,
   xc11 may be an integer from 1 to 10, wherein, when xc11 is 2 or more, two or more of Ar₄₀₂ may be identical to or different from each other,
   CY₄₀₁ and CY₄₀₂ may each independently be a π electron-rich C₃-C₆₀ cyclic group (for example, a benzene group, a naphthalene group, a fluorene group, a carbazole group, a benzocarbazole group, an indolocarbazole group, a dibenzofuran group, a dibenzothiophene group, a dibenzosilole group, a benzonaphthofuran group, a benzonaphthothiophene group, or a benzonaphthosilole group),
   A₂₁ may be a single bond, O, S, N(R₄₁₁), C(R₄₁₁)(R₄₁₂), or Si(R₄₁₁)(R₄₁₂),
   A₂₂ may be a single bond, O, S, N(R₄₁₁), C(R₄₁₁)(R₄₁₂), or Si(R₄₁₁)(R₄₁₂),
   at least one of A₂₁ and A₂₂ in Formula 12 may not be a single bond,
   R₄₀₁, R₄₀₂, R₄₁₁, and R₄₁₂ may each independently be:
      hydrogen, deuterium, a C₁-C₂₀ alkyl group, or a C₁-C₂₀ alkoxy group;
      a C₁-C₂₀ alkyl group or a C₁-C₂₀ alkoxy group, each substituted with deuterium, a phenyl group, a naphthyl group, a fluorenyl group, a carbazolyl group, a dibenzofuranyl group, a dibenzothiophenyl group, or any combination thereof;
      π electron-rich C₃-C₆₀ cyclic group, unsubstituted or substituted with deuterium, a C₁-C₂₀ alkyl group, a C₁-C₂₀ alkoxy group, a phenyl group, a naphthyl group, a fluorenyl group, a carbazolyl group, a dibenzofuranyl group, a dibenzothiophenyl group, a biphenyl group, or any combination thereof; or
      -Si(Q404)(Q405)(Q406),
      e1 and e2 may each independently be an integer from 0 to 10,
      Q₄₀₁ to Q₄₀₆ may each independently be hydrogen, deuterium, a C₁-C₂₀ alkyl group, a phenyl group, a naphthyl group, a fluorenyl group, a carbazolyl group, a dibenzofuranyl group, a dibenzothiophenyl group, a biphenyl group, a terphenyl group, or a triphenylenyl group, and
      * indicates a binding site to a neighboring atom.

In an embodiment, Ar₃₀₁ and L₃₀₁ in Formula E-1 may each independently be a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an iso-benzothiazole group, a benzoxazole group, a benzoisoxazole group, a triazole group, a tetrazole group, an oxadiazole group, a triazine group, a thiadiazole group, an imidazopyridine group, an imidazopyrimidine group, or an azacarbazole group, each unsubstituted or substituted with deuterium, - F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a biphenyl group, a terphenyl group, a naphthyl group, a cyano group-containing phenyl group, a cyano group-containing biphenyl group, a cyano group-containing terphenyl group, a cyano group-containing naphthyl group, a pyridinyl group, a phenylpyridinyl group, a diphenylpyridinyl group, a biphenylpyridinyl group, a di(biphenyl)pyridinyl group, a pyrazinyl group, a phenylpyrazinyl group, a diphenylpyrazinyl group, a biphenylpyrazinyl group, a di(biphenyl)pyrazinyl group, a pyridazinyl group, a phenylpyridazinyl group, a diphenylpyridazinyl group, a biphenylpyridazinyl group, a di(biphenyl)pyridazinyl group, a pyrimidinyl group, phenylpyrimidinyl group, a diphenylpyrimidinyl group, a biphenylpyrimidinyl group, a di(biphenyl)pyrimidinyl group, a triazinyl group, a phenyltriazinyl group, a diphenyltriazinyl group, a biphenyltriazinyl group, a di(biphenyl)triazinyl group, -Si(Q₃₁)(Q₃₂)(Q₃₃), -N(Q₃₁)(Q₃₂), -B(Q₃₁)(Q₃₂), - C(=O)(Q₃₁), -S(=O)₂(Q₃₁), -P(=O)(Q₃₁)(Q₃₂), or any combination thereof,
at least one of L₃₀₁(s) in the number of xb1 may each independently be an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a phthalazine group, a naphthyridine group, a quinoxaline group, a quinazoline group, a cinnoline group, a phenantridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, or an azacarbazole group, each unsubstituted or substituted with deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a biphenyl group, a terphenyl group, a naphthyl group, a cyano-containing phenyl group, a cyano-containing a biphenyl group, a cyano-containing terphenyl group, a cyano-containing a naphthyl group, a pyridinyl group, a phenylpyridinyl group, a diphenylpyridinyl group, a biphenylpyridinyl group, a di(biphenyl)pyridinyl group, a pyrazinyl group, a phenylpyrazinyl group, a diphenylpyrazinyl group, a biphenylpyrazinyl group, a di(biphenyl)pyrazinyl group, a pyridazinyl group, a phenylpyridazinyl group, a diphenylpyridazinyl group, a biphenylpyridazinyl group, a di(biphenyl)pyridazinyl group, a pyrimidinyl group, a phenylpyrimidinyl group, a diphenylpyrimidinyl group, a biphenylpyrimidinyl group, a di(biphenyl)pyrimidinyl group, a triazinyl group, a phenyltriazinyl group, a diphenyltriazinyl group, a biphenyltriazinyl group, a di(biphenyl)triazinyl group, - Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), - P(=O)(Q₃₁)(Q₃₂), or any combination thereof,
R₃₀₁ may be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a biphenyl group, a terphenyl group, a tetraphenyl group, a naphthyl group, a cyano group-containing phenyl group, a cyano group-containing biphenyl group, a cyano group-containing terphenyl group, a cyano group-containing tetraphenyl group, a cyano group-containing naphthyl group, a pyridinyl group, a phenylpyridinyl group, a diphenylpyridinyl group, a biphenylpyridinyl group, a di(biphenyl)pyridinyl group, a pyrazinyl group, a phenylpyrazinyl group, a diphenylpyrazinyl group, a biphenylpyrazinyl group, a di(biphenyl)pyrazinyl group, a pyridazinyl group, a phenylpyridazinyl group, a diphenylpyridazinyl group, a biphenylpyridazinyl group, a di(biphenyl)pyridazinyl group, a pyrimidinyl group, a phenylpyrimidinyl group, a diphenylpyrimidinyl group, a biphenylpyrimidinyl group, a di(biphenyl)pyrimidinyl group, a triazinyl group, a phenyltriazinyl group, a diphenyltriazinyl group, a biphenyltriazinyl group, a di(biphenyl)triazinyl group, - Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₃₁ to Q₃₃ may each independently be a C₁-C₁₀ alkyl group, a C₁-C₁₀ alkoxy group, a phenyl group, a biphenyl group, a terphenyl group, or a naphthyl group.

In one or more embodiments,
Ar₃₀₁ may be: a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, or a dibenzothiophene group, each unsubstituted or substituted with deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a biphenyl group, a terphenyl group, a naphthyl group, a cyano group-containing phenyl group, a cyano group-containing biphenyl group, a cyano group-containing terphenyl group, a cyano group-containing naphthyl group, a pyridinyl group, a phenylpyridinyl group, a diphenylpyridinyl group, a biphenylpyridinyl group, a di(biphenyl)pyridinyl group, a pyrazinyl group, a phenylpyrazinyl group, a diphenylpyrazinyl group, a biphenylpyrazinyl group, a di(biphenyl)pyrazinyl group, a pyridazinyl group, a phenylpyridazinyl group, a diphenylpyridazinyl group, a biphenylpyridazinyl group, a di(biphenyl)pyridazinyl group, a pyrimidinyl group, a phenylpyrimidinyl group, a diphenylpyrimidinyl group, a biphenylpyrimidinyl group, a di(biphenyl)pyrimidinyl group, a triazinyl group, a phenyltriazinyl group, a diphenyltriazinyl group, a biphenyltriazinyl group, a di(biphenyl)triazinyl group, - Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), - P(=O)(Q₃₁)(Q₃₂), or any combination thereof; or
a group represented by one of Formulae 5-1 to 5-3 and 6-1 to 6-33, and
L₃₀₁ may be a group represented by one Formulae 5-1 to 5-3 and Formula 6-1 to 6-33: 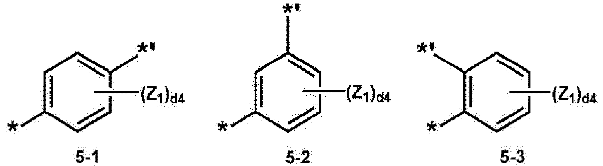 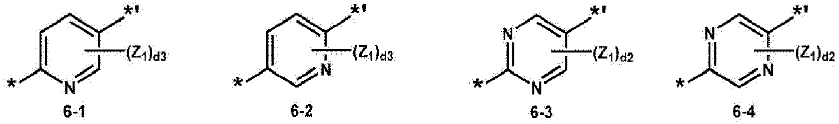 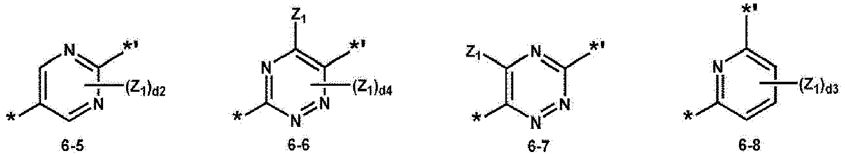 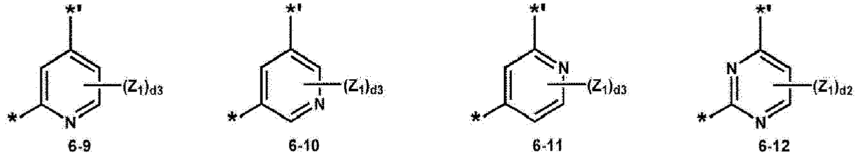 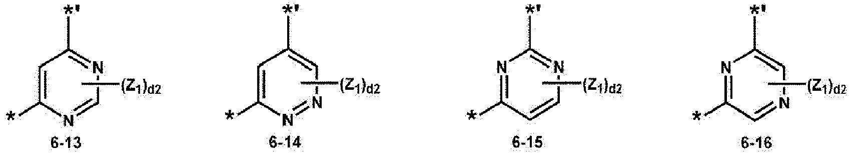 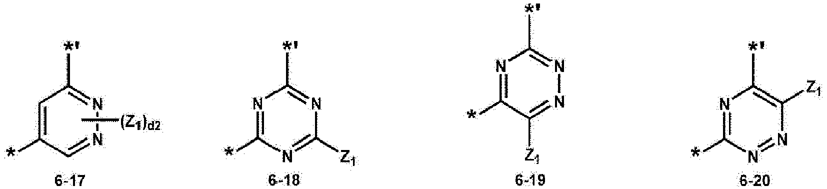 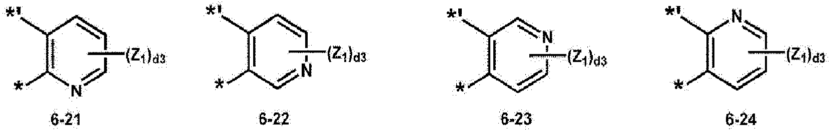 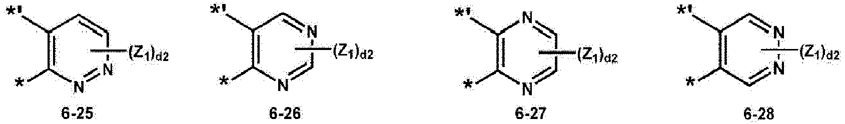 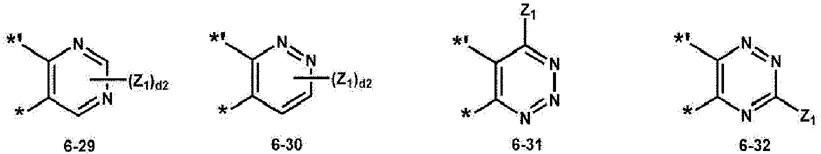 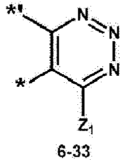
wherein, in Formulae 5-1 to 5-3 and 6-1 to 6-33,
Z₁ may be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a biphenyl group, a terphenyl group, a naphthyl group, a cyano group-containing phenyl group, a cyano group-containing biphenyl group, a cyano group-containing terphenyl group, a cyano group-containing naphthyl group, a pyridinyl group, a phenylpyridinyl group, a diphenylpyridinyl group, a biphenylpyridinyl group, a di(biphenyl)pyridinyl group, a pyrazinyl group, a phenylpyrazinyl group, a diphenylpyrazinyl group, a biphenylpyrazinyl group, a di(biphenyl)pyrazinyl group, a pyridazinyl group, a phenylpyridazinyl group, a diphenylpyridazinyl group, a biphenylpyridazinyl group, a di(biphenyl)pyridazinyl group, a pyrimidinyl group, a phenylpyrimidinyl group, a diphenylpyrimidinyl group, a biphenylpyrimidinyl group, a di(biphenyl)pyrimidinyl group, a triazinyl group, a phenyltriazinyl group, a diphenyltriazinyl group, a biphenyltriazinyl group, a di(biphenyl)triazinyl group, - Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂),
d4 may be 0, 1, 2, 3, or 4,
d3 may be 0, 1, 2, or 3,
d2 may be 0, 1, or 2, and
* and *' each indicate a binding site to a neighboring atom.
Q₃₁ to Q₃₃ are each the same as described herein.

In one or more embodiments, L₃₀₁ may be a group represented by one of Formulae 5-2, 5-3, and 6-8 to 6-33.

In one or more embodiments, R₃₀₁ may be a cyano group or a group represented by one of Formulae 7-1 to 7-18, and at least one of Ar₄₀₂(s) in the number of xc11 may be a group represented by one of Formulae 7-1 to 7-18: 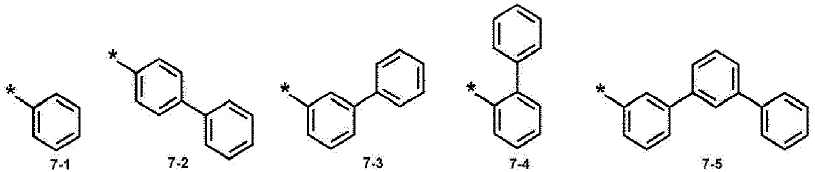 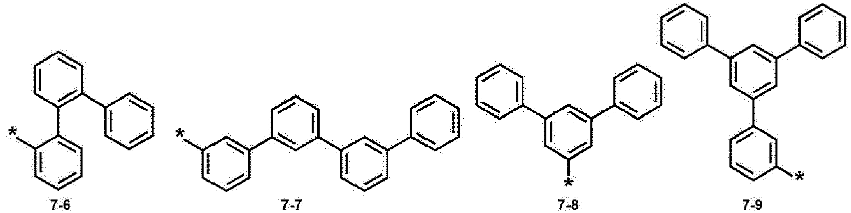 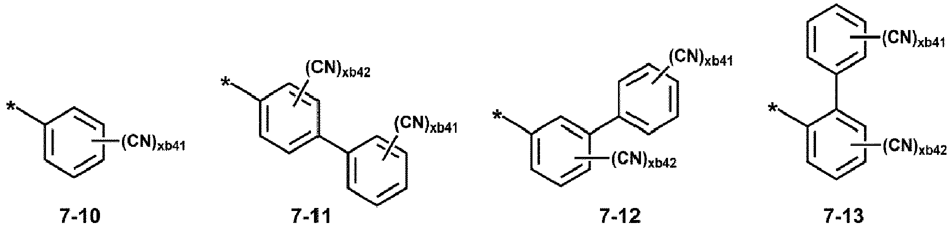 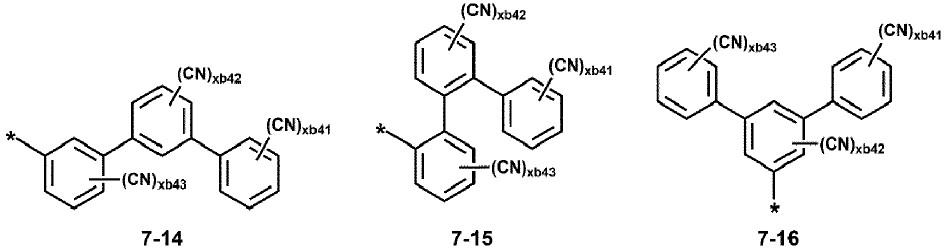 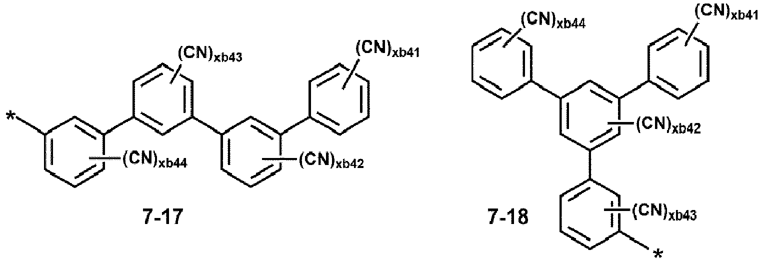
wherein, in Formulae 7-1 to 7-18,
xb41 to xb44 may each be 0, 1, or 2, wherein xb41 in Formula 7-10 may not be 0, xb41+xb42 in Formulae 7-11 to 7-13 may not be 0, xb41+xb42+xb43 in Formulae 7-14 to 7-16 may not be 0, xb41+xb42+xb43+xb44 in Formulae 7-17 and 7-18 may not be 0, and * indicates a binding site to a neighboring atom.

In Formula E-1, two or more of Ar₃₀₁ may be identical to or different from each other, and two or more of L₃₀₁ may be identical to or different from each other. In Formula H-1, two or more of L₄₀₁ may be identical to or different from each other, and two or more of Ar₄₀₂ may be identical to or different from each other.

In an embodiment, the electron-transporting host may be represented by Formula E-2:

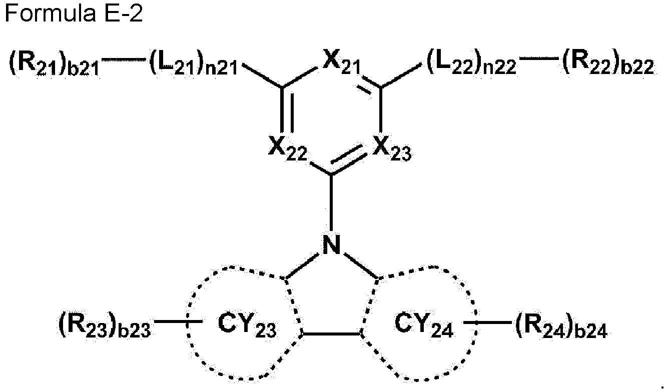

In an embodiment, in Formula E-2, CY₂₃ and CY₂₄ may each independently be a C₆-C₃₀ carbocyclic group or a C₁-C₃₀ heterocyclic group.

In an embodiment, CY₂₃ and CY₂₄ may each independently be a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group.

In an embodiment, CY₂₃ and CY₂₄ may each independently be a benzene group, a naphthalene group, or a pyridine group.

In an embodiment, in Formula E-2, X₂₁ may be N or C(R₂₁ₐ), X₂₂ may be N or C(R₂₂ₐ), and X₂₃ may be N or C(R₂₃ₐ).

In an embodiment, in Formula E-2, at least one of X₂₁ to X₂₃ may be N.

For example, one of X₂₁ to X₂₃ may be N, two of X₂₁ to X₂₃ may each be N, and X₂₁ to X₂₃ may each be N.

In an embodiment, in Formula E-2, L₂₁ and L₂₂ may each independently be a single bond, a substituted or unsubstituted C₃-C₆₀ carbocyclic group, or a substituted or unsubstituted C₁-C₆₀ heterocyclic group.

For example, L₂₁ and L₂₂ may each independently be: a single bond; or
a benzene group, a naphthalene group, an anthracene group, a phenanthrene group, a triphenylene group, a pyrene group, a chrysene group, a cyclopentadiene group, a 1,2,3,4-tetrahydronaphthalene group, a thiophene group, a furan group, an indole group, a benzoborole group, a benzophosphole group, an indene group, a benzosilole group, a benzogermole group, a benzothiophene group, a benzoselenophene group, a benzofuran group, a carbazole group, a dibenzoborole group, a dibenzophosphole group, a fluorene group, a dibenzosilole group, a dibenzogermole group, a dibenzothiophene group, a dibenzoselenophene group, a dibenzofuran group, a dibenzothiophene 5-oxide group, a 9H-a fluorene-9-one group, a dibenzothiophene 5,5-dioxide group, an azaindole group, an azabenzoborole group, an azabenzophosphole group, an azaindene group, an azabenzosilole group, an azabenzogermole group, an azabenzothiophene group, an azabenzoselenophene group, an azabenzofuran group, an azacarbazole group, an azadibenzoborole group, an azadibenzophosphole group, an azafluorene group, an azadibenzosilole group, an azadibenzogermole group, an azadibenzothiophene group, an azadibenzoselenophene group, an azadibenzofuran group, an azadibenzothiophene 5-oxide group, an aza-9H-fluoren-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrrole group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, a benzothiadiazole group, a 5,6,7,8-tetrahydroisoquinoline group, or a 5,6,7,8-tetrahydroquinoline group, each unsubstituted or substituted with at least one R₁₀ₐ. Here, R₁₀ₐ is the same as described in connection with R₂₃ in the present specification.

In an embodiment, in Formula E-2, L₂₁ and L₂₂ may each independently be: a single bond; or a group represented by one of Formulae L-1 to L-12: 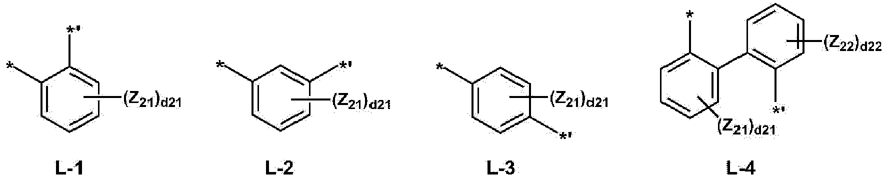 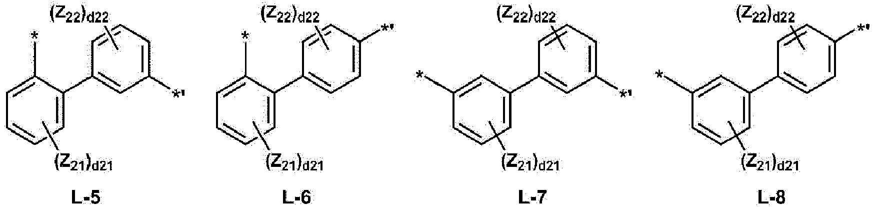 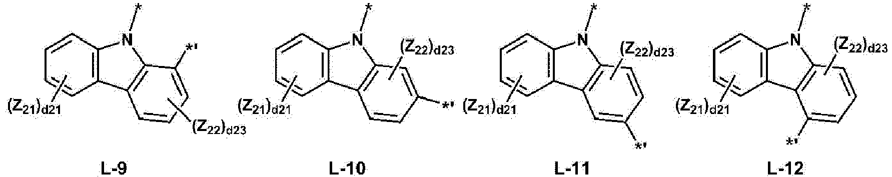
wherein, in Formulae L-1 to L-12,
Z₂₁ and Z₂₂ are each the same as described in connection with R₂₁,
d21 and d22 may each independently be an integer from 0 to 4,
d23 may be an integer from 0 to 3, and
* and *' each indicate a binding site to a neighboring atom.

In an embodiment, in Formula E-2, n21 and n22 indicate the number of L₂₁(s) and L₂₂(s), respectively, and may each independently be an integer from 0 to 5 (e.g., 0, 1, 2, or 3). When n21 is 2 or more, two or more of L₂₁ may be identical to or different from each other, and when n22 is 2 or more, two or more of L₂₂ may be identical to or different from each other.

In an embodiment, in Formula E-2, R₂₁ to R₂₄, R₂₁ₐ, R₂₂ₐ, and R₂₃ₐ may each independently be hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, a substituted or unsubstituted monovalent non-aromatic condensed heteropolycyclic group, -Si(Q₁)(Q₂)(Q₃), - Ge(Q₁)(Q₂)(Q₃), -N(Q₄)(Q₅), -B(Q₆)(Q₇), or -P(=O)(Q₈)(Q₉).

At least one substituent of the substituted C₆-C₃₀ carbocyclic group, the substituted C₁-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted C₁-C₆₀ heteroaryloxy group, the substituted C₁-C₆₀ heteroarylthio group, the substituted monovalent non-aromatic condensed polycyclic group, and the substituted monovalent non-aromatic condensed heteropolycyclic group may be:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -Si(Q₁₁)(Q₁₂)(Q₁₃), -Ge(Q₁₁)(Q₁₂)(Q₁₃), -N(Q₁₄)(Q₁₈), -B(Q₁₆)(Q₁₇), - P(=O)(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, or a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -Si(Q₂₁)(Q₂₂)(Q₂₃), -Ge(Q₂₁)(Q₂₂)(Q₂₃), -N(Q₂₄)(Q₂₅), -B(Q₂₆)(Q₂₇), - P(=O)(Q₂₈)(Q₂₉), or any combination thereof; or
-Si(Q₃₁)(Q₃₂)(Q₃₃), -Ge(Q₃₁)(Q₃₂)(Q₃₃), -N(Q₃₄)(Q₃₅), -B(Q₃₆)(Q₃₇), or - P(=O)(Q₃₈)(Q₃₉), and
Q1 to Q9, Q11 to Q19, Q21 to Q29, and Q31 to Q39 may each independently be: hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₂-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group; or a substituted or unsubstituted monovalent non-aromatic condensed heteropolycyclic group.

In Formula E-2, b21 to b24 indicate the number of R₂₁(s) to R₂₄(s), respectively, and may each independently be an integer from 0 to 10 (e.g., 0, 1, 2, or 3). When b21 is 2 or more, two or more of R₂₁ may be identical to or different from each other, when b22 is 2 or more, two or more of R₂₂ may be identical to or different from each other, when b23 is 2 or more, two or more of R₂₃ may be identical to or different from each other, and when b24 is 2 or more, two or more of R₂₄ may be identical to or different from each other.

For example, in Formula E-2, R₂₁ to R₂₄, R₂₁ₐ, R₂₂ₐ, and R₂₃ₐ may each independently be:
hydrogen, deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, or a C₁-C₂₀ alkoxy group;
a C₁-C₂₀ alkyl group or a C₁-C₂₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a phenyl group, a naphthyl group, a pyridinyl group, a pyrimidinyl group, or a combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purynyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, or an imidazopyrimidinyl group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a phenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purynyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, -Si(Q₃₁)(Q₃₂)(Q₃₃), or any combination thereof; or
-Si(Q₁)(Q₂)(Q₃), -Ge(Q₁)(Q₂)(Q₃), -N(Q₄)(Q₅), -B(Q₆)(Q₇), or -P(=O)(Q₈)(Q₉), and
Q₁ to Q₉ and Q₃₁ to Q₃₃ may each independently be:
   -CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂;
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group; or
   an n-propyl group, an isopropyl group, an n-butyl group, an isobutyl group, a sec-butyl group, a tert-butyl group, an n-pentyl group, an isopentyl group, a sec-pentyl group, a tert-pentyl group, a phenyl group, or a naphthyl group, each substituted with deuterium, a C₁-C₁₀ alkyl group, a phenyl group, or a combination thereof.

In an embodiment, R₂₁ to R₂₄, R₂₁ₐ, R₂₂ₐ, and R₂₃ₐ may each independently be:
hydrogen, deuterium, -F, -Cl, -Br, -I, -CD₃, -CD₂H, -CDH₂, -CF₃, -CF₂H, -CFH₂, a C₁-C₆₀ alkyl group, a C₂-C₆₀ alkenyl group, a C₂-C₆₀ alkynyl group, or a C₁-C₆₀ alkoxy group; or
a group represented by one of Formulae 9-1 to 9-61, 9-201 to 9-240, 10-1 to 10-129, and 10-201 to 10-355; or
   -N(Q₁)(Q₂), -Si(Q₃)(Q₄)(Q₅) or -Ge(Q₃)(Q₄)(Q₅), and
   Q₁ to Q₅ are each the same as described herein: 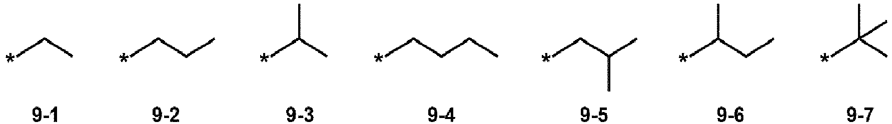 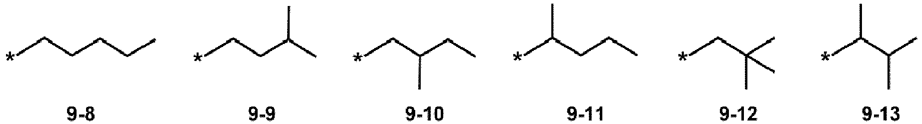 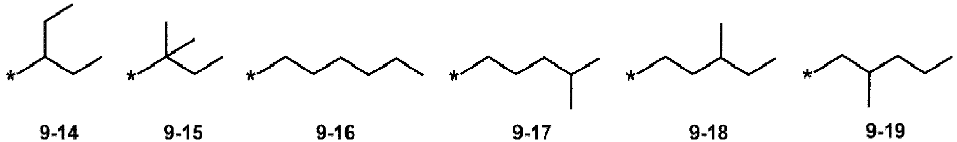 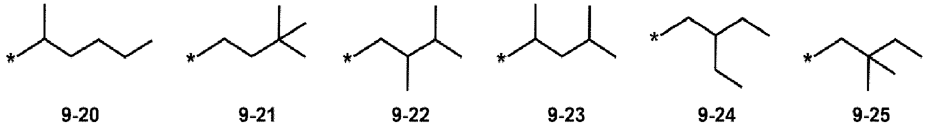 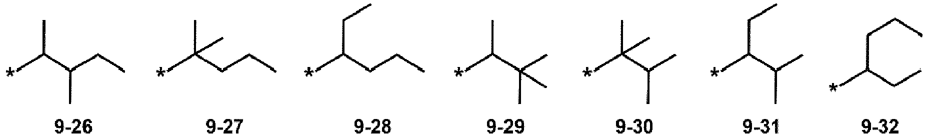 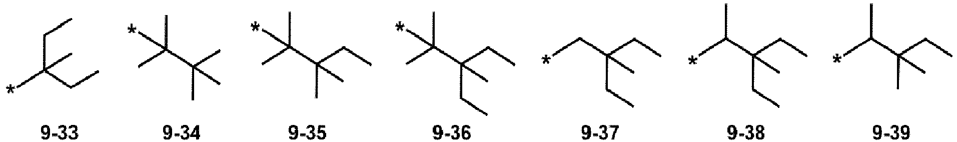 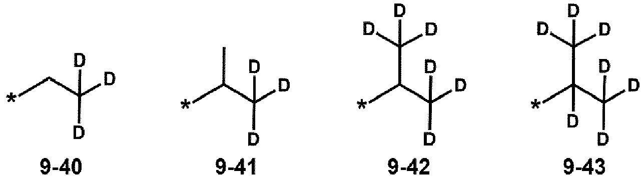 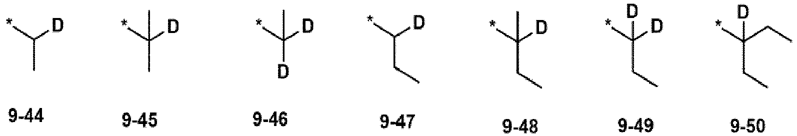 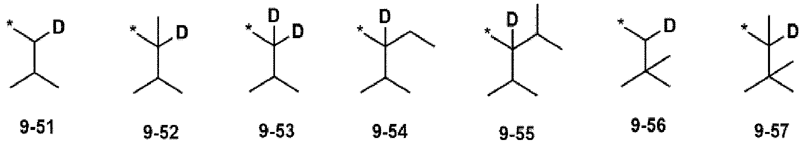 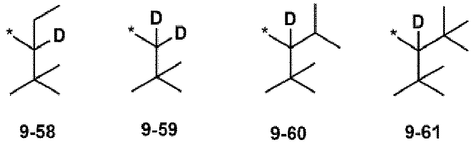 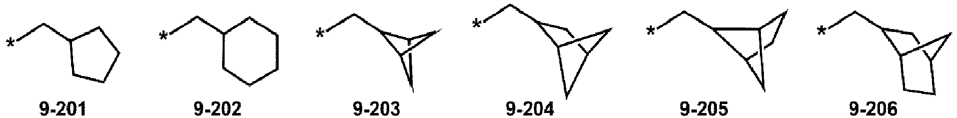 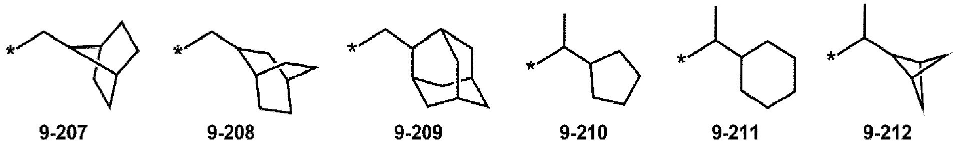 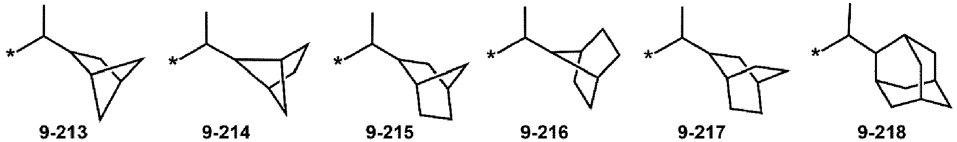 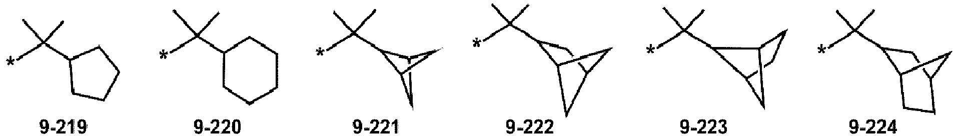 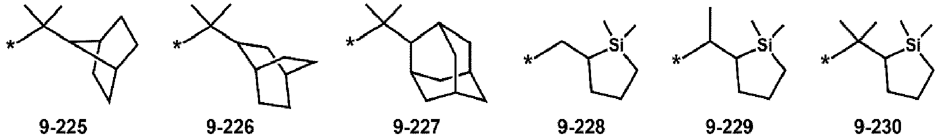 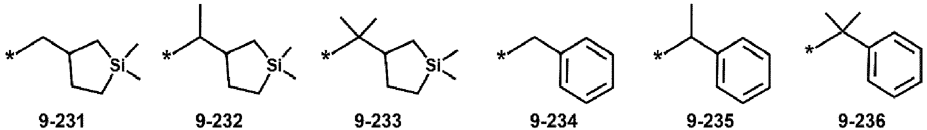 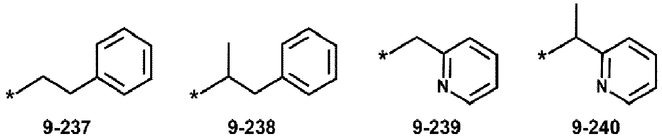 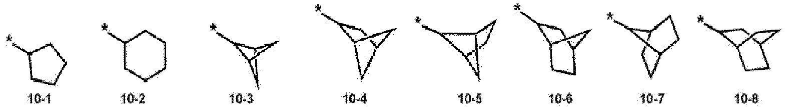 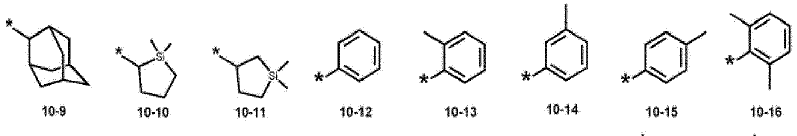 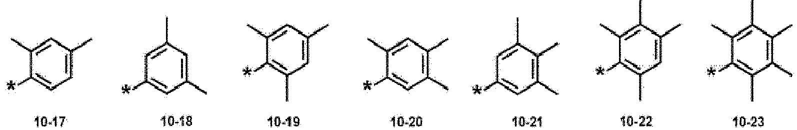 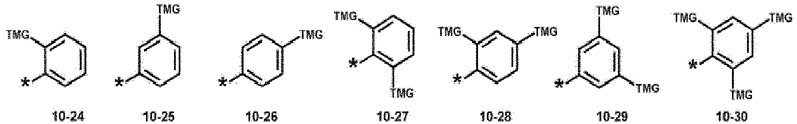 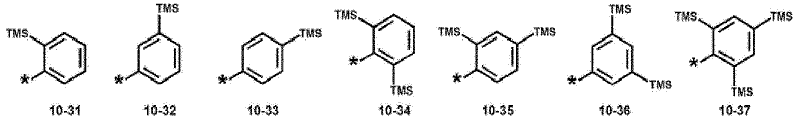 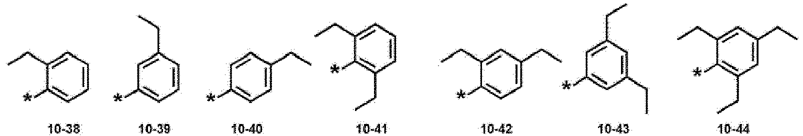 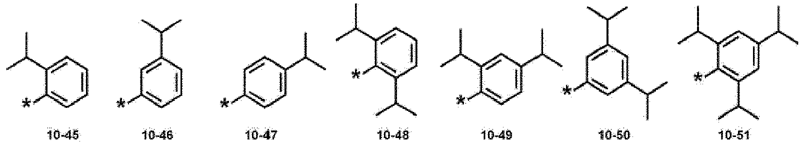 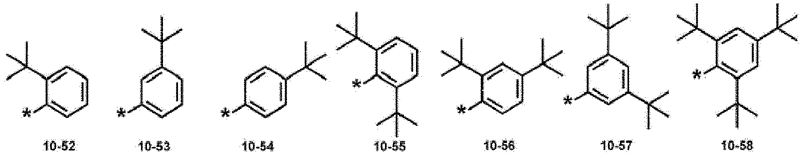 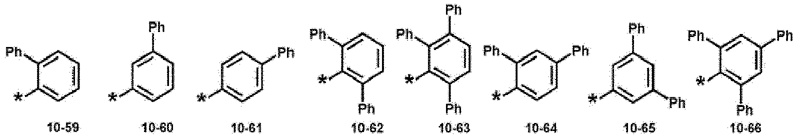 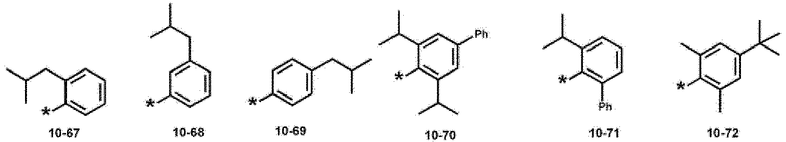 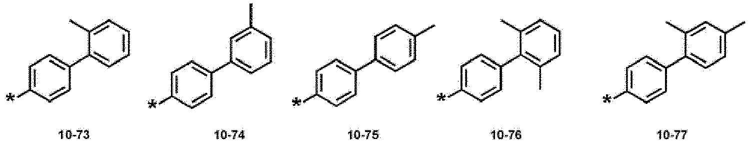 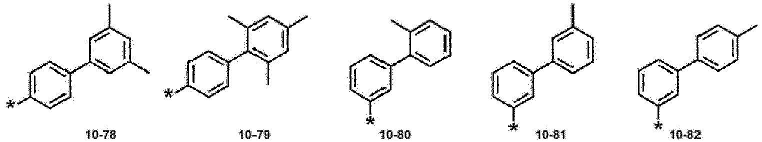 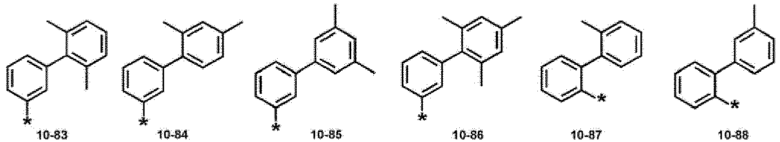 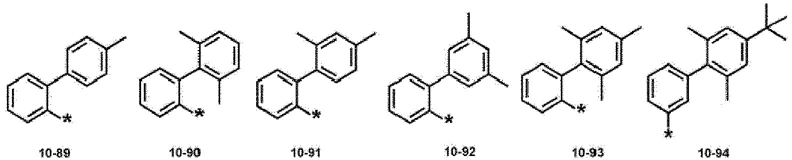 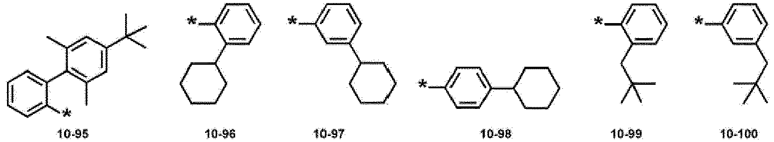 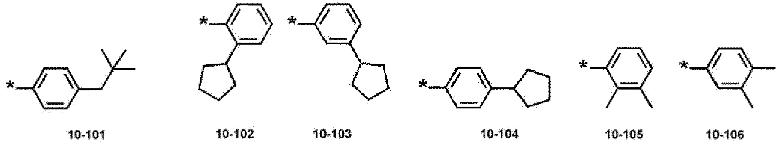 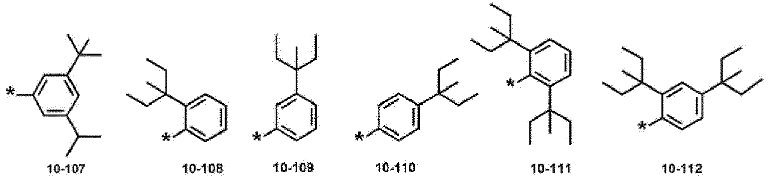 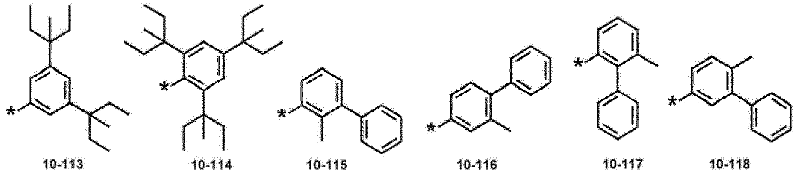 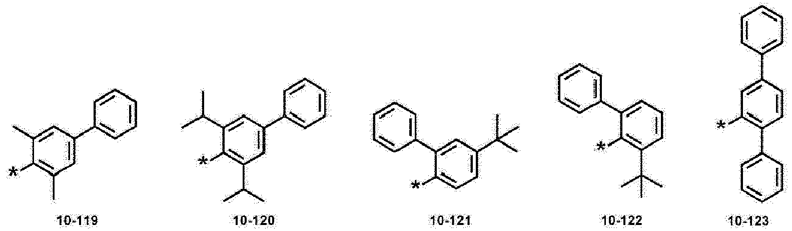 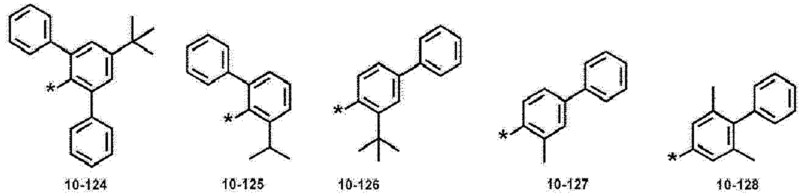 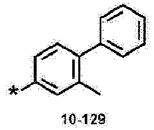 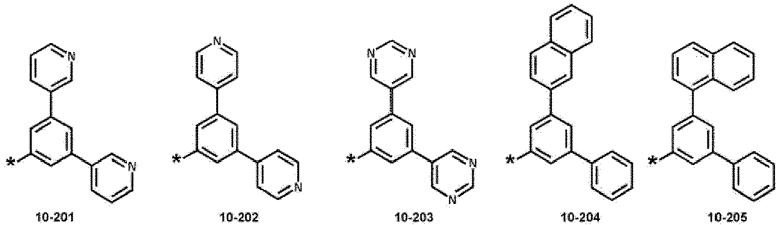 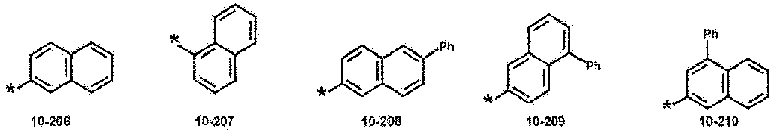 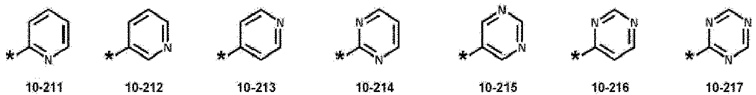 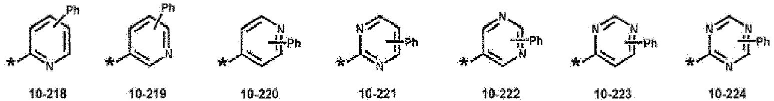 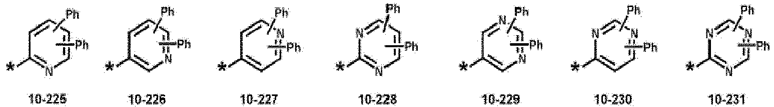 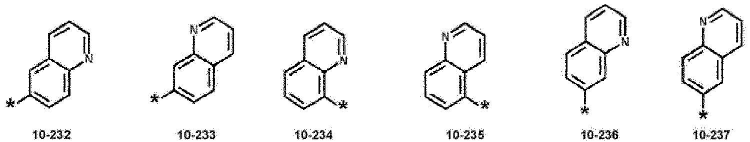 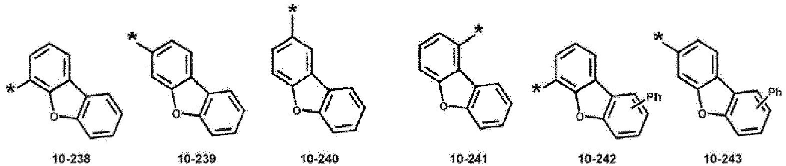 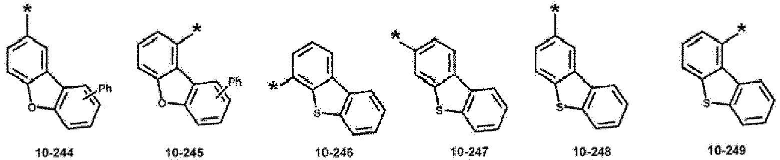 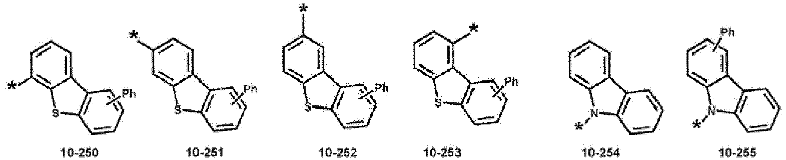 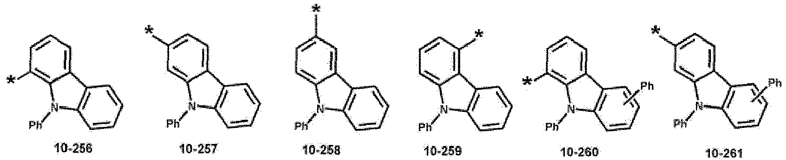 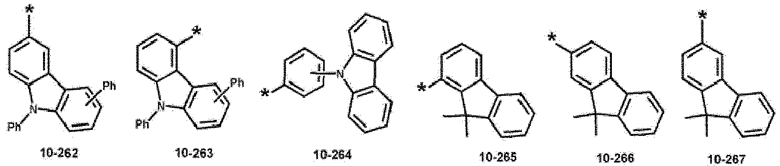 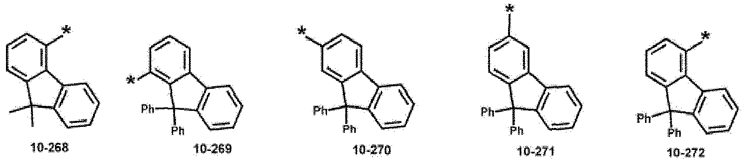 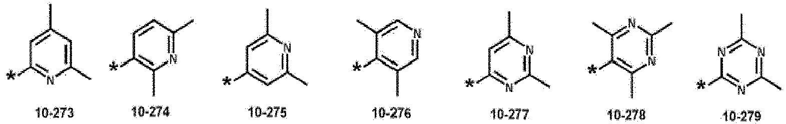 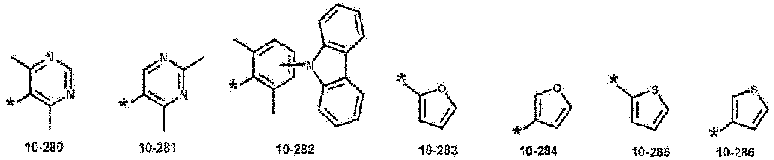 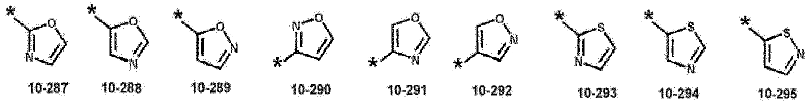 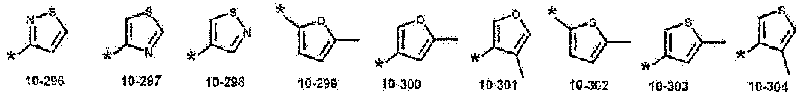 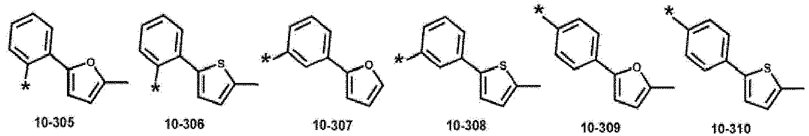 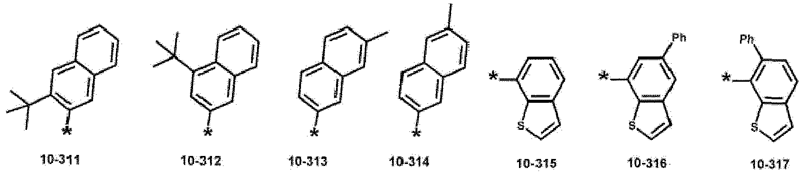 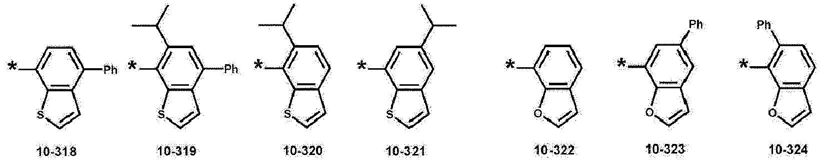 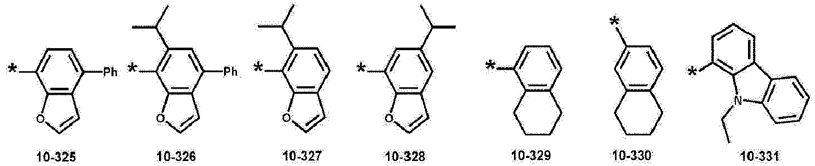 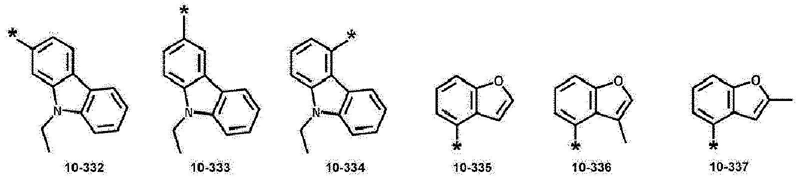 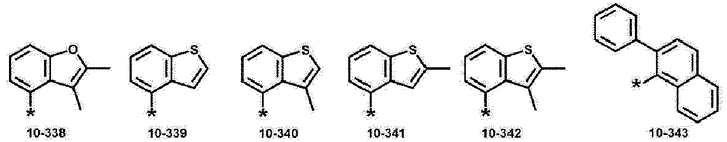 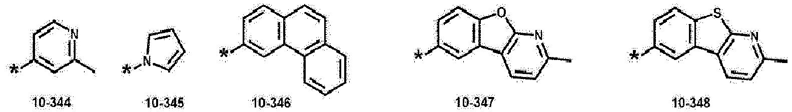 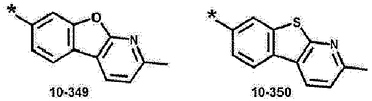 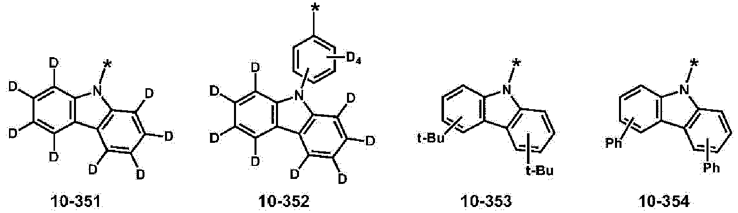 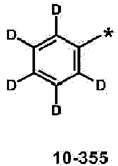
   wherein, in Formulae 9-1 to 9-61, 9-201 to 9-240, 10-1 to 10-129, and 10-201 to 10-355, * indicates a binding site to a neighboring atom, Ph represents a phenyl group, TMS represents a trimethylsilyl group, TMG represents a trimethylgermyl group, and t-Bu represents a t-butyl group.

In an embodiment, R₂₁ and R₂₂ may each independently be: a phenyl group, a naphthyl group, or a carbazolyl group, each unsubstituted or substituted with deuterium, a C₁-C₁₀ alkyl group, a phenyl group, -Si(Q₃₁)(Q₃₂)(Q₃₃) or any combination thereof; or -Si(Q₁)(Q₂)(Q₃),
Q₁ to Q₃ may each independently be a phenyl group or a naphthyl group, each unsubstituted or substituted with deuterium, a C₁-C₁₀ alkyl group, a phenyl group, - Si(Q₃₁)(Q₃₂)(Q₃₃), or any combination thereof, and
Q₃₁ to Q₃₃ may each independently be a phenyl group or a naphthyl group, each unsubstituted or substituted with deuterium, a C₁-C₁₀ alkyl group, a phenyl group, or any combination thereof.

In an embodiment, R₂₁ and R₂₂ may each independently be a C₆-C₆₀ aryl group unsubstituted or substituted with at least one R₁₀ₐ, a group represented by Formula E-2A, or a group represented by Formula E-2B, and
R₁₀ₐ is the same as described in connection with R₂₃ in Formula E-2: 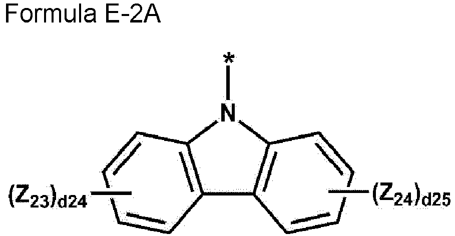 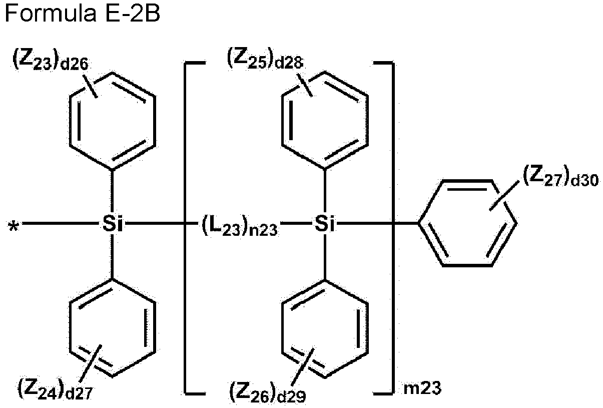
wherein, in Formulae E-2A and E-2B,
Z₂₃ to Z₂₇ are each the same as described in connection with R₂₃,
d24 and d25 may each independently be an integer from 1 to 4,
d26 to d30 may each independently be an integer from 1 to 5,
L₂₃ is the same as described in connection with L₂₁,
n23 may be an integer from 1 to 5,
m23 may be an integer from 0 to 3, and
* indicates a binding site to a neighboring atom.

Examples of the electron-transporting host include, for example, compounds of Groups HE1 to HE8:

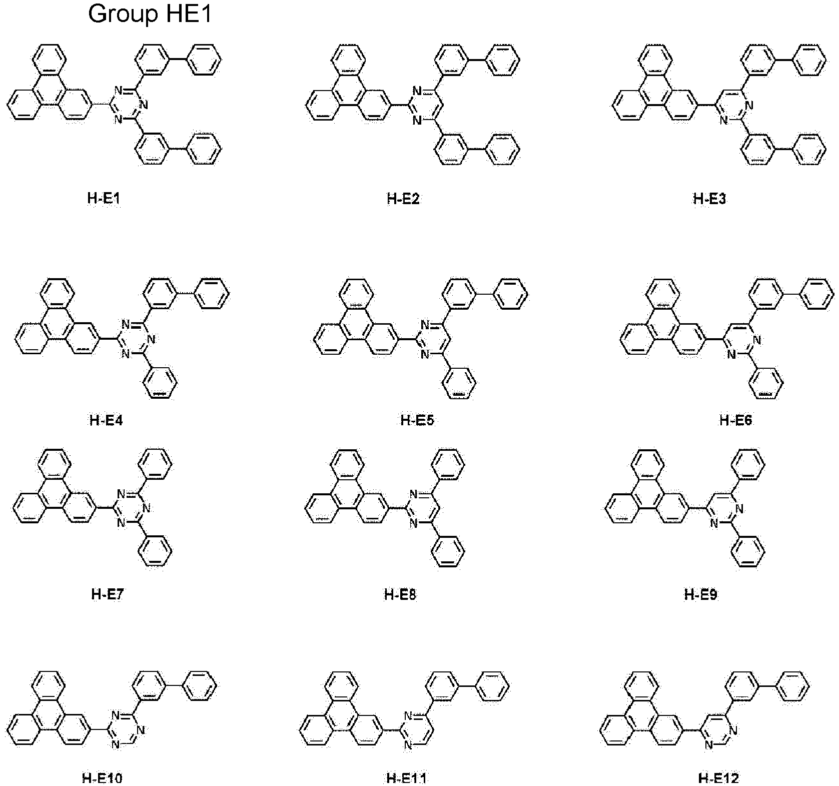

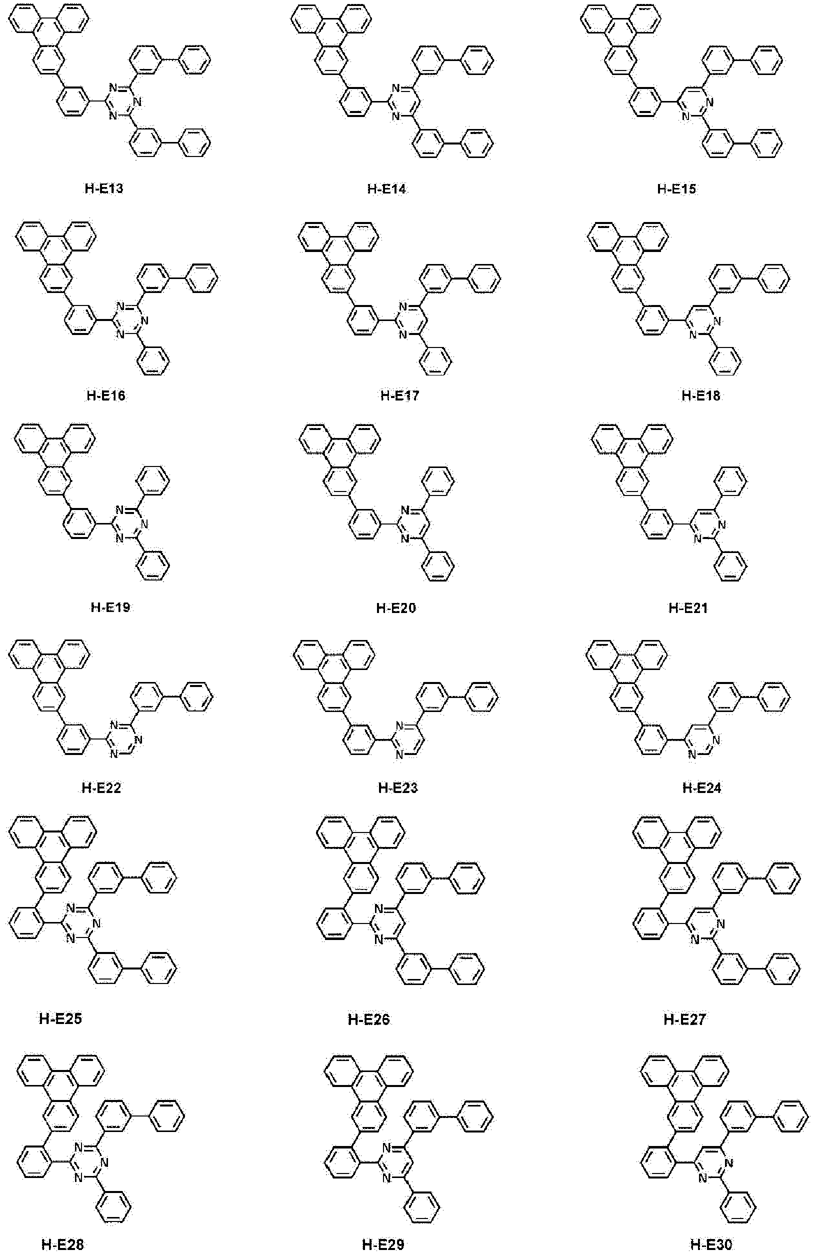

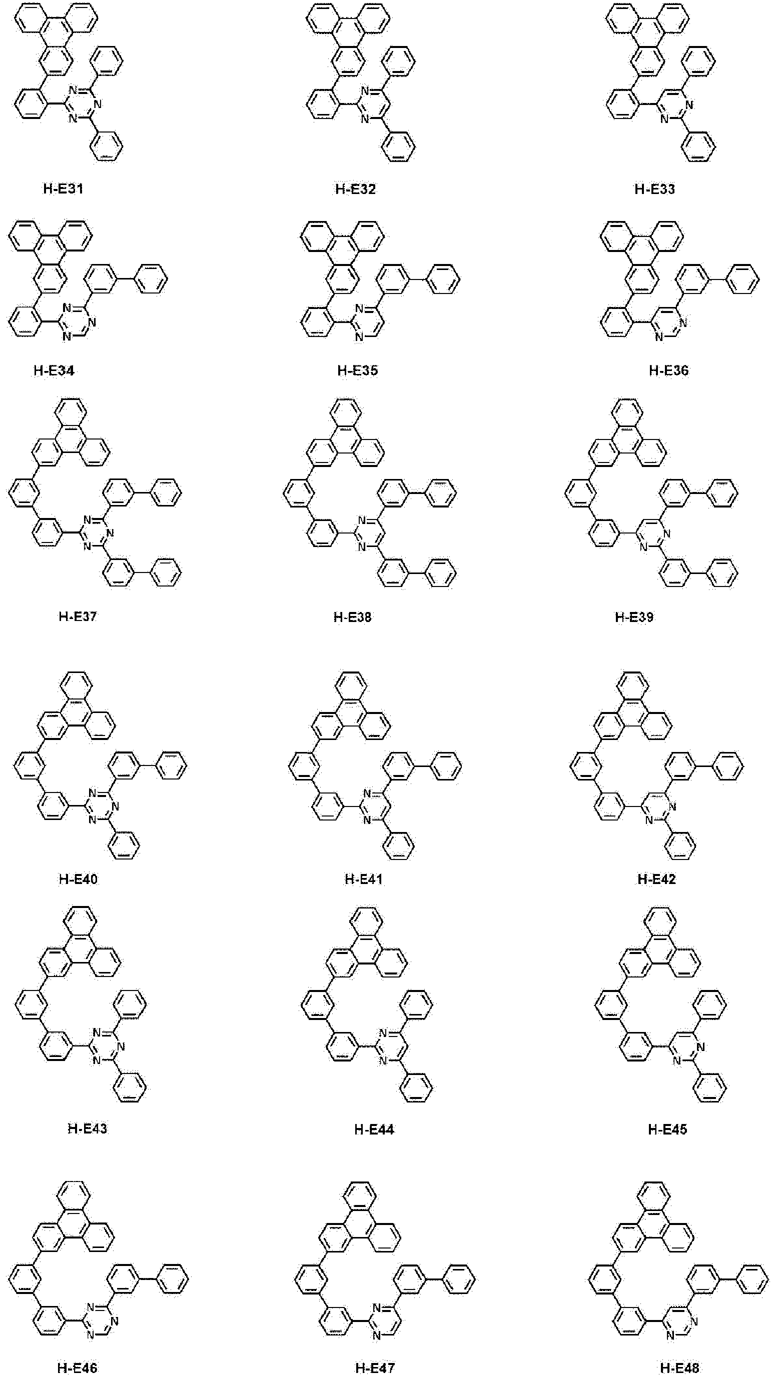

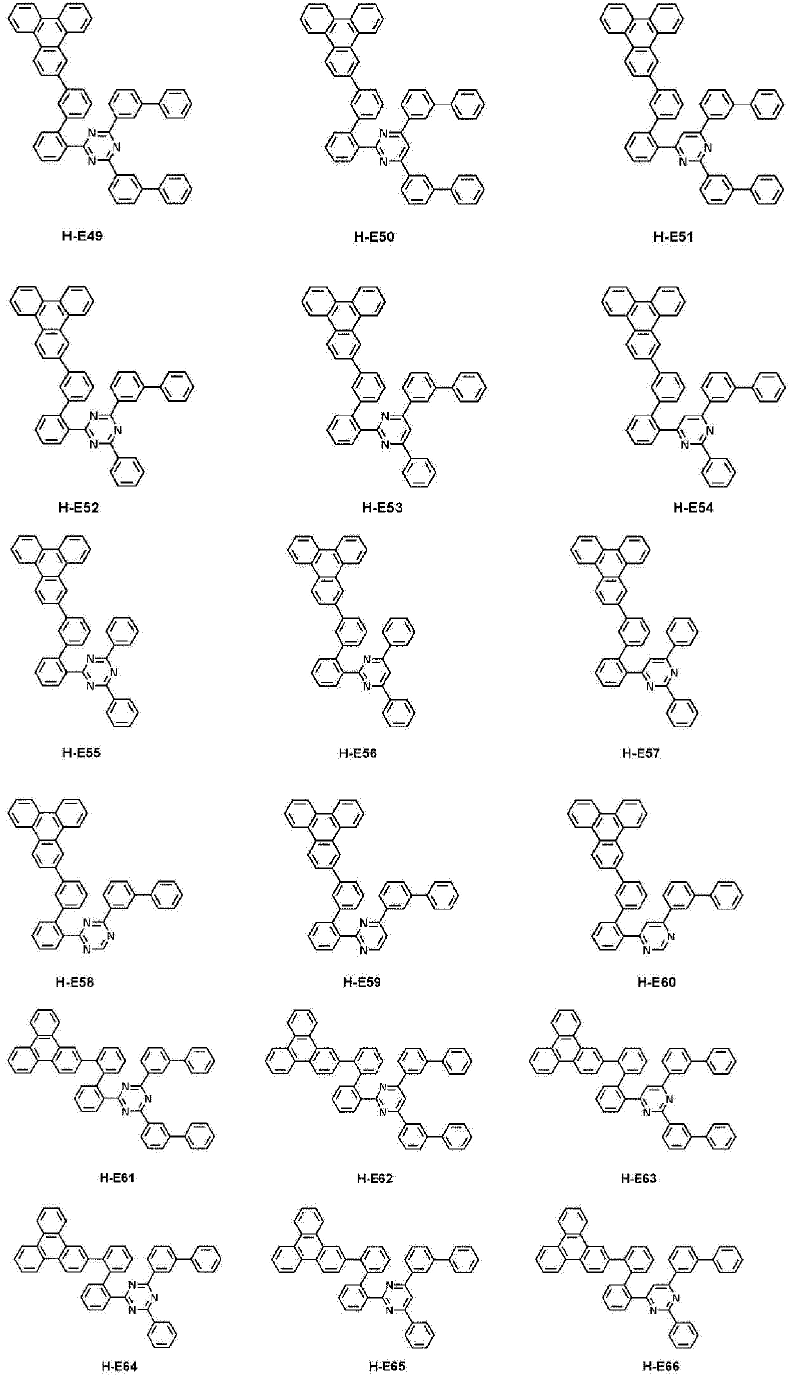

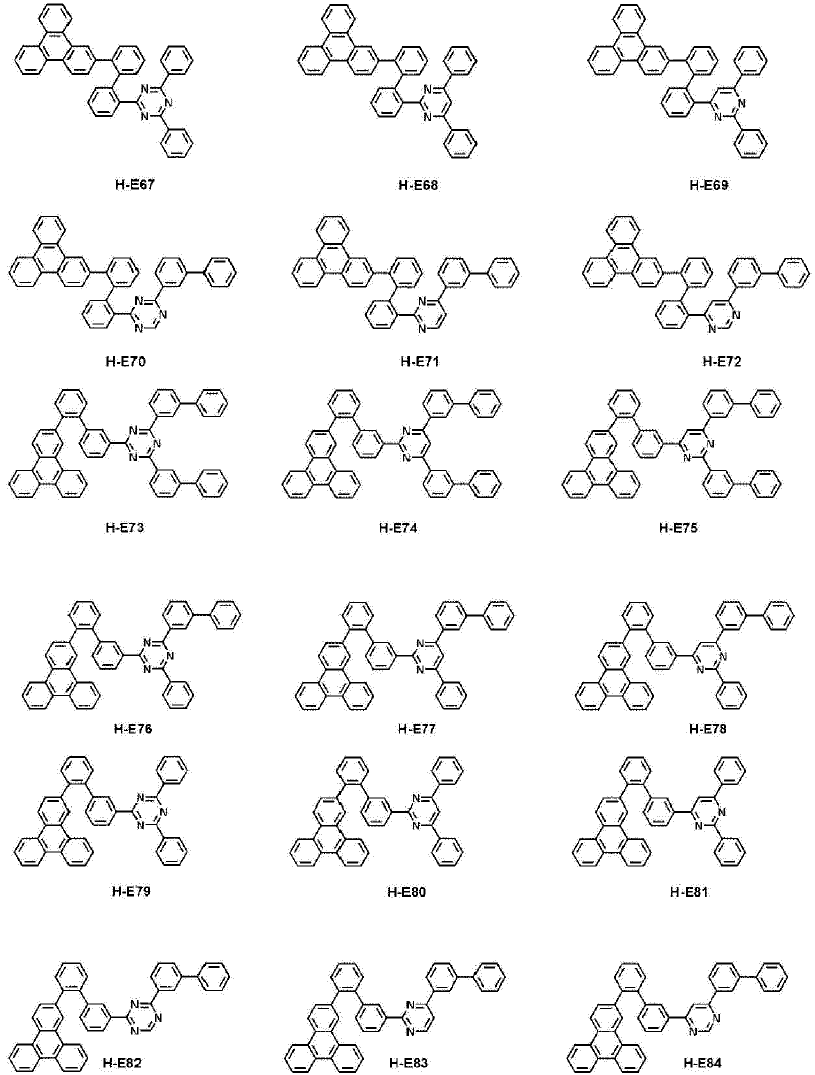

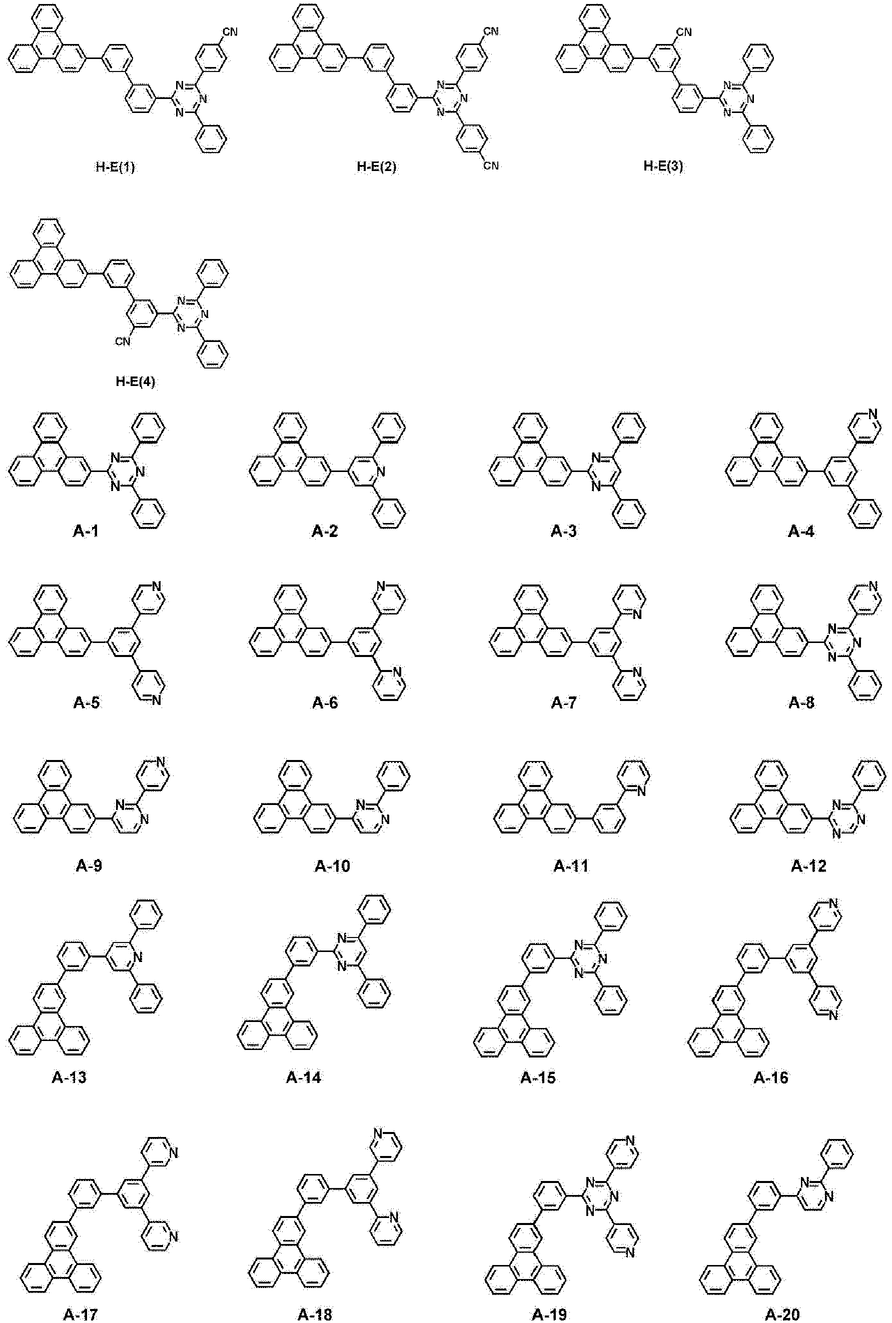

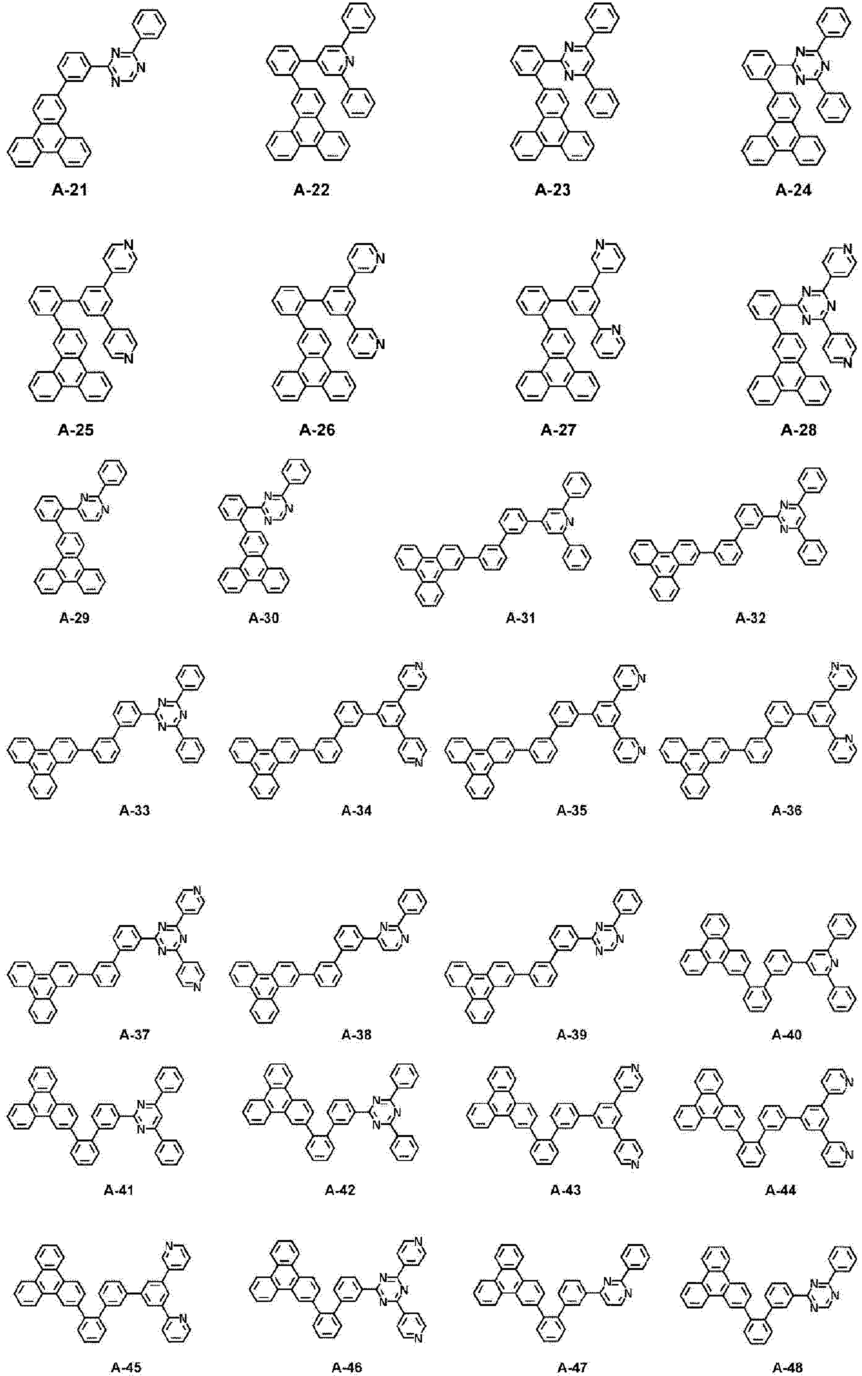

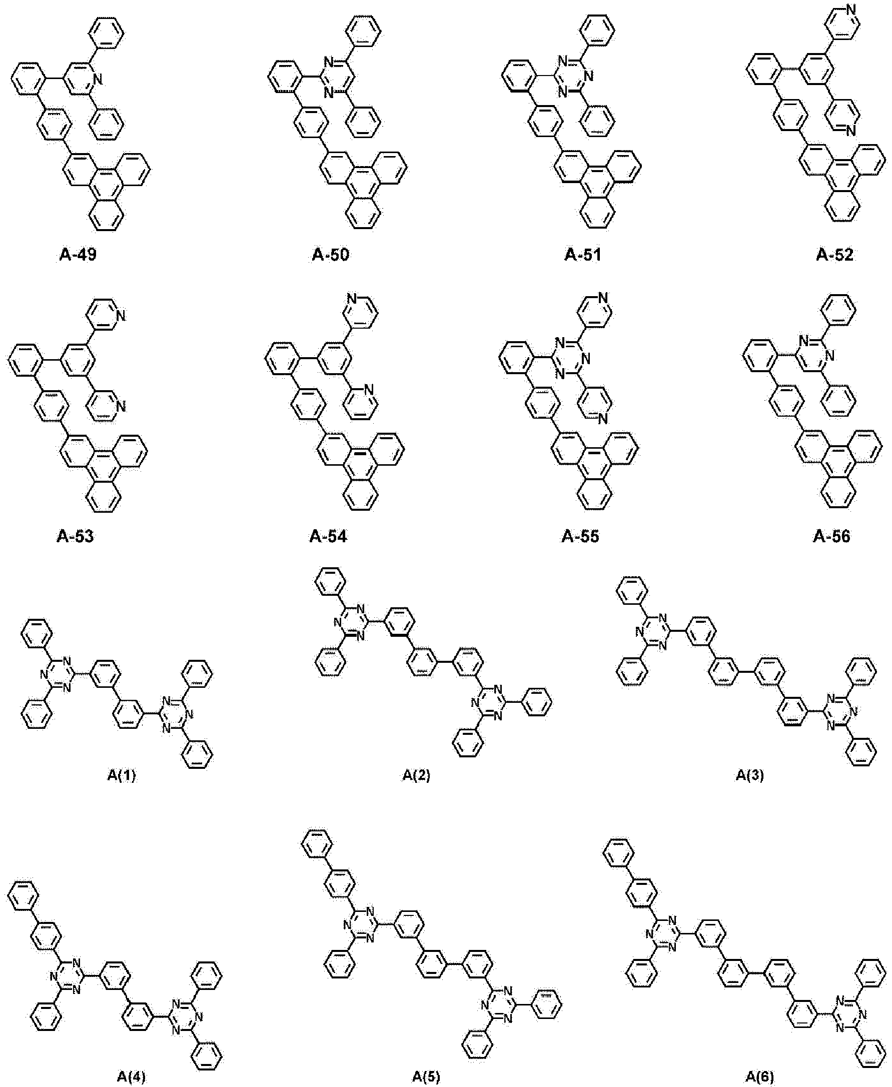

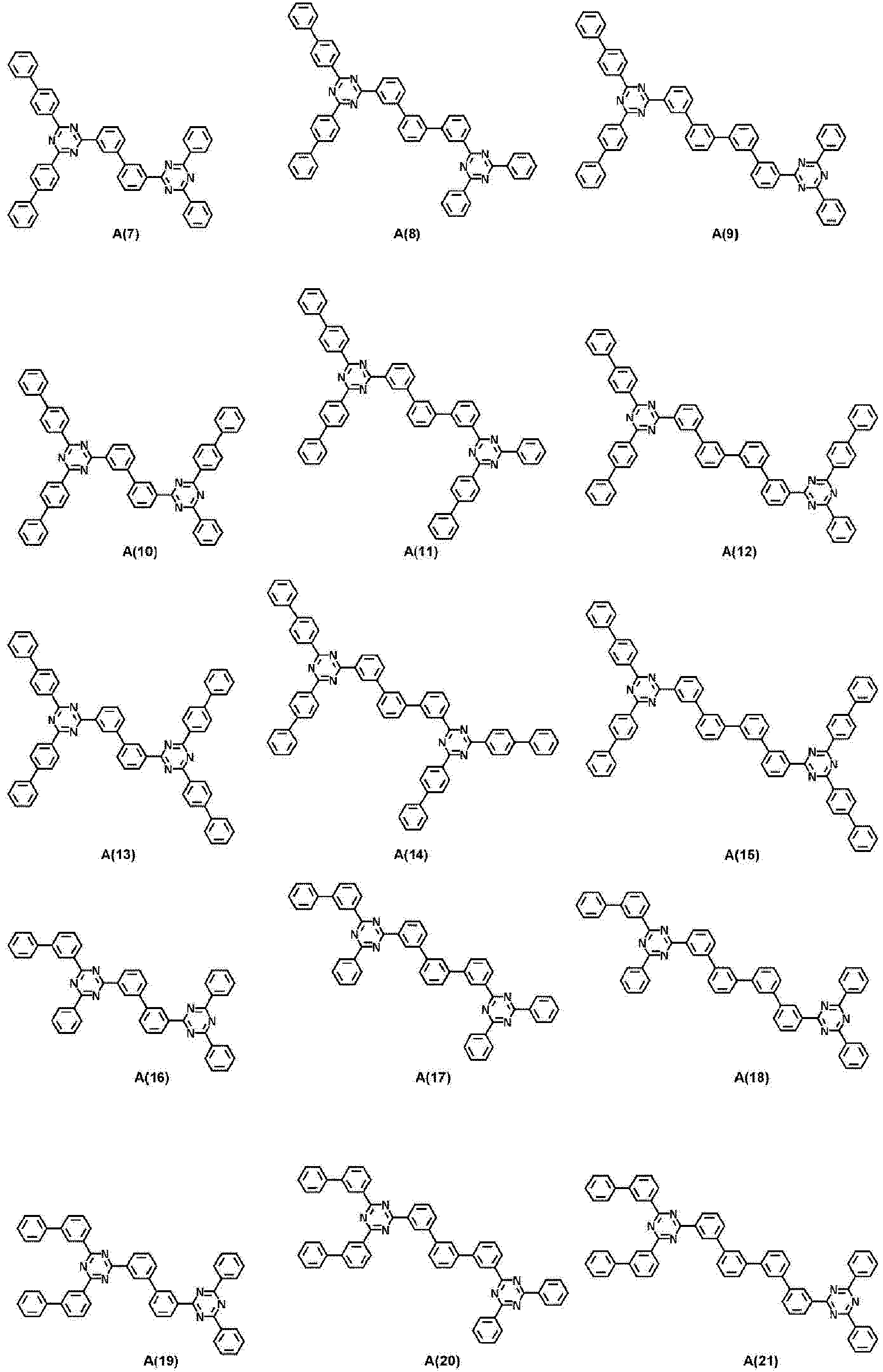

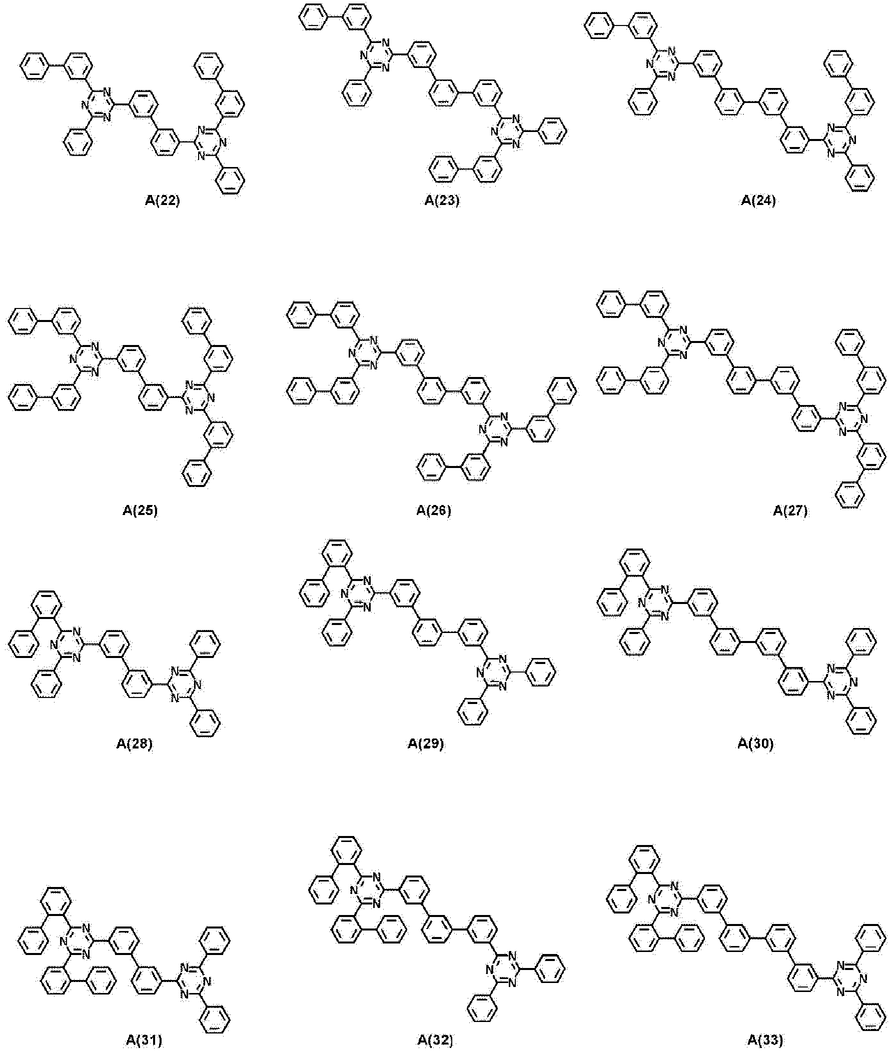

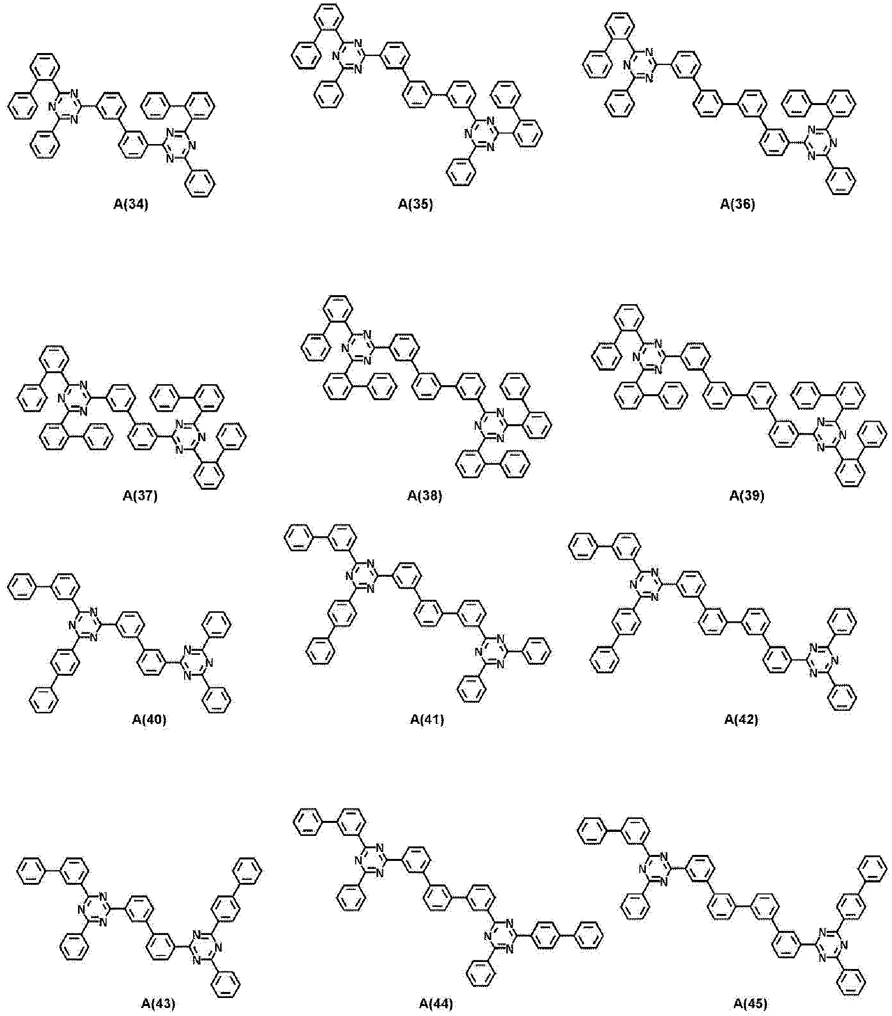

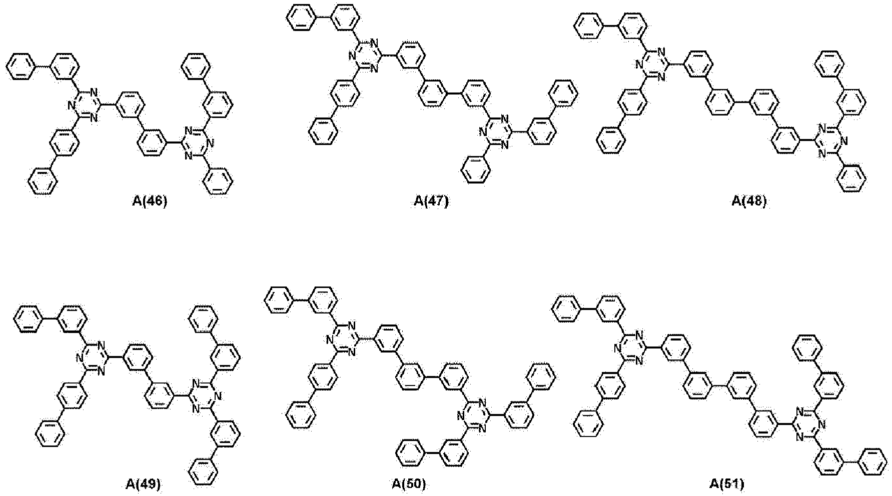

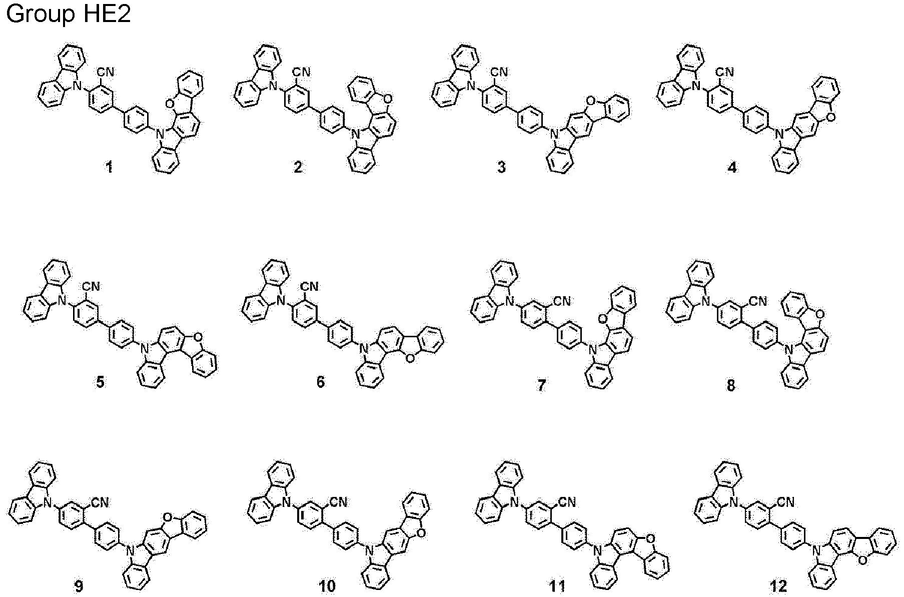

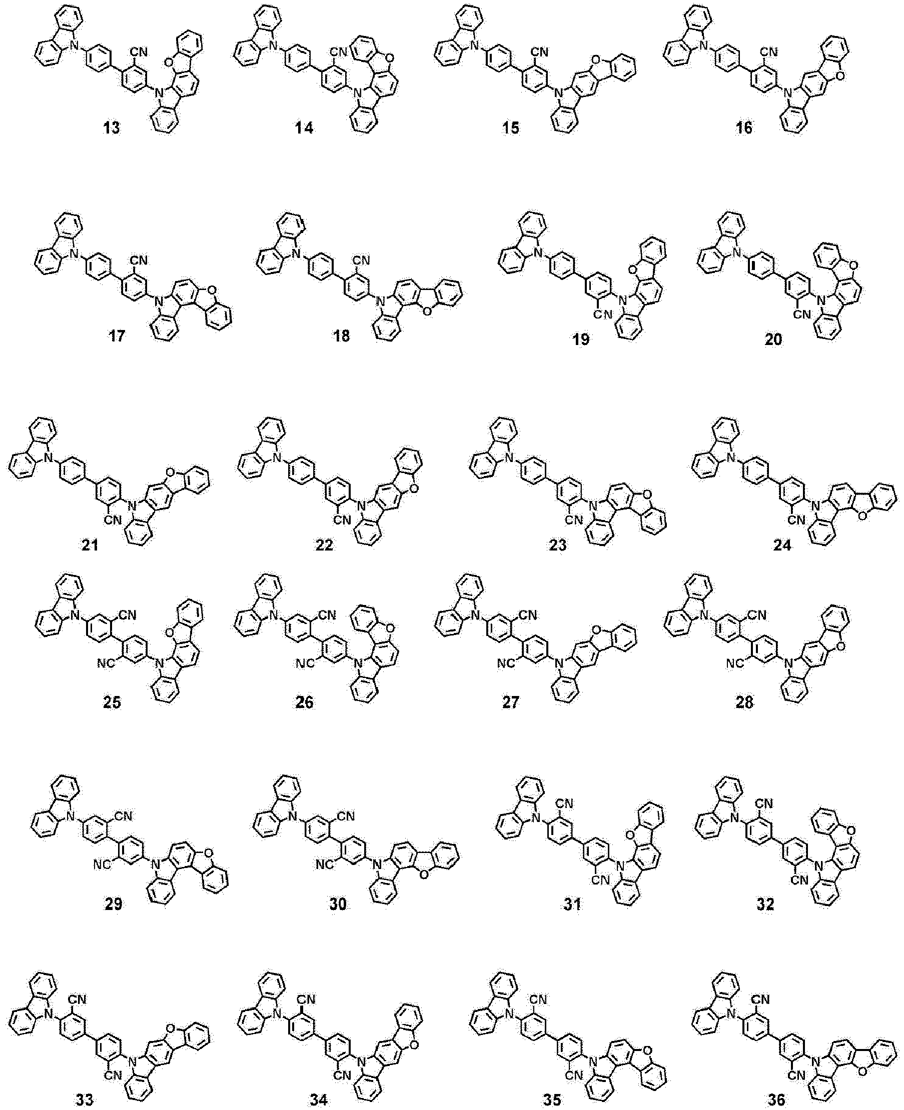

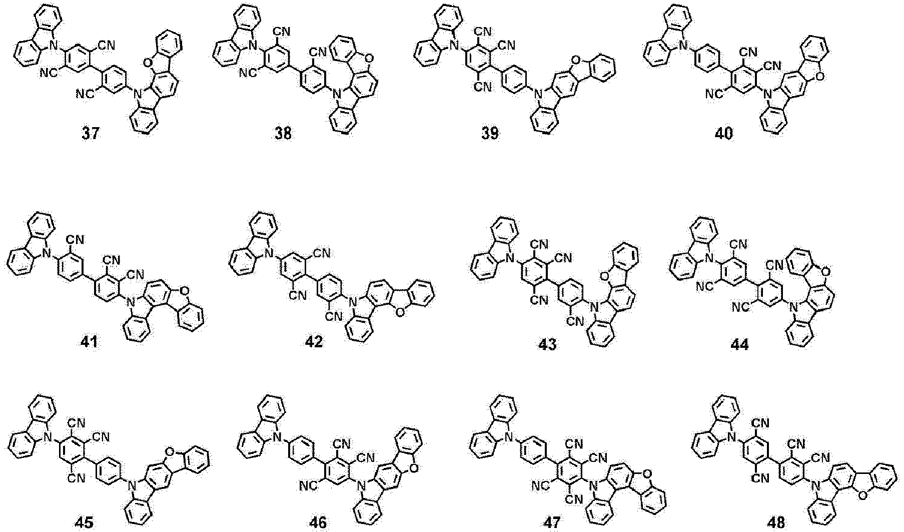

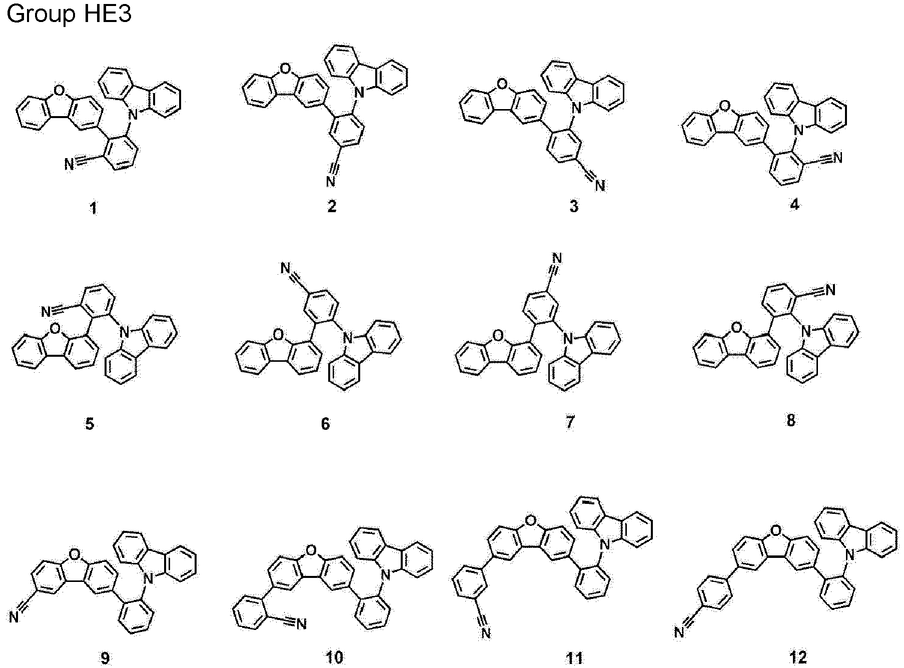

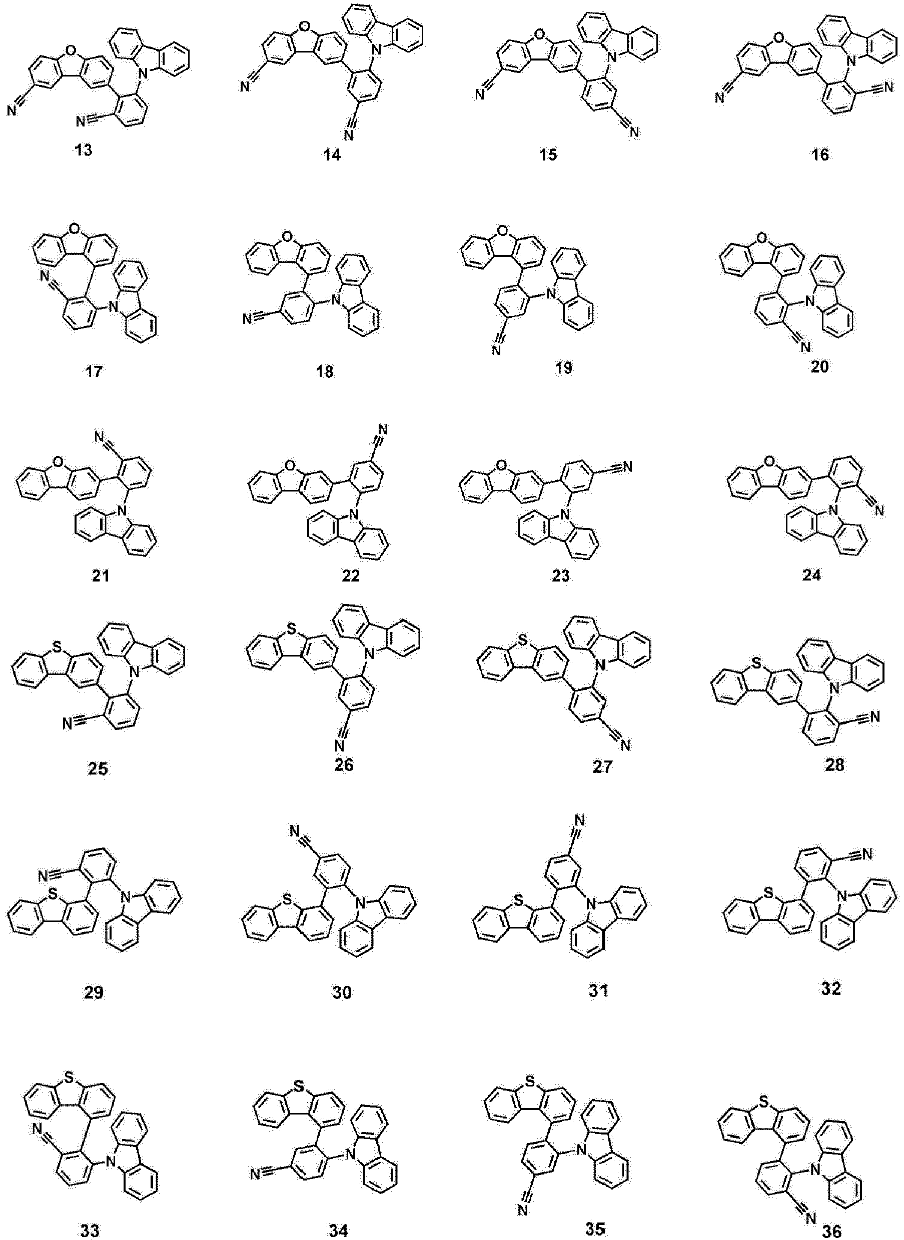

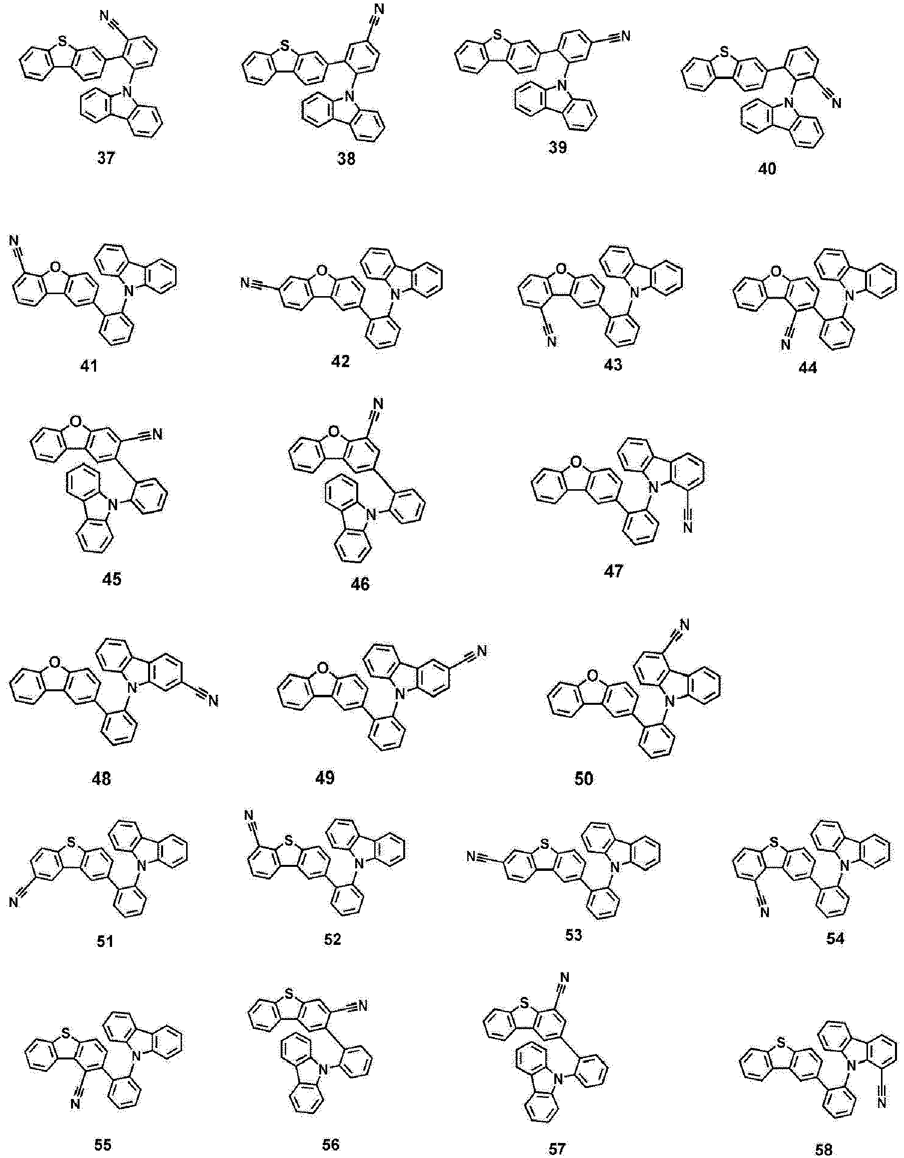

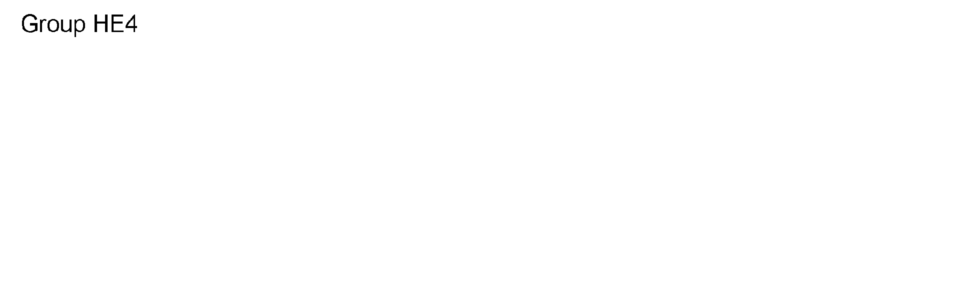

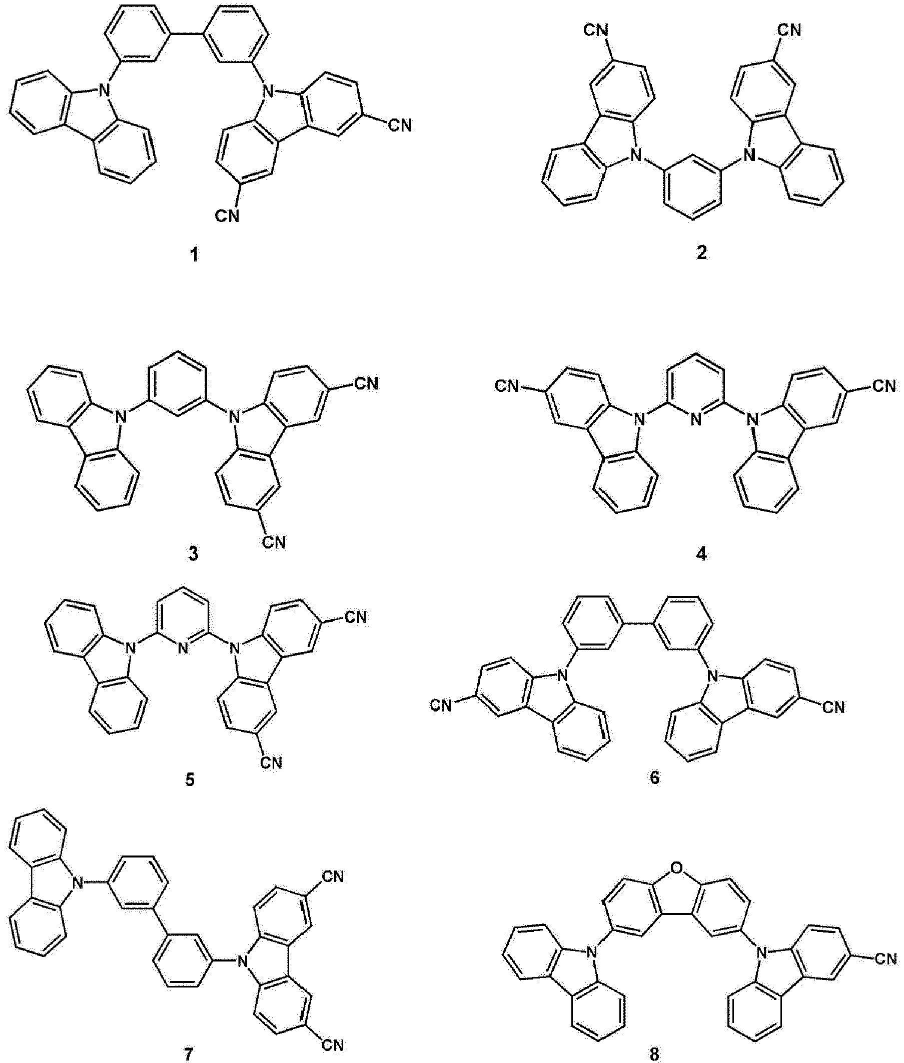

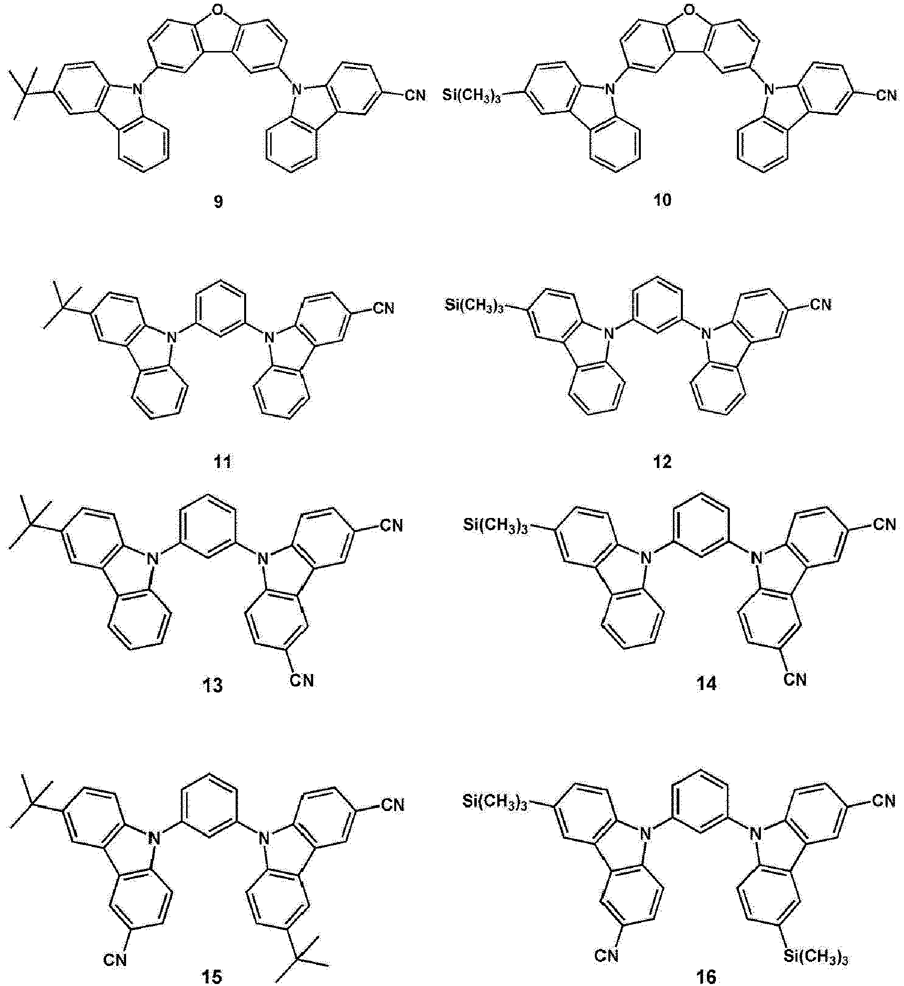

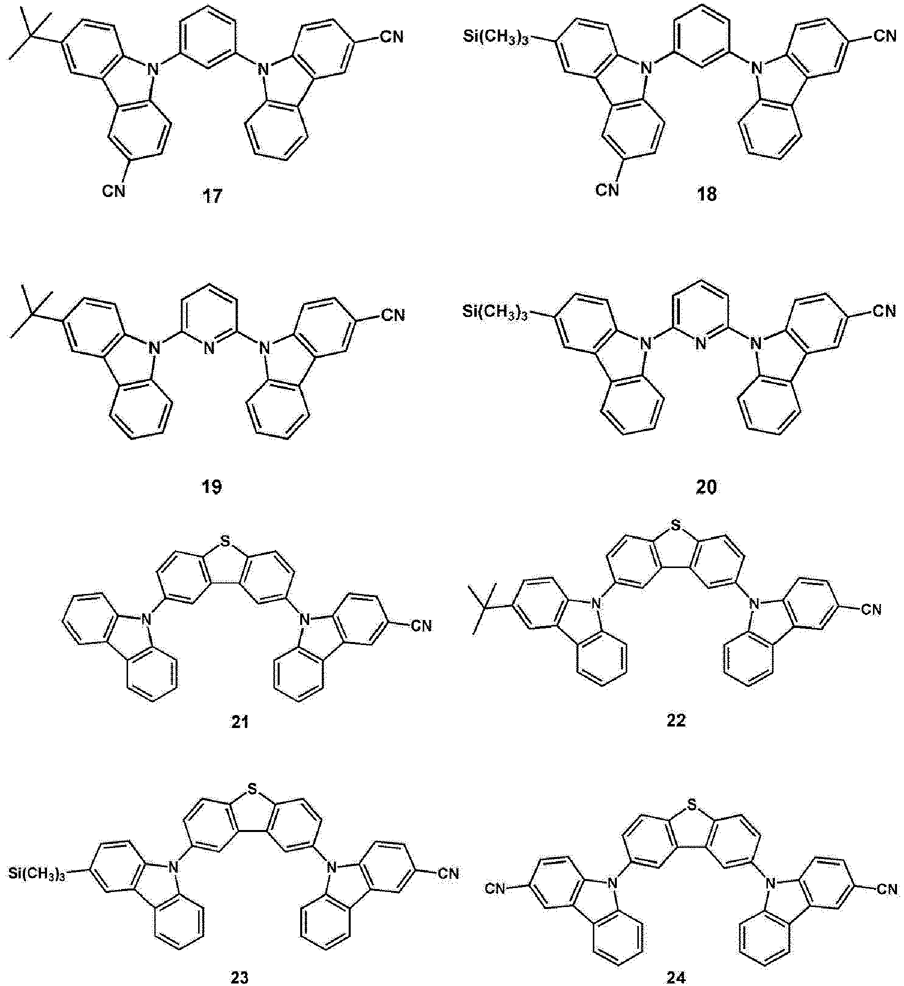

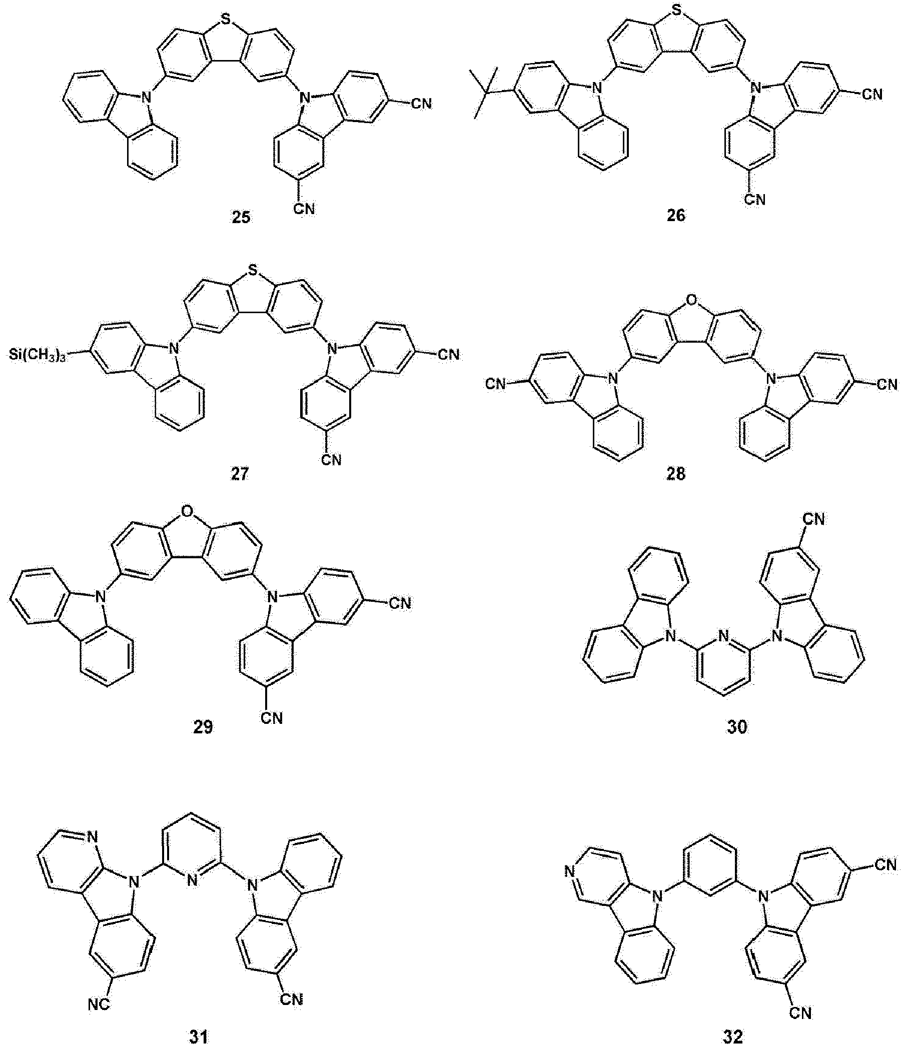

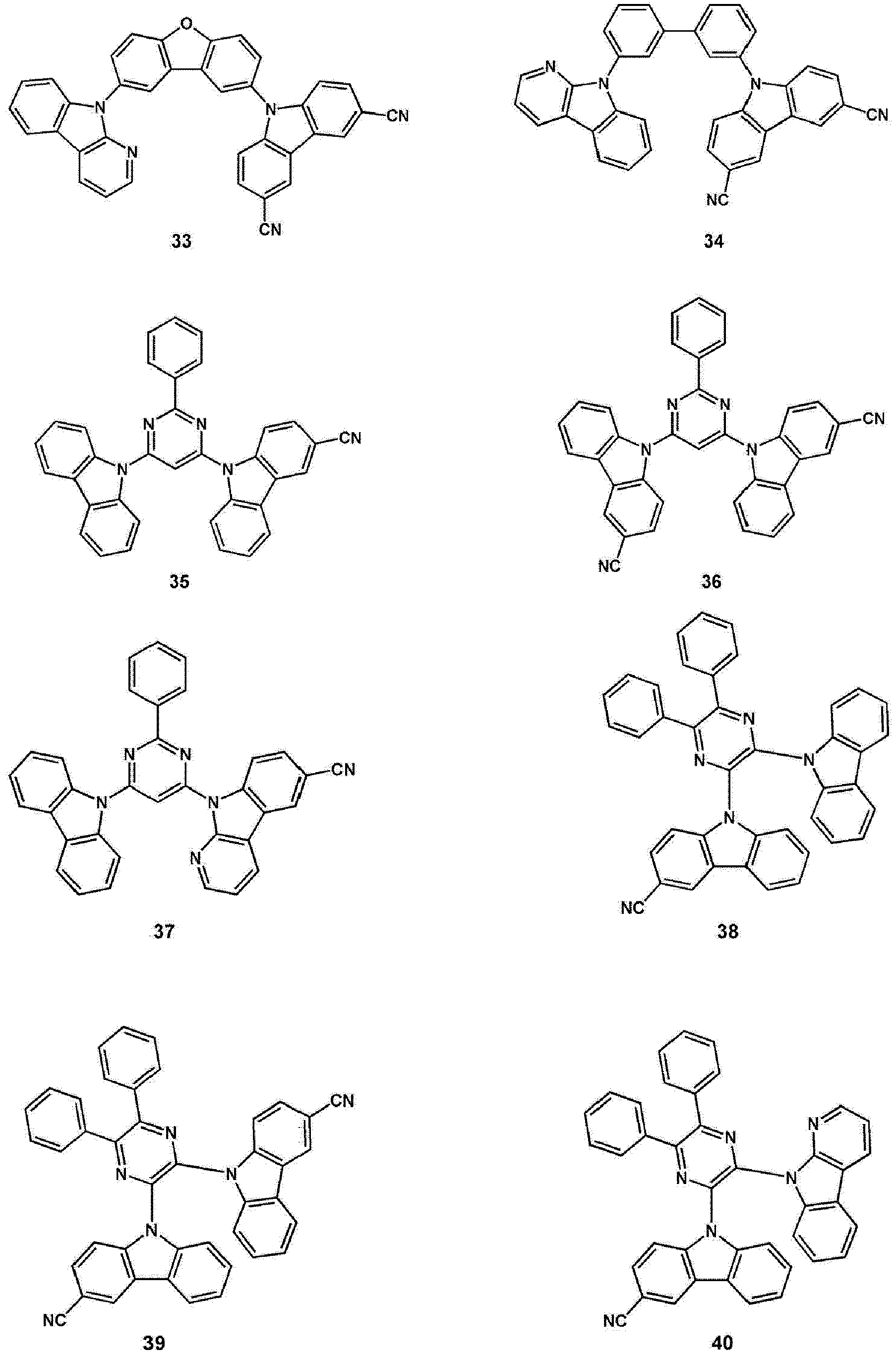

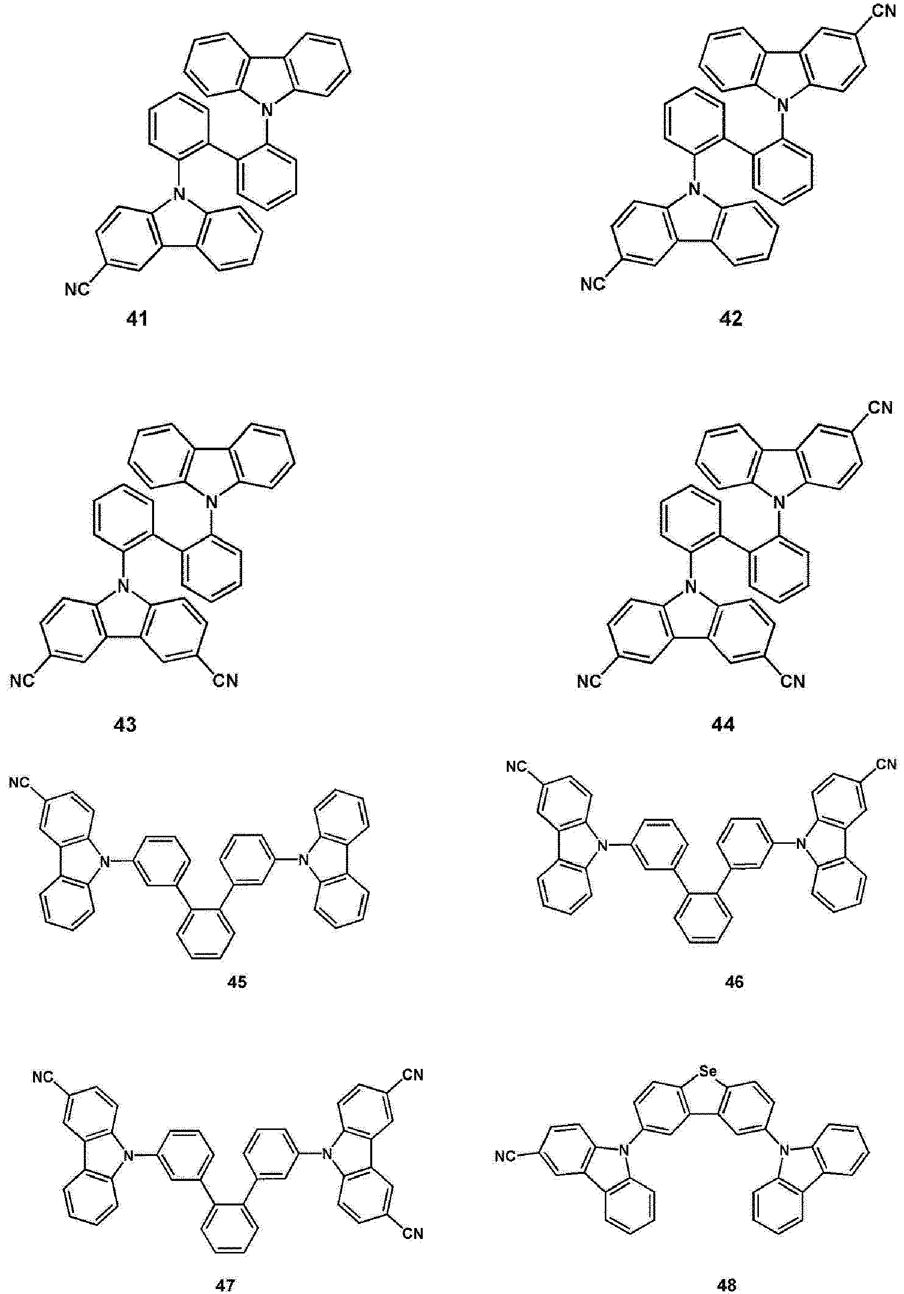

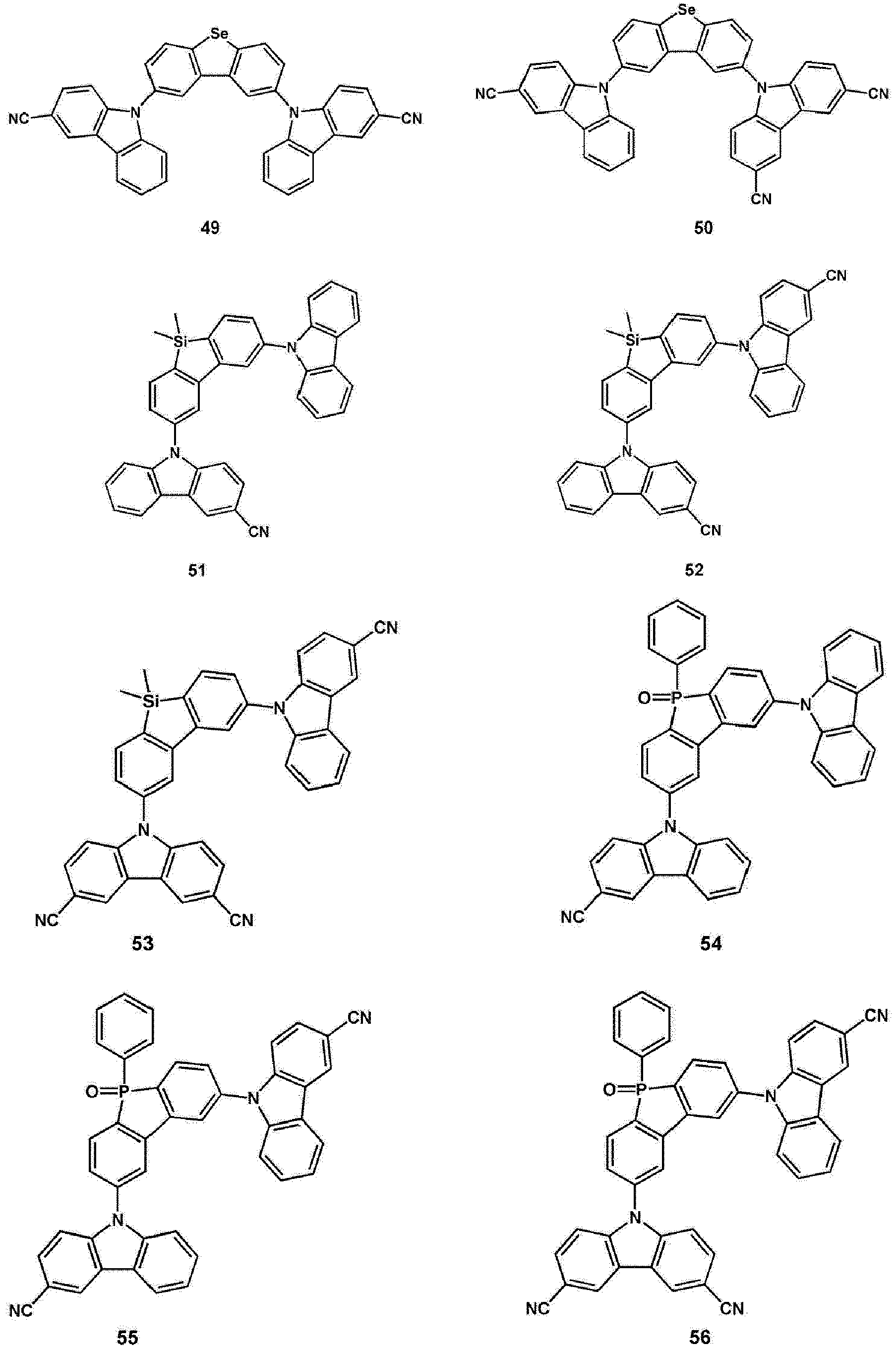

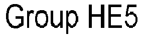

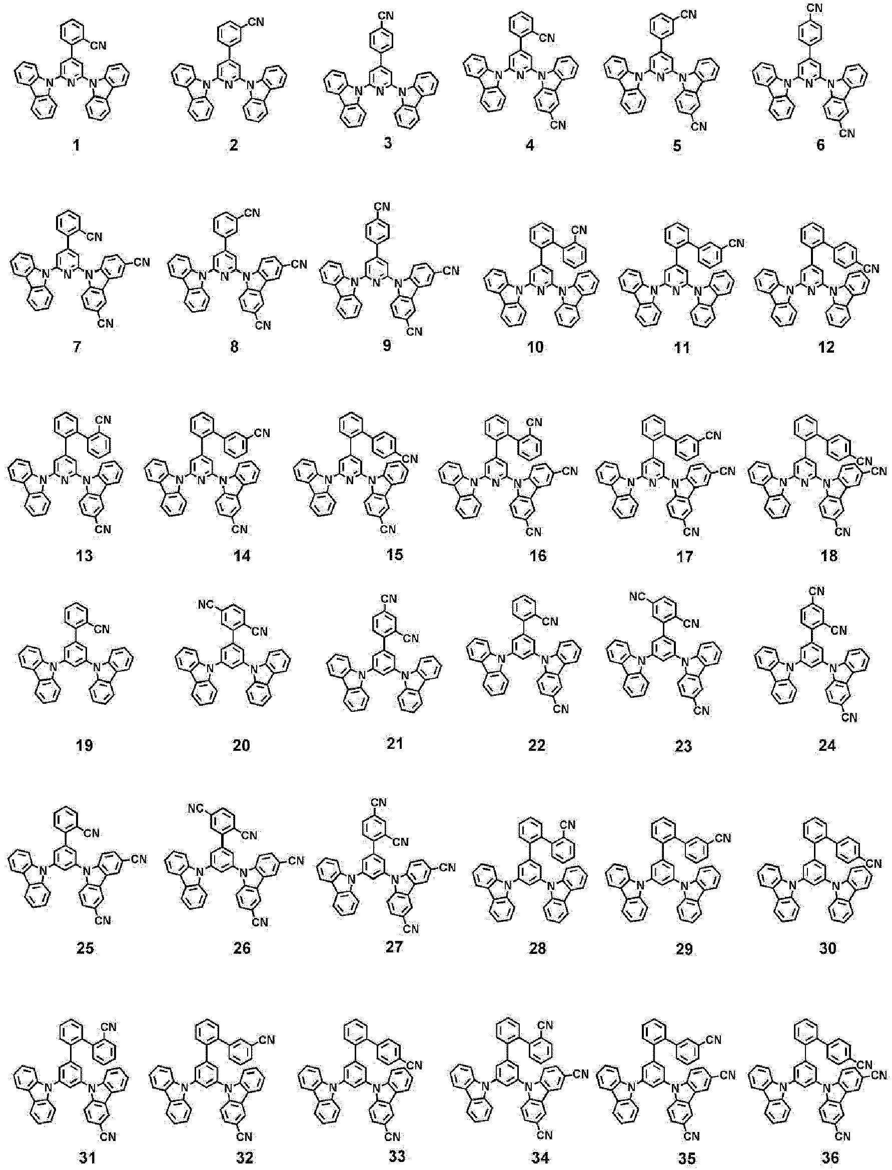

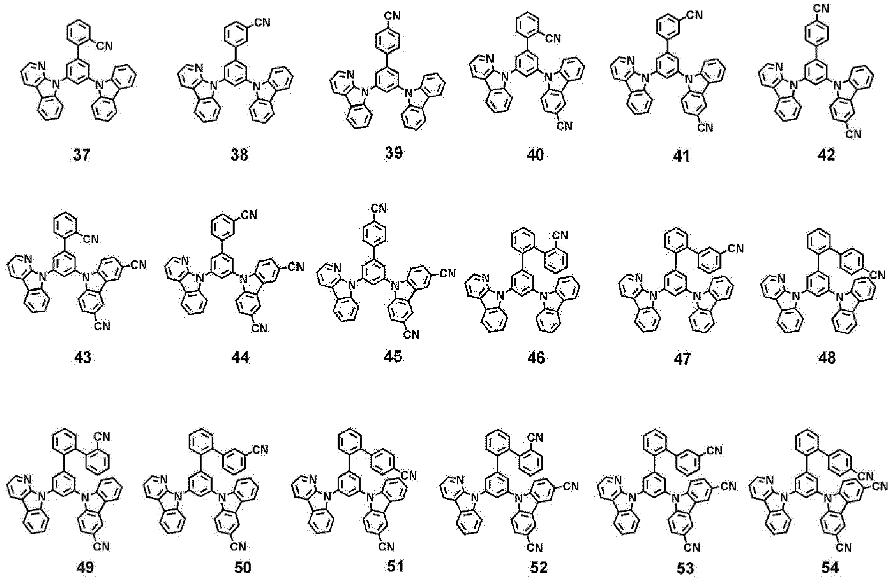

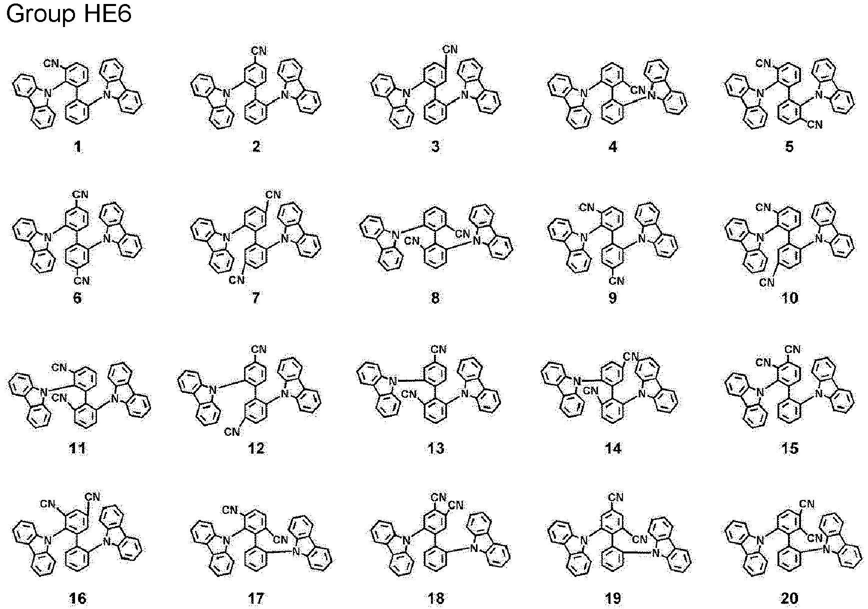

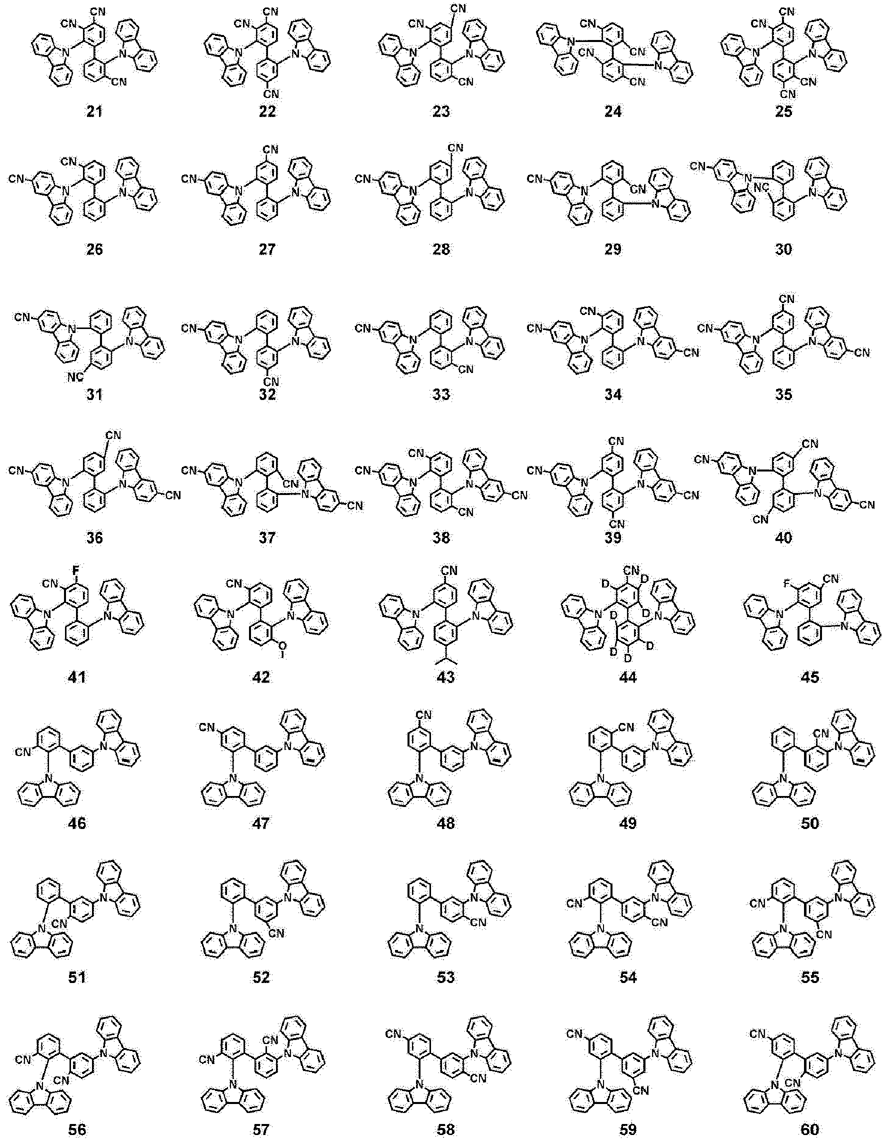

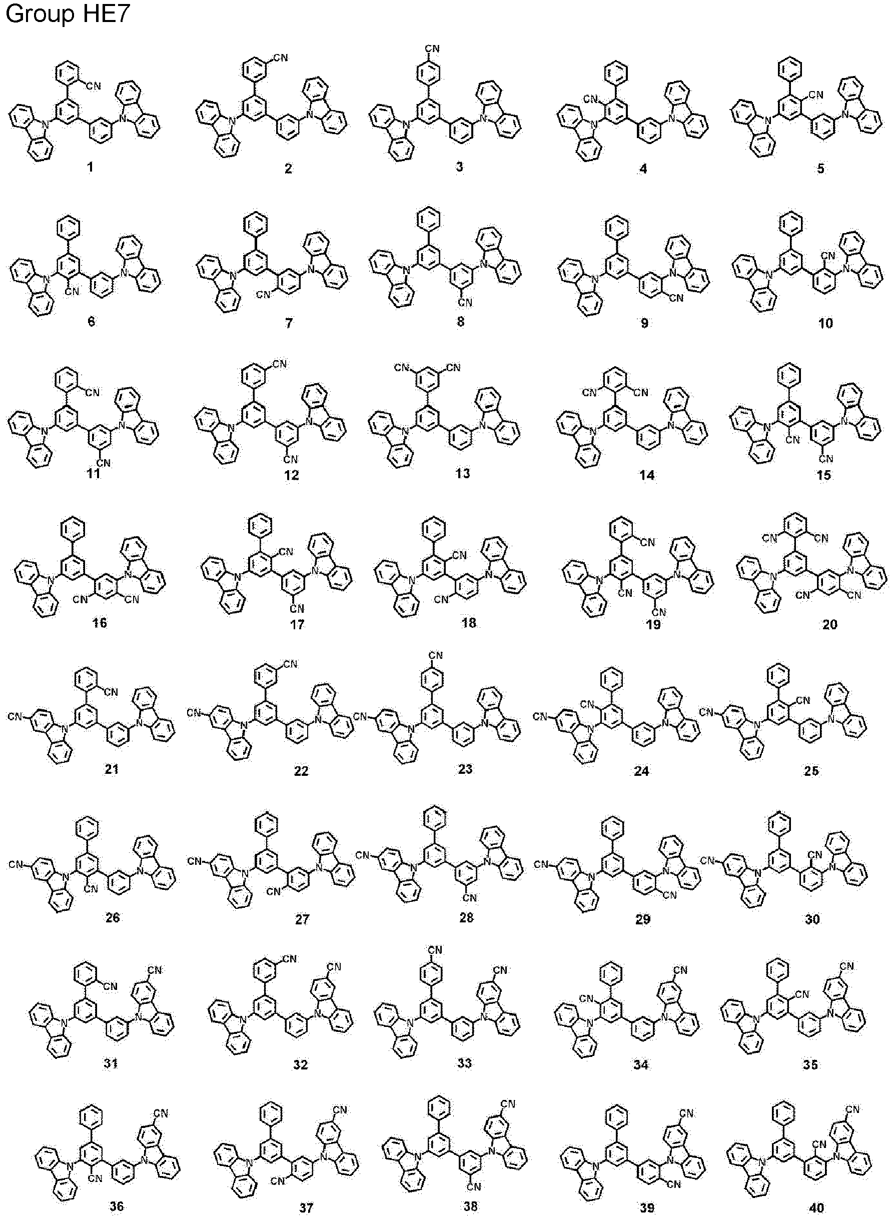

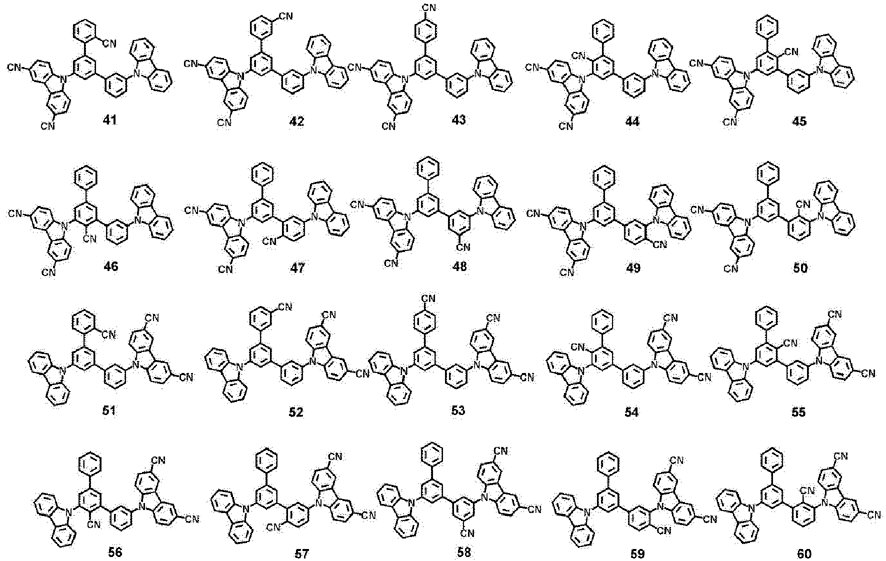

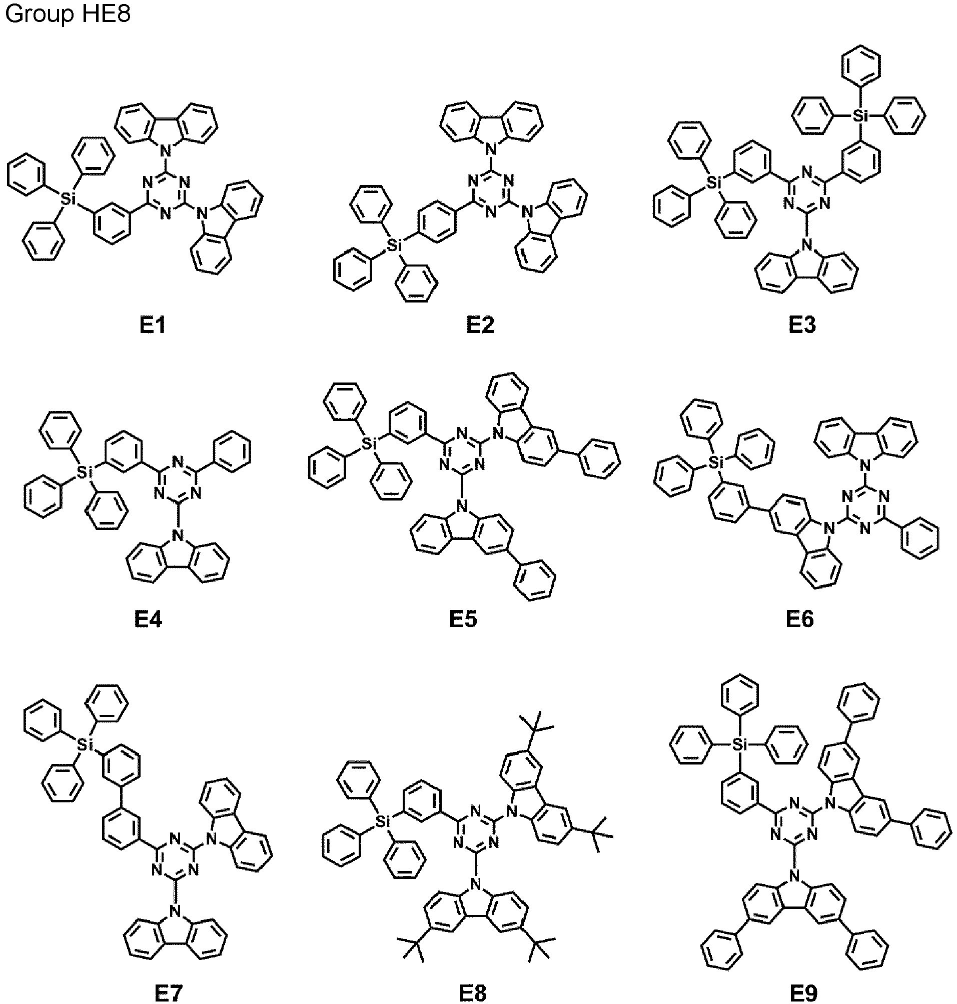

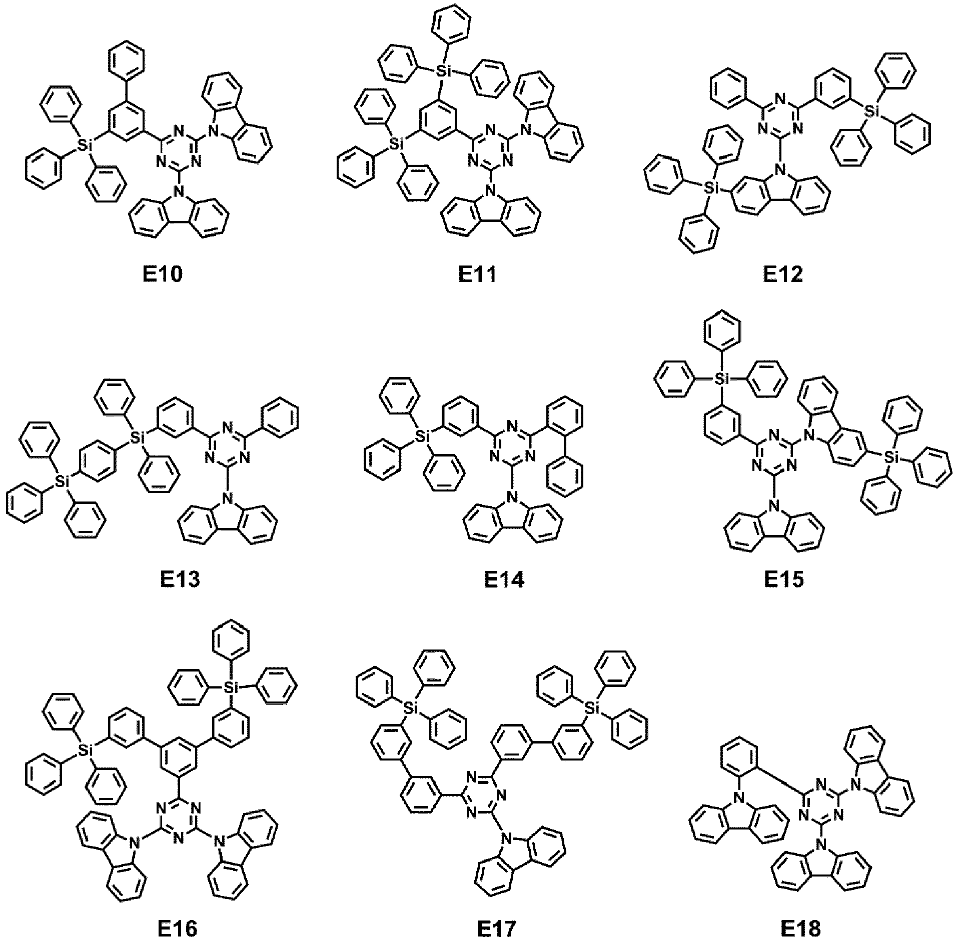

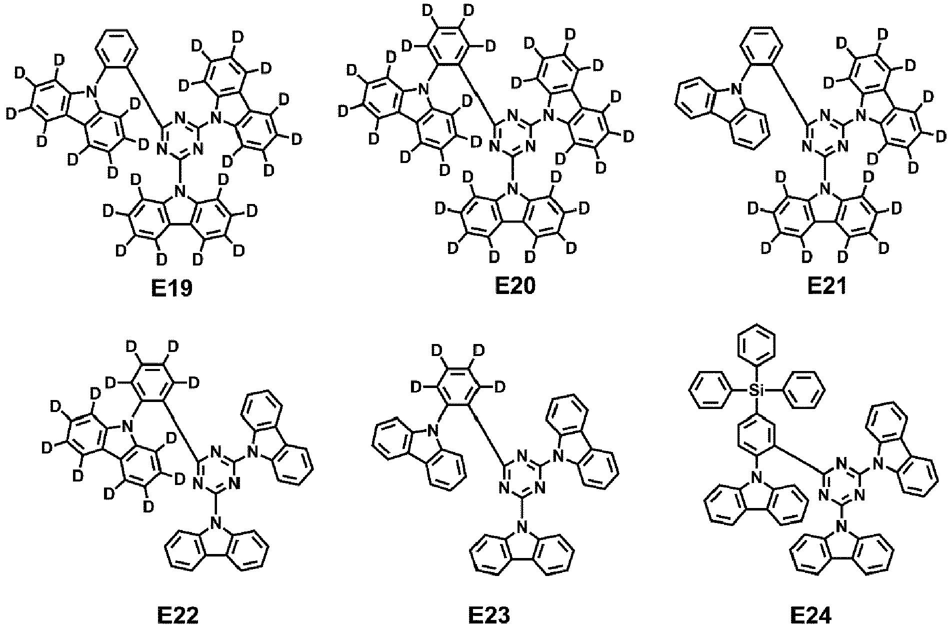

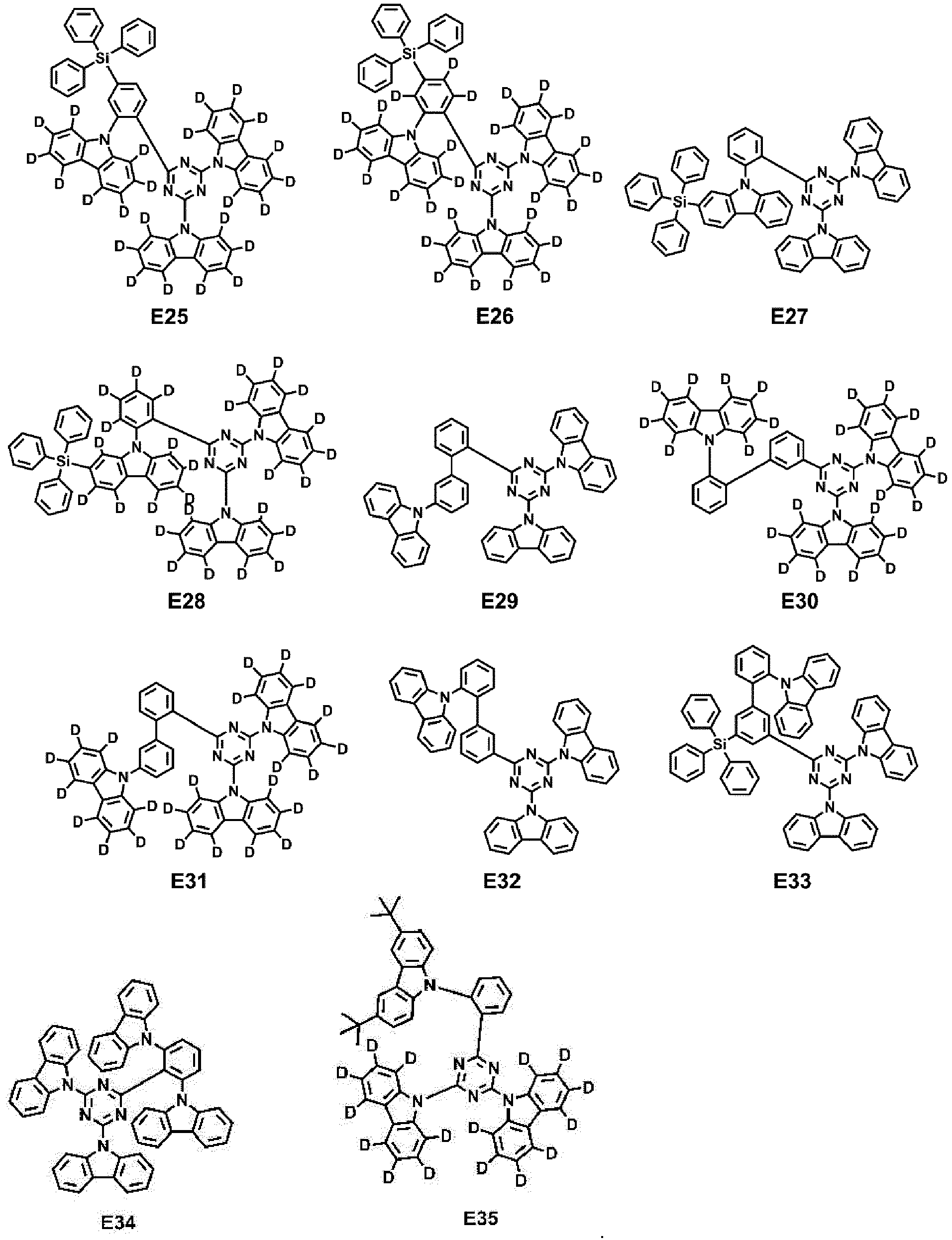

In an embodiment, the hole-transporting host may include at least one of Compounds H-H1 to H-H103:

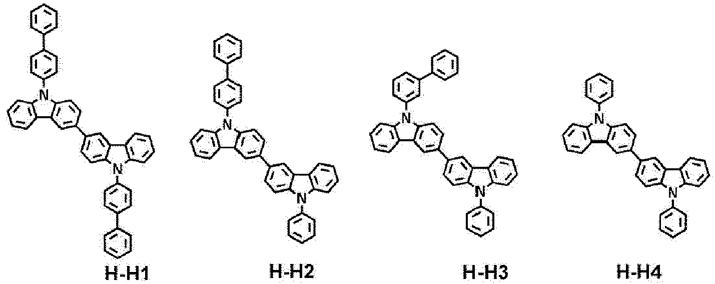

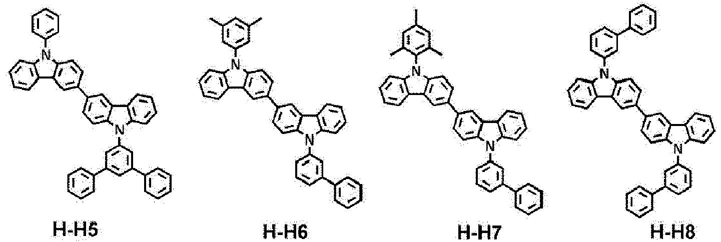

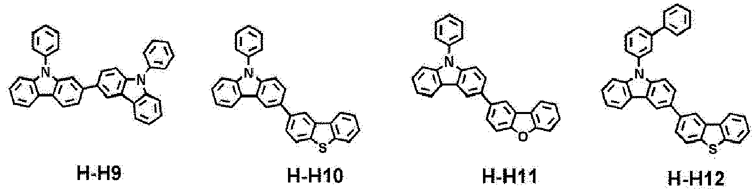

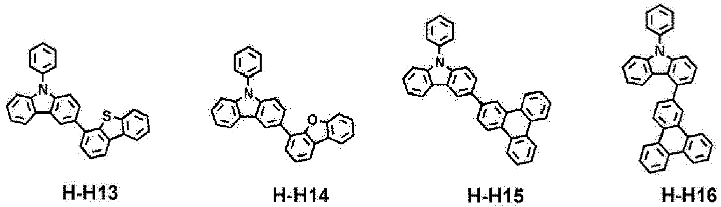

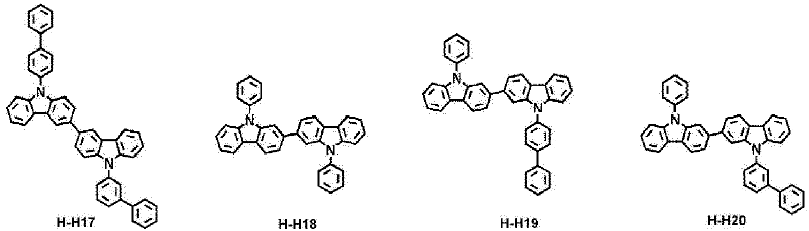

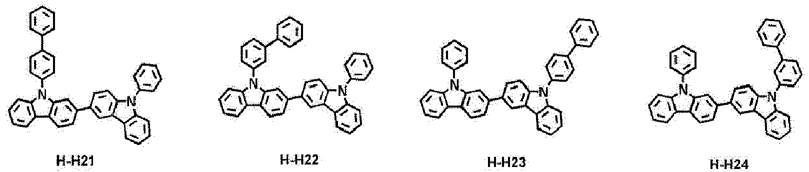

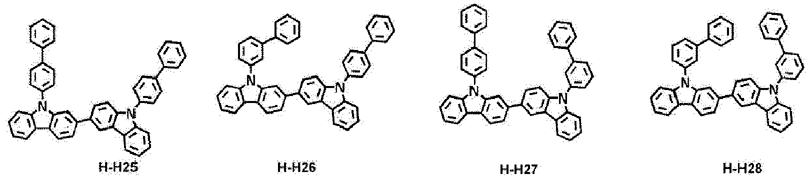

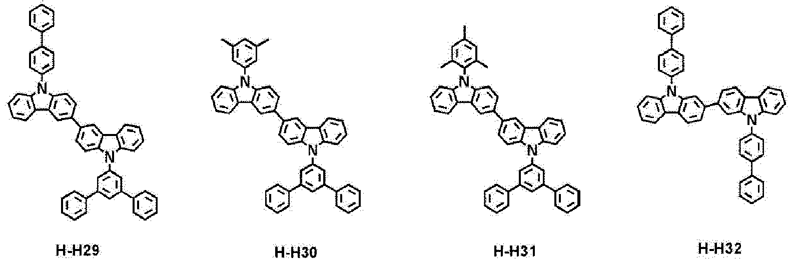

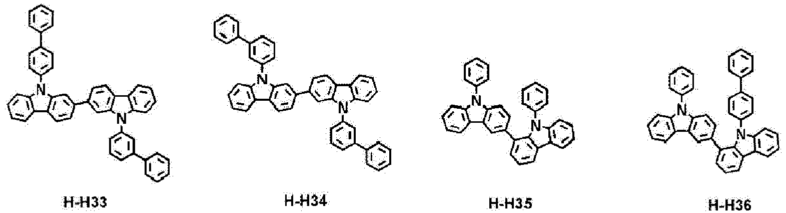

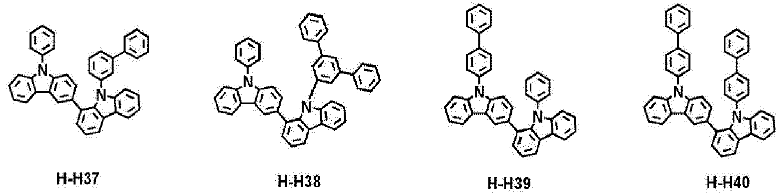

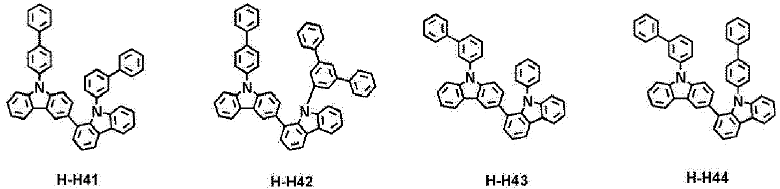

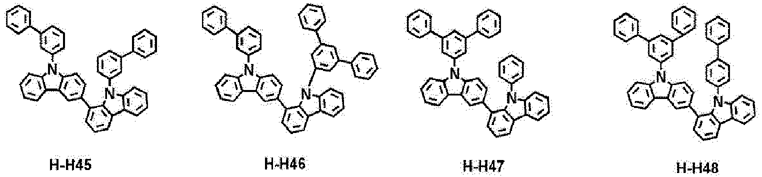

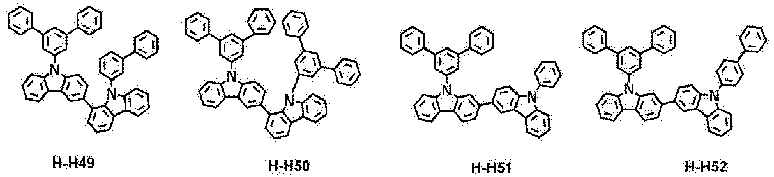

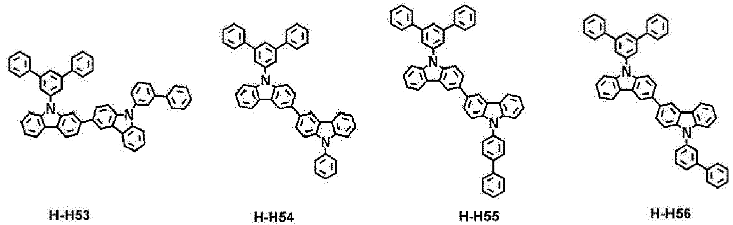

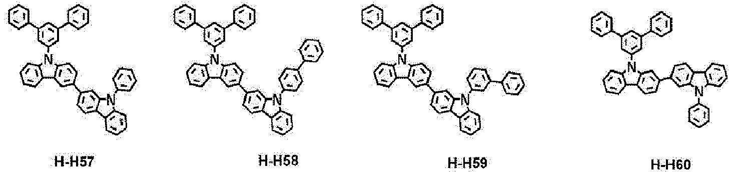

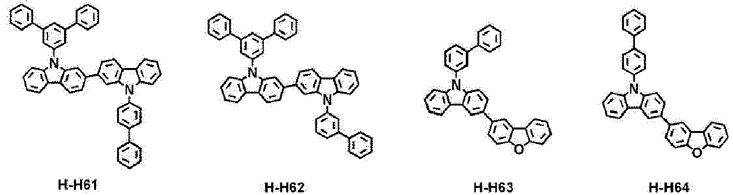

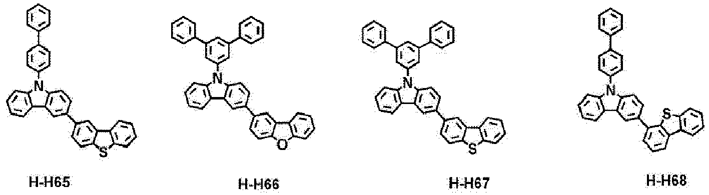

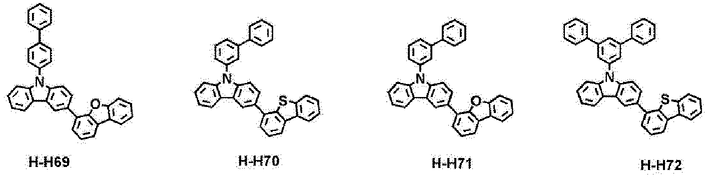

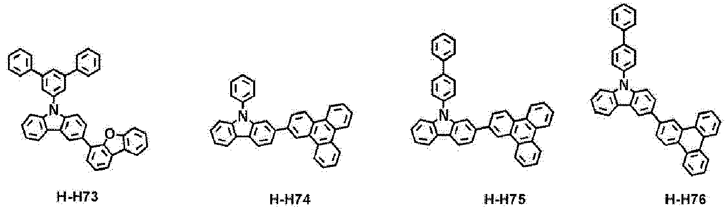

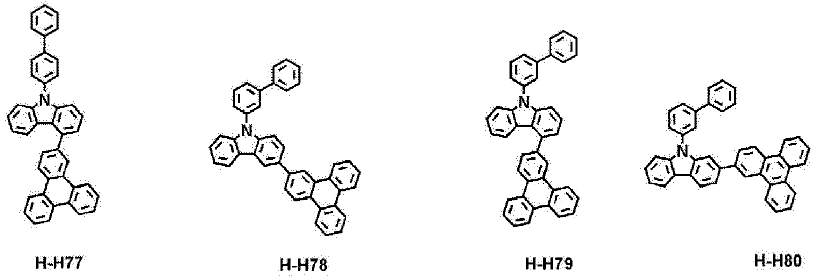

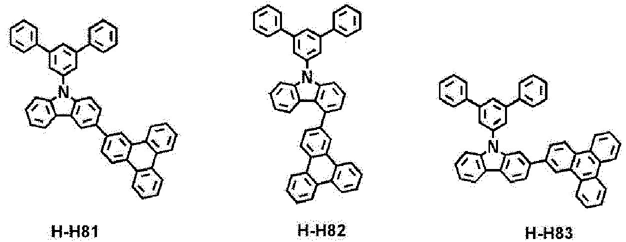

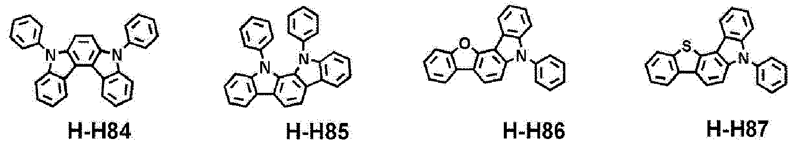

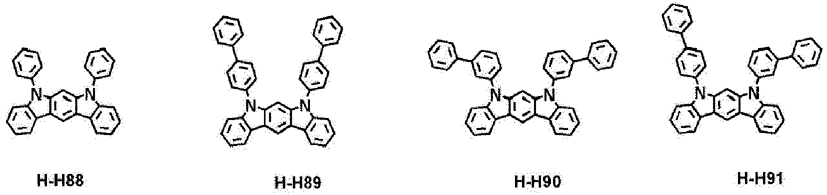

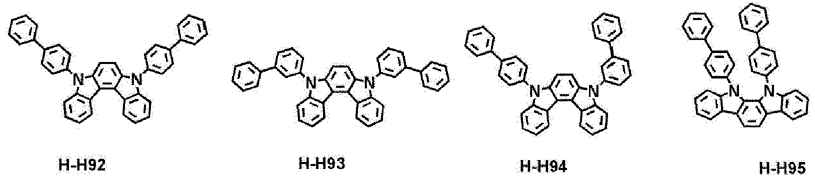

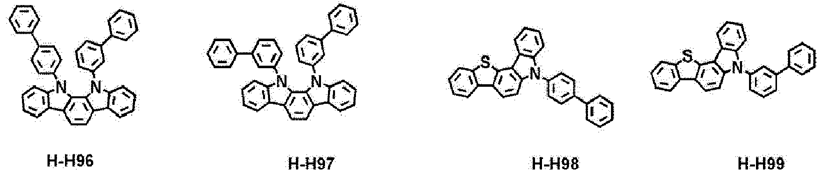

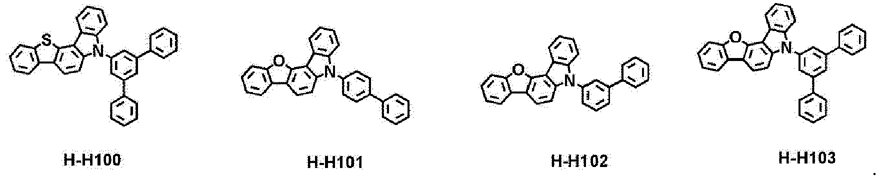

In an embodiment, the bipolar host may be a compound of Group HEH1: Group HEH1

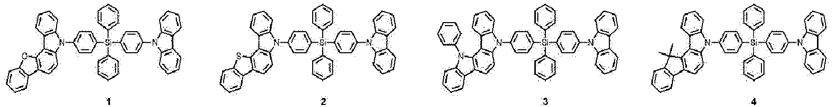

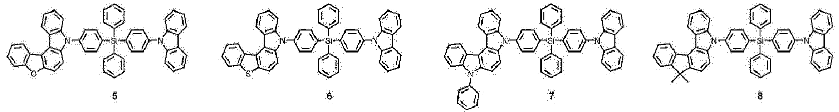

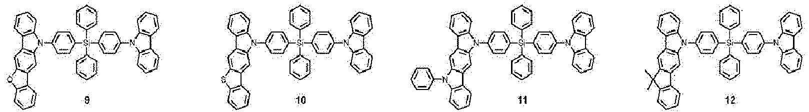

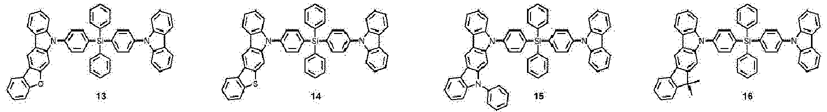

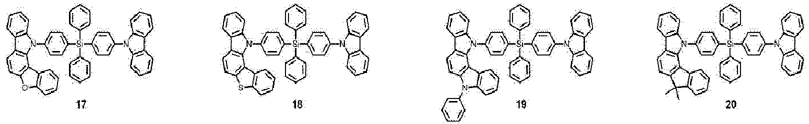

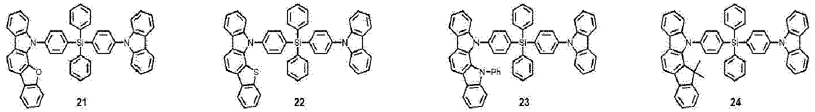

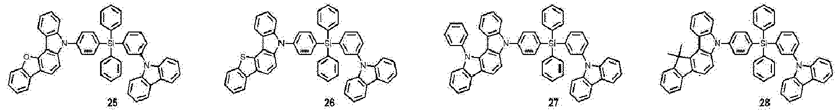

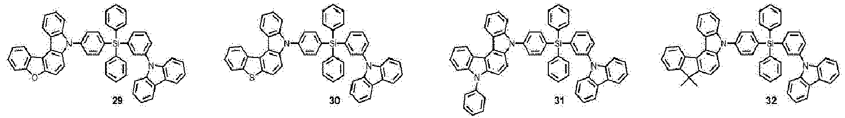

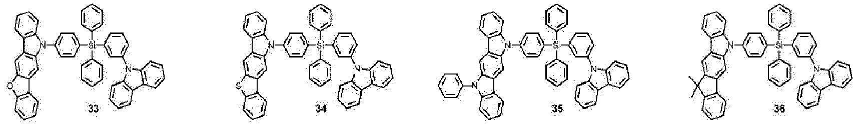

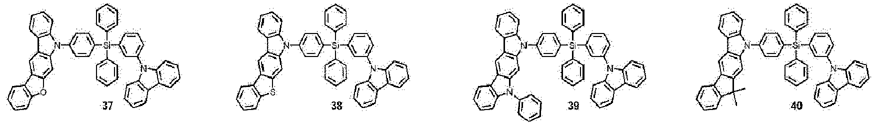

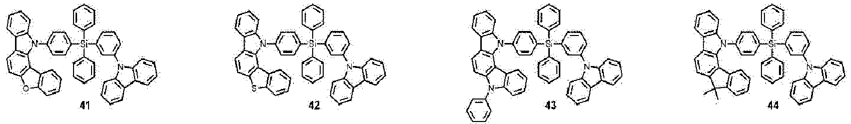

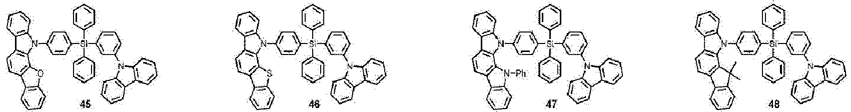

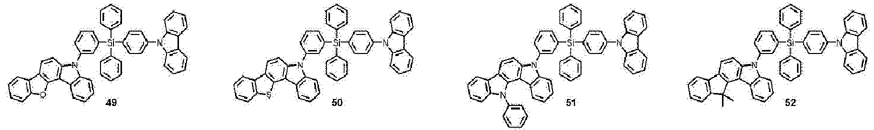

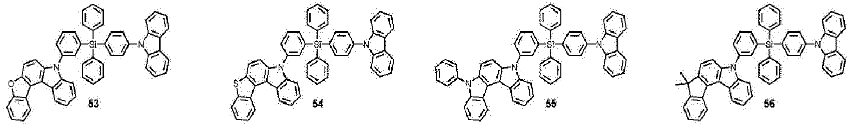

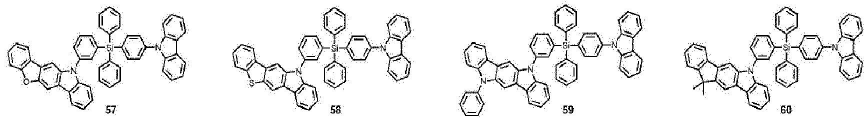

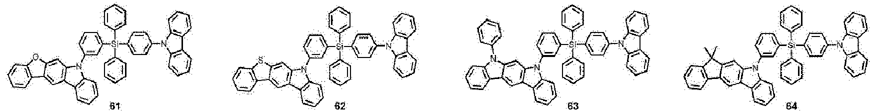

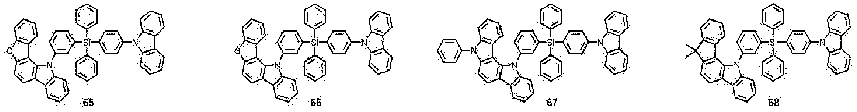

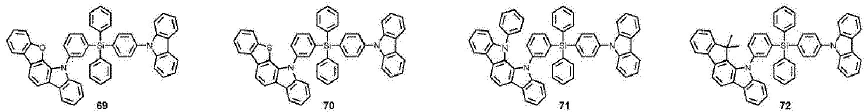

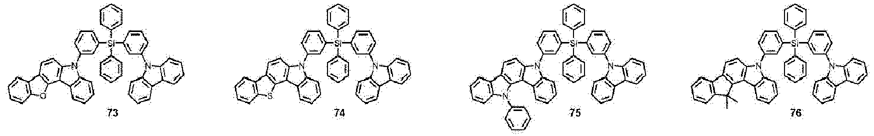

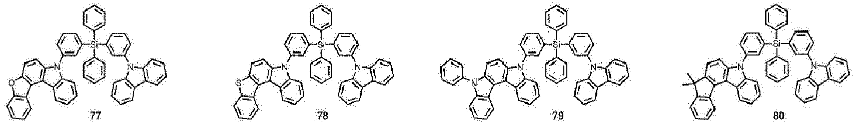

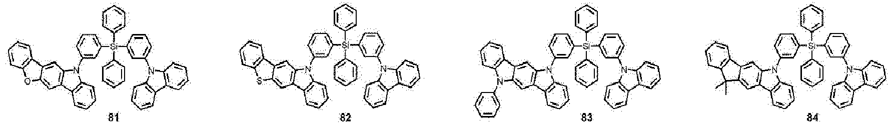

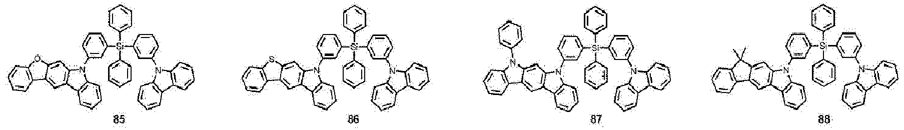

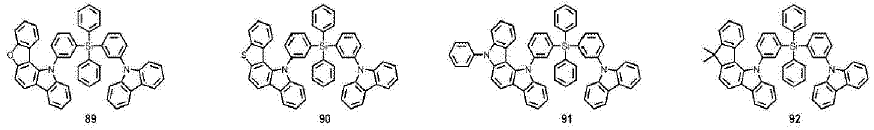

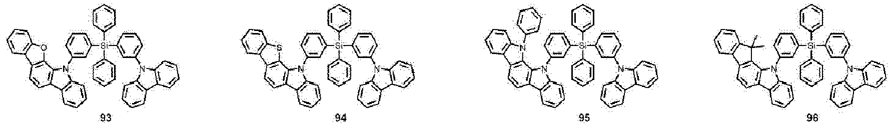

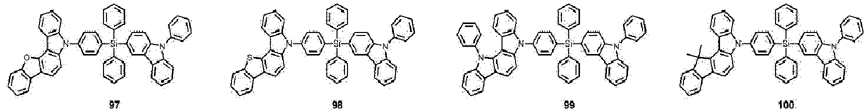

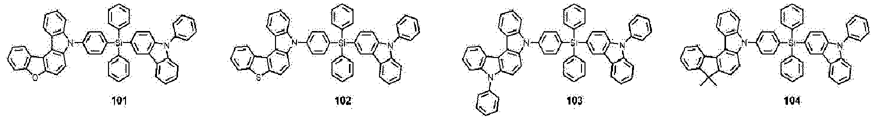

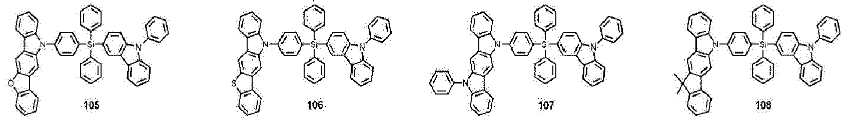

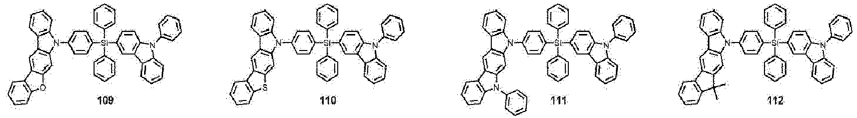

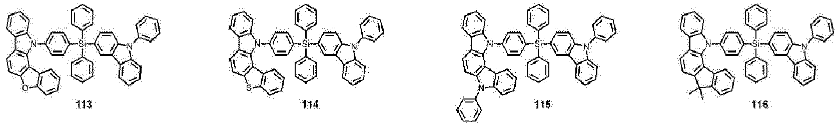

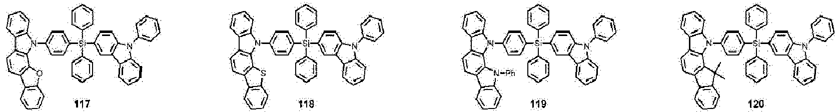

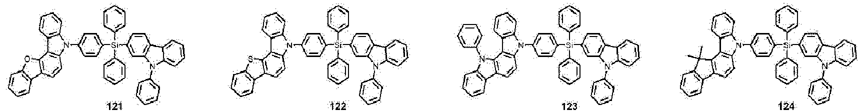

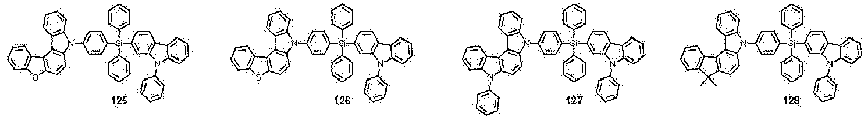

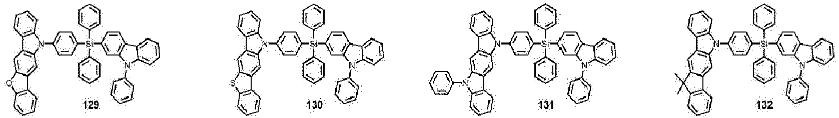

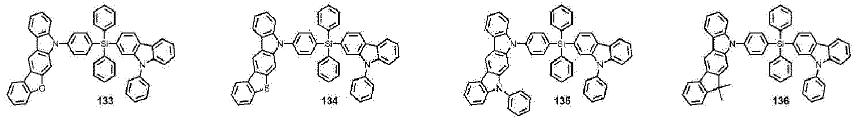

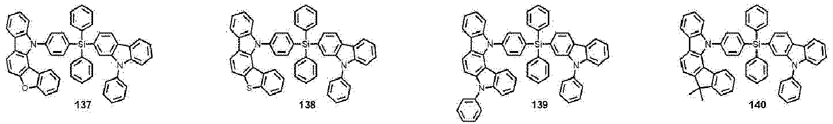

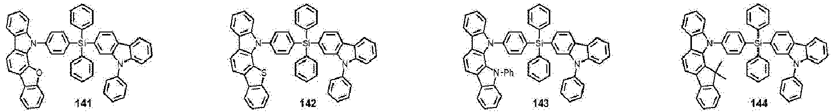

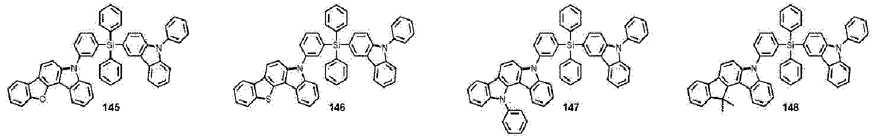

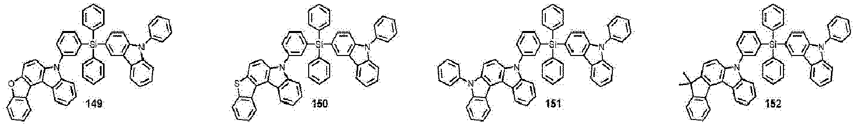

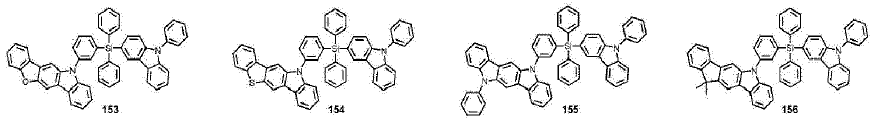

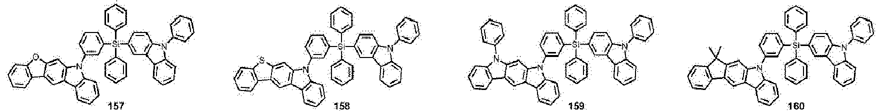

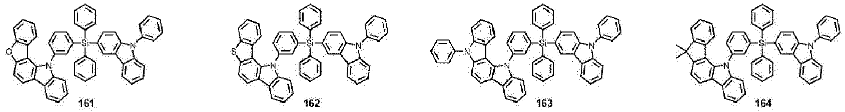

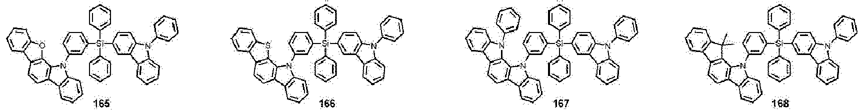

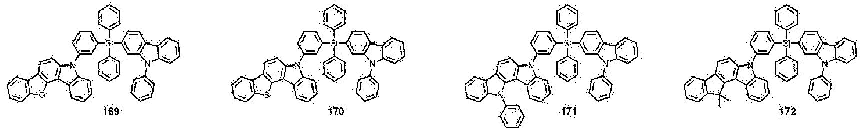

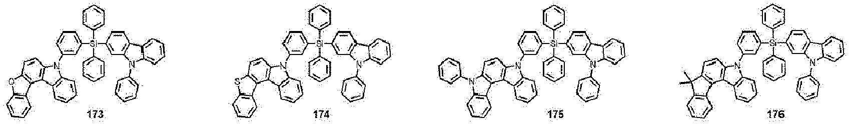

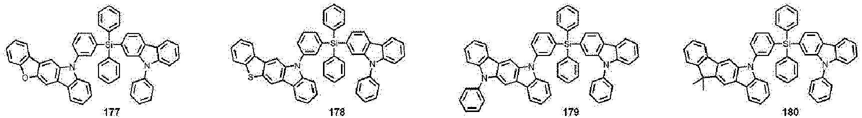

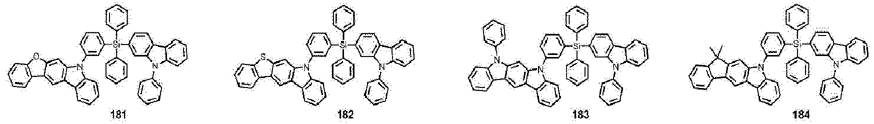

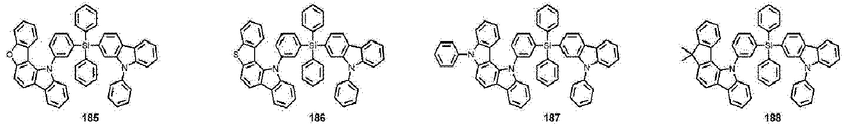

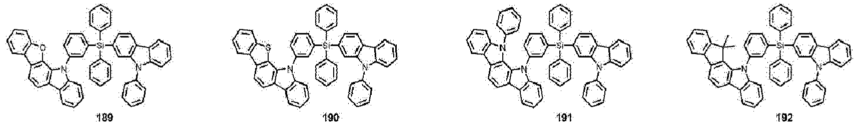

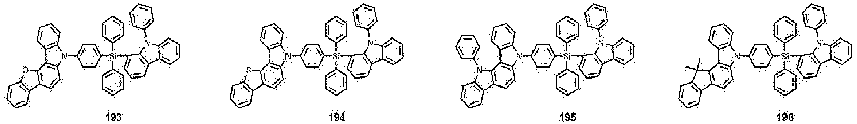

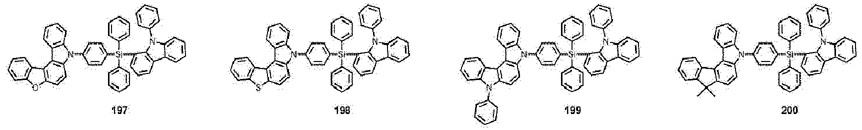

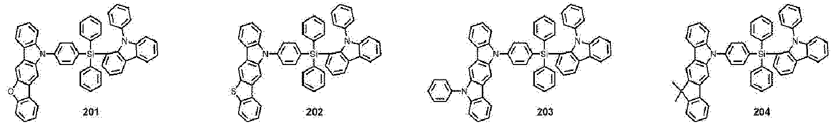

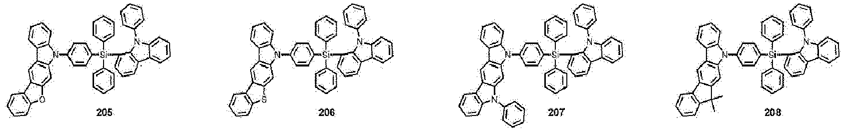

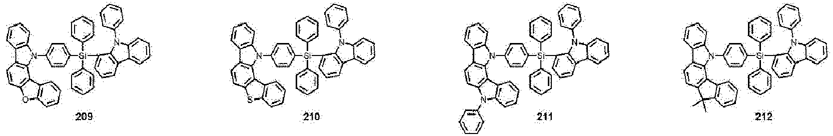

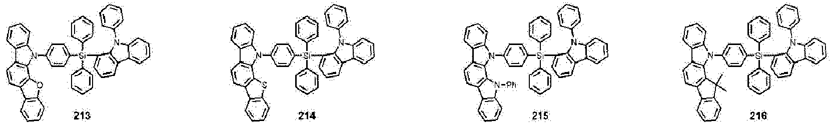

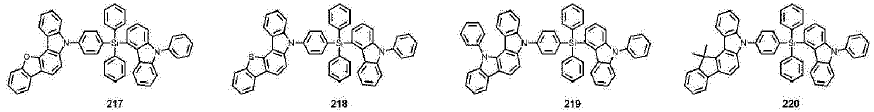

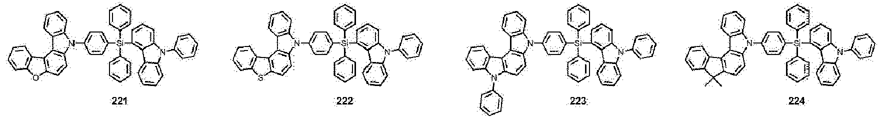

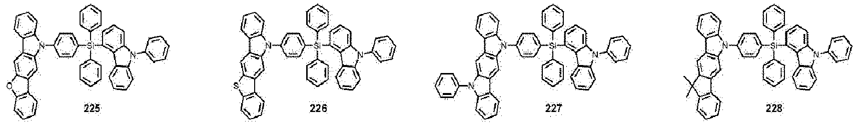

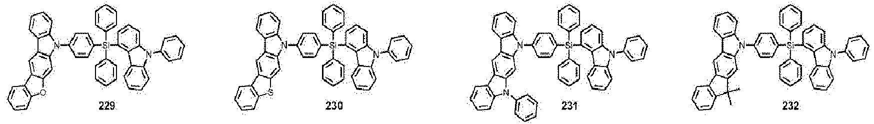

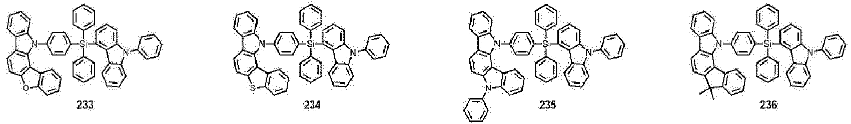

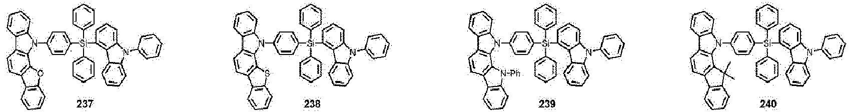

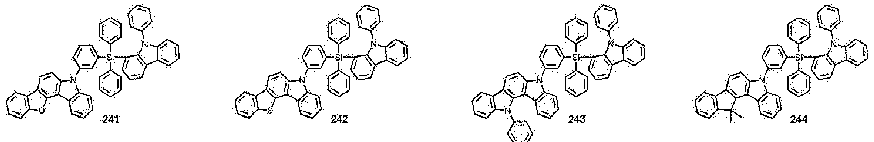

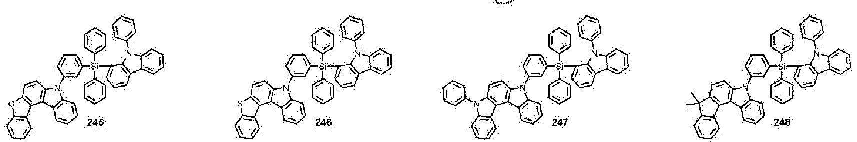

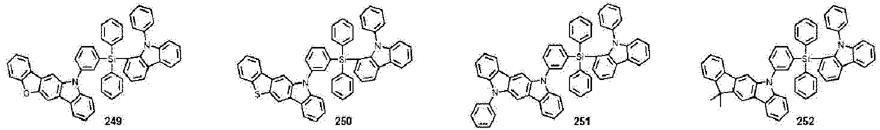

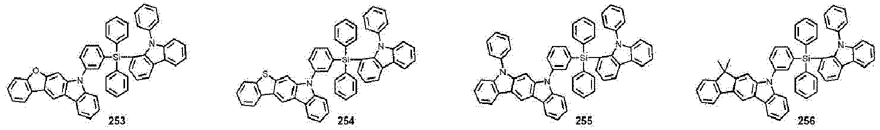

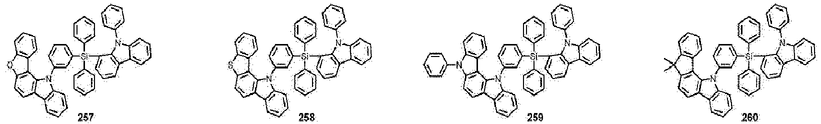

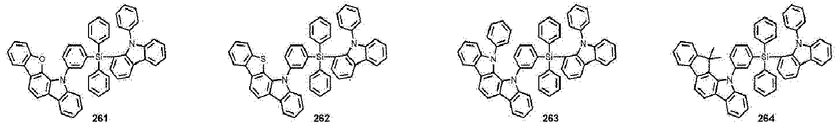

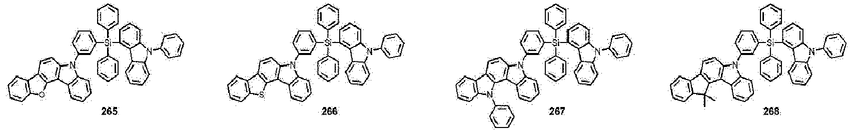

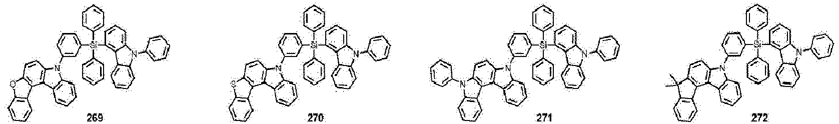

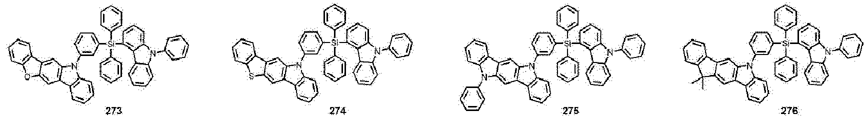

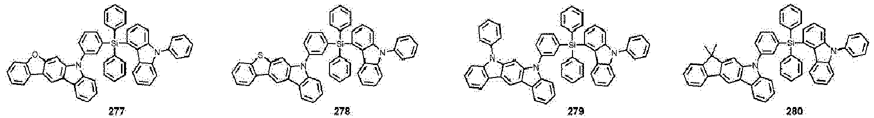

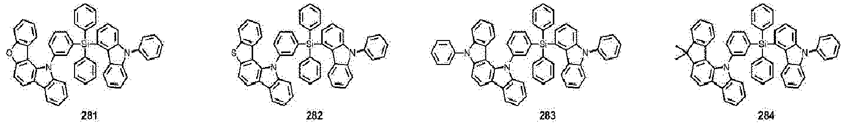

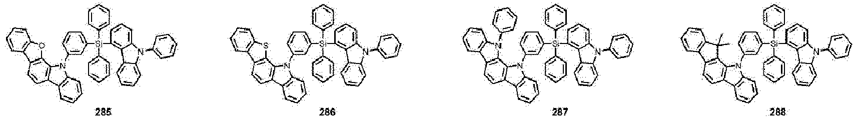

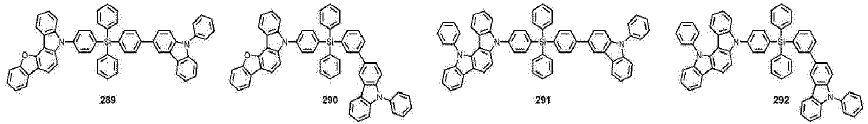

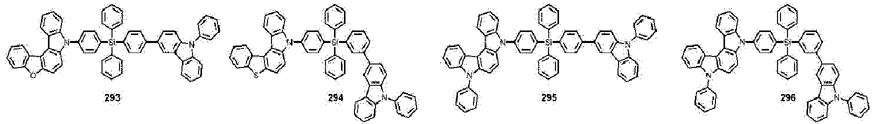

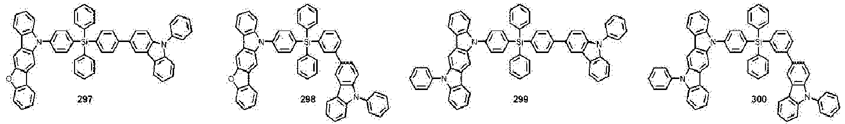

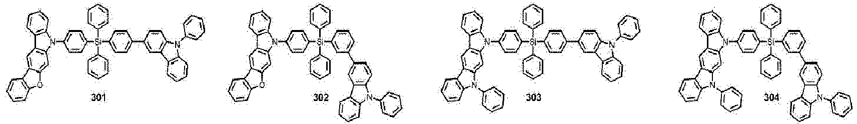

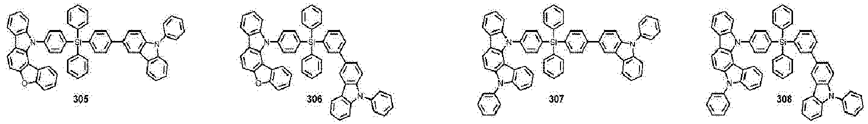

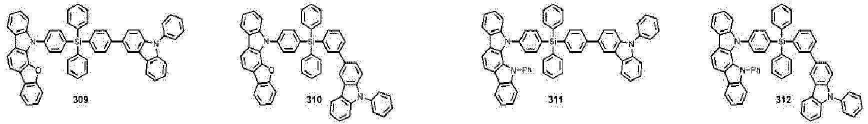

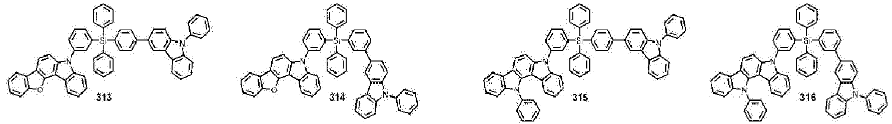

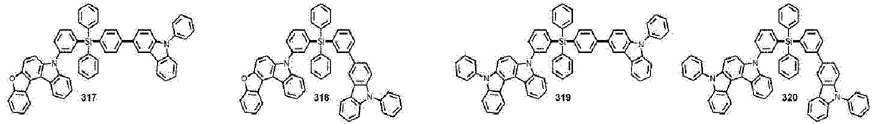

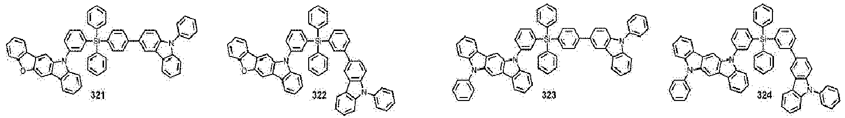

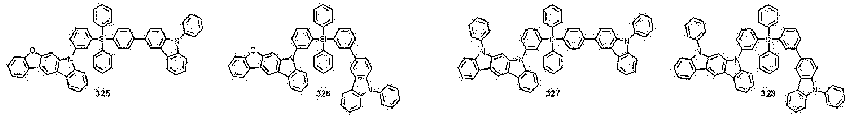

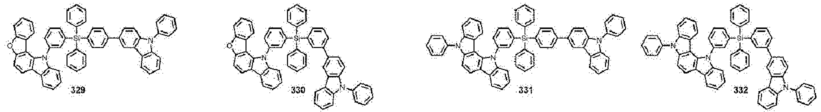

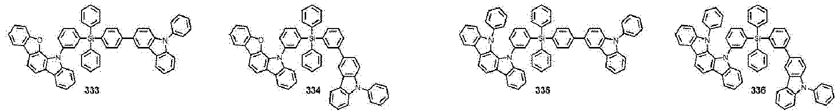

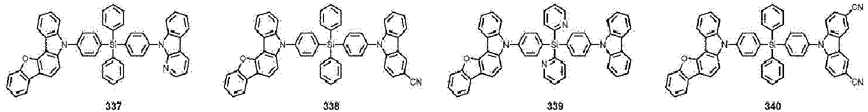

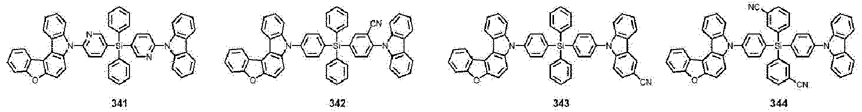

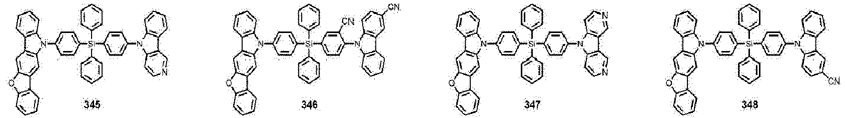

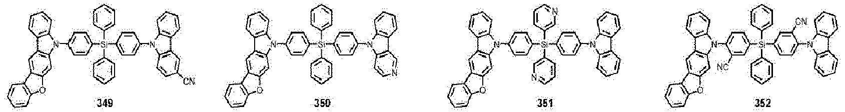

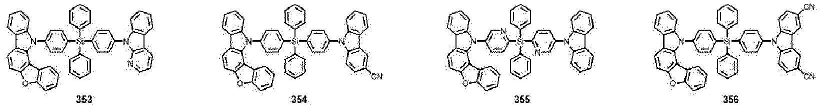

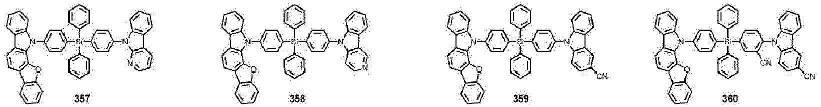

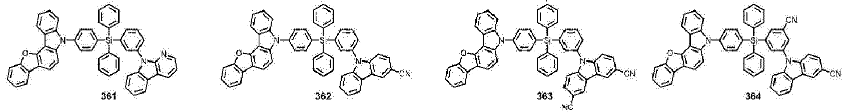

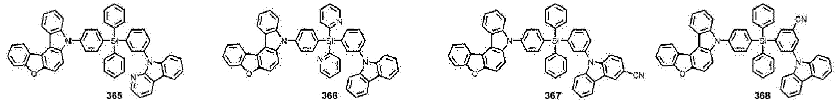

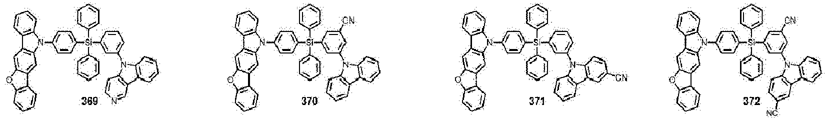

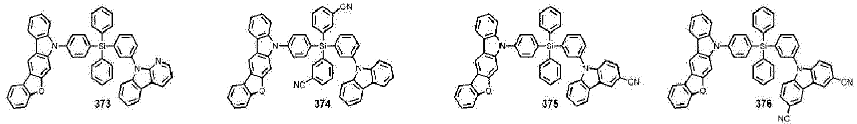

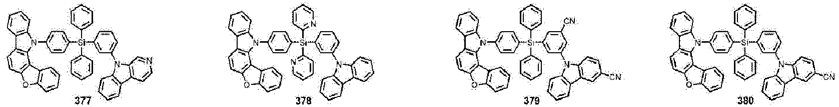

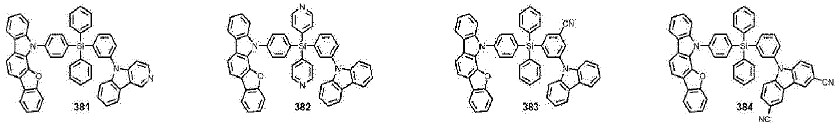

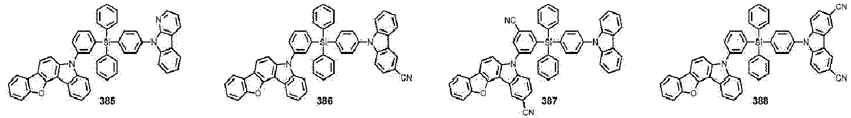

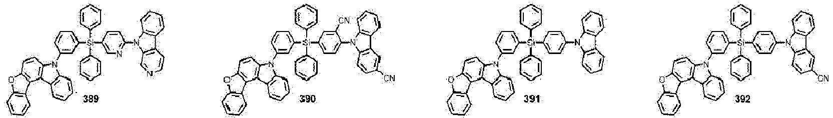

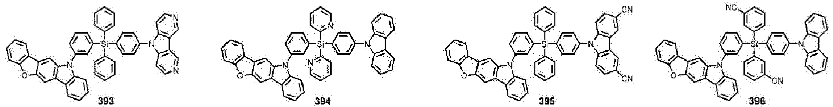

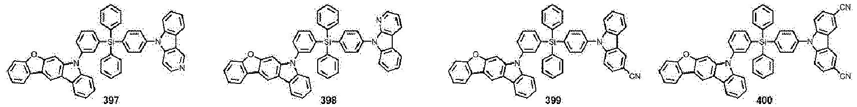

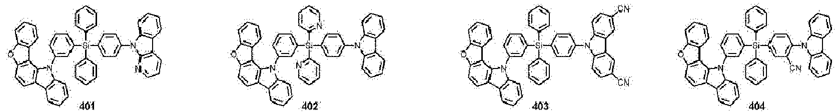

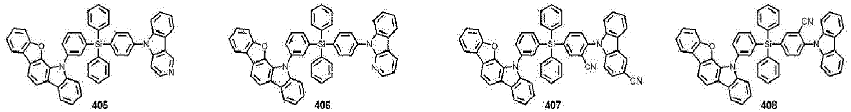

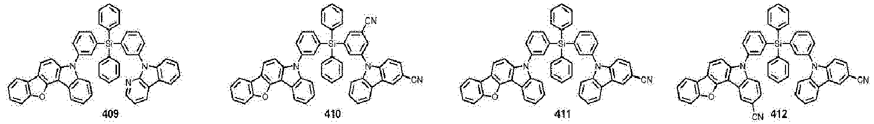

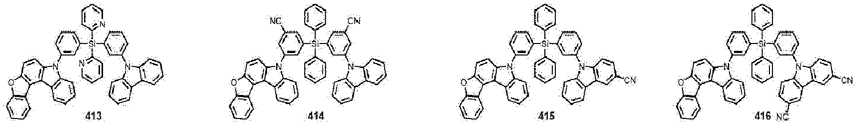

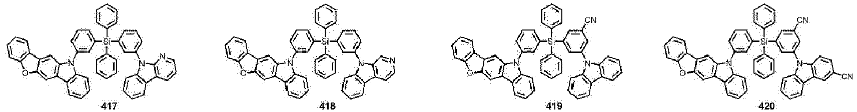

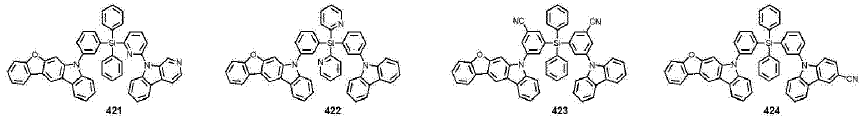

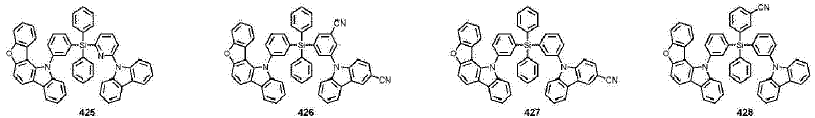

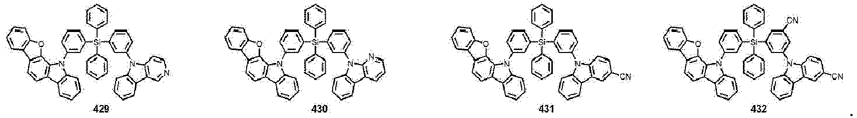

The term "Ph" as used herein is a phenyl group.

In an embodiment, an example of the hole-transporting host may be Compound H1. In an embodiment, an example of the electron-transporting host may be Compound H2:

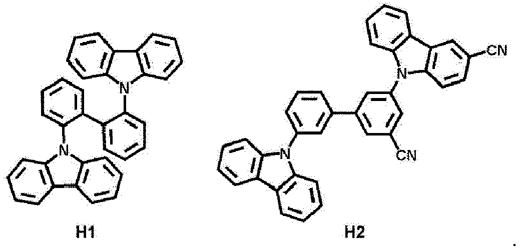

### [Dopant in emission layer 15]

The dopant included in the emission layer 15 may include a phosphorescent dopant, a fluorescent dopant, or any combination thereof.

In an embodiment, the emission layer 15 may include a host, a fluorescent dopant, and a phosphorescent dopant, and the host may include the heterocyclic compound. In this regard, the phosphorescent dopant may be a sensitizer compound that is used together with a fluorescent dopant to transfer excitons to the fluorescent dopant.

In an embodiment, the phosphorescent dopant may be a blue dopant.

### Phosphorescent dopant

In an embodiment, the phosphorescent dopant may include a transition metal and a tetradentate ligand. In one or more embodiments, the phosphorescent dopant may include a transition metal and at least one of a monodentate ligand, a bidentate ligand, and a tridentate ligand.

In one or more embodiments, the phosphorescent dopant may include an organometallic compound represented by Formula 31: 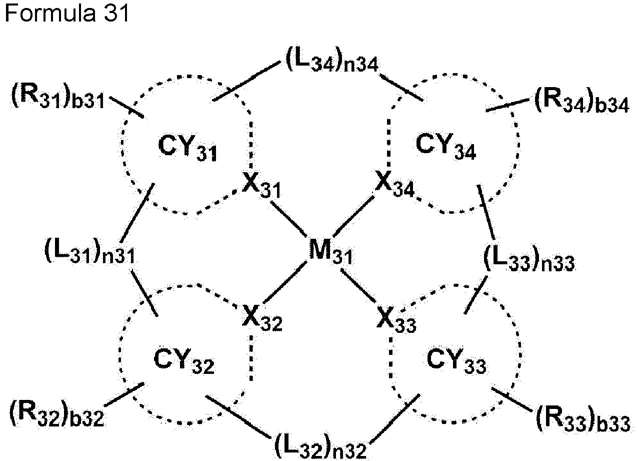 wherein, in Formula 31, M₃₁ may be a transition metal.

In an embodiment, M₃₁ may be Pt, Pd, or Au.

In Formula 31, X₃₁ to X₃₄ may each independently be C or N, and
two of a bond between X₃₁ and M₃₁, a bond between X₃₂ and M₃₁, a bond between X₃₃ and M₃₁, and a bond between X₃₄ and M₃₁ may be coordinate bonds, and the other two may be covalent bonds.

In an embodiment, a bond between X₃₁ and M₃₁ may be a coordinate bond.

In an embodiment, X₃₁ may be C, and a bond between X₃₁ and M₃₁ may be a coordinate bond. That is, X₃₁ in Formula 3 may be C in a carbene moiety.

In Formula 31, CY₃₁ to CY₃₄ may each independently be a C₅-C₃₀ carbocyclic group or a C₁-C₃₀ heterocyclic group.

In an embodiment, CY₃₁ to CY₃₄ may each independently be:
i) a first ring, ii) a second ring, iii) a condensed cyclic group in which two or more first rings are condensed with each other, iv) a condensed cyclic group in which two or more second rings are condensed with each other or v) a condensed cyclic group in which at least one first ring is condensed with at least one second ring,
wherein the first ring may be a cyclopentane group, a cyclopentadiene group, a furan group, a thiophene group, a pyrrole group, a silole group, an oxazole group, an isoxazole group, an oxadiazole group, an isoxadiazole group, an oxatriazole group, an isoxatriazole group, a thiazole group, an isothiazole group, a thiadiazole group, an isothiadiazole group, a thiatriazole group, an isothiatriazole group, a pyrazole group, an imidazole group, a triazole group, a tetrazole group, an azasilole group, a diazasilole group, or a triazasilole group, and
the second ring may be an adamantane group, a norbornane group, a norbornene group, a cyclohexane group, a cyclohexene group, a benzene group, a pyridine group, a pyrimidine group, a pyrazine group, a pyridazine group, a triazine group, an oxazine group, a thiazine group, a dihydropyrazine group, a dihydropyridine group, or a dihydroazasilane group.

In Formula 31, L₃₁ may be a single bond, a double bond, *-N(R₃₅ₐ)-*', *-B(R₃₅ₐ)-*', *-P(R₃₅ₐ)-*', *-C(R₃₅ₐ)(R_{35b})-*', *-Si(R₃₅ₐ)(R_{35b})-*', *-Ge(R₃₅ₐ)(R_{35b})-*', *-S-*', *-Se-*', *-O-*', *-C(=O)-*', *-S(=O)-*', *-S(=O)₂-*', *-C(R₃₅ₐ)=*', *=C(R₃₅ₐ)-*', *-C(R₃₅ₐ)=C(R_{35b})-*', *-C(=S)-*', *-C=C-*', a C₅-C₃₀ carbocyclic group unsubstituted or substituted with at least one R₁₀ₐ, or a C₁-C₃₀ heterocyclic group unsubstituted or substituted with at least one R₁₀ₐ,
L₃₂ may be a single bond, a double bond, *-N(R₃₆ₐ)-*', *-B(R₃₆ₐ)-*', *-P(R₃₆ₐ)-*', *-C(R₃₆ₐ)(R_{36b})-*', *-Si(R₃₆ₐ)(R_{36b})-*', *-Ge(R₃₆ₐ)(R_{36b})-*', *-S-*', *-Se-*', *-O-*', *-C(=O)-*', *-S(=O)-*', *-S(=O)₂-*_{'}, *-C(R₃₆ₐ)=*', *=C(R₃₆ₐ)-*', *-C(R₃₆ₐ)=C(R_{36b})-*', *-C(=S)-*', *-C=C-*', a C₅-C₃₀ carbocyclic group unsubstituted or substituted with at least one R₁₀ₐ, or a C₁-C₃₀ heterocyclic group unsubstituted or substituted with at least one R₁₀ₐ,
L₃₃ may be a single bond, a double bond, *-N(R₃₇ₐ)-*', *-B(R₃₇ₐ)-*', *-P(R₃₇ₐ)-*', *-C(R₃₇ₐ)(R_{37b})-*', *-Si(R₃₇ₐ)(R_{37b})-*', *-Ge(R₃₇ₐ)(R_{37b})-*', *-S-*', *-Se-*', *-O-*', *-C(=O)-*', *-S(=O)-*', *-S(=O)₂-*_{'}, *-C(R₃₇ₐ)=*', *=C(R₃₇ₐ)-*', *-C(R₃₇ₐ)=C(R_{37b})-*', *-C(=S)-*', *-C=C-*', a C₅-C₃₀ carbocyclic group unsubstituted or substituted with at least one R₁₀ₐ, or a C₁-C₃₀ heterocyclic group unsubstituted or substituted with at least one R₁₀ₐ, and
L₃₄ may be a single bond, a double bond, *-N(R₃₈ₐ)-*', *-B(R₃₈ₐ)-*', *-P(R₃₈ₐ)-*', *-C(R₃₈ₐ)(R_{38b})-*', *-Si(R₃₈ₐ)(R_{38b})-*', *-Ge(R₃₈ₐ)(R_{38b})-*', *-S-*', *-Se-*', *-O-*', *-C(=O)-*', *-S(=O)-*', *-S(=O)₂-*', *-C(R₃₈ₐ)=*', *=C(R₃₈ₐ)-*', *-C(R₃₈ₐ)=C(R_{38b})-*', *-C(=S)-*', *-C=C-*', a C₅-C₃₀ carbocyclic group unsubstituted or substituted with at least one R₁₀ₐ, or a C₁-C₃₀ heterocyclic group unsubstituted or substituted with at least one R₁₀ₐ.

In Formula 31, n31 to n34 may each independently be an integer from 0 to 5, wherein three or more of n31 to n34 may each independently be an integer from 1 to 5.

In Formula 31, when n31 is 0, L₃₁ is absent, when n32 is 0, L₃₂ is absent, when n33 is 0, L₃₃ is absent, and when n34 is 0, L₃₄ is absent.

In Formula 31, when n31 is 2 or more, two or more of L₃₁ may be identical to or different from each other, when n32 is 2 or more, two or more of L₃₂ may be identical to or different from each other, when n33 is 2 or more, two or more of L₃₃ may be identical to or different from each other, and when n34 is 2 or more, two or more of L₃₄ may be identical to or different from each other.

In Formula 31, R₃₁ to R₃₄, R₃₅ₐ, R_{35b}, R₃₆ₐ, R_{36b}, R₃₇ₐ, R_{37b}, R₃₈ₐ, and R_{38b} may each independently be hydrogen, deuterium, -F, -Cl, -Br, -I, -SF₅, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₇-C₆₀ arylalkyl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₂-C₆₀ heteroarylalkyl group, a substituted or unsubstituted monovalent non-aromatic condensed polycyclic group, a substituted or unsubstituted monovalent non-aromatic condensed heteropolycyclic group, - N(Q₁)(Q₂), -Si(Q₃)(Q₄)(Q₅), -Ge(Q₃)(Q₄)(Q₅), -B(Q₆)(Q₇), -P(=O)(Q₈)(Q₉), or-P(Q₈)(Q₉).

In an embodiment, R₃₁ to R₃₄, R₃₅ₐ, R_{35b}, R₃₆ₐ, R_{36b}, R₃₇ₐ, R_{37b}, R₃₈ₐ, and R_{38b} may each independently be: hydrogen, deuterium, -F, -Cl, -Br, -I, -CD₃, -CD₂H, -CDH₂, -CF₃, -CF₂H, -CFH₂, a cyano group, a nitro group, an amino group, a C₁-C₂₀ alkyl group, or a C₁-C₂₀ alkoxy group;
a C₁-C₂₀ alkyl group or a C₁-C₂₀ alkoxy group, each substituted with at least one of deuterium, -F, -Cl, -Br, -I, -CD₃, -CD₂H, -CDH₂, -CF₃, -CF₂H, -CFH₂, a cyano group, a nitro group, an amino group, and a phenyl group; or
a cyclopentyl group, a cyclohexyl group, a cycloheptyl group, a cyclopentenyl group, a cyclohexenyl group, a cycloheptenyl group, a phenyl group, a biphenyl group, a naphthyl group, a fluorenyl group, a phenanthrenyl group, or an anthracenyl group, each unsubstituted or substituted with at least one of deuterium, -F, -Cl, -Br, -I, -CD₃, -CD₂H, -CDH₂, -CF₃, -CF₂H, -CFH₂, a cyano group, a nitro group, an amino group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a cycloheptenyl group, a phenyl group, a biphenyl group, a naphthyl group, a fluorenyl group, a phenanthrenyl group, and an anthracenyl group.

In an embodiment, the phosphorescent dopant may include an organometallic compound represented by Formula 31-1 or 31-2:

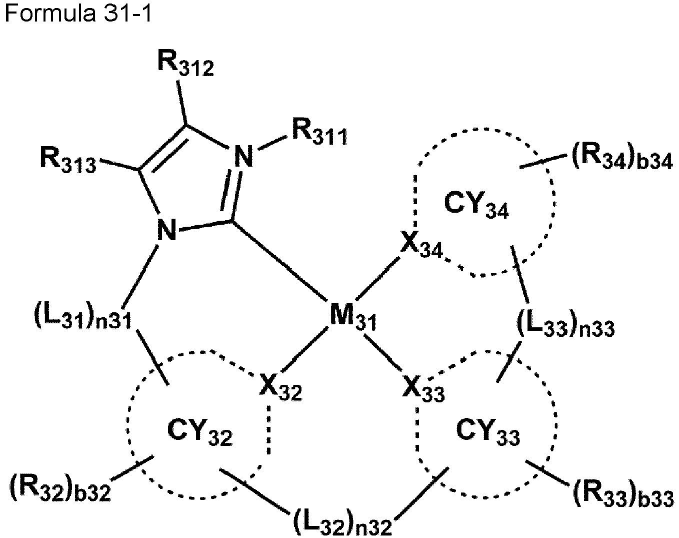

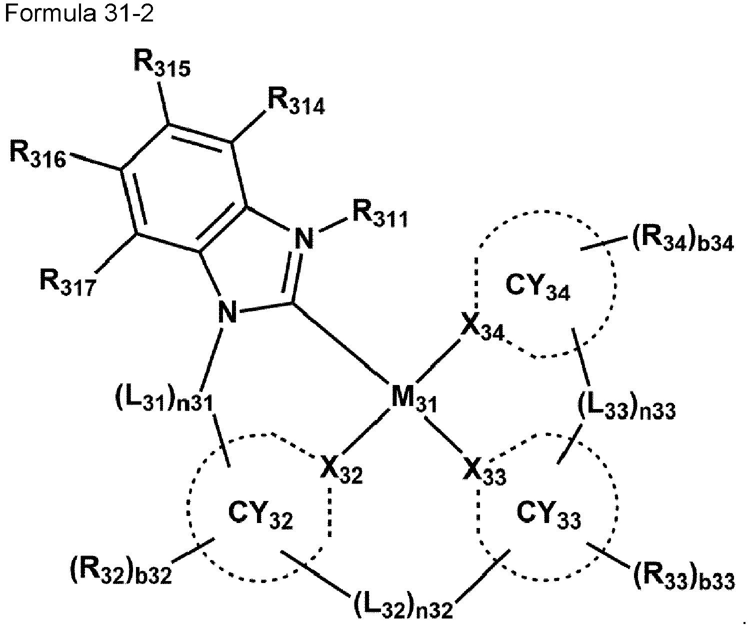

In Formula 31, b31 to b34 may each independently be an integer from 0 to 20.

In Formula 31, at least two of R₃₁(s) in the number of b31 may optionally be bonded to each other to form a C₅-C₃₀ carbocyclic group unsubstituted or substituted with at least one R₁₀ₐ or a C₁-C₃₀ heterocyclic group unsubstituted or substituted with at least one R₁₀ₐ,
at least two of R₃₂(s) in the number of b32 may optionally be bonded to each other to form a C₅-C₃₀ carbocyclic group unsubstituted or substituted with at least one R₁₀ₐ or a C₁-C₃₀ heterocyclic group unsubstituted or substituted with at least one R₁₀ₐ,
at least two of R₃₃(s) in the number of b33 may optionally be bonded to each other to form a C₅-C₃₀ carbocyclic group unsubstituted or substituted with at least one R₁₀ₐ or a C₁-C₃₀ heterocyclic group unsubstituted or substituted with at least one R₁₀ₐ, and
at least two of R₃₄(s) in the number of b34 may optionally be bonded to each other to form a C₅-C₃₀ carbocyclic group unsubstituted or substituted with at least one R₁₀ₐ or a C₁-C₃₀ heterocyclic group unsubstituted or substituted with at least one R₁₀ₐ.

At least two of R₃₁ to R₃₄, R₃₅ₐ, R_{35b}, R₃₆ₐ, R_{36b}, R₃₇ₐ, R_{37b}, R₃₈ₐ, and R_{38b} may optionally be bonded to each other to form a C₅-C₃₀ carbocyclic group unsubstituted or substituted with at least one R₁₀ₐ or a C₁-C₃₀ heterocyclic group unsubstituted or substituted with at least one R₁₀ₐ, and
R₁₀ₐ is the same as described in connection with R₃₁ in the present specification.

In Formulae 31-1 and 31-2,
M₃₁, CY₃₂, CY₃₃, CY₃₄, X₃₂, X₃₃, X₃₄, L₃₁, L₃₂, L₃₃, n31, n32, n33, R₃₂, R₃₃, R₃₄, a32, a33, and a34 are each the same as described herein, and
R₃₁₁ to R₃₁₇ are each independently the same as described in connection with R₃₁.

In an embodiment, in Formulae 31-1 and 31-2, R₃₁₁ to R₃₁₇ may each independently be:
hydrogen, deuterium, -F, -Cl, -Br, -I, -CD₃, -CD₂H, -CDH₂, -CF₃, -CF₂H, -CFH₂, -SF₅, a hydroxyl group, a cyano group, a nitro group, an amidino group, a hydrazine group, a hydrazone group, a carboxylic acid group or a salt thereof, a sulfonic acid group or a salt thereof, or a phosphoric acid group or a salt thereof;
a C₁-C₂₀ alkyl group or a C₁-C₂₀ alkoxy group, each unsubstituted or substituted with deuterium, -F, -Cl, -Br, -I, -CD₃, -CD₂H, -CDH₂, -CF₃, -CF₂H, -CFH₂, - SF₆, a hydroxyl group, a cyano group, a nitro group, an amidino group, a hydrazine group, a hydrazone group, or any combination thereof;
a C₃-C₁₀ cycloalkyl group, a C₁-C₁₀ heterocycloalkyl group, a C₆-C₆₀ aryl group, a C₇-C₆₀ arylalkyl group, C₆-C₆₀ aryloxy group, a C₆-C₆₀ arylthio group, a C₁-C₆₀ heteroaryl group, a substituted or unsubstituted C₂-C₆₀ heteroaryl alkyl group, a monovalent non-aromatic condensed polycyclic group, a monovalent non-aromatic condensed heteropolycyclic group, each unsubstituted or substituted with deuterium, -F, -Cl, -Br, -I, -CD₃, -CD₂H, -CDH₂, -CF₃, -CF₂H, -CFH₂, -SF₅, a hydroxyl group, a cyano group, a nitro group, an amidino group, a hydrazine group, a hydrazone group, a C₁-C₂₀ alkyl group, a C₁-C₂₀ alkoxy group, or any combination thereof; or
-Si(Q₁)(Q₂)(Q₃), -Ge(Q₁)(Q₂)(Q₃), -N(Q₄)(Q₅), -B(Q₆)(Q₇), -P(=O)(Q₈)(Q₉), or - P(Q₈)(Q₉).

For example, in Formulae 31-1 and 31-2, at least one of R₃₁₁ to R₃₁₇ may include
a C₁-C₂₀ alkyl group, a C₆-C₆₀ aryl group, or a C₇-C₆₀ arylalkyl group, each unsubstituted or substituted with at least one of a methyl group, an ethyl group, an n-propyl group, an isopropyl group, an n-butyl group, an isobutyl group, a sec-butyl group, a tert-butyl group, a phenyl group, a cumyl group, or a combination thereof.

In one or more embodiments, the phosphorescent dopant may include an organometallic compound represented by Formula 51:

Formula 51 M₅₁(L₅₁)ₙ₅₁(L₅₂)ₙ₅₂

wherein M₅₁ in Formula 51 may be a transition metal.

In an embodiment, M₅₁ may be a first-row transition metal, a second-row transition metal, or a third-row transition metal of the Periodic Table of Elements.

In one or more embodiments, M₅₁ may be iridium (Ir), platinum (Pt), osmium (Os), titanium (Ti), zirconium (Zr), hafnium (Hf), europium (Eu), terbium (Tb), thulium (Tm), or rhodium (Rh).

In one or more embodiments, M₅₁ may be Ir, Pt, Os, or Rh.

In Formula 51, L₅₁ may be a ligand represented by Formula 51A, and L₅₂ may be a ligand represented by Formula 51B: 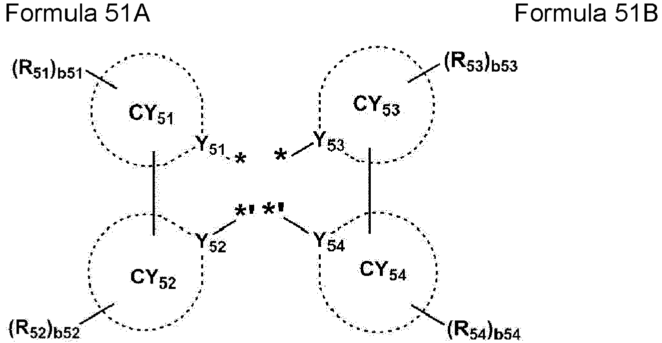 wherein Formulae 51A and 51B are each the same as described herein.

In Formula 51, n51 may be 1, 2, or 3, wherein, when n51 is 2 or more, two or more of L₅₁ may be identical to or different from each other.

In Formula 52, n52 may be 0, 1, or 2, wherein, when n52 is 2 or more, two or more of L₅₂ may be identical to or different from each other.

In Formula 51, the sum of n51 and n52 may be 2 or 3. For example, the sum of n51 and n52 may be 3.

In an embodiment, in Formula 51, i) M₅₁ may be Ir, and the sum of n51+n52 may be 3; or ii) M₅₁ may be Pt, and the sum of n51 +n52 may be 2.

In one or more embodiments, in Formula 51, M₅₁ may be Ir, and regarding n51 and n52, i) n51 may be 1, and n52 may be 2; or ii) n51 may be 2, and n52 may be 1.

In Formula 51, L₅₁ and L₅₂ may be different from each other.

In Formulae 51A and 51B, Y₅₁ to Y₅₄ may each independently be C or N. For example, Y₅₁ and Y₅₃ may each be N, and Y₅₂ and Y₅₄ may each be C.

In Formulae 51A and 51B, CY₅₁ to CY₅₄ may each independently be a C₅-C₃₀ carbocyclic group or a C₁-C₃₀ heterocyclic group.

For example, CY₅₁ to CY₅₄ may each independently be i) a third ring, ii) a fourth ring, iii) a condensed cyclic group in which two or more third rings are condensed with each other, iv) a condensed cyclic group in which two or more fourth rings are condensed with each other, or v) a condensed cyclic group in which at least one third ring is condensed with at least one fourth ring,
wherein the third ring may be a cyclopentane group, a cyclopentene group, a furan group, a thiophene group, a pyrrole group, a silole group, a borole group, a phosphole group, a germole group, a selenophene group, an oxazole group, an oxadiazole group, an oxatriazole group, a thiazole group, a thiadiazole group, a thiatriazole group, a pyrazole group, an imidazole group, a triazole group, a tetrazole group, or an azasilole group, and
the fourth ring may be an adamantane group, a norbornane group, a norbornene group, a cyclohexane group, a cyclohexene group, a benzene group, a pyridine group, a pyrimidine group, a pyrazine group, a pyridazine group, or a triazine group.

In one or more embodiments, in Formulae 51A and 5B, ring CY₁ to ring CY₄ may each independently be a cyclopentane group, a cyclohexane group, a cyclohexene group, a benzene group, a naphthalene group, an anthracene group, a phenanthrene group, a triphenylene group, a pyrene group, a chrysene group, a 1,2,3,4-tetrahydronaphthalene group, a cyclopentadiene group, a pyrrole group, a furan group, a thiophene group, a silole group, a borole group, a phosphole group, a germole group, a selenophene group, an indene group, an indole group, a benzofuran group, a benzothiophene group, a benzosilole group, a benzoborole group, a benzophosphole group, a benzogermole group, a benzoselenophene group, a fluorene group, a carbazole group, a dibenzofuran group, a dibenzothiophene group, a dibenzosilole group, a dibenzoborole group, a dibenzophosphole group, a dibenzogermole group, a dibenzoselenophene group, a benzofluorene group, a benzocarbazole group, a naphthobenzofuran group, a naphthobenzothiophene group, a naphthobenzosilole group, a naphthobenzoborole group, a naphthobenzophosphole group, a naphthobenzogermole group, a naphthobenzoselenophene group, a dibenzofluorene group, a dibenzocarbazole group, a dinaphthofuran group, a dinaphthothiophene group, a dinaphthosilole group, a dinaphthoborole group, a dinaphthophosphole group, a dinaphthogermole group, a dinaphthoselenophene group, an indenophenanthrene group, an indolophenanthrene group, a phenanthrobenzofuran group, a phenanthrobenzothiophene group, a phenanthrobenzosilole group, a phenanthrobenzoborole group, a phenanthrobenzophosphole group, a phenanthrobenzogermole group, a phenanthrobenzoselenophene group, a dibenzothiophene 5-oxide group, a 9H-fluorene-9-one group, a dibenzothiophene group, a 5,5-dioxide group, an azaindene group, an azaindole group, an azabenzofuran group, an azabenzothiophene group, an azabenzosilole group, an azabenzoborole group, an azabenzophosphole group, an azabenzogermole group, an azabenzoselenophene group, an azafluorene group, an azacarbazole group, an azadibenzofuran group, an azadibenzothiophene group, an azadibenzosilole group, an azadibenzoborole group, an azadibenzophosphole group, an azadibenzogermole group, an azadibenzoselenophene group, an azabenzofluorene group, an azabenzocarbazole group, an azanaphthobenzofuran group, an azanaphthobenzothiophene group, an azanaphthobenzosilole group, an azanaphthobenzoborole group, an azanaphthobenzophosphole group, an azanaphthobenzogermole group, an azanaphthobenzoselenophene group, an azadibenzofluorene group, an azadibenzocarbazole group, an azadinaphthofuran group, an azadinaphthothiophene group, an azadinaphthosilole group, an azadinaphthoborole group, an azadinaphthophosphole group, an azadinaphthogermole group, an azadinaphthoselenophene group, an azaindenophenanthrene group, an azaindolophenanthrene group, an azaphenanthrobenzofuran group, an azaphenanthrobenzothiophene group, an azaphenanthrobenzosilole group, an azaphenanthrobenzoborole group, an azaphenanthrobenzophosphole group, an azaphenanthrobenzogermole group, an azaphenanthrobenzoselenophene group, an azadibenzothiophene 5-oxide group, an aza9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a benzoquinoline group, a benzoisoquinoline group, a benzoquinoxaline group, a benzoquinazoline group, a phenanthroline group, a phenantridine group, a pyrazole group, an imidazole group, a triazole group, an oxazole group, an isoxazole group, a thiazole group, an isothiazole group, an oxadiazole group, a thiadiazole group, an azasilole group, an azaborole group, an azaphosphole group, an azagermole group, an azaselenophene group, a benzopyrrole group, a benzopyrazole group, a benzimidazole group, a benzoxazole group, a benzisoxazole group, a benzothiazole group, a benzisothiazole group, a benzioxadiazole group, a benzothiadiazole group, a pyridinopyrrole group, a pyridinopyrazole group, a pyridinoimidazole group, a pyridinoxazole group, a pyridinoisoxazole group, a pyridinothiazole group, a pyridinoisothiazole group, a pyridinoxadiazole group, a pyridinothiadiazole group, a pyrimidinopyrrole group, a pyrimidinopyrazole group, a pyrimidinoimidazole group, a pyrimidinoxazole group, a pyrimidinoisoxazole group, a pyrimidinothiazole group, a pyrimidinoisothiazole group, a pyrimidinoxadiazole group, a pyrimidinothiadiazole group, a 5,6,7,8-tetrahydroisoquinoline group, a 5,6,7,8-tetrahydroquinoline group, an adamantane group, a norbornane group, a norbornene group, a benzene group condensed with a cyclohexane group, a benzene group condensed with a norbornane group, a pyridine group condensed with a cyclohexane group, or a pyridine group condensed with a norbornane group.

In an embodiment, CY₅₁ and CY₅₃ may be different from each other.

In one or more embodiments, CY₅₂ and CY₅₄ may be different from each other.

In one or more embodiments, CY₅₁ to CY₅₄ may be different from each other.

In an embodiment, in Formulae 51A and 51B, R₅₁ to R₅₄ may each independently be hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₁-C₆₀ alkylthio group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -Ge(Q₁)(Q₂)(Q₃), -N(Q₄)(Q₅), - B(Q₆)(Q₇), -P(=O)(Q₈)(Q₉), or -P(Q₈)(Q₉). Q₁ to Q₉ are each the same as described herein.

In an embodiment, in Formulae 51A and 51B, R₅₁ to R₅₄ may each independently be:
hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, a C₁-C₂₀ alkoxy group, or a C₁-C₂₀ alkylthio group;
a C₁-C₂₀ alkyl group, a C₁-C₂₀ alkoxy group, or a C₁-C₂₀ alkylthio group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cyclooctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a pyridinyl group, a pyrimidinyl group, or any combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, or an azadibenzothiophenyl group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a (phenyl)C₁-C₁₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.2]octyl group, a (C₁-C₂₀ alkyl)cyclopentyl group, a (C₁-C₂₀ alkyl)cyclohexyl group, a (C₁-C₂₀ alkyl)cycloheptyl group, a (C₁-C₂₀ alkyl)cyclooctyl group, a (C₁-C₂₀ alkyl)adamantanyl group, a (C₁-C₂₀ alkyl)norbornanyl group, a (C₁-C₂₀ alkyl)norbornenyl group, a (C₁-C₂₀ alkyl)cyclopentenyl group, a (C₁-C₂₀ alkyl)cyclohexenyl group, a (C₁-C₂₀ alkyl)cycloheptenyl group, a (C₁-C₂₀ alkyl)bicyclo[1.1.1]pentyl group, a (C₁-C₂₀ alkyl)bicyclo[2.1.1]hexyl group, a (C₁-C₂₀ alkyl)bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, a benzoisothiazolyl group, a benzoxazolyl group, a benzoisoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, an azacarbazolyl group, an azadibenzofuranyl group, an azadibenzothiophenyl group, or any combination thereof; or
-Ge(Q₁)(Q₂)(Q₃), -N(Q₄)(Q₅), -B(Q₆)(Q₇), -P(=O)(Q₈)(Q₉), or -P(Q₈)(Q₉), and
Q₁ to Q₉ may each independently be:
   -CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂; or
   an n-propyl group, an isopropyl group, an n-butyl group, a sec-butyl group, an isobutyl group, a tert-butyl group, an n-pentyl group, a tert-pentyl group, an neopentyl group, an isopentyl group, a see-pentyl group, a 3-pentyl group, a see-isopentyl group, a phenyl group, a biphenyl group, or a naphthyl group, each unsubstituted or substituted with deuterium, a C₁-C₁₀ alkyl group, a phenyl group, or any combination thereof.

In one or more embodiments, R₅₁ to R₅₄ may each independently be:
hydrogen, deuterium, -F, or a cyano group;
a C₁-C₂₀ alkyl group unsubstituted or substituted with deuterium, a cyano group, a C₃-C₁₀ cycloalkyl group, a deuterated C₃-C₁₀ cycloalkyl group, a fluorinated C₃-C₁₀ cycloalkyl group, a (C₁-C₂₀ alkyl)C₃-C₁₀ cycloalkyl group, a C₁-C₁₀ heterocycloalkyl group, a deuterated C₁-C₁₀ heterocycloalkyl group, a fluorinated C₁-C₁₀ heterocycloalkyl group, a (C₁-C₂₀ alkyl)C₁-C₁₀ heterocycloalkyl group, a phenyl group, a deuterated a phenyl group, a fluorinated a phenyl group, a (C₁-C₂₀ alkyl)phenyl group, a biphenyl group, a deuterated a biphenyl group, a fluorinated a biphenyl group, a (C₁-C₂₀ alkyl)biphenyl group, a dibenzofuranyl group, a deuterated a dibenzofuranyl group, a fluorinated a dibenzofuranyl group, a (C₁-C₂₀ alkyl)dibenzofuranyl group, a dibenzothiophenyl group, a deuterated a dibenzothiophenyl group, a fluorinated a dibenzothiophenyl group, a (C₁-C₂₀ alkyl)dibenzothiophenyl group, or any combination thereof;
a C₃-C₁₀ cycloalkyl group, a C₁-C₁₀ heterocycloalkyl group, a phenyl group, or a biphenyl group, each unsubstituted or substituted with deuterium, a cyano group, a C₁-C₂₀ alkyl group, a deuterated C₁-C₂₀ alkyl group, fluorinated C₁-C₂₀ alkyl group, a C₁-C₂₀ alkoxy group, a deuterated C₁-C₂₀ alkoxy group, a fluorinated C₁-C₂₀ alkoxy group, a C₃-C₁₀ cycloalkyl group, a deuterated C₃-C₁₀ cycloalkyl group, a fluorinated C₃-C₁₀ cycloalkyl group, a (C₁-C₂₀ alkyl)C₃-C₁₀ cycloalkyl group, a C₁-C₁₀ heterocycloalkyl group, a deuterated C₁-C₁₀ heterocycloalkyl group, a fluorinated C₁-C₁₀ heterocycloalkyl group, a (C₁-C₂₀ alkyl)C₁-C₁₀ heterocycloalkyl group, a phenyl group, a deuterated phenyl group, a fluorinated a phenyl group, a (C₁-C₂₀ alkyl)phenyl group, a biphenyl group, a deuterated biphenyl group, a fluorinated a biphenyl group, a (C₁-C₂₀ alkyl)biphenyl group, a dibenzofuranyl group, a deuterated dibenzofuranyl group, a fluorinated a dibenzofuranyl group, a (C₁-C₂₀ alkyl)dibenzofuranyl group, a dibenzothiophenyl group, a deuterated dibenzothiophenyl group, a fluorinated a dibenzothiophenyl group, a (C₁-C₂₀ alkyl)dibenzothiophenyl group, or any combination thereof; or
-Si(Q₃)(Q₄)(Q₅) or -Ge(Q₃)(Q₄)(Q₅).

In Formulae 51A and 51B, b51 to b54 indicate the number of R₅₁(s) to R₅₄(s), respectively, and may each independently be an integer from 0 to 20. When b51 is 2 or more, two or more of R₅₁ may be identical to or different from each other, when b52 is 2 or more, two or more of R₅₂ may be identical to or different from each other, when b53 is 2 or more, two or more of R₅₃ may be identical to or different from each other, and when b54 is 2 or more, two or more of R₅₄ may be identical to or different from each other. For example, b51 to b54 may each independently be an integer from 0 to 8.

In an embodiment, the phosphorescent dopant may include one of Compounds P1 to P52:

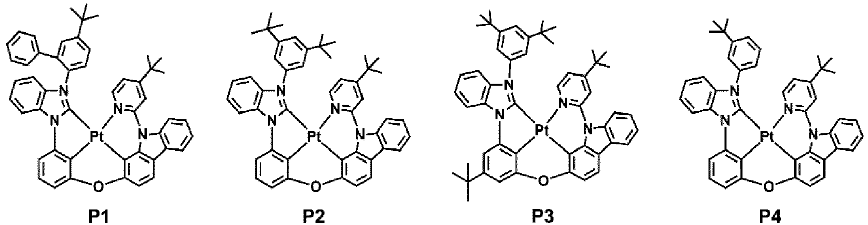

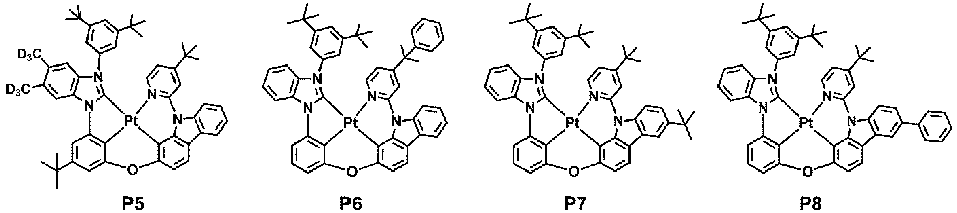

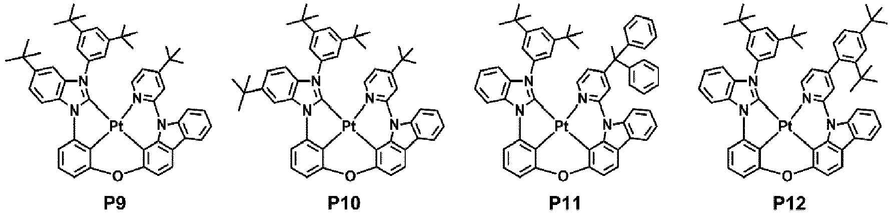

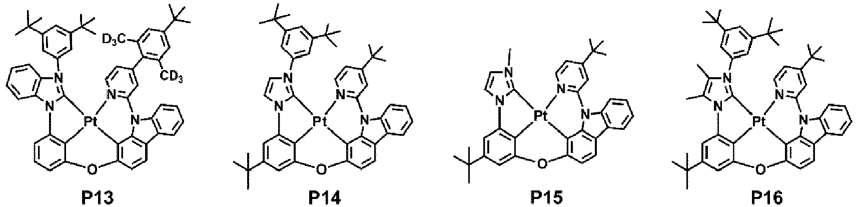

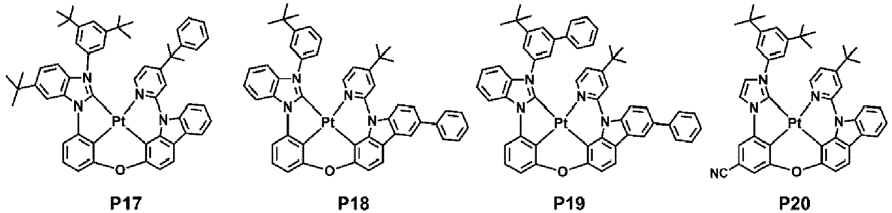

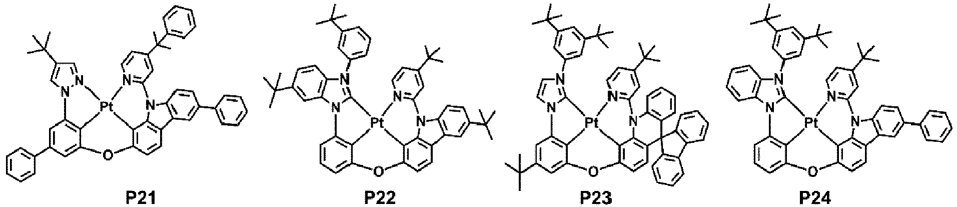

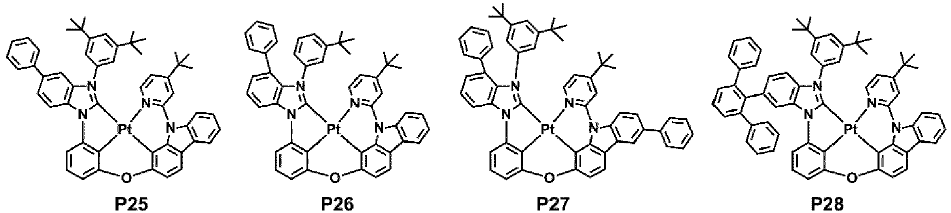

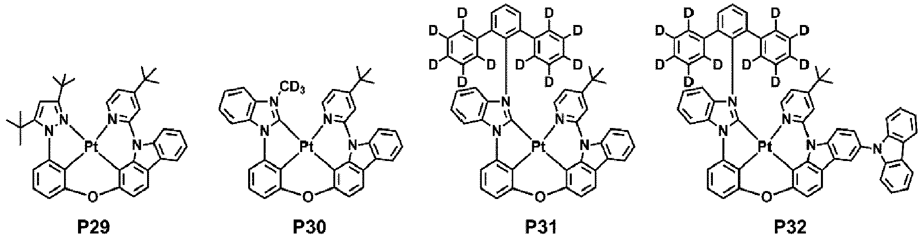

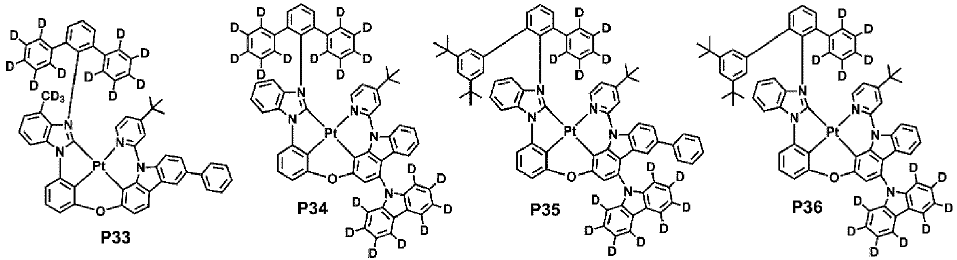

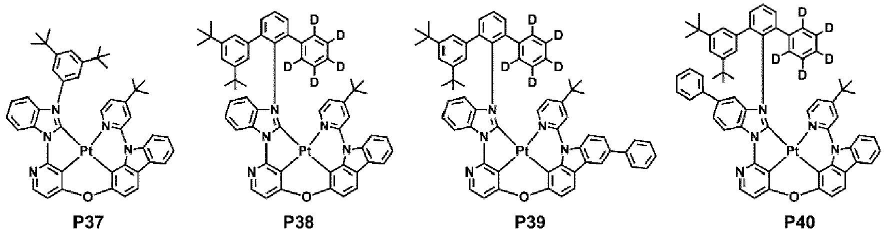

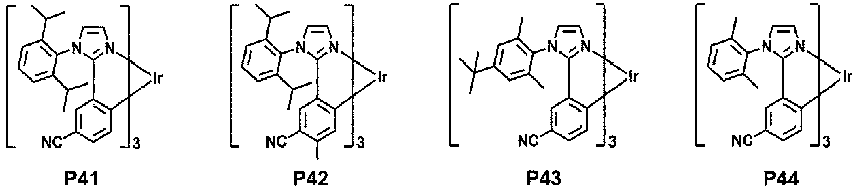

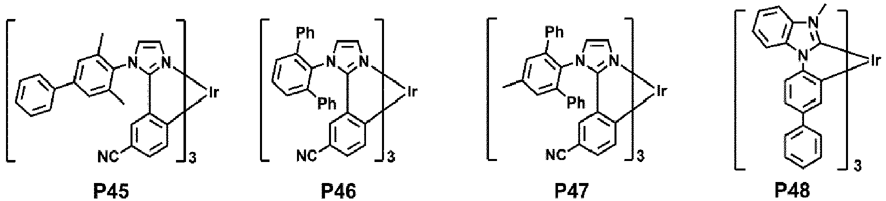

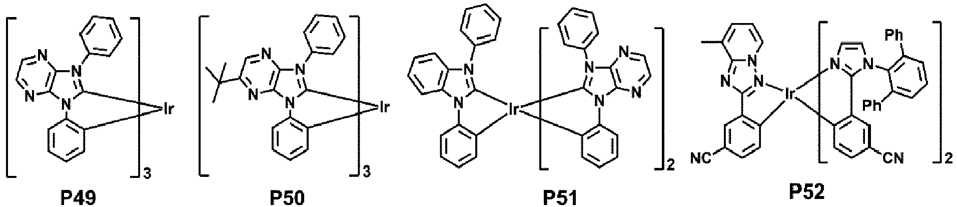

When the phosphorescent dopant is selected from Compounds P1 to P52, exciplex formation with the aforementioned host compound may be facilitated. For example, the phosphorescent dopant may have, by including bulky substituents (e.g., a tert-butyl group, a cumyl group, etc.), an energy level close to that of the host compound, and thus exciplex formation may be facilitated. In the case of the phosphorescent dopant, a gap between a LUMO level of the electron-transporting host and a HOMO level of the phosphorescent dopant may be reduced, thereby facilitating the exciplex formation.

### Fluorescent dopant

In an embodiment, the emission layer 15 may further include a fluorescent dopant. For example, the fluorescent dopant may be a thermally activated delayed fluorescence dopant and a blue dopant.

The fluorescent dopant may have a difference between a triplet energy level and a singlet energy level of less than or equal to 0.4 eV.

For example, the fluorescent dopant may be a thermally activated delayed fluorescence dopant and a blue dopant.

In an embodiment, the fluorescent dopant may be a luminescence emitter that may emit light by receiving excitons from the exciplex of the host and the phosphorescent dopant according to an embodiment so that the received excitons transition to a ground state.

In an embodiment, the fluorescent dopant may be a compound represented by Formula 41: 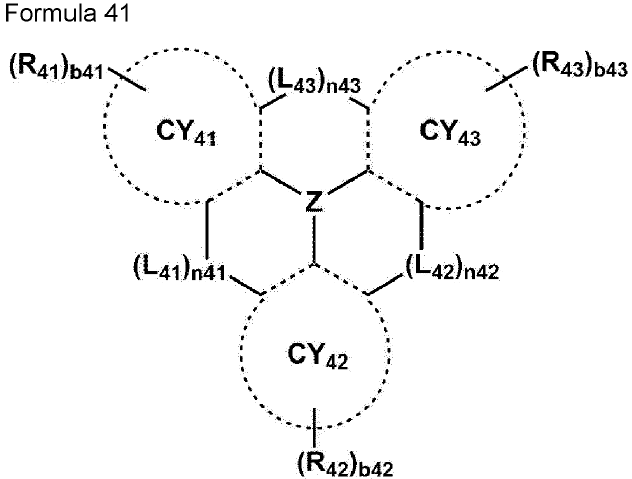
wherein, in Formula 41,
Z may be B or N,
CY₄₁ to CY₄₃ may each independently be a C₅-C₃₀ carbocyclic group or a C₁-C₃₀ heterocyclic group,
L₄₁ may be *-N(R₄₄)-*', *-B(R₄₄)-*', *-P(R₄₄)-*', *-C(R₄₄)(R₄₅)-*', *-Si(R₄₄)(R₄₅)-*', *-Ge(R₄₄)(R₄₅)-*', *-O-*', *-S-*', *-Se-*', *-C(=O)-*', or *-S(=O)₂-*',
L₄₂ may be *-N(R₄₆)-*', *-B(R₄₆)-*', *-P(R₄₆)-*', *-C(R₄₆)(R₄₇)-*', *-Si(R₄₆)(R₄₇)-*', *-Ge(R₄₆)(R₄₇)-*', *-O-*', *-S-*', *-Se-*', *-C(=O)-*', or *-S(=O)₂-*',
L₄₃ may be *-N(R₄₈)-*', *-B(R₄₈)-*', *-P(R₄₈)-*', *-C(R₄₈)(R₄₉)-*', *-Si(R₄₈)(R₄₉)-*', *-Ge(R₄₈)(R₄₉)-*', *-O-*', *-S-*', *-Se-*', *-C(=O)-*', or *-S(=O)₂-*',
n41 to n43 may each independently be 0 or 1,
when n41 is 0, L₄₁ is absent, when n42 is 0, L₄₂ is absent, and when n43 is 0, L₄₃ is absent,
R₄₁ to R₄₉ may each independently be hydrogen, deuterium, -F, -Cl, -Br, -I, - SF₅, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₇-C₆₀ arylalkyl group, a substituted or unsubstituted C₆-C₆₀ aryloxy group, a substituted or unsubstituted C₆-C₆₀ arylthio group, a substituted or unsubstituted C₂-C₆₀ heteroarylalkyl group, a substituted or unsubstituted C₆-C₆₀ aryl group, a substituted or unsubstituted monovalent non-aromatic condensed polycyclic group, a substituted or unsubstituted monovalent non-aromatic condensed heteropolycyclic group, - Si(Q₁)(Q₂)(Q₃), -Ge(Q₁)(Q₂)(Q₃), -N(Q₄)(Q₅), -B(Q₆)(Q₇), -P(=O)(Q₈)(Q₉), or-P(Q₈)(Q₉),
b41 to b43 may each independently be an integer from 1 to 20,
at least two of R₄₁(s) in the number of b41 may optionally be bonded to each other to form a C₅-C₃₀ carbocyclic group unsubstituted or substituted with at least one R_{10b} or a C₁-C₃₀ heterocyclic group unsubstituted or substituted with at least one R_{10b},
at least two of R₄₂(s) in the number of b42 may optionally be bonded to each other to form a C₅-C₃₀ carbocyclic group unsubstituted or substituted with at least one R_{10b} or a C₁-C₃₀ heterocyclic group unsubstituted or substituted with at least one R_{10b},
at least two of R₄₃(s) in the number of b43 may optionally be bonded to each other to form a C₅-C₃₀ carbocyclic group unsubstituted or substituted with at least one R_{10b} or a C₁-C₃₀ heterocyclic group unsubstituted or substituted with at least one R_{10b},
at least two of R₄₁ to R₄₉ may optionally be bonded to each other to form a C₅-C₃₀ carbocyclic group unsubstituted or substituted with at least one R_{10b} or a C₁-C₃₀ heterocyclic group unsubstituted or substituted with at least one R_{10b},
R_{10b} is the same as described in connection with R₄₁,
* and *' each indicate a binding site to a neighboring atom,
at least one substituent of the substituted C₅-C₃₀ carbocyclic group, the substituted C₁-C₃₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted C₁-C₆₀ heteroaryloxy group, the substituted C₁-C₆₀ heteroarylthio group, the substituted monovalent non-aromatic condensed polycyclic group, and the substituted monovalent non-aromatic condensed heteropolycyclic group may be:
   deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
   a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -Si(Q₁₁)(Q₁₂)(Q₁₃), -Ge(Q₁₁)(Q₁₂)(Q₁₃), -N(Q₁₄)(Q₁₆), -B(Q₁₆)(Q₁₇), - P(=O)(Q₁₈)(Q₁₉), or any combination thereof;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, or a monovalent non-aromatic condensed heteropolycyclic group;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a C₁-C₆₀ heteroaryloxy group, a C₁-C₆₀ heteroarylthio group, a monovalent non-aromatic condensed polycyclic group, a monovalent non-aromatic condensed heteropolycyclic group, -Si(Q₂₁)(Q₂₂)(Q₂₃), -Ge(Q₂₁)(Q₂₂)(Q₂₃), -N(Q₂₄)(Q₂₅), -B(Q₂₆)(Q₂₇), - P(=O)(Q₂₈)(Q₂₉), or any combination thereof; or
   -si(Q₃₁)(Q₃₂)(Q₃₃), -Ge(Q₃₁)(Q₃₂)(Q₃₃), -N(Q₃₄)(Q₃₅), -B(Q₃₆)(Q₃₇), or - P(=O)(Q₃₈)(Q₃₉), and
   Q₁ to Q₉, Q₁₁ to Q₁₉, Q₂₁ to Q₂₉, and Q₃₁ to Q₃₉ may each independently be hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted C₁-C₆₀ heteroaryloxy group, a substituted or unsubstituted C₁-C₆₀ heteroarylthio group, a substituted or unsubstituted monovalent non-aromatic condensed polycyclic group, or a substituted or unsubstituted monovalent non-aromatic condensed heteropolycyclic group.

In an embodiment, R₄₁ to R₄₉ may each independently be:
hydrogen, deuterium, -F, -Cl, -Br, -I, a hydroxyl group, a cyano group, a nitro group, a C₁-C₂₀ alkyl group, or a C₁-C₂₀ alkoxy group;
a C₁-C₆₀ alkyl group or a C₁-C₆₀ alkoxy group, each substituted with at least one of deuterium, -F, -Cl, -Br, -I, a hydroxyl group, a cyano group, a nitro group, an amino group, a phenyl group, a biphenyl group, a naphthyl group, a fluorenyl group, a phenanthrenyl group, an anthracenyl group, a fluoranthenyl group, a triphenylenyl group, a pyrenyl group, and a chrysenyl group; or
a cyclopentyl group, a cyclohexyl group, a cycloheptyl group, a cyclopentenyl group, a cyclohexenyl group, a cycloheptenyl group, a phenyl group, a biphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a phenanthrolinyl group, a benzimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, or a carbazolyl group, each unsubstituted or substituted with at least one of deuterium, -F, -Cl, -Br, -I, a hydroxyl group, a cyano group, a nitro group, an amino group, a C₁-C₆₀ alkyl group, a C₁-C₆₀ alkoxy group, a cyclopentyl group, a cyclohexyl group, a cycloheptyl group, a cyclopentenyl group, a cyclohexenyl group, a cycloheptenyl group, a phenyl group, a biphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a phenanthrolinyl group, a benzimidazolyl group, a benzofuranyl group, a benzothiophenyl group, a benzoisothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, and a carbazolyl group.

In an embodiment, Formula 41 may be selected from Formulae 41-1 to 41-9: 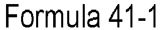 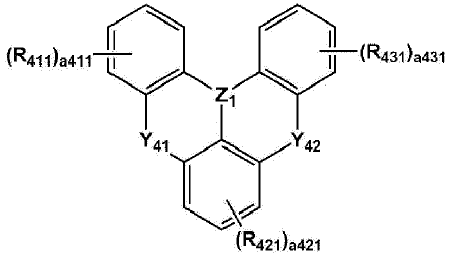 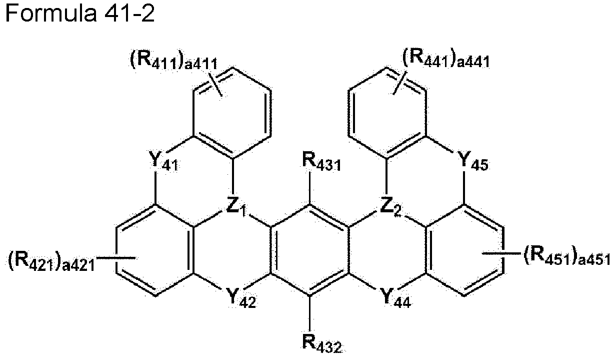 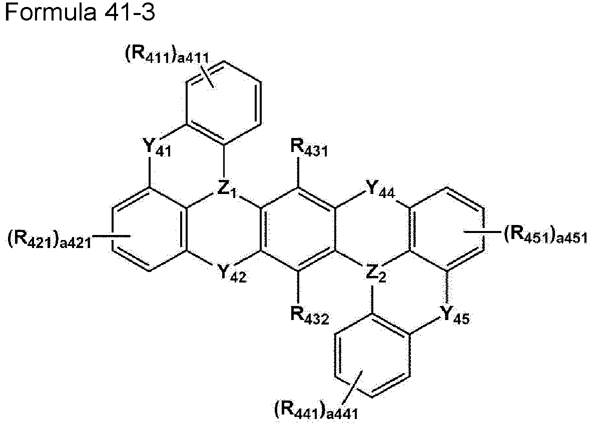 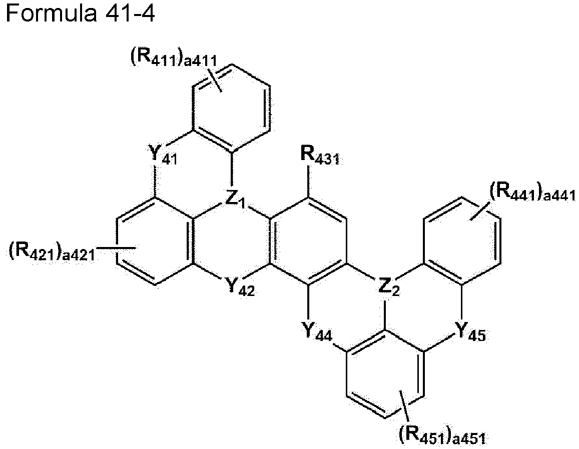 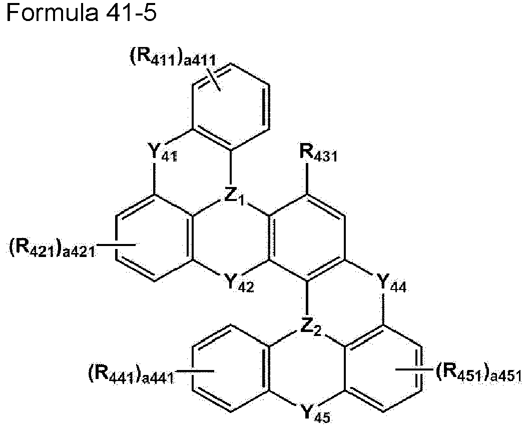 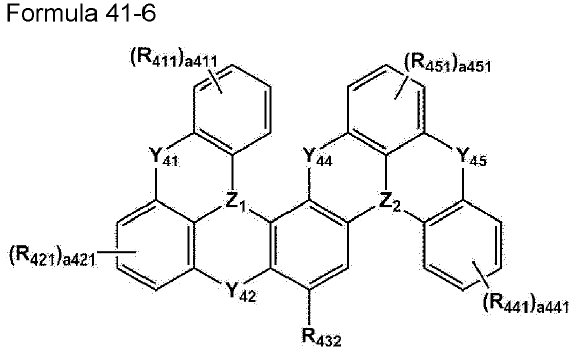 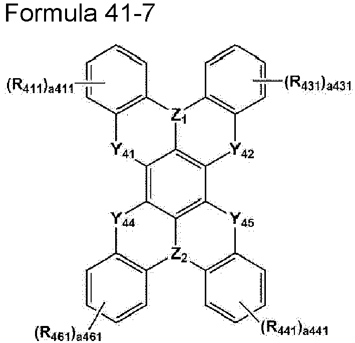 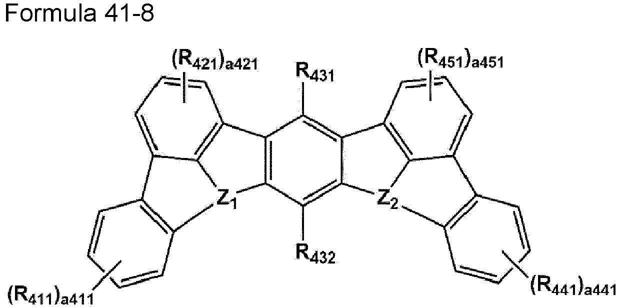 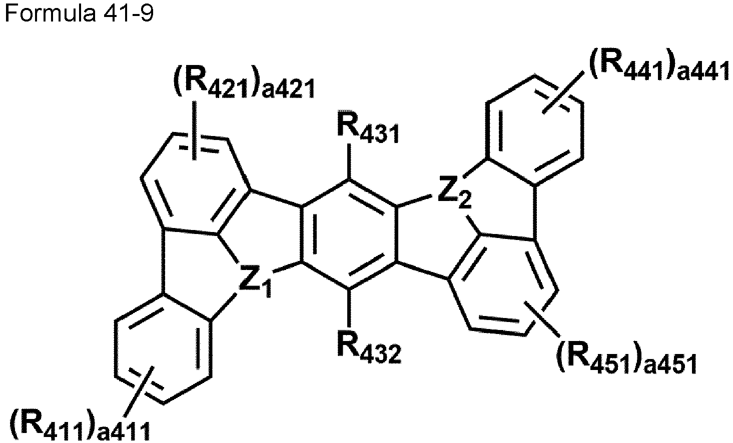
wherein, in Formulae 41-1 to 41-9,
Z₁ and Z₂ are each the same as described in connection with Z in Formula 41,
Y₄₁ and Y₄₂ are each the same as described in connection with L₄₁,
Y₄₄ and Y₄₅ are the same as described in connection with Y₄₁ and Y₄₂ in Formula 41, respectively,
R₄₁₁ is the same as described in connection with R₄₁ in Formula 41, R₄₂₁ is the same as described in connection with R₄₂ in Formula 41, R₄₃₁ and R₄₃₂ are each the same as described in connection with R₄₃ in Formula 41, R₄₄₁ is the same as described in connection with R₄₁ in Formula 41, R₄₅₁ is the same as described in connection with R₄₂ in Formula 41, and R₄₆₁ is the same as described in connection with R₄₃ in Formula 41,
a411 may be an integer from 0 to 4,
a421 may be an integer from 0 to 3,
a431 may be an integer from 0 to 4,
a441 may be an integer from 0 to 4,
a451 may be an integer from 0 to 3, and
a461 may be an integer from 0 to 4.

In an embodiment, the fluorescent dopant may be selected from Compounds D1 to D30:

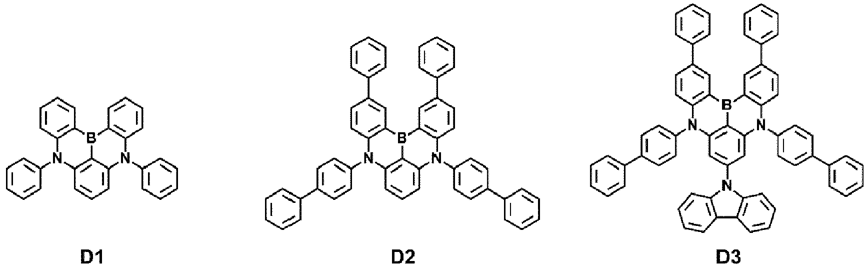

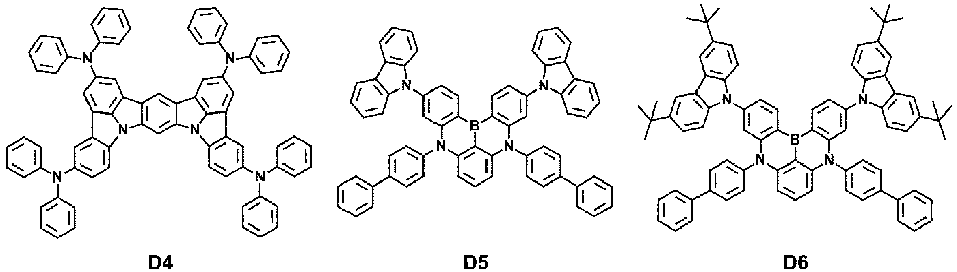

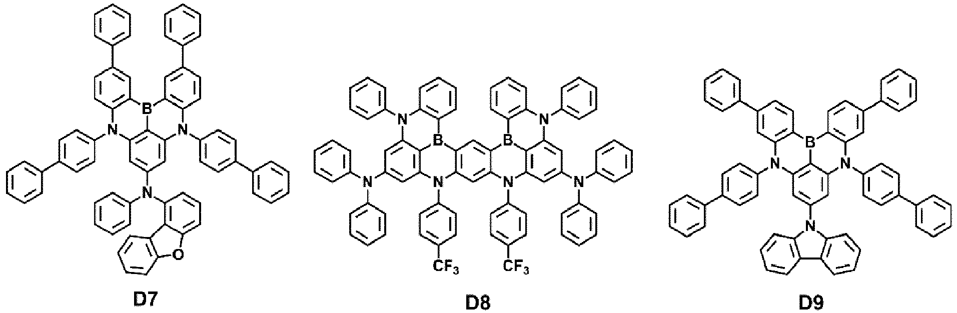

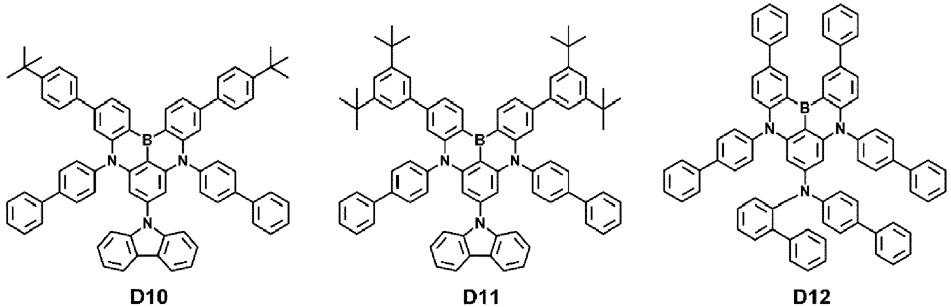

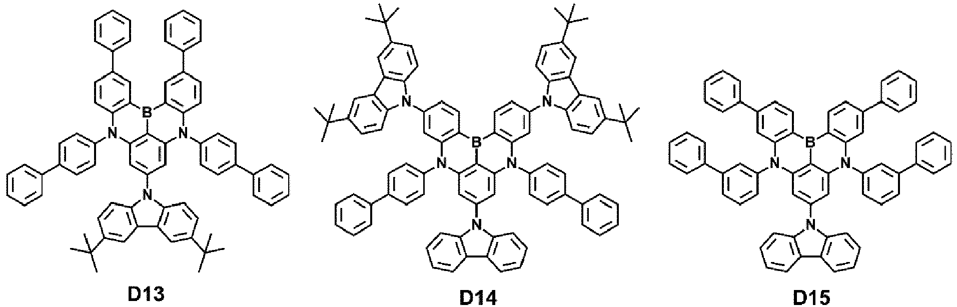

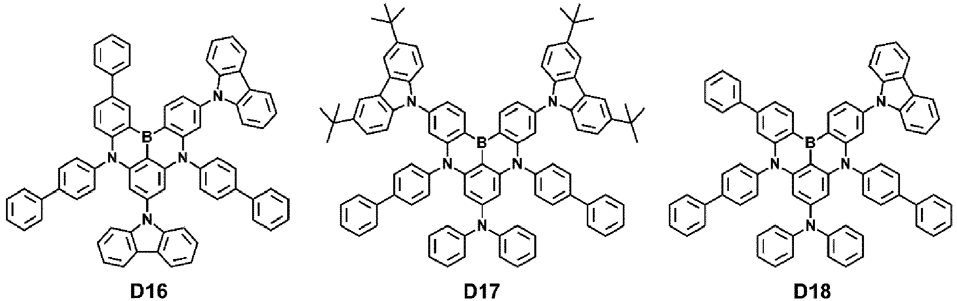

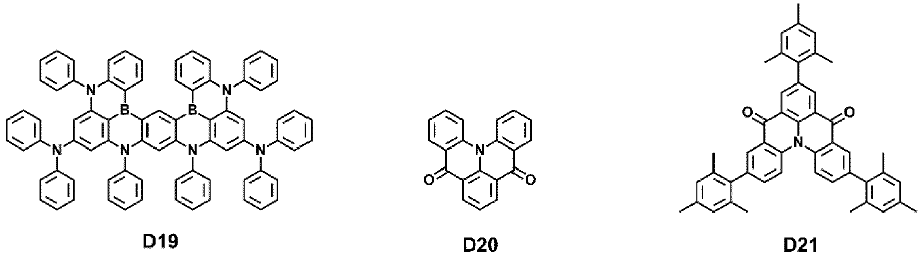

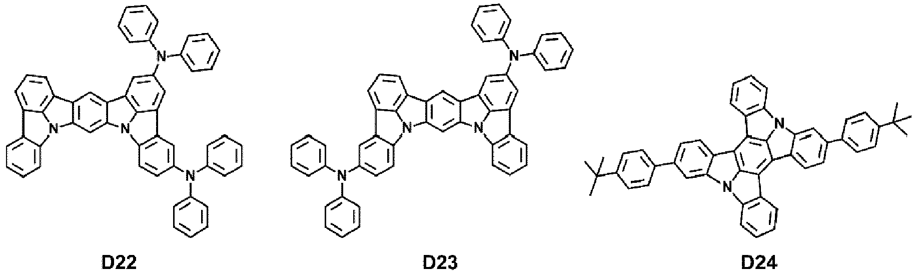

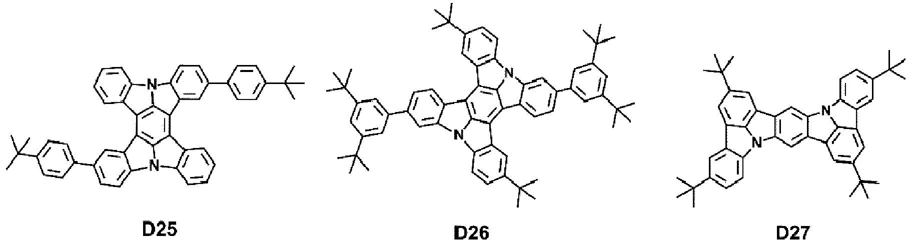

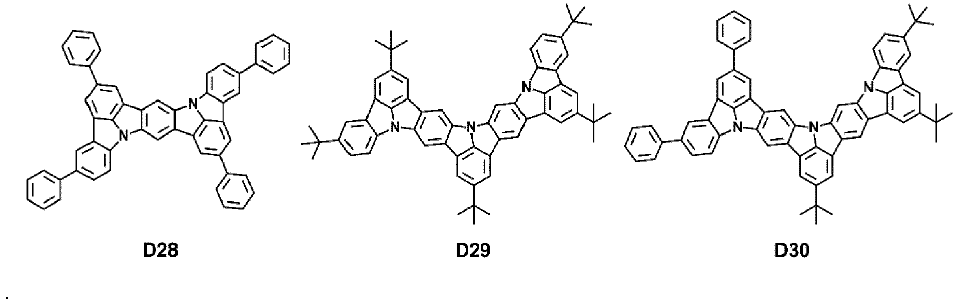

In an embodiment, the fluorescent dopant may be included in the emission layer in an amount in a range of about 0 wt% to about 5 wt%.

### [Hole transport region 12]

The hole transport region 12 may be arranged between the first electrode 11 and the emission layer 15 of the organic light-emitting device 10.

The hole transport region 12 may have a single-layer structure or a multi-layer structure.

For example, the hole transport region 12 may have a hole injection layer, a hole transport layer, a hole injection layer/hole transport layer structure, a hole injection layer/first hole transport layer/second hole transport layer structure, a hole injection layer/first hole transport layer/second hole transport layer/electron blocking layer structure, a hole transport layer/organic layer structure, a hole injection layer/hole transport layer/organic layer structure, a hole transport layer/electron blocking layer structure, or a hole injection layer/hole transport layer/electron blocking layer structure.

The hole transport region 12 may include any compound having hole-transporting properties.

The hole transport region 12 may include the heterocyclic compound represented by Formula 1. For example, the heterocyclic compound may be included in a hole transport layer, a hole transport layer, an electron blocking layer, or any combination thereof of the hole transport region 12.

For example, the hole transport region 12 may include an amine-based compound.

In an embodiment, the hole transport region 12 may include m-MTDATA, TDATA, 2-TNATA, NPB, β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor-sulfonic acid (PANI/CSA), polyaniline/poly(4-styrene sulfonate) (PANI/PSS), a compound represented by one of Formulae 201 to 205, or any combination thereof: 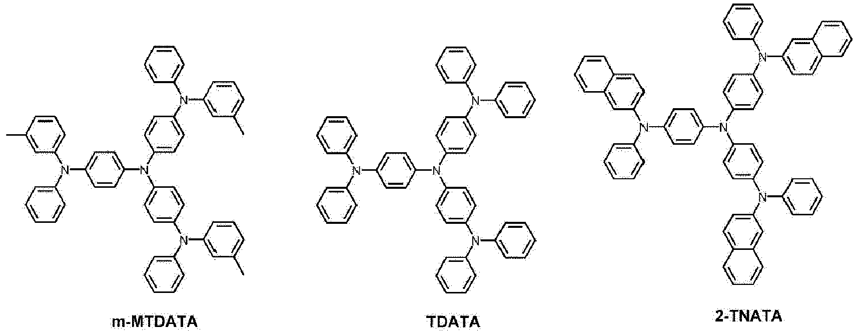 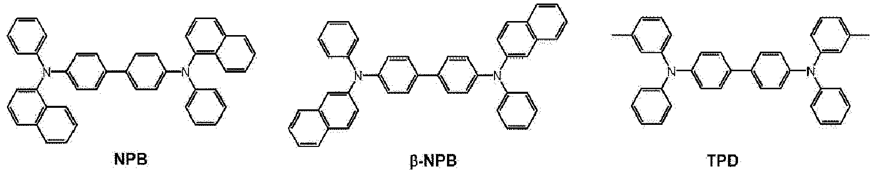 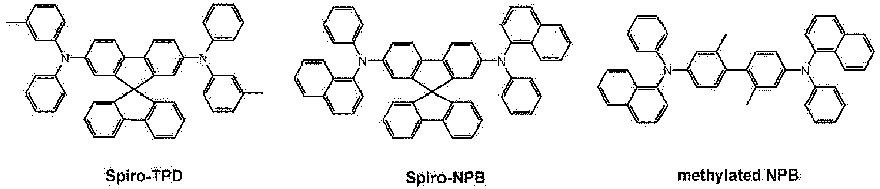 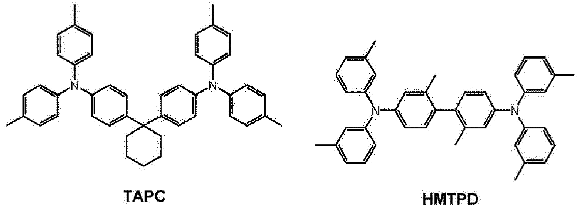 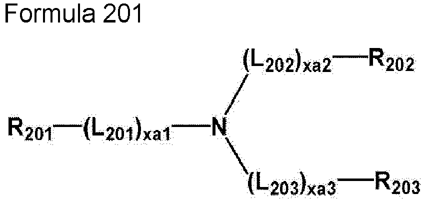 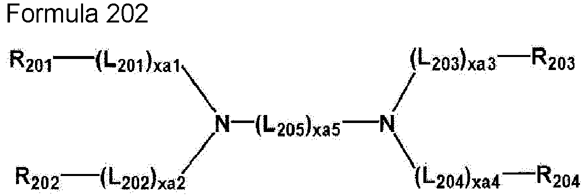 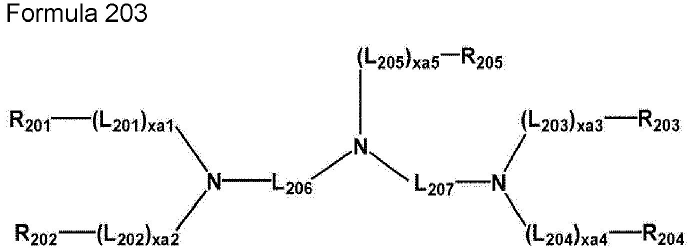 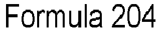 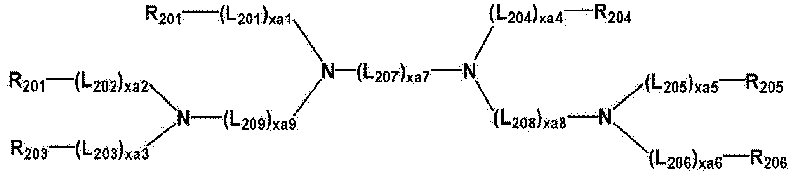 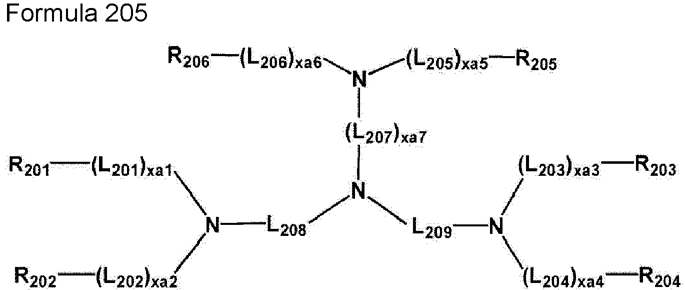
wherein, in Formulae 201 to 205,
L₂₀₁ to L₂₀₉ may each independently be *-O-*', *-S-*', a substituted or unsubstituted C₅-C₆₀ carbocyclic group, or a substituted or unsubstituted C₁-C₆₀ heterocyclic group,
xa1 to xa9 may each independently be an integer from 0 to 5, and
R₂₀₁ to R₂₀₆ may each independently be a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, or a substituted or unsubstituted monovalent non-aromatic condensed heteropolycyclic group, wherein neighboring two groups of R₂₀₁ to R₂₀₆ may optionally be linked to each other via a single bond, a dimethyl-methylene group, or a diphenyl-methylene group.

For example,
L₂₀₁ to L₂₀₉ may each independently be a benzene group, a heptalene group, an indene group, a naphthalene group, an azulene group, a heptalene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentacene group, a rubicene group, a corogen group, an ovalene group, a pyrrole group, an isoindole group, an indole group, a furan group, a thiophene group, a benzofuran group, a benzothiophene group, a benzocarbazole group, a dibenzocarbazole group, a dibenzofuran group, a dibenzothiophene group, a dibenzothiophene sulfone group, a carbazole group, a dibenzosilole group, an indenocarbazole group, an indolocarbazole group, a benzofurocarbazole group, a benzothienocarbazole group, or a triindolobenzene group, each unsubstituted or substituted with deuterium, a C₁-C₁₀ alkyl group, a C₁-C₁₀ alkoxy group, a phenyl group, a naphthyl group, a fluorenyl group, a carbazolyl group, a dibenzofuranyl group, a dibenzothiophenyl group, a triphenylenyl group, a biphenyl group, a terphenyl group, a tetraphenyl group, -Si(Q₁₁)(Q₁₂)(Q₁₃), or any combination thereof,
xa1 to xa9 may each independently be 0, 1, or 2,
R₂₀₁ to R₂₀₆ may each independently be a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an indeno carbazolyl group, an indolocarbazolyl group, a benzofurocarbazolyl group, or a benzothienocarbazolyl group, each unsubstituted or substituted with deuterium, -F, -Cl, -Br, -I, a hydroxyl group, a cyano group, a nitro group, an amidino group, a hydrazine group, a hydrazone group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), -N(Q₃₁)(Q₃₂), or any combination thereof, and
Q₁₁ to Q₁₃ and Q₃₁ to Q₃₃ may each independently be a C₁-C₁₀ alkyl group, a C₁-C₁₀ alkoxy group, a phenyl group, a biphenyl group, a terphenyl group, or a naphthyl group.

In one or more embodiments, the hole transport region 12 may include a carbazole-containing amine-based compound.

In one or more embodiments, the hole transport region 12 may include a carbazole-containing amine-based compound and a carbazole-free amine-based compound.

The carbazole-containing amine-based compound may include, for example, compounds represented by Formula 201 including a carbazole group and further including at least one of a dibenzofuran group, a dibenzothiophene group, a fluorene group, a spiro-bifluorene group, an indenocarbazole group, an indolocarbazole group, a benzofurocarbazole group, and a benzothienocarbazole group.

The carbazole-free amine-based compound may include, for example, compounds represented by Formula 201 not including a carbazole group and including at least one of a dibenzofuran group, a dibenzothiophene group, a fluorene group, a spiro-bifluorene group, an indenocarbazole group, an indolocarbazole group, a benzofurocarbazole group, and a benzothienocarbazole group.

In one or more embodiments, the hole transport region 12 may include a compound represented by Formula 201, a compound represented by Formula 202, or any combination thereof.

In one or more embodiments, the hole transport region 12 may include a compound represented by Formula 201-1, 202-1, or 201-2, or any combination thereof: 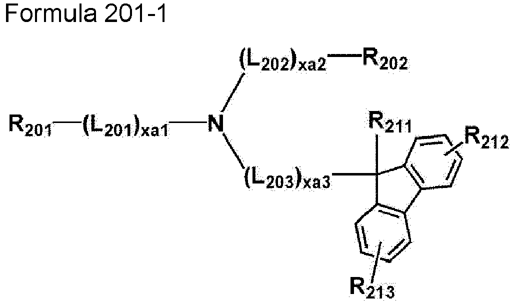 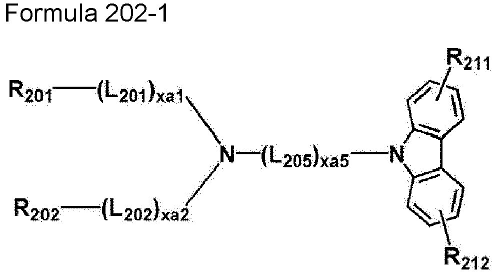 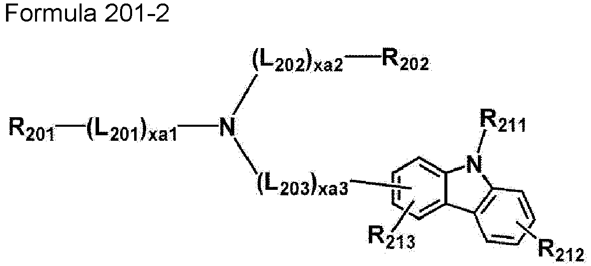 wherein, in Formulae 201-1, 202-1, and 201-2, L₂₀₁ to L₂₀₃, L₂₀₅, xa1 to xa3, xa5, R₂₀₁ and R₂₀₂ are each the same as described herein, and R₂₁₁ to R₂₁₃ may each independently be hydrogen, deuterium, -F, -Cl, -Br, -I, a hydroxyl group, a cyano group, a nitro group, an amidino group, a hydrazine group, a hydrazone group, a C₁-C₂₀ alkyl group, a C₁-C₂₀ alkoxy group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a dimethylfluorenyl group, a diphenylfluorenyl group, a triphenylenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, or a pyridinyl group.

In an embodiment, the hole transport region 12 may include one of Compounds HT1 to HT39 or any combination thereof:

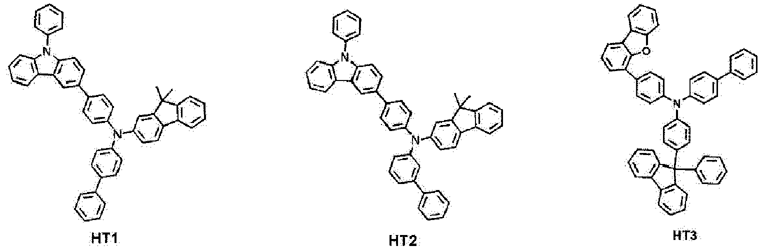

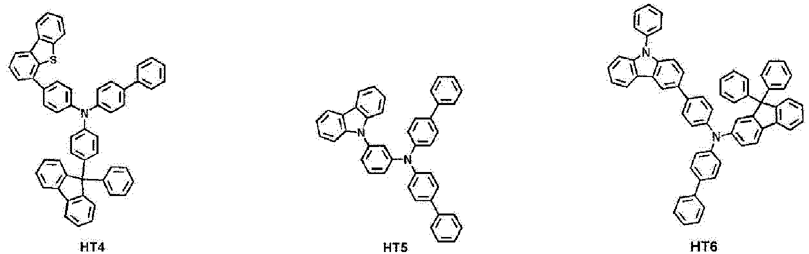

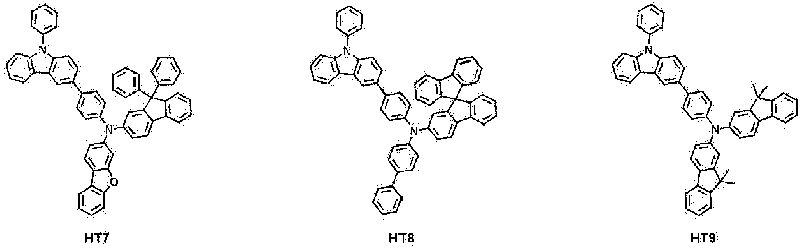

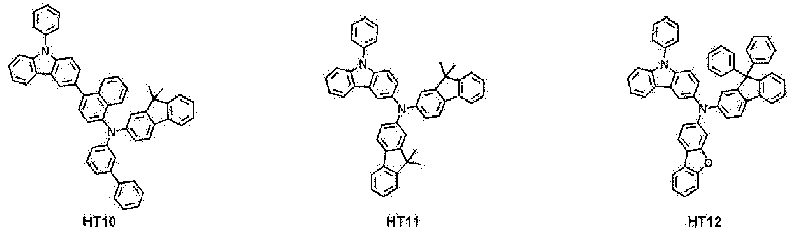

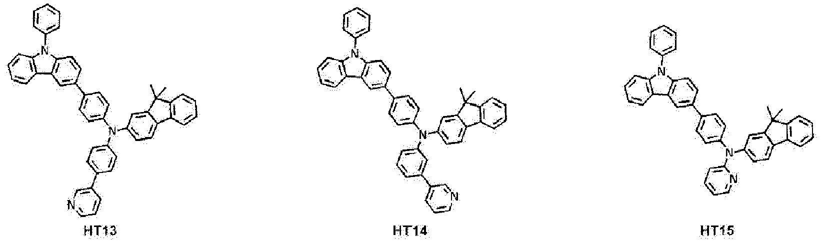

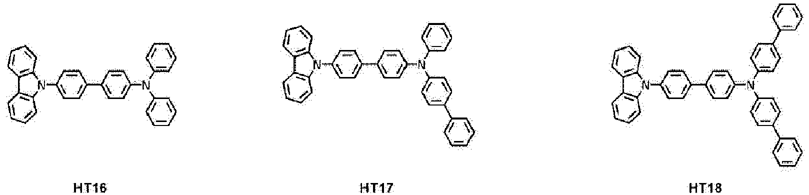

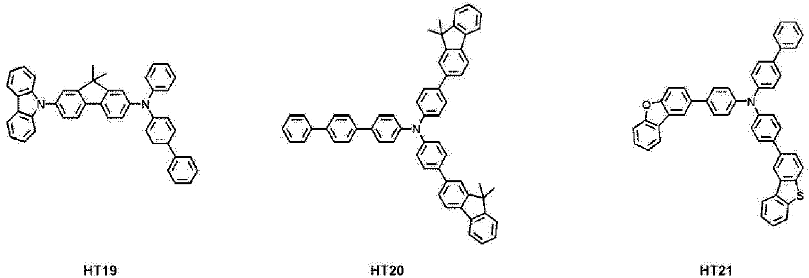

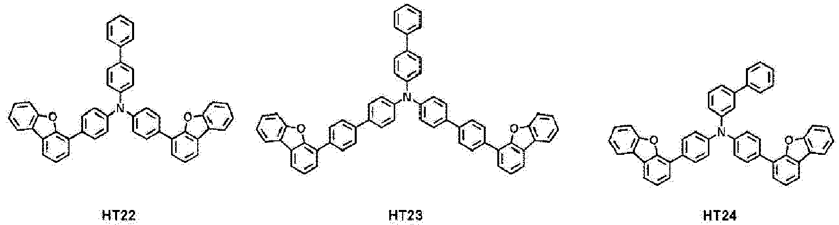

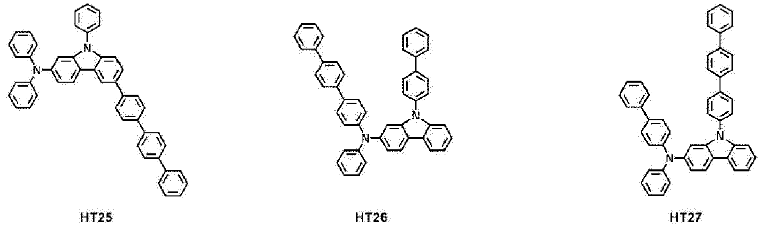

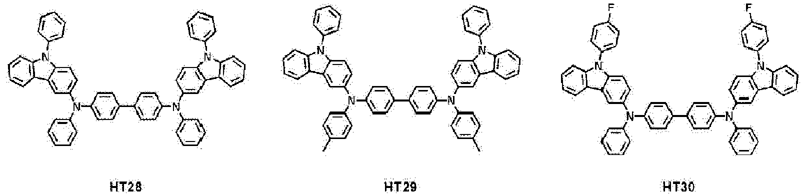

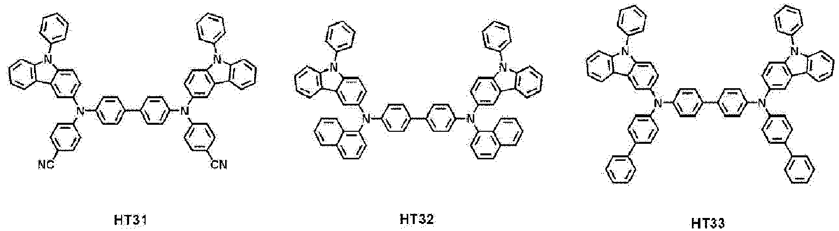

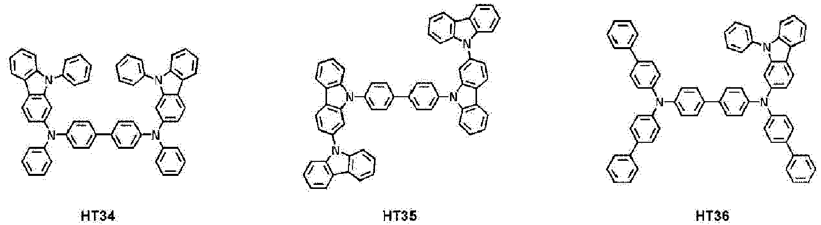

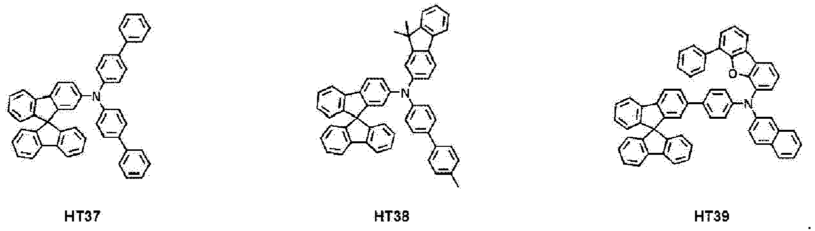

In one or more embodiments, the hole transport region 12 of the organic light-emitting device 10 may further include a p-dopant. When the hole transport region 12 further includes a p-dopant, the hole transport region 12 may have a matrix (for example, at least one of compounds represented by Formulae 201 to 205) and a p-dopant included in the matrix. The p-dopant may be uniformly or non-uniformly doped in the hole transport region 12.

In an embodiment, the LUMO energy level of the p-dopant may be less than or equal to -3.5 eV.

The p-dopant may include a quinone derivative, a metal oxide, a cyano group-containing compound, or any combination thereof.

For example, the p-dopant may include:
a quinone derivative, such as tetracyanoquinodimethane (TCNQ), 2,3,5,6-tetrafluoro-7,7,8,8-tetracyanoquinodimethane (F4-TCNQ), or F6-TCNNQ;
a metal oxide, such as tungsten oxide or molybdenum oxide;
1,4,5,8,9,12-hexaazatriphenylene-hexacarbonitrile (HAT-CN);
a compound represented by Formula 221; or
any combination thereof: 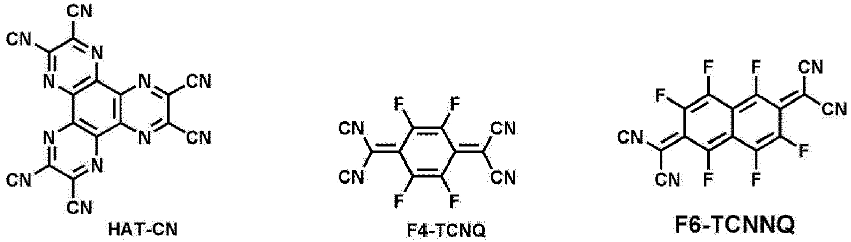 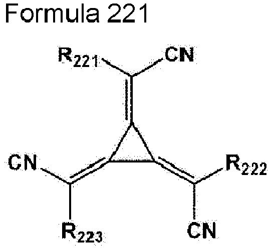
wherein, in Formula 221,
R₂₂₁ to R₂₂₃ may each independently be a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, or a substituted or unsubstituted monovalent non-aromatic condensed heteropolycyclic group, wherein at least one substituent of R₂₂₁ to R₂₂₃ may be: a cyano group; -F; -Cl; -Br; -I; a C₁-C₂₀ alkyl group substituted with -F; a C₁-C₂₀ alkyl group substituted with -Cl; a C₁-C₂₀ alkyl group substituted with -Br; a C₁-C₂₀ alkyl group substituted with -I; or any combination thereof.

The compound represented by Formula 221 may include, for example, Compound HT-D2:

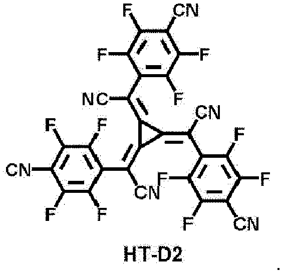

The hole transport region 12 may have a thickness in a range about 100 Å to about 10,000 Å, for example, about 400 Å to about 2,000 Å, and the emission layer 15 may have a thickness in a range of about 100 Å to about 3,000 Å, for example, about 300 Å to about 1,000 Å. When the thickness of each of the hole transport region 12 and the emission layer 15 is within these ranges, satisfactory hole transportation characteristics and/or luminescence characteristics may be obtained without a substantial increase in driving voltage.

The hole transport region 12 may further include a buffer layer.

The buffer layer may compensate for an optical resonance distance according to a wavelength of light emitted from the emission layer 15, and thus, efficiency of a formed organic light-emitting device may be improved.

The hole transport region 12 may further include an electron blocking layer. The electron blocking layer may include a known material, for example, mCP or DBFPO:

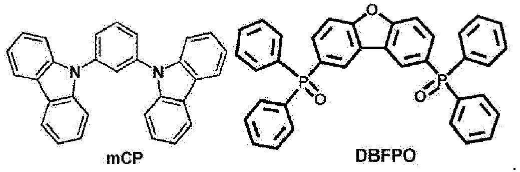

### [Electron transport region 17]

The electron transport region 17 is arranged between the emission layer 15 and the second electrode 19 of the organic light-emitting device 10.

The electron transport region 17 may have a single-layer structure or a multi-layer structure.

For example, the electron transport region 17 may have an electron transport layer, an electron transport layer/electron injection layer structure, a buffer layer/electron transport layer structure, hole blocking layer/electron transport layer structure, a buffer layer/electron transport layer/electron injection layer structure, or a hole blocking layer/electron transport layer/electron injection layer structure. The electron transport region 17 may further include an electron control layer.

The electron transport region 17 may include the heterocyclic compound represented by Formula 1. For example, the heterocyclic compound may be included in a buffer layer or the like of the electron transport region 17.

The electron transport region 17 may include known electron-transporting materials.

The electron transport region 17 (for example, a buffer layer, a hole blocking layer, an electron control layer, or an electron transport layer in the electron transport region) may include a metal-free compound containing at least one π electron-deficient nitrogen-containing C₁-C₆₀ cyclic group. The π electron-deficient nitrogen-containing C₁-C₆₀ cyclic group is the same as described herein.

For example, the electron transport region 17 may include a compound represented by Formula 601:

Formula 601 [Ar₆₀₁]ₓₑ₁₁-[(L₆₀₁)ₓₑ₁-R₆₀₁]ₓₑ₂₁.

wherein, in Formula 601,
Ar₆₀₁ and L₆₀₁ may each independently be a C₅-C₆₀ carbocyclic group unsubstituted or substituted with at least one R₆₀₁ₐ or a C₁-C₆₀ heterocyclic group unsubstituted or substituted with at least one R₆₀₁ₐ,
xe11 may be 1, 2, or 3,
xe1 may be an integer from 0 to 5,
R₆₀₁ₐ and R₆₀₁ may each independently be a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₆₀₁)(Q₆₀₂)(Q₆₀₃), - -C(=O)(Q₆₀₁), -S(=O)₂(Q₆₀₁), or -P(=O)(Q₆₀₁)(Q₆₀₂),
Q₆₀₁ to Q₆₀₃ may each independently be a C₁-C₁₀ alkyl group, a C₁-C₁₀ alkoxy group, a phenyl group, a biphenyl group, a terphenyl group, or a naphthyl group, and
xe21 may be an integer from 1 to 5.

In an embodiment, at least one of Ar₆₀₁(s) in the number of xe11 and R₆₀₁(s) in the number of xe21 may include the π electron-deficient nitrogen-containing C₁-C₆₀ cyclic group.

In an embodiment, Ar₆₀₁ and L₆₀₁ in Formula 601 may each independently be a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, or an azacarbazole group, each unsubstituted or substituted with deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -S(=O)₂(Q₃₁), -P(=O)(Q₃₁)(Q₃₂), or any combination thereof, and
Q₃₁ to Q₃₃ may each independently be a C₁-C₁₀ alkyl group, a C₁-C₁₀ alkoxy group, a phenyl group, a biphenyl group, a terphenyl group, or a naphthyl group.

When xe11 in Formula 601 is 2 or more, two or more of Ar₆₀₁ may be linked to each other via a single bond.

In one or more embodiments, Ar₆₀₁ in Formula 601 may be an anthracene group.

In one or more embodiments, the compound represented by Formula 601 may be represented by Formula 601-1: 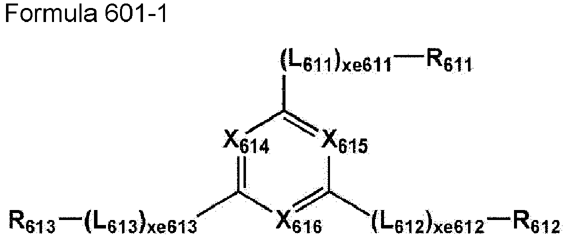
wherein, in Formula 601-1,
X₆₁₄ may be N or C(R₆₁₄), X₆₁₅ may be N or C(R₆₁₆), X₆₁₆ may be N or C(R₆₁₆), and at least one of X₆₁₄ to X₆₁₆ may be N,
L₆₁₁ to L₆₁₃ are each independently the same as described in connection with L601,
xe611 to xe613 are each independently the same as described in connection with xe1,
R₆₁₁ to R₆₁₃ are each independently the same as described in connection with R₆₀₁, and
R₆₁₄ to R₆₁₆ may each independently be hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, or a naphthyl group.

In one or more embodiments, xe1 and xe611 to xe613 in Formulae 601 and 601-1 may each independently be 0, 1, or 2.

In one or more embodiments, R₆₀₁ and R₆₁₁ to R₆₁₃ in Formulae 601 and 601-1 may each independently be a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, or an azacarbazolyl group, each unsubstituted or substituted with deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, or any combination thereof; or
-S(=O)₂(Q₆₀₁) or -P(=O)(Q₆₀₁)(Q₆₀₂), and
Q₆₀₁ and Q₆₀₂ are the same as described herein.

The electron transport region 17 may include one of Compounds ET1 to ET36 or any combination thereof:

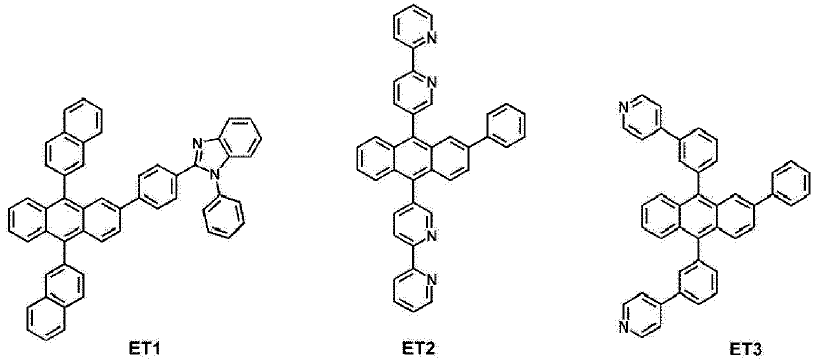

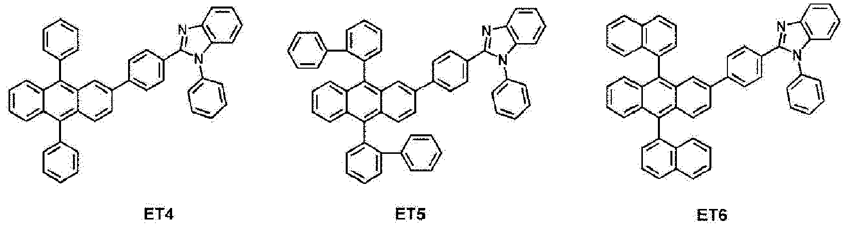

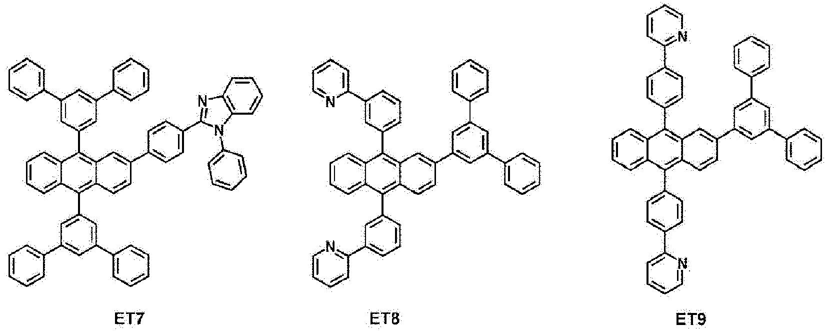

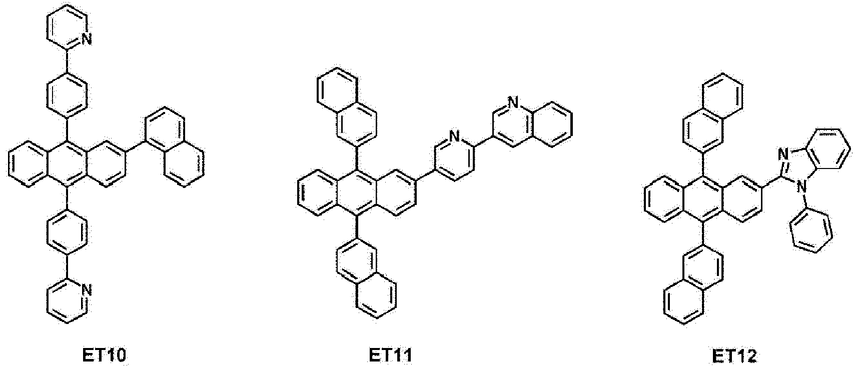

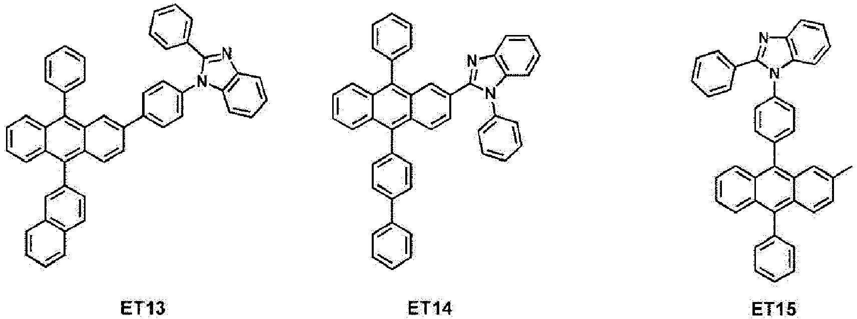

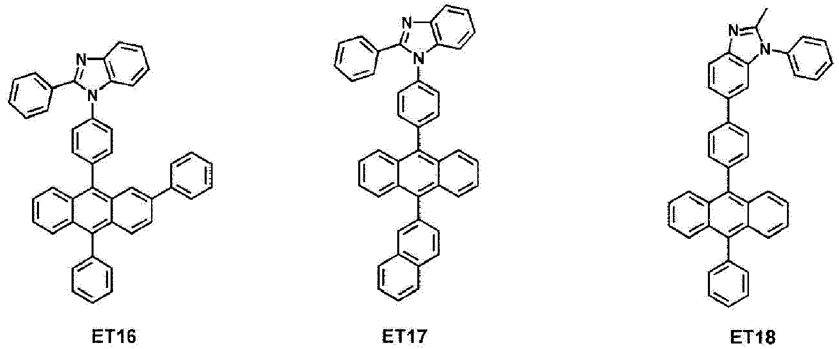

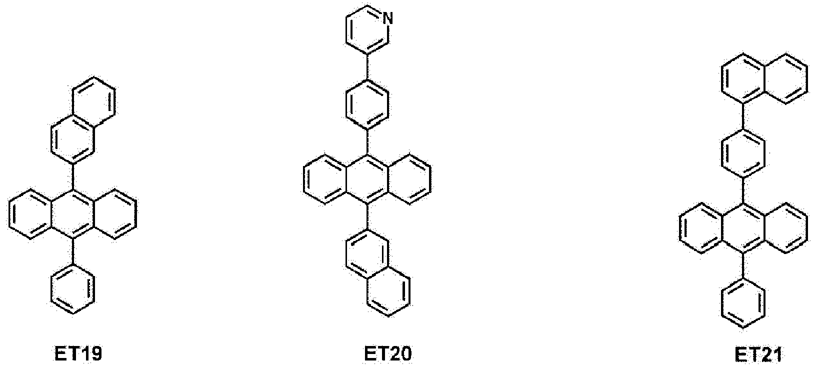

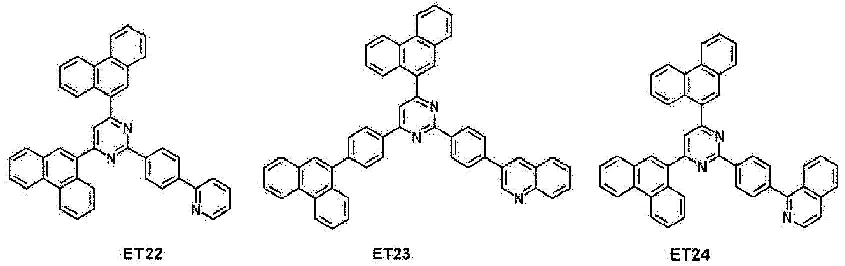

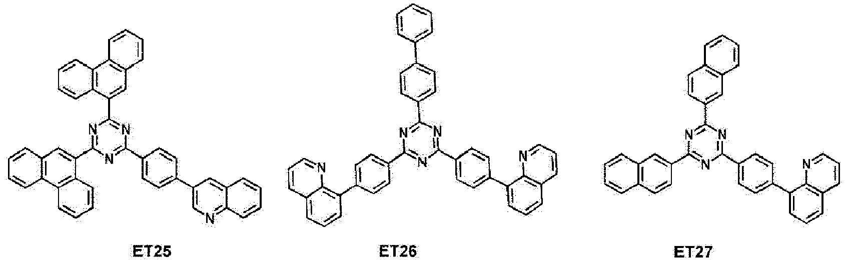

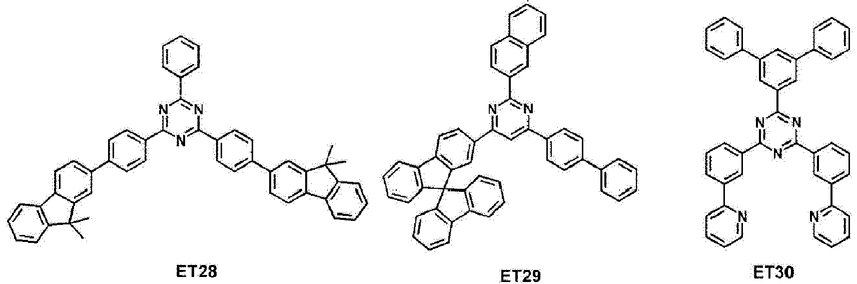

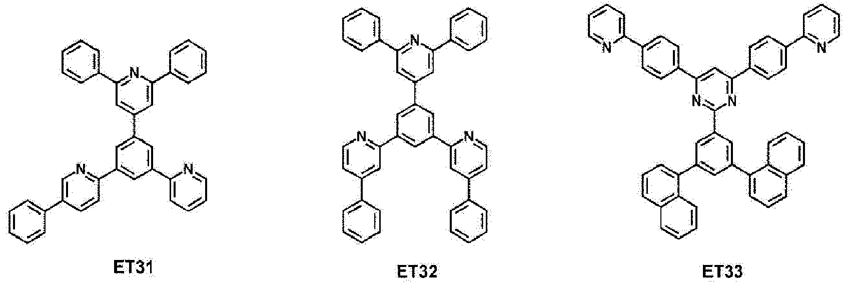

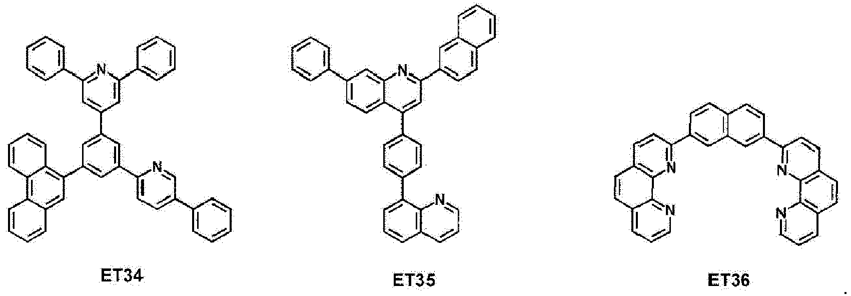

In one or more embodiments, the electron transport region 17 may include 2,9-dimethyl-4,7-diphenyl-1,10-phenanthroline (BCP), 4,7-diphenyl-1,10-phenanthroline (Bphen), Alq₃, BAlq, 3-(biphenyl-4-yl)-5-(4-tert-butylphenyl)-4-phenyl-4H-1,2,4-triazole (TAZ), NTAZ, DBFPO, or any combination thereof. For example, when the electron transport region 17 includes a hole blocking layer, the hole blocking layer may include BCP or Bphen:

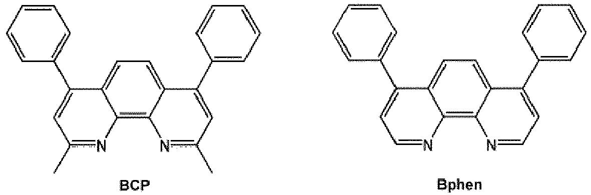

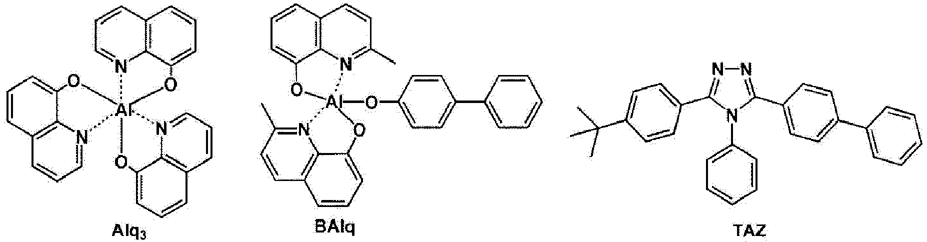

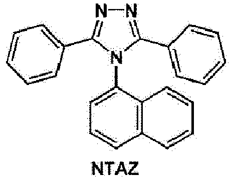

Thicknesses of the buffer layer, the hole blocking layer, and the electron control layer may each independently be in a range of about 20 Å to about 1,000 Å, for example, about 30 Å to about 300 Å. When the thicknesses of the buffer layer, the hole blocking layer, and the electron control layer are within these ranges, excellent hole blocking characteristics or excellent electron control characteristics may be obtained without a substantial increase in driving voltage.

A thickness of the electron transport layer may be in a range of about 100 Å to about 1,000 Å, for example, about 150 Å to about 500 Å. When the thickness of the electron transport layer is within these ranges, satisfactory electron transporting characteristics may be obtained without a substantial increase in driving voltage.

The electron transport region 17 (for example, the electron transport layer in the electron transport region 17) may further include, in addition to the aforementioned materials, a metal-containing material.

The metal-containing material may include an alkali metal complex, an alkaline earth metal complex, or any combination thereof. A metal ion of the alkali metal complex may include a Li ion, a Na ion, a K ion, a Rb ion, a Cs ion, or any combination thereof, and a metal ion of the alkaline earth metal complex may include a Be ion, a Mg ion, a Ca ion, a Sr ion, a Ba ion, or any combination thereof. A ligand coordinated with the metal ion of the alkali metal complex or the alkaline earth-metal complex may include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxydiphenyloxadiazole, a hydroxydiphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

For example, the metal-containing material may include a Li complex. The Li complex may include, for example, Compound ET-D1 (LiQ) or ET-D2:

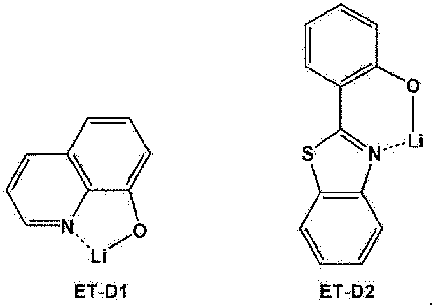

The electron transport region 17 may include an electron injection layer that facilitates the injection of electrons from the second electrode 19. The electron injection layer may directly contact the second electrode 19.

The electron injection layer may have i) a single-layer structure consisting of a single layer including a single material, ii) a single-layer structure consisting of a single layer including multiple materials that are different from each other, or iii) a multi-layer structure consisting of multiple layers including multiple materials that are different from each other.

The electron injection layer may include an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof.

The alkali metal may include Li, Na, K, Rb, Cs, or any combination thereof. In an embodiment, the alkali metal may be Li, Na, or Cs. In one or more embodiments, the alkali metal may be Li or Cs.

The alkaline earth metal may include Mg, Ca, Sr, Ba, or any combination thereof.

The rare earth metal may include Sc, Y, Ce, Tb, Yb, Gd, or any combination thereof.

The alkali metal compound, the alkaline earth metal compound, and the rare earth metal compound may include oxides and halides (for example, fluorides, chlorides, bromides, or iodides) of the alkali metal, the alkaline earth metal, and the rare earth metal, or any combination thereof.

The alkali metal compound may include: one of alkali metal oxides such as Li₂O, Cs₂O, K₂O, and the like; one of alkali metal halides such as LiF, NaF, CsF, KF, Lil, Nal, Csl, KI, and the like; or any combination thereof. In an embodiment, the alkali metal compound may include LiF, Li₂O, NaF, Lil, Nal, Csl, KI, or any combination thereof.

The alkaline earth-metal compound may include one of alkaline earth-metal compounds, such as BaO, SrO, CaO, BaₓSr₁₋ₓO (wherein 0<x<1), or BaₓCa₁₋ₓO (wherein 0<x<1), or any combination thereof. In an embodiment, the alkaline earth metal compound may include BaO, SrO, CaO, or any combination thereof.

The rare earth metal compound may include YbF₃, ScF₃, ScO₃, Y₂O₃, Ce₂O₃, GdF₃, TbF₃, or any combination thereof. In an embodiment, the rare earth metal compound may include YbF₃, ScF₃, TbF₃, YbI₃, ScI₃, TbI₃, or any combination thereof.

The alkali metal complex, the alkaline earth metal complex, and the rare earth metal complex may include an ion of alkali metal, alkaline earth metal, and rare earth metal as described above, and a ligand coordinated with a metal ion of the alkali metal complex, the alkaline earth metal complex, or the rare earth metal complex may include hydroxy quinoline, hydroxy isoquinoline, hydroxy benzoquinoline, hydroxy acridine, hydroxy phenanthridine, hydroxy phenyloxazole, hydroxy phenylthiazole, hydroxy diphenyloxadiazole, hydroxy diphenylthiadiazole, hydroxy phenylpyridine, hydroxy phenylbenzimidazole, hydroxy phenylbenzothiazole, bipyridine, phenanthroline, cyclopentadiene, or any combination thereof.

The electron injection layer may consist of an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof, as described above. In one or more embodiments, the electron injection layer may further include an organic material. When the electron injection layer further includes an organic material, an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combination thereof may be homogeneously or non-homogeneously dispersed in a matrix including the organic material.

A thickness of the electron injection layer may be in a range of about 1 Å to about 100 Å, and, for example, about 3 Å to about 90 Å. When the thickness of the electron injection layer is within these ranges, satisfactory electron injection characteristics may be obtained without a substantial increase in driving voltage.

### [Second electrode 19]

The second electrode 19 is arranged on the aforementioned organic layer 10A. The second electrode 19 may be a cathode which is an electron injection electrode, and in this regard, a material for forming the second electrode 19 may be selected from a metal, an alloy, an electrically conductive compound, and a combination thereof, which have a relatively low work function.

The second electrode 19 may include Li, Ag, Mg, Al, Al-Li, Ca, Mg-In, Mg-Ag, ITO, IZO, or any combination thereof. The second electrode 19 may be a transmissive electrode, a semi-transmissive electrode, or a reflective electrode.

The second electrode 19 may have a single-layer structure having a single layer or a multi-layer structure including two or more layers.

### Explanation of terms

The term "C₁-C₆₀ alkyl group" as used herein refers to a linear or branched saturated aliphatic hydrocarbons monovalent group having 1 to 60 carbon atoms, and the term "C₁-C₆₀ alkylene group" as used here refers to a divalent group having the same structure as the C₁-C₆₀ alkyl group.

Examples of the C₁-C₆₀ alkyl group, the C₁-C₂₀ alkyl group, and/or the C₁-C₁₀ alkyl group are a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, or a tert-decyl group, each unsubstituted or substituted with a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, or any combination thereof.

The term "C₁-C₆₀ alkoxy group" as used herein refers to a monovalent group represented by -OA₁₀₁ (wherein A₁₀₁ is the C₁-C₆₀ alkyl group), and examples thereof are a methoxy group, an ethoxy group, a propoxy group, a butoxy group, and a pentoxy group.

The term "C₂-C₆₀ alkenyl group" as used herein refers to a hydrocarbon group formed by substituting at least one carbon-carbon double bond in the middle or at the terminus of the C₂-C₆₀ alkyl group, and examples thereof are an ethenyl group, a propenyl group, and a butenyl group. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group formed by substituting at least one carbon-carbon triple bond in the middle or at the terminus of the C₂-C₆₀ alkyl group, and examples thereof are an ethynyl group, and a propynyl group. The term "C₂-C₆₀ alkynylene group" as used herein refers to a divalent group having the same structure as the C₂-C₆₀ alkynyl group.

The term "C₃-C₁₀ cycloalkyl group" as used herein refers to a monovalent saturated hydrocarbon cyclic group having 3 to 10 carbon atoms, and the term "C₃-C₁₀ cycloalkylene group" as used herein refers to a divalent group having the same structure as the C₃-C₁₀ cycloalkyl group.

Examples of the C₃-C₁₀ cycloalkyl group are a cyclopropyl group, a cyclobutyl group, a cyclopentyl group, cyclohexyl group, a cycloheptyl group, a cyclooctyl group, an adamantanyl group, a norbornanyl group (a bicyclo[2.2.1]heptyl group), a bicyclo[1.1.1]pentyl group, a bicyclo[2.1.1]hexyl group, and a bicyclo[2.2.2]octyl group.

The term "C₁-C₁₀ heterocycloalkyl group" as used herein refers to a monovalent monocyclic group that includes at least one heteroatom selected from B, N, O, P, Si, S, Se, Ge, and B as a ring-forming atom and 1 to 10 carbon atoms, and the term "the C₁-C₁₀ heterocycloalkylene group" as used herein refers to a divalent group having the same structure as the C₁-C₁₀ heterocycloalkyl group.

Examples of the C₁-C₁₀ heterocycloalkyl group are a silolanyl group, a silinanyl group, tetrahydrofuranyl group, a tetrahydro-2H-pyranyl group, and a tetrahydrothiophenyl group.

The term "C₃-C₁₀ cycloalkenyl group" as used herein refers to a monovalent cyclic group that includes 3 to 10 carbon atoms and at least one carbon-carbon double bond in the ring thereof and has no aromaticity, and examples thereof are a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent monocyclic group that has at least one hetero atom selected from B, N, O, P, Si, S, Se, Ge, and B as a ring-forming atom, 1 to 10 carbon atoms, and at least one carbon-carbon double bond in the ring thereof. Examples of the C₁-C₁₀ heterocycloalkenyl group are a 2,3-dihydrofuranyl group and a 2,3-dihydrothiophenyl group. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Examples of the C₆-C₆₀ aryl group are a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

The term "C₁-C₆₀ heteroaryl group" as used herein refers to a monovalent group that includes at least one heteroatom selected from B, N, O, P, Si, S, Se, Ge, and B as a ring-forming atom and a heterocyclic aromatic system having 1 to 60 carbon atoms, and the term "C₁-C₆₀ heteroarylene group" as used herein refers to a divalent group that includes at least one heteroatom selected from B, N, O, P, Si, S, Se, Ge, and B as a ring-forming atom and a heterocyclic aromatic system having 1 to 60 carbon atoms. Examples of the C₁-C₆₀ heteroaryl group are a pyridinyl group, a pyrimidinyl group, a pyrazinyl group, a pyridazinyl group, a triazinyl group, a quinolinyl group, and an isoquinolinyl group. When the C₆-C₆₀ heteroaryl group and the C₆-C₆₀ heteroarylene group each include two or more rings, the rings may be fused to each other.

The term "C₆-C₆₀ aryloxy group" as used herein indicates -OA₁₀₂ (wherein A₁₀₂ is the C₆-C₆₀ aryl group), and the term "C₆-C₆₀ arylthio group" as used herein indicates -SA₁₀₃ (wherein A₁₀₃ is the C₆-C₆₀ aryl group).

The term "monovalent non-aromatic condensed polycyclic group" as used herein refers to a monovalent group in which two or more rings are condensed with each other, only carbon is used as a ring-forming atom (for example, the number of carbon atoms may be 8 to 60), and the whole molecule is a non-aromaticity group. An example of the monovalent non-aromatic condensed polycyclic group is a fluorenyl group. The term "divalent non-aromatic condensed polycyclic group" as used herein refers to a divalent group having the same structure as the monovalent non-aromatic condensed polycyclic group described above.

The term "monovalent non-aromatic condensed heteropolycyclic group" as used herein refers to a monovalent group having two or more rings condensed with each other, a heteroatom selected from N, O, P, Si, S, Se, Ge, and B, other than carbon atoms (for example, having 1 to 60 carbon atoms), as a ring-forming atom, and no aromaticity in the entire molecular structure thereof. An example of the monovalent non-aromatic condensed heteropolycyclic group is a carbazolyl group. The term "divalent non-aromatic condensed heteropolycyclic group" as used herein refers to a divalent group having the same structure as the monovalent non-aromatic condensed heteropolycyclic group described above.

The term "π electron-depleted nitrogen-containing C₁-C₆₀ cyclic group" as used herein refers to a cyclic group having 1 to 60 carbon atoms and including at least one *-N=*' (wherein * and *' each indicate a binding site to a neighboring atom) as a ring-forming moiety. For example, the π electron-depleted nitrogen-containing C₁-C₆₀ cyclic group may be a) a first ring, b) a condensed ring in which at least two first rings are condensed, or c) a condensed ring in which at least one first ring and at least one second ring are condensed.

The term "π electron-rich C₃-C₆₀ cyclic group" as used herein refers to a cyclic group having 3 to 60 carbon atoms and not including at least one *-N=*' (wherein * and *' each indicate a binding site to a neighboring atom) as a ring-forming moiety. For example, the π electron-rich C₃-C₆₀ cyclic group may be a) a second ring or b) a condensed ring in which at least two second rings are condensed.

The term "C₅-C₆₀ cyclic group" as used herein refers to a monocyclic or polycyclic group having 5 to 60 carbon atoms, e.g., a) a third ring or b) a condensed ring in which at least two third rings are condensed.

The "C₁-C₆₀ heterocyclic group" as used herein refers to a monocyclic or polycyclic group including at least one heteroatom and 1 to 60 carbon atoms, e.g., a) a fourth ring, b) a condensed ring in which at least two fourth rings are condensed, or c) a condensed ring in which at least one third ring is condensed with at least one fourth ring.

The "first ring" as used herein may be an imidazole group, a pyrazole group, a thiazole group, an isothiazole group, an oxazole group, an isoxazole group, a pyridine group, a pyrazine group, a pyridazine group, a pyrimidine group, a triazole group, a tetrazole group, an oxadiazole group, a triazine group, or a thiadiazole group.

The "second ring" as used herein may be a benzene group, a cyclopentadiene group, a pyrrole group, a furan group, a thiophene group, or a silole group.

The "third ring" as used herein may be a cyclopentane group, a cyclopentadiene group, an indene group, an adamantane group, a norbornene group, a bicyclo[1.1.1]pentane group, a bicyclo[2.1.1]hexane group, a bicyclo[2.2.1]heptane group (a norbornane group), a bicyclo[2.2.2]octane group, a cyclohexane group, a cyclohexene group, or a benzene group.

The "fourth ring" as used herein may be a furan group, a thiophene group, a pyrrole group, a silole group, an oxazole group, an isoxazole group, an oxadiazole group, an isoxadiazole group, oxatriazole group, an isoxatriazole group, a thiazole group, an isothiazole group, a thiadiazole group, an isothiadiazole group, a thiatriazole group, an isotriazole group, a pyrazole group, an imidazole group, a triazole group, a tetrazole group, an azasilole group, a diazasilole group, a triazasilole group, a pyridine group, a pyrimidine group, a pyrazine group, a pyridazine group, or a triazine group.

For example, the π electron-depleted nitrogen-containing C₁-C₆₀ cyclic group may be an imidazole group, a pyrazole group, a thiazole group, an isothiazole group, an oxazole group, an isoxazole group, a pyridine group, a pyrazine group, a pyridazine group, a pyrimidine group, an indazole group, a purine group, a quinoline group, an isoquinoline group, a benzoquinoline group, a benzoisoquinoline group, a phthalazine group, a naphthyridine group, a quinoxaline group, a benzoquinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, an azacarbazole group, an azadibenzofuran group, an azadibenzothiophene group, an azadibenzosilole group, an acridine group, or a pyridopyrazine group.

For example, the π electron-rich C₃-C₆₀ cyclic group may be a benzene group, a heptalene group, an indene group, a naphthalene group, an azulene group, an indacene group, an acenaphthylene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentacene group, a hexacene group, a pentaphene group, a rubicene group, a coronene group, an ovalene group, a pyrrole group, a furan group, a thiophene group, an isoindole group, an indole group, an indene group, a benzofuran group, a benzothiophene group, a benzosilole group, a naphthopyrrole group, a naphthofuran group, a naphthothiophene group, a naphthosilole group, a benzocarbazole group, a dibenzocarbazole group, a dibenzofuran group, a dibenzothiophene group, a carbazole group, a dibenzosilole group, an indenocarbazole group, an indolocarbazole group, a benzofurocarbazole group, a benzothienocarbazole group, a benzosilolocarbazole group, a triindolobenzene group, a pyrrolophenanthrene group, a furanophenanthrene group, a thienophenanthrene group, a benzonaphthofuran group, a benzonapthothiophene group, an (indolo)phenanthrene group, a (benzofurano)phenanthrene group, or a (benzothieno)phenanthrene group.

For example, the C₅-C₆₀ carbocyclic group may be a cyclopentane group, a cyclohexane group, a cyclohexene group, a benzene group, a naphthalene group, an anthracene group, a phenanthrene group, a triphenylene group, a pyrene group, a chrysene group, a 1,2,3,4-tetrahydronaphthalene group, cyclopentadiene group, an indene group, a fluorene group, a 5,6,7,8-tetrahydroisoquinoline group, a 5,6,7,8-tetrahydroquinoline group, an adamantane group, a norbornane group, or a norbornene group.

For example, the C₁-C₆₀ heterocyclic group may be a thiophene group, a furan group, a pyrrole group, a cyclopentadiene group, a silole group, a borole group, a phosphole group, a selenophene group, a germole group, a benzothiophene group, a benzofuran group, an indole group, an indene group, a benzosilole group, a benzoborole group, a benzophosphole group, a benzoselenophene group, a benzogermole group, a dibenzothiophene group, a dibenzofuran group, a carbazole group, a dibenzosilole group, a dibenzoborole group, a dibenzophosphole group, a dibenzoselenophene group, a dibenzogermole group, a dibenzothiophene 5-oxide group, a 9H-fluorene-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluorene-9-one group, an azadibenzothiophene 5,5-dioxide group, a pyridine group, a pyrimidine group, a pyrazine group, a pyridazine group, a triazine group, a quinoline group, an isoquinoline group, a quinoxaline group, a quinazoline group, a phenanthroline group, a pyrazole group, an imidazole group, a triazole group, an oxazole group, an isoxazole group, a thiazole group, an isothiazole group, an oxadiazole group, a thiadiazole group, a benzopyrazole group, a benzimidazole group, a benzoxazole group, a benzothiazole group, a benzoxadiazole group, or a benzothiadiazole group.

The terms "a π electron-deficient nitrogen-containing C₁-C₆₀ cyclic group, a π electron-rich C₃-C₆₀ cyclic group, a C₅-C₆₀ cyclic group, and a C₁-C₆₀ heterocyclic group" as used herein each refer to a part of a condensed ring or a monovalent, a divalent, a trivalent, a tetravalent, a pentavalent, or a hexavalent group, depending on the formula structure.

Substituents of the substituted π electron-deficient nitrogen-containing C₁-C₆₀ cyclic group, the substituted π electron-rich C₃-C₆₀ cyclic group, the substituted C₅-C₆₀ cyclic group, the substituted C₁-C₆₀ heterocyclic group, the substituted C₁-C₆₀ alkylene group, the substituted C₂-C₆₀ alkenylene group, the substituted C₂-C₆₀ alkynylene group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may each independently be:
deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a carboxylic acid or a salt thereof, a sulfonic acid or a salt thereof, a phosphoric acid or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇₋C₆₀ alkylaryl group, a C₆-C₆₀ aryloxy group, a C₆-C₆₀ arylthio group, a C₁-C₆₀ heteroaryl group, a C₂-C₆₀ alkylheteroaryl group, a monovalent non-aromatic condensed polycyclic group, a monovalent non-aromatic condensed heteropolycyclic group, -N(Q₁₁)(Q₁₂), - Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), -P(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇₋C₆₀ alkylaryl group, a C₆-C₆₀ aryloxy group, a C₆-C₆₀ arylthio group, a C₁-C₆₀ heteroaryl group, a C₂-C₆₀ alkylheteroaryl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, -CF₂H, -CFH₂, a hydroxyl group, a cyano group, a nitro group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₇₋C₆₀ alkylaryl group, a C₆-C₆₀ aryloxy group, a C₆-C₆₀ arylthio group, a C₁-C₆₀ heteroaryl group, a C₂-C₆₀ alkylheteroaryl group, a monovalent non-aromatic condensed polycyclic group, a monovalent non-aromatic condensed heteropolycyclic group, -N(Q₂₁)(Q₂₂), - Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), -P(Q₂₈)(Q₂₉), or any combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉); or
any combination thereof.
Q₁ to Q9, Q₁₁ to Q19, Q21 to Q29, and Q31 to Q39 described herein may each independently be: hydrogen; deuterium; -F; -Cl; -Br; -I; a hydroxyl group; a cyano group; a nitro group; an amidino group; a hydrazine group; a hydrazone group; a carboxylic acid or a salt thereof; a sulfonic acid or a salt thereof; a phosphoric acid or a salt thereof; a C₁-C₆₀ alkyl group which is unsubstituted or substituted with deuterium, a C₁-C₆₀ alkyl group, a C₆-C₆₀ aryl group, or any combination thereof; a C₂-C₆₀ alkenyl group; a C₂-C₆₀ alkynyl group; a C₁-C₆₀ alkoxy group; a C₃-C₁₀ cycloalkyl group; a C₁-C₁₀ heterocycloalkyl group; a C₃-C₁₀ cycloalkenyl group; a C₁-C₁₀ heterocycloalkenyl group; a C₆-C₆₀ aryl group which is unsubstituted or substituted with deuterium, a C₁-C₆₀ alkyl group, a C₆-C₆₀ aryl group, or any combination thereof; a C₆-C₆₀ aryloxy group; a C₆-C₆₀ arylthio group; a C₁-C₆₀ heteroaryl group; a monovalent non-aromatic condensed polycyclic group; or a monovalent non-aromatic condensed heteropolycyclic group.

For example, Q₁ to Q₉, Q₁₁ to Q₁₉, Q₂₁ to Q₂₉, and Q₃₁ to Q₃₉ described herein may each independently be:
-CH₃, -CD₃, -CD₂H, -CDH₂, -CH₂CH₃, -CH₂CD₃, -CH₂CD₂H, -CH₂CDH₂, - CHDCH₃, -CHDCD₂H, -CHDCDH₂, -CHDCD₃, -CD₂CD₃, -CD₂CD₂H, or -CD₂CDH₂; or
an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, a phenyl group, a biphenyl group, or a naphthyl group, each unsubstituted or substituted with deuterium, a C₁-C₁₀ alkyl group, a phenyl group, or any combination thereof.

The term "room temperature" as used herein refers to a temperature of about 25 °C.

The terms "a biphenyl group, a terphenyl group, and a tetraphenyl group" as used herein each refer to a monovalent group having two, three, and four phenyl groups linked via a single bond, respectively.

Hereinafter, a compound and an organic light-emitting device according to embodiments are described in detail with reference to Synthesis Examples and Examples. However, the organic light-emitting device is not limited thereto. The wording "'B' was used instead of 'A'" used in describing Synthesis Examples means that an amount of 'A' used was identical to an amount of 'B' used, in terms of a molar equivalent.

### [Examples]

### Synthesis Example 1. Synthesis of Compound 6

Compound 6 was synthesized according to the following reaction scheme:

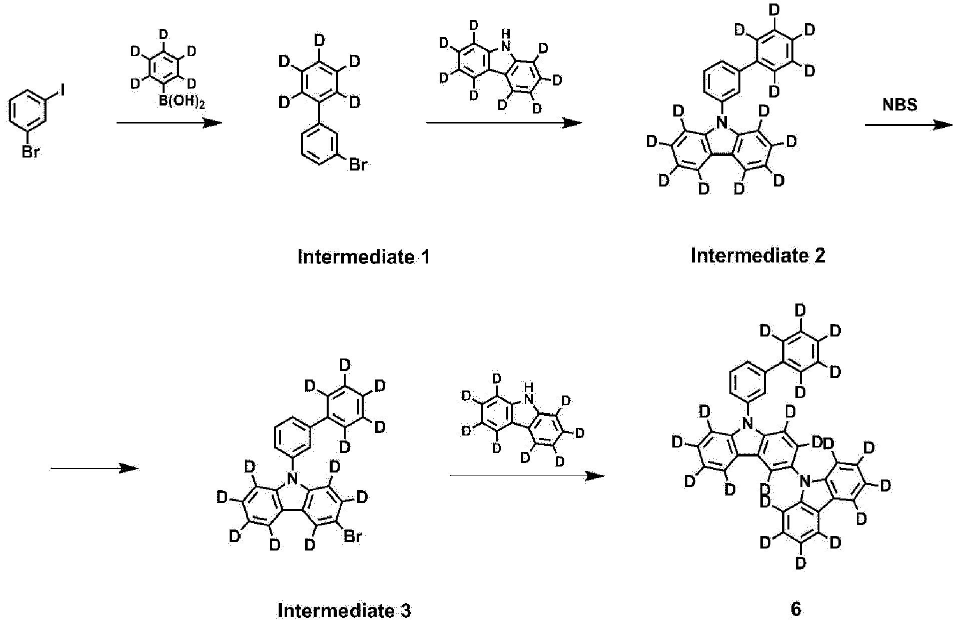

### (1) Synthesis of Intermediate 1

1-bromo-3-iodobenzene (35 g, 123.7 mmol), (phenyl-d5)boronic acid (13 g, 103 mmol), tetrakis(triphenylphosphine)palladium(0) (5.95 g, 5.15 mmol), and sodium bicarbonate (21.6 g, 257 mmol) were mixed with 400 mL of toluene, 100 mL of ethanol, and 100 mL of distilled water, and the mixed solution was heated at 120 °C for 16 hours. After completion of the reaction, the reaction solution was cooled to room temperature, and an organic layer was obtained by extraction using ethyl acetate, dried using anhydrous magnesium sulfate (MgSO₄), concentrated, and then subjected to a silica column, so as to synthesize Intermediate 1. (28.3 g, 118.8 mmol, yield of 96 %)
LCMS (calculated: 237.02, found(M+1): 238.04 m/z)

### (2) Synthesis of Intermediate 2

Intermediate 1 (28.3 g, 118.8 mmol), 9H-carbazole-1,2,3,4,5,6,7,8-d8 (20.8 g, 118.8 mmol), sodium tert-butoxide (17.1 g, 178.3 mmol), tris(dibenzylideneacetone)dipalladium(0) (5.4 g, 5.9 mmol), and tri-tert-butylphosphine (Tri-tert-butylphosphine) (4.8 mL, 11.9 mmol) were mixed with 550 mL of toluene, and the mixed solution was heated at 130 °C for 16 hours. After completion of the reaction, the reaction solution was cooled to room temperature, and an organic layer was obtained by extraction using ethyl acetate, dried using anhydrous MgSO₄, concentrated, and then subjected to a silica column, so as to synthesize Intermediate 2. (19.5 g, 60.5 mmol, yield of 51 %)
LCMS (calculated: 332.22, found(M+1): 333.25 m/z)

### (3) Synthesis of Intermediate 3

Intermediate 2 (19.5 g, 60.5 mmol) was mixed with 600 mL of dimethyl formamide, and the mixed solution was stirred at 0 °C. While maintaining the temperature at 0 °C, N-bromosuccinimide (10.2 g, 57.5 mmol) dissolved in 50 mL of dimethyl formamide was added dropwise thereto and stirred at room temperature for 16 hours. After completion of the reaction, the reaction solution was cooled to room temperature, and an organic layer was obtained by extraction using ethyl acetate, dried using anhydrous MgSO₄, concentrated, and then subjected to a silica column, so as to synthesize Intermediate 3. (23.2 g, 56.6 mmol, yield of 93 %)
LCMS (calculated: 409.12, found(M+1): 410.13 m/z)

### (4) Synthesis of Compound 6

Intermediate 3 (23.2 g, 56.6 mmol), 9H-carbazole-1,2,3,4,5,6,7,8-d8 (10.4 g, 59.4 mmol), sodium tert-butoxide (8.2 g, 84.9 mmol), tris(dibenzylideneacetone)dipalladium(0) (2.1 g, 2.3 mmol), and tri-tert-butylphosphine (1.8 mL, 4.5 mmol) were mixed with 280 mL of toluene, and the mixed solution was heated at 130 °C for 16 hours. After completion of the reaction, the reaction solution was cooled to room temperature, and an organic layer was obtained by extraction using ethyl acetate, dried using anhydrous MgSO₄, concentrated, and then subjected to a silica column, so as to synthesize Compound 6 (19.5 g, 38.6 mmol, yield of 68 %).
LCMS (calculated: 504.32, found(M+1): 505.33 m/z)

### Synthesis Example 2. Synthesis of Compound 26

Compound 26 was synthesized according to the following reaction scheme:

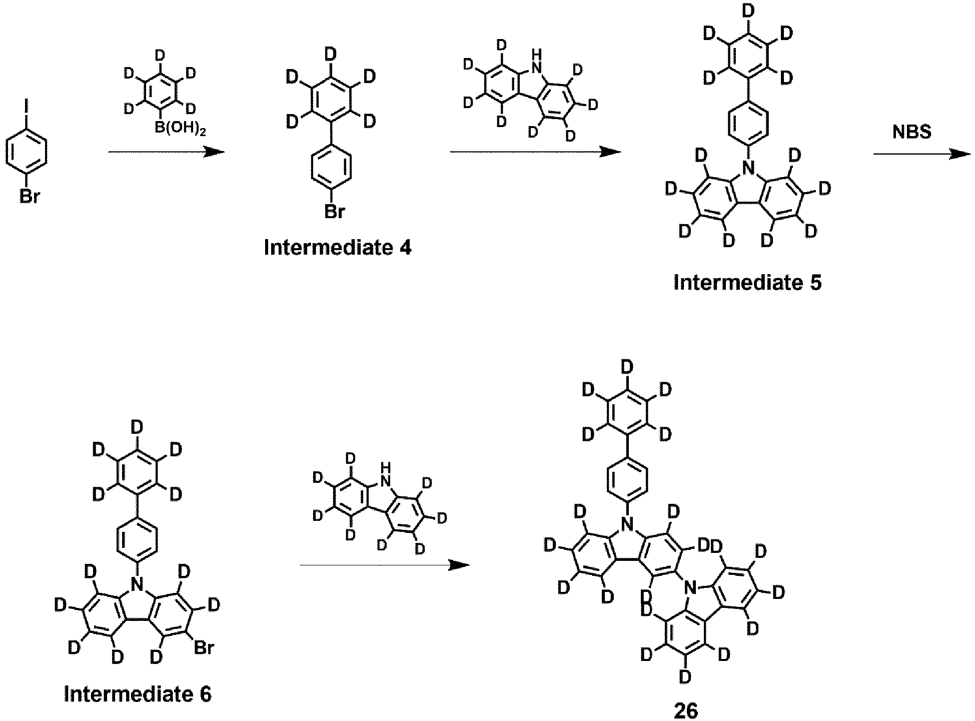

### (1) Synthesis of Intermediate 4

Intermediate 4 was synthesized (yield of 85 %) in the same manner as used to synthesize Intermediate 1 of Synthesis Example 1, except that 1-bromo-4-iodobenzene was used instead of 1-bromo-3-iodobenzene.
LCMS (calculated: 237.02, found(M+1): 238.04 m/z)

### (2) Synthesis of Compound 26

Compound 26 was synthesized in the same manner as used to synthesize Compound 6 of Synthesis Example 1, except that, in the synthesis of Compound 6, Intermediate 4 was used instead of Intermediate 1, Intermediate 5 was used instead of Intermediate 2, and Intermediate 6 was used instead of Intermediate 3.
LCMS (calculated: 504.32, found(M+1): 505.33 m/z)

### Example 1

A glass substrate on which a 1,500 Å-thick indium tin oxide (ITO) electrode (first electrode, anode) was formed was cleaned by ultrasonication using distilled water. After the completion of ultrasonication using distilled water, cleaning by ultrasonication using a solvent, such as isopropyl alcohol, acetone, and methanol, was performed, and the glass substrate was dried and transferred to a plasma cleaner. The glass substrate was cleaned by using oxygen plasma for 5 minutes, and then transferred to a vacuum laminator.

Compound HT3 and Compound HT-D2 were co-deposited on the ITO electrode on the glass substrate to form a hole injection layer having a thickness of 100 Å, Compound HT3 was deposited on the hole injection layer to form a hole transport layer having a thickness of 1,300 Å, and mCP was deposited on the hole transport layer to form an electron blocking layer having a thickness of 100 Å, so as to form a hole transport region.

On the hole transport region, a host and Compound P31 as a dopant were co-deposited at a weight ratio of 85:15 to form an emission layer having a thickness of 300 Å. For the host, a first host (Compound 6) and a second host (Compound E1) were used, and a weight ratio thereof was adjusted to 6:4.

BCP was vacuum-deposited on the emission layer to form a hole blocking layer having a thickness of 100 Å, Compound ET3 and LiQ were co-deposited on the hole blocking layer to form an electron transport layer having a thickness of 300 Å, LiQ was deposited on the electron transport layer to form an electron injection layer having a thickness of 10 Å, and an Al second electrode (cathode) having a thickness of 1,200 Å was formed on the electron injection layer, thereby completing the manufacture of an organic light-emitting device.

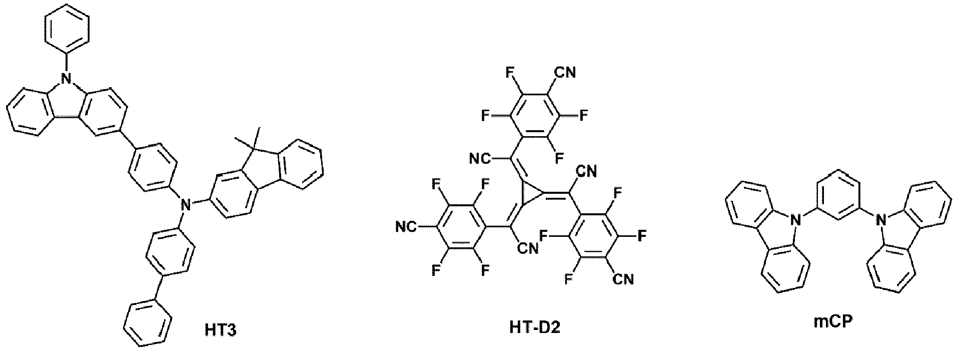

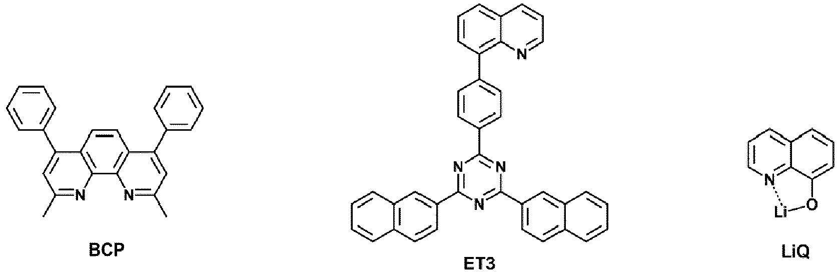

### Example 2 and Comparative Examples 1 to 4

Organic light-emitting devices were manufactured in the same manner as in Example 1, except that compounds shown in Table 2 were each used instead of Compound 6 in forming the emission layer.

### Evaluation Example 1: Characterization of organic light-emitting device

For each of the organic light-emitting devices of Examples 1 and 2 and Comparative Examples 1 to 4, the lifespan (T₉₅ at 1,000 nit, hr) was evaluated with a current-voltage meter (Keithley 2400) and a luminance meter (Minolta Cs-1000A), and results thereof are shown in Table 2. Here, the lifespan (T₉₅) is a measure of the time required for the luminance to decline to 95 % of the initial luminance of 100 %. The lifespans (T₉₅ at 1,000nit, hr) of the organic light-emitting devices of Examples 1 and 2 and Comparative Examples 1 to 4 were represented as relative values (%) compared to the lifespan of the organic light-emitting device of Comparative Example 3.

**Table 2**

| | Host in emission layer | | Dopant | Lifespan (T₉₅) |
|---|---|---|---|---|
| | First host | Second host | | |
| Example 1 | 6 | E1 | P31 | 134 % |
| Comparative Example 1 | CE1 | E1 | P31 | 89 % |
| Example 2 | 26 | E1 | P31 | 121 % |
| Comparative Example 2 | CE2 | E1 | P31 | 79 % |
| Comparative Example 3 | CE3 | E1 | P31 | 100 % |
| Comparative Example 4 | CE4 | E1 | P31 | 74% |

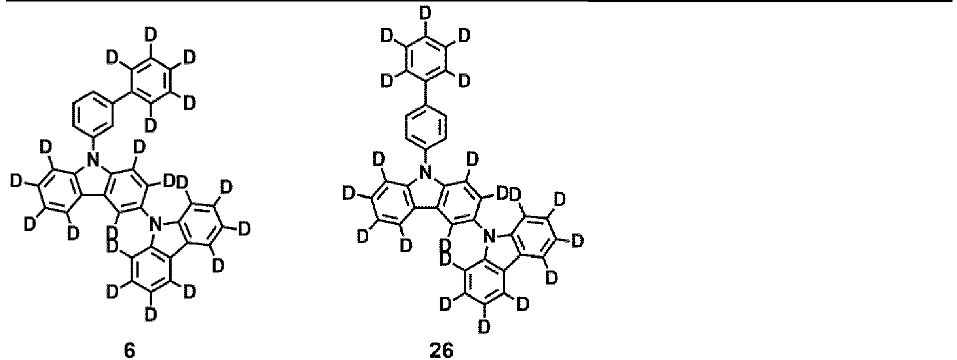

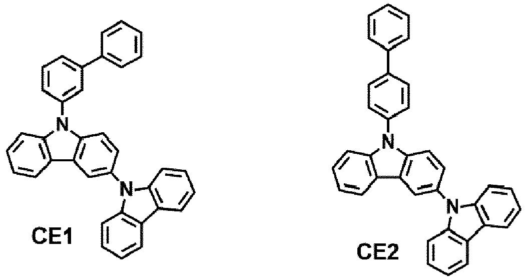

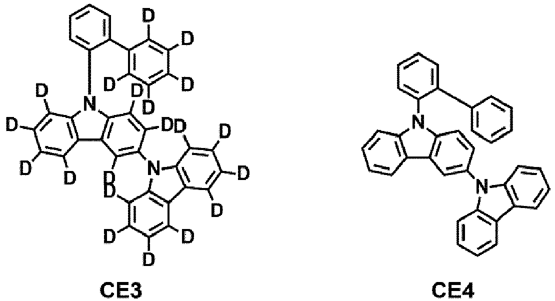

Referring to Table 2, it was confirmed that the organic light-emitting devices of Examples 1 and 2 had excellent external quantum efficiency and lifespan effect compared to the organic light-emitting devices of Comparative Examples 1 to 4.

### Example 3

A glass substrate on which a 1,500 Å-thick indium tin oxide (ITO) electrode (first electrode, anode) was formed was cleaned by ultrasonication using distilled water. After the completion of ultrasonication using distilled water, cleaning by ultrasonication using a solvent, such as isopropyl alcohol, acetone, and methanol, was performed, and the glass substrate was dried and transferred to a plasma cleaner. The glass substrate was cleaned by using oxygen plasma for 5 minutes, and then transferred to a vacuum laminator.

Compound HT3 and Compound HT-D2 were co-deposited on the ITO electrode on the glass substrate to form a hole injection layer having a thickness of 100 Å, Compound HT3 was deposited on the hole injection layer to form a hole transport layer having a thickness of 1,300 Å, and mCP was deposited on the hole transport layer to form an electron blocking layer having a thickness of 100 Å, so as to form a hole transport region.

On the hole transport region, a host, a phosphorescent dopant (Compound P31), and a fluorescent dopant (Compound D3) were co-deposited at a weight ratio of 85:14:1 to form an emission layer having a thickness of 300 Å. For the host, a first host (Compound 6) and a second host (Compound E1) were used, and a weight ratio thereof was adjusted to 6:4.

BCP was vacuum-deposited on the emission layer to form a hole blocking layer having a thickness of 100 Å, Compound ET3 and LiQ were co-deposited on the hole blocking layer to form an electron transport layer having a thickness of 300 Å, LiQ was deposited on the electron transport layer to form an electron injection layer having a thickness of 10 Å, and an Al second electrode (cathode) having a thickness of 1,200 Å was formed on the electron injection layer, thereby completing the manufacture of an organic light-emitting device.

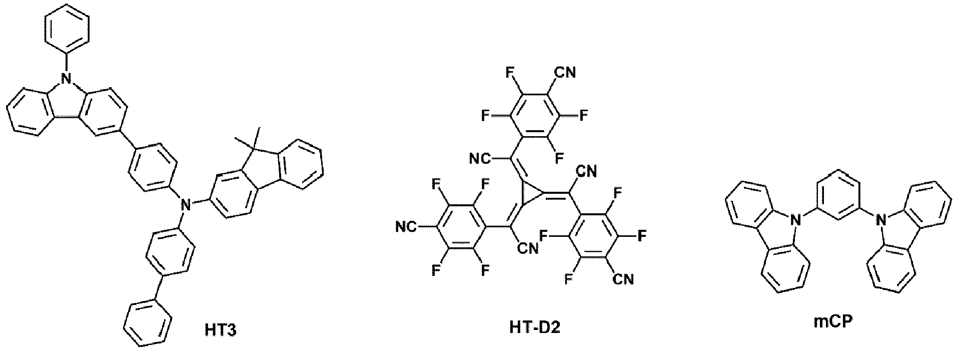

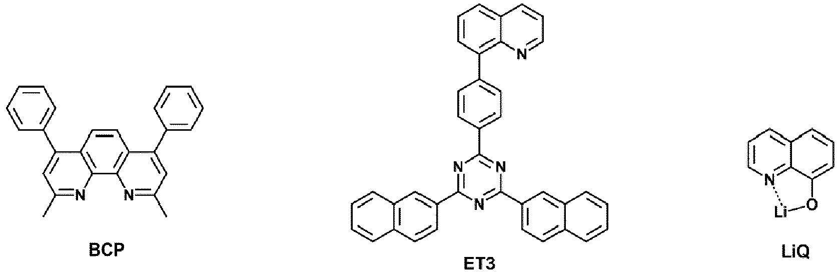

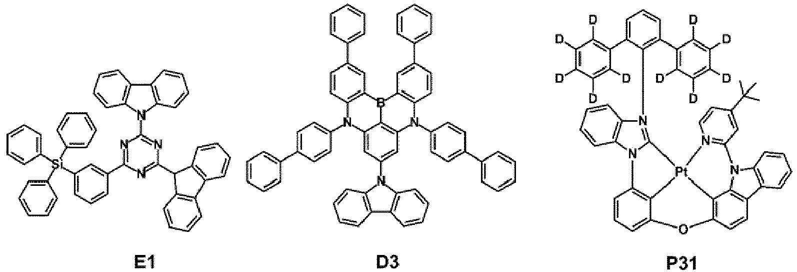

### Example 4 and Comparative Examples 5 to 8

Organic light-emitting devices were manufactured in the same manner as in Example 3, except that compounds shown in Table 3 were each used instead of Compound 6 in forming the emission layer.

### Evaluation Example 2: Characterization of organic light-emitting device

For each of the organic light-emitting devices of Examples 3 and 4 and Comparative Examples 5 to 8, the lifespan (T₉₅ at 1,000 nit, hr) was evaluated with a current-voltage meter (Keithley 2400) and a luminance meter (Minolta Cs-1000A), and results thereof are shown in Table 3. Here, the lifespan (T₉₅) is a measure of the time required for the luminance to decline to 95 % of the initial luminance of 100 %. The lifespans of the organic light-emitting devices of Examples 3 and 4 and Comparative Examples 5 to 8 were represented as relative values (%) compared to the lifespan of the organic light-emitting device of Comparative Example 7.

**Table 3**

| | Host in emission layer | | Dopant in emission layer | | Lifespan (T₉₅) |
|---|---|---|---|---|---|
| | First host | Second host | Phosphorescent dopant | Fluorescent dopant | |
| Example 3 | 6 | E1 | P31 | D3 | 163 % |
| Comparative Example 5 | CE1 | E1 | P31 | D3 | 115 % |
| Example 4 | 26 | E1 | P31 | D3 | 160 % |
| Comparative Example 6 | CE2 | E1 | P31 | D3 | 112 % |
| Comparative Example 7 | CE3 | E1 | P31 | D3 | 100 % |
| Comparative Example 8 | CE4 | E1 | P31 | D3 | 78 % |

Referring to Table 3, it was confirmed that the organic light-emitting devices of Examples 3 and 4 had excellent lifespan effect compared to the organic light-emitting devices of Comparative Examples 5 to 8.

According to the one or more embodiments, use of a heterocyclic compound represented by Formula 1 may provide an organic light-emitting device having long lifespan characteristics and an electronic apparatus including the organic light-emitting device.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims.

## Claims

1. A heterocyclic compound represented by Formula 1: 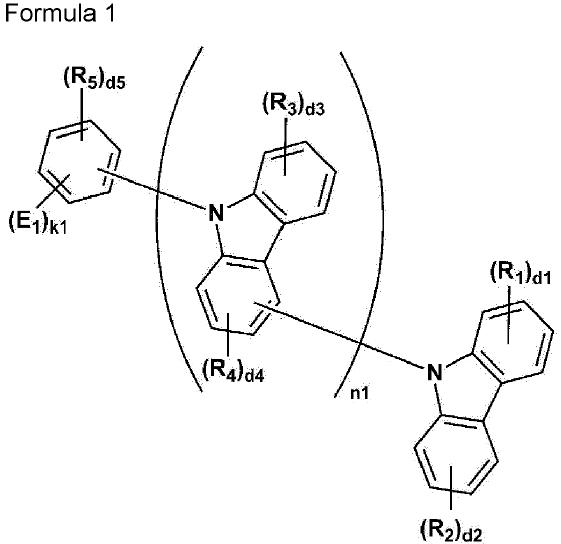 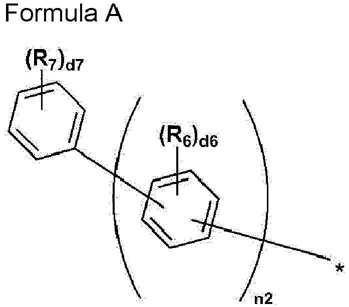
wherein, in Formula 1,
E₁ is a group represented by Formula A, and
k1 is an integer from 1 to 5,
in Formulae 1 and A,
n1 is 1,
n2 is 0,
R₁ to R₄ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or - P(Q₈)(Q₉),
R₅ to R₇ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryloxy group, a substituted or unsubstituted C₆-C₆₀ arylthio group, -N(Q₁)(Q₂), -Si(Q₃)(Q₄)(Q₅), - Ge(Q₃)(Q₄)(Q₅), -B(Q₆)(Q₇), -P(=O)(Q₈)(Q₉), or -P(Q₈)(Q₉),
d1, d2, d3, d5, and d6 are each independently an integer from 0 to 4,
d4 is an integer from 0 to 3,
d7 is an integer from 0 to 5,
the heterocyclic compound represented by Formula 1 comprises at least one deuterium, and
a moiety represented by 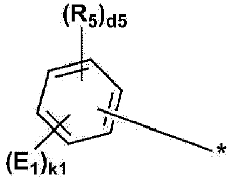 in Formula 1 is a group represented by Formula 4-2 or 4-3: 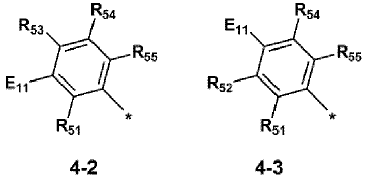
wherein, in Formulae 4-2 and 4-3,
E₁₁ is the same as described in connection with E₁,
R₅₁ to R₅₅ are each independently the same as described in connection with R₅,
* indicates a binding site to a neighboring atom,
substituents of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group are each:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), -Si(Q₁₃)(Q₁₄)(Q₁₅), -Ge(Q₁₃)(Q₁₄)(Q₁₅), - B(Q₁₆)(Q₁₇), -P(=O)(Q₁₈)(Q₁₉), -P(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with at least one of deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), - Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), -P(Q₂₈)(Q₂₉) or any combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), - P(=O)(Q₃₈)(Q₃₉), or -P(Q₃₈)(Q₃₉); or
any combination thereof, and
Q₁ to Q9, Q₁₁ to Q19, Q21 to Q29, and Q31 to Q39 are each independently hydrogen, deuterium, -F, -Cl, -Br, -I, a hydroxyl group, a cyano group, a nitro group, an amidino group, a hydrazine group, a hydrazone group, a carboxylic acid or a salt thereof, a sulfonic acid or a salt thereof, a phosphoric acid or a salt thereof, a C₁-C₆₀ alkyl group which is unsubstituted or substituted with deuterium, a C₁-C₆₀ alkyl group, a C₆-C₆₀ aryl group, or any combination thereof, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group which is unsubstituted or substituted with deuterium, a C₁-C₆₀ alkyl group, a C₆-C₆₀ aryl group, or any combination thereof, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group.

2. The heterocyclic compound of claim 1, wherein the heterocyclic compound represented by Formula 1 satisfies at least one of Conditions 1 to 7:
Condition 1
At least one of R₁(s) in the number of d1 is deuterium;
Condition 2
At least one of R₂(s) in the number of d2 is deuterium;
Condition 3
At least one of R₃(s) in the number of n1*d3 is deuterium;
Condition 4
At least one of R₄(s) in the number of n1*d4 is deuterium;
Condition 5
At least one of R₅(s) in the number of d5 is deuterium;
Condition 6
At least one of R₆(s) in the number of k1*n2*d6 is deuterium; and
Condition 7
A least one of R₇(s) in the number of k1*d7 is deuterium.

3. The heterocyclic compound of claims 1 or 2, wherein the heterocyclic compound represented by Formula 1 satisfies at least one of Conditions 1' to 7':
Condition 1'
R₁(s) in the number of d1 are all deuterium, and d1 is 4;
Condition 2'
R₂(s) in the number of d2 are all deuterium, and d2 is 4;
Condition 3'
R₃(s) in the number of n1*d3 are all deuterium, and d3 is 4;
Condition 4'
R₄(s) in the number of n1*d4 are all deuterium, and d4 is 3;
Condition 5'
R₅(s) in the number of d5 are all deuterium,
k1 is 1, and d5is 4;
Condition 6'
R₆(s) in the number of k1*n2*d6 are all deuterium, and d6 is 4; and
Condition 7'
R₇(s) in the number of k1*d7 are all deuterium, and d7 is 4.

4. The heterocyclic compound of any of claims 1-3, wherein a substitution ratio of deuterium in the heterocyclic compound represented by Formula 1 is greater than or equal to 10 %.

5. The heterocyclic compound of any of claims 1-4, wherein a moiety represented by 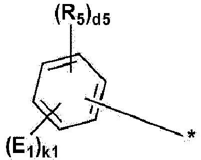 in Formula 1 is a group represented by one of Formulae 4-201 to 4-279 and 4-301 to 4-356: 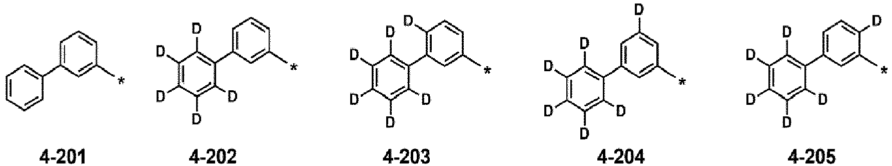 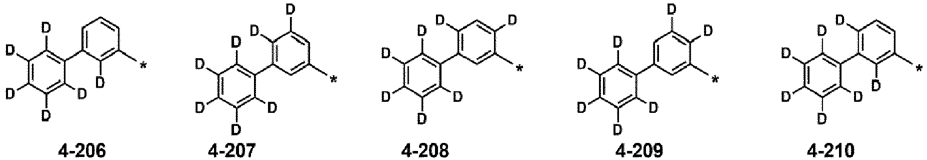 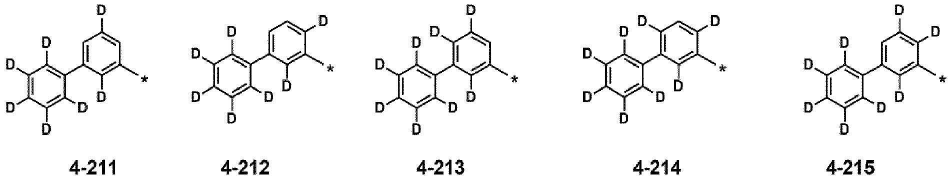 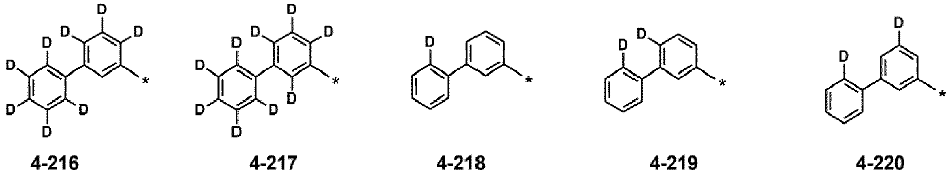 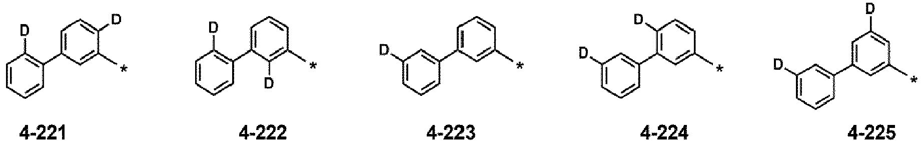 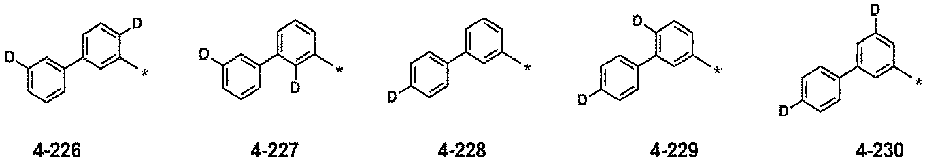 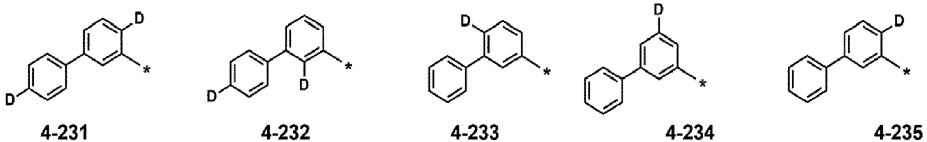 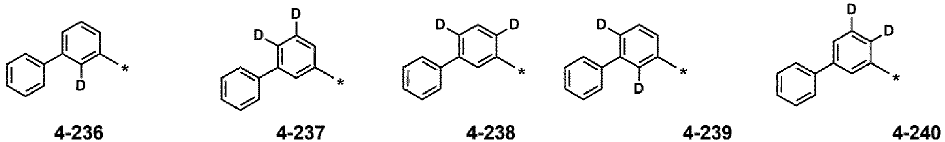 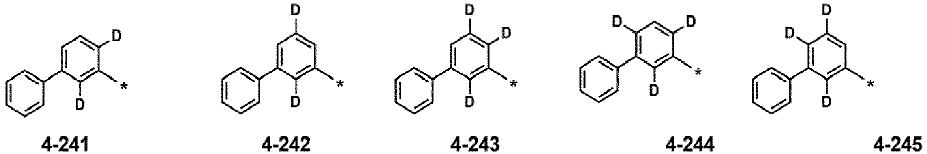 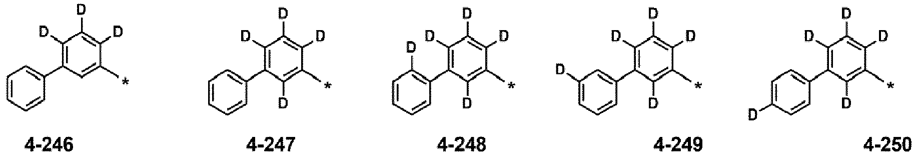 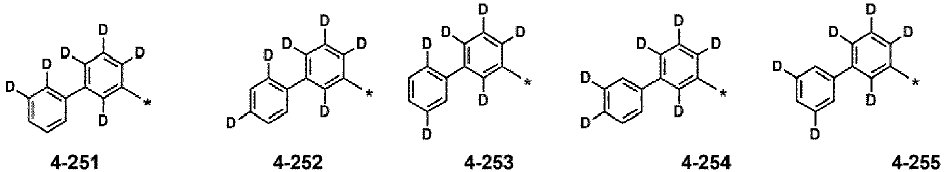 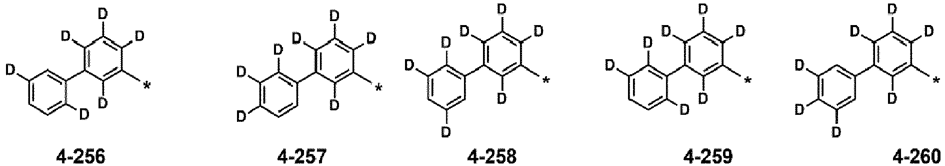 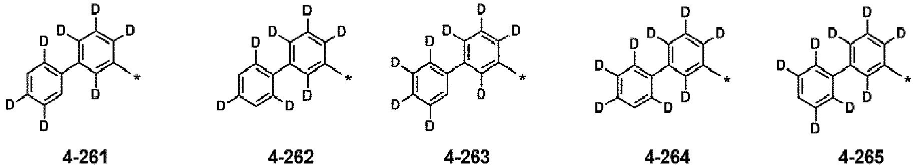 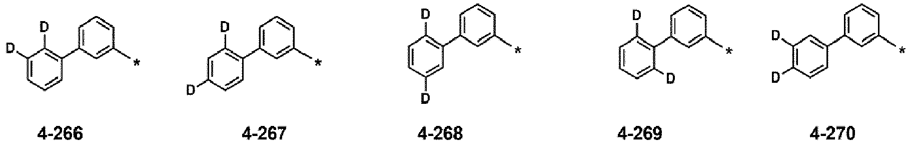 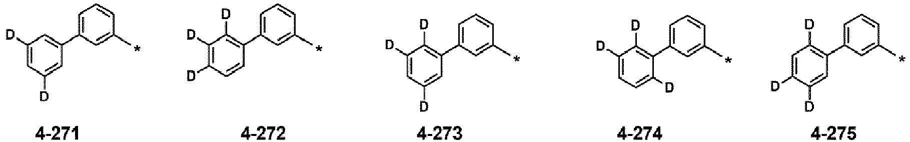 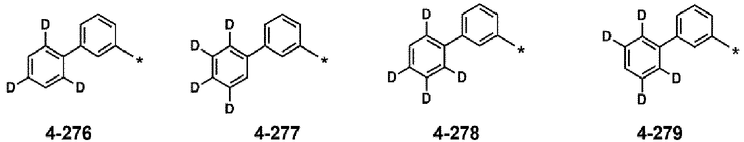 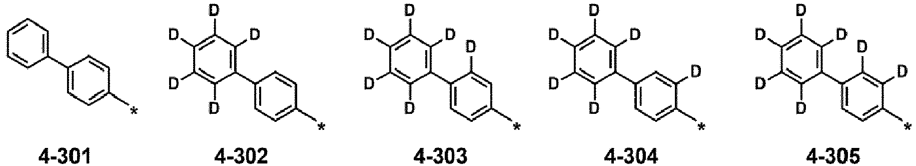 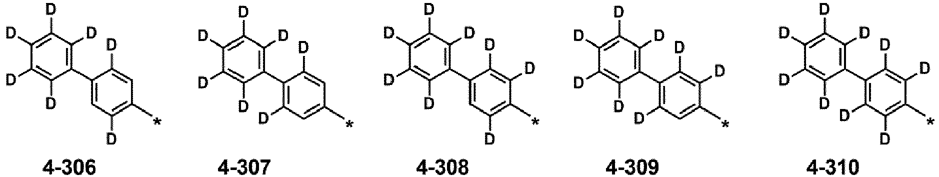 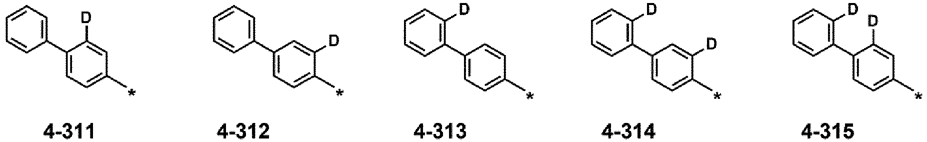 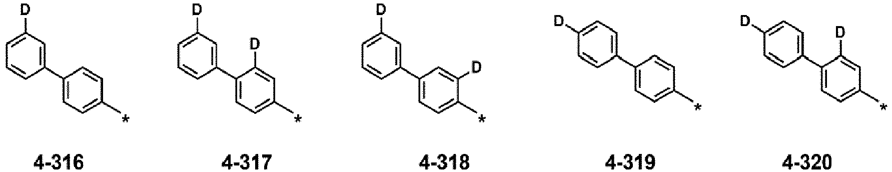 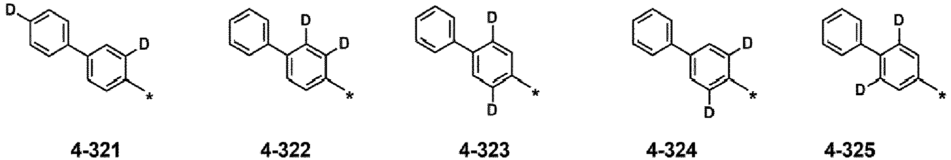 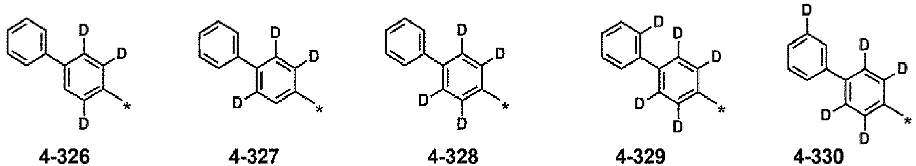 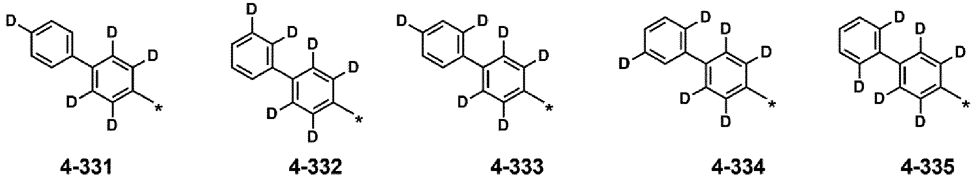 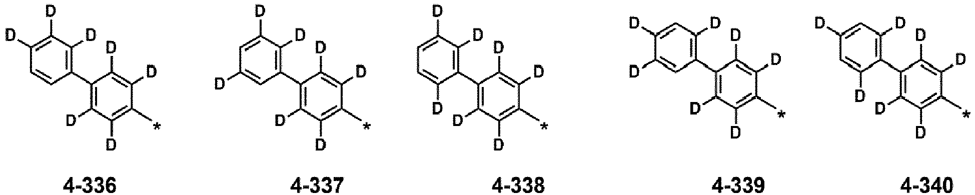 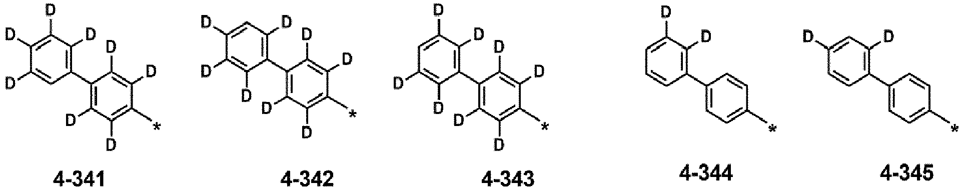 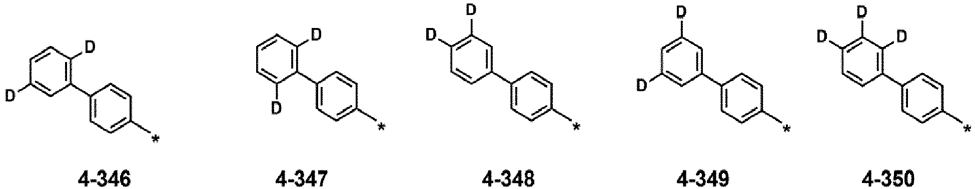 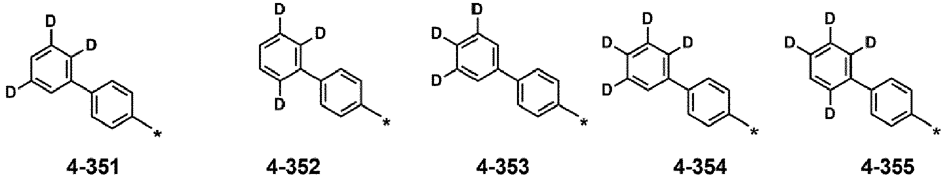 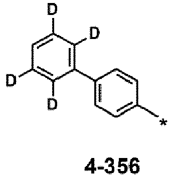
wherein, in Formulae 4-201 to 4-279 and 4-301 to 4-356,
D is a deuterium atom, and
* indicates a binding site to a neighboring atom.

6. The heterocyclic compound of any of claims 1-5, wherein a moiety represented by 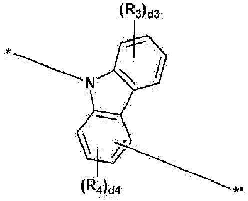 in Formula 1 is a group represented by one of Formulae 2-1 to 2-4: 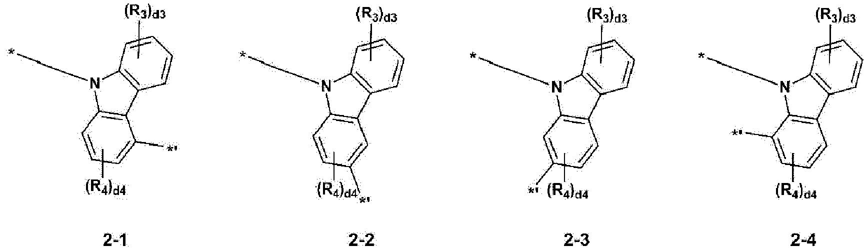
wherein, in Formulae 2-1 to 2-4,
R₃, R₄, d3, and d4 are each the same as defined for claim 1, and
* and *' each indicate a binding site to a neighboring atom.

7. The heterocyclic compound of any of claims 1-6, wherein a moiety represented by 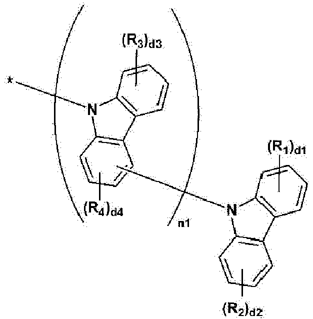 in Formula 1 is a group represented by one of Formulae 3-1 to 3-4: 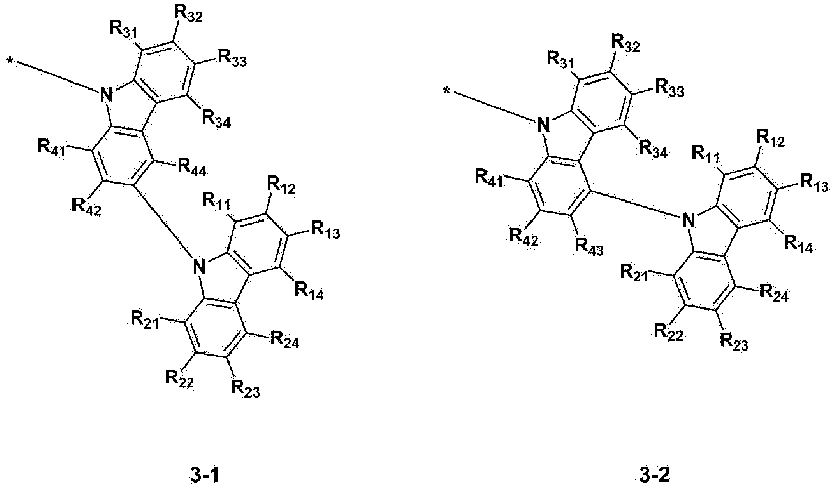 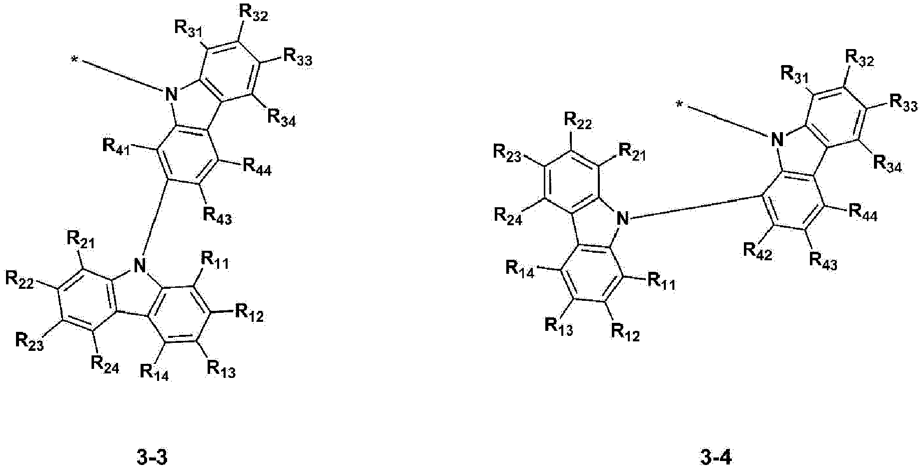
wherein, in Formulae 3-1 to 3-4,
R₁₁ to R₁₄ are each the same as defined for R₁ in claim 1,
R₂₁ to R₂₄ are each the same as defined for R₂ in claim 1,
R₃₁ to R₃₄ are each the same as defined for R₃ in claim 1,
R₄₁ to R₄₄ are each the same as defined for R₄ in claim 1, and
* indicates a binding site to a neighboring atom.

8. The heterocyclic compound of claim 7, wherein the group represented by one of Formulae 3-1 to 3-4 satisfies one of Conditions 8-1 to 8-4:
Condition 8-1
At least one of R₁₁ to R₁₄ is deuterium;
Condition 8-2
At least one of R₂₁ to R₂₄ is deuterium;
Condition 8-3
At least one of R₃₁ to R₃₄ is deuterium; and
Condition 8-4
At least one of R₄₁ to R₄₄ is deuterium.

9. The heterocyclic compound of any of claims 1-8, wherein a moiety represented by 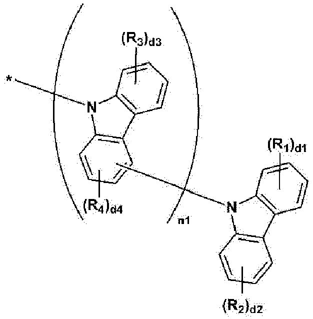 in Formula 1 is a group represented by one of Formulae 3-101 to 3-112, 3-201 to 3-212, 3-301 to 3-312, and 3-401 to 3-412: 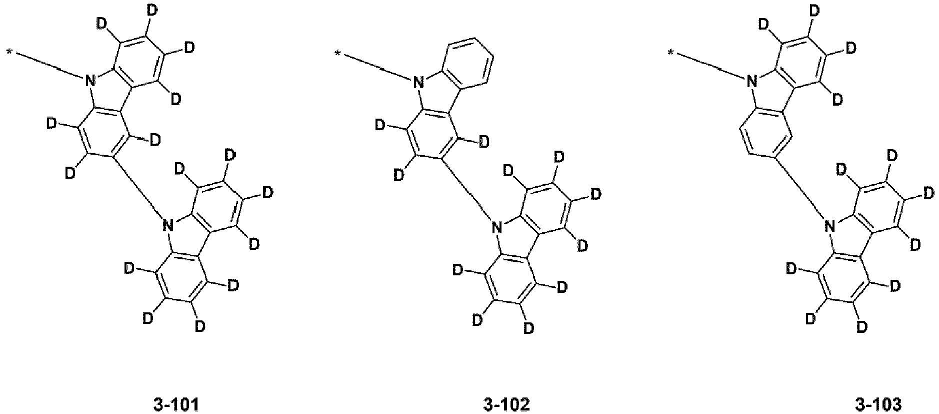 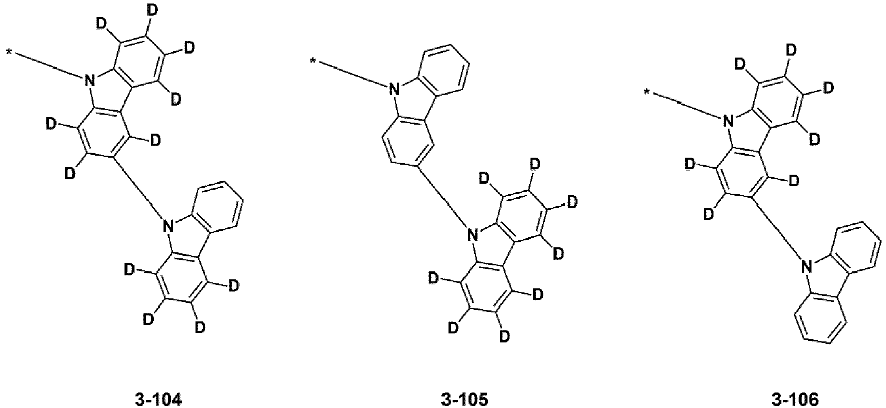 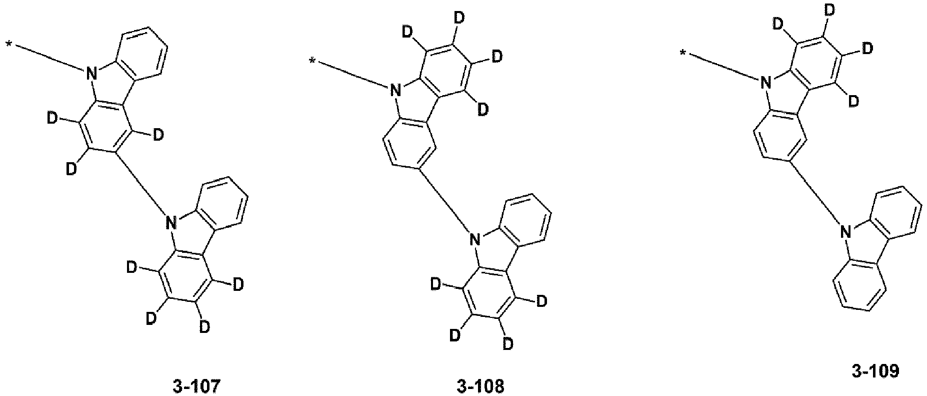 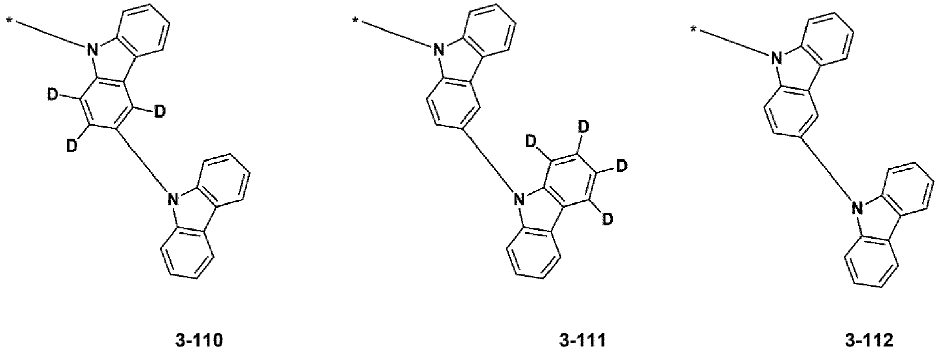 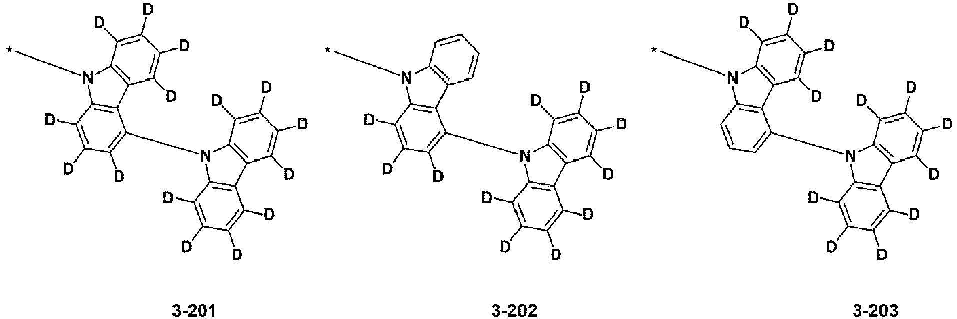 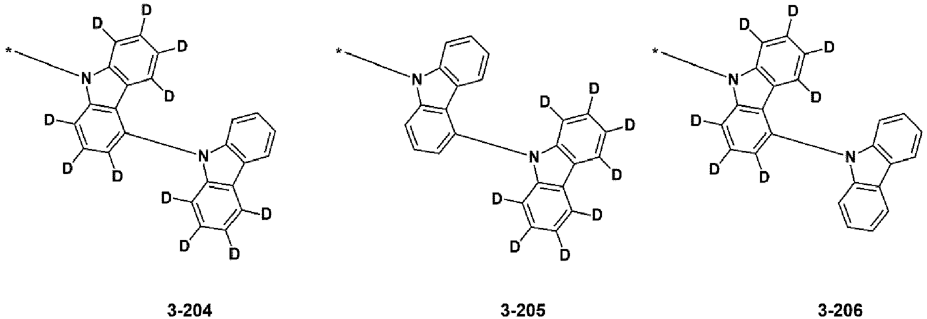 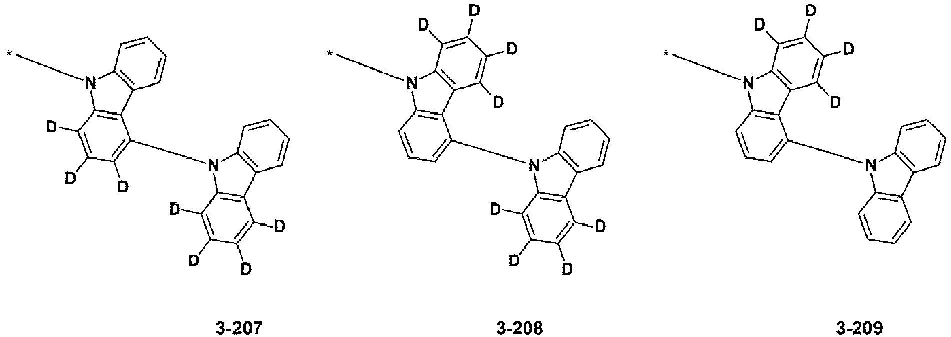 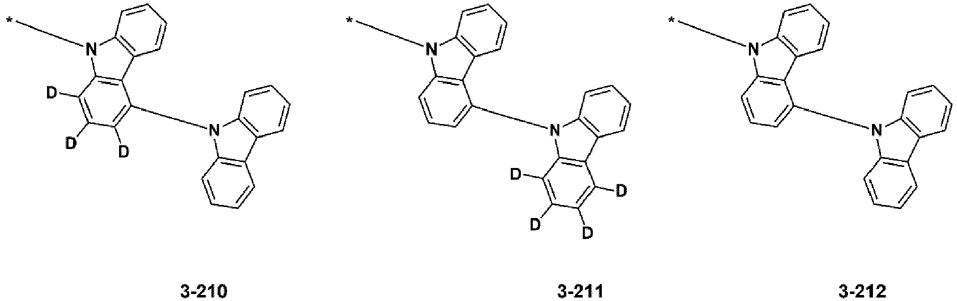 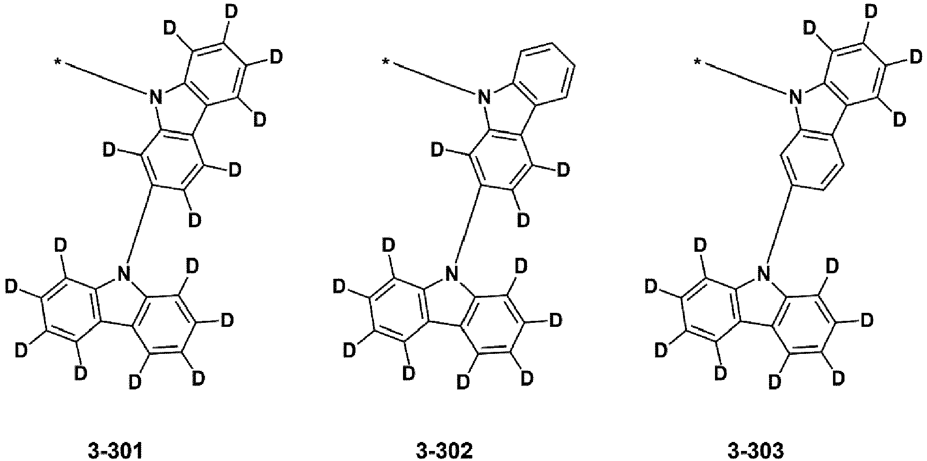 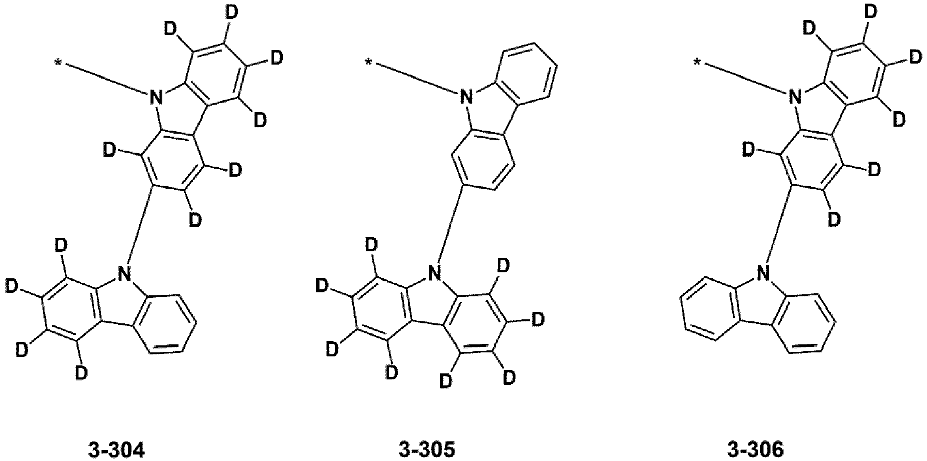 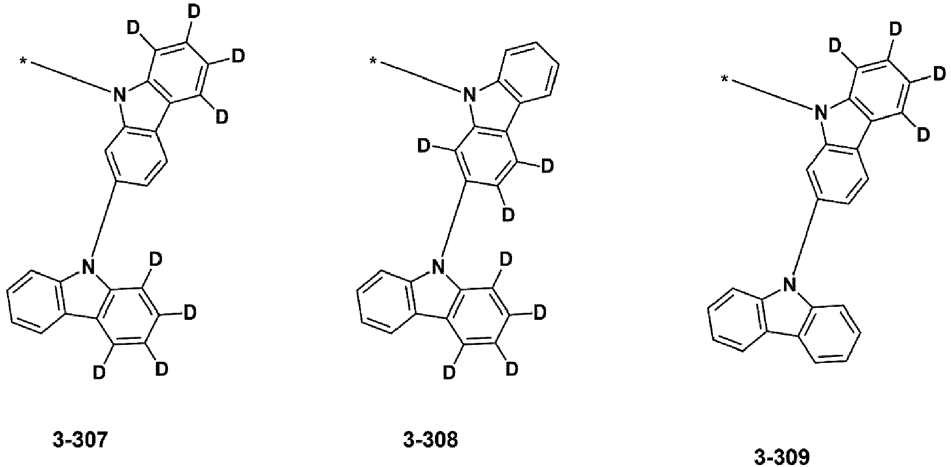 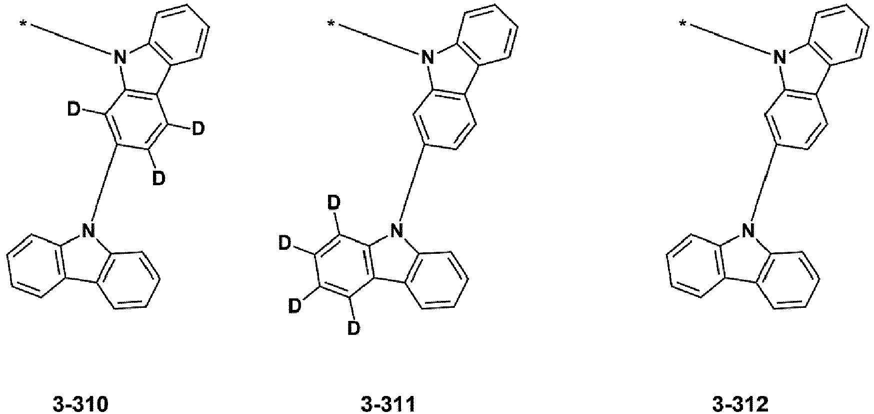 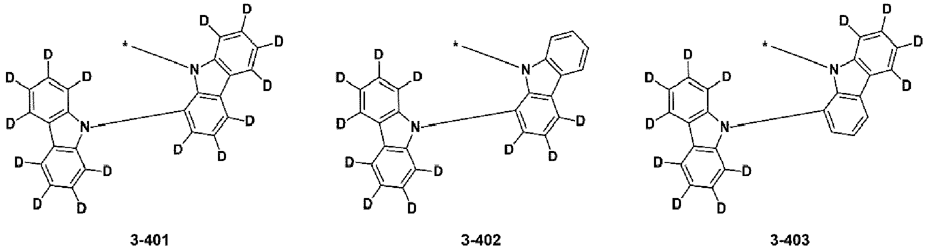 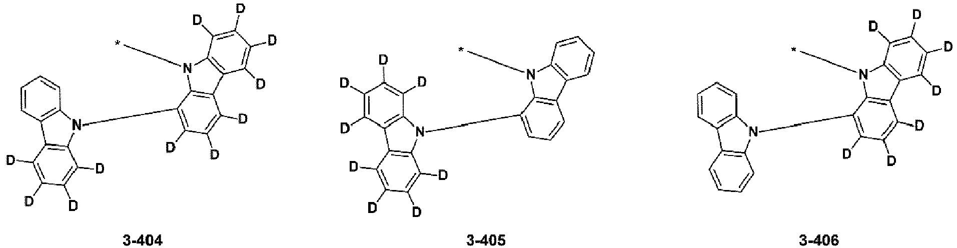 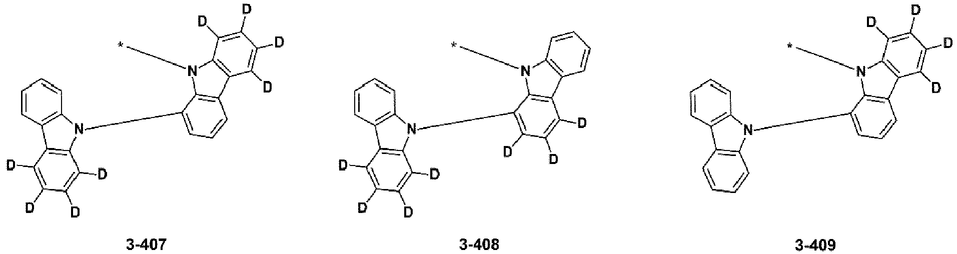 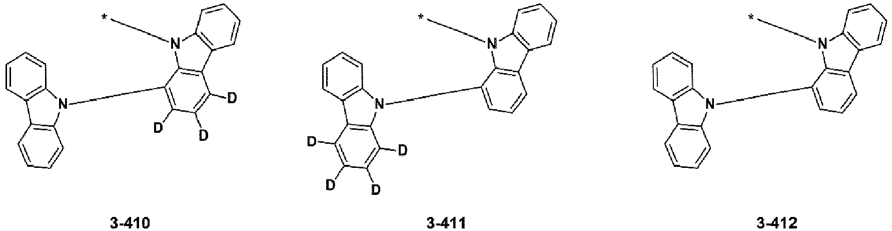
wherein, in Formulae 3-101 to 3-112, 3-201 to 3-212, 3-301 to 3-312, and 3-401 to 3-412,
D is a deuterium atom, and
* indicates a binding site to a neighboring atom.

10. The heterocyclic compound of any of claims 1-9, wherein the heterocyclic compound represented by Formula 1 is represented by Formula 1-1 or 1-2: 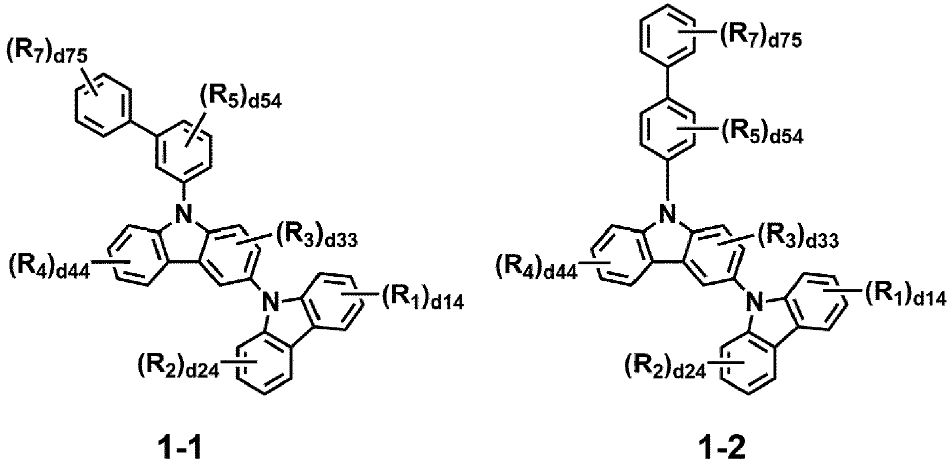
wherein, in Formulae 1-1 and 1-2,
R₁ to R₅ are each the same as defined for claim 1,
d75 is an integer from 0 to 5,
d14, d24, d34, and d54 are each independently an integer from 0 to 4,
d43 is an integer from 0 to 3, and
the heterocyclic compounds represented by Formulae 1-1 and 1-2 each comprise at least one deuterium; and/or
wherein
R₁ and R₂ are each independently hydrogen, deuterium, -F, -Cl, -Br, -I, -SF₅, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryloxy group, a substituted or unsubstituted C₆-C₆₀ arylthio group, -N(Q₁)(Q₂), -Si(Q₃)(Q₄)(Q₅), - Ge(Q₃)(Q₄)(Q₅), -B(Q₆)(Q₇), -P(=O)(Q₈)(Q₉), or -P(Q₈)(Q₉), and
Q₁ to Q₉ are each the same as defined for claim 1.

11. An organic light-emitting device comprising:
a first electrode;
a second electrode, and
an organic layer arranged between the first electrode and the second electrode and comprising an emission layer,
wherein the organic layer comprises at least one of the heterocyclic compound of any of claims 1-10.

12. The organic light-emitting device of claim 11, wherein
the organic layer further comprises a hole transport region between the first electrode and the emission layer and an electron transport region between the emission layer and the second electrode,
the hole transport region comprises a hole injection layer, a hole transport layer, an electron blocking layer, an auxiliary layer, or any combination thereof, and
the electron transport region comprises a buffer layer, a hole blocking layer, an electron transport layer, an electron injection layer, or any combination thereof;
preferably wherein the hole transport region and/or the electron transport region comprises the heterocyclic compound.

13. The organic light-emitting device of claims 11 or 12, wherein the emission layer comprises the heterocyclic compound;
preferably wherein the emission layer further comprises a phosphorescent dopant and/or a fluorescent dopant.

14. The organic light-emitting device of claim 13, wherein the emission layer further comprises an electron-transporting host.

15. An electronic apparatus comprising the organic light-emitting device of any of claims 11-14.
